(19) Europäisches Patentamt
European Patent Office
Office européen des brevets

(11) EP 4 778 565 A2

(12) EUROPEAN PATENT APPLICATION

(43) Date of publication:
22.07.2026 Bulletin 2026/30

(51) International Patent Classification (IPC):
*A61N 1/36* $^{(2006.01)}$

(21) Application number: 26163624.5

(22) Date of filing: 28.04.2023

(52) Cooperative Patent Classification (CPC):
A61N 1/36062; A61N 1/36067; A61N 1/36135

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR

(30) Priority: 29.04.2022 EP 22170983

(62) Document number(s) of the earlier application(s) in accordance with Art. 76 EPC:
23723512.2 / 4 514 449

(71) Applicant: Ecole Polytechnique Fédérale de Lausanne (EPFL)
1015 Lausanne (CH)

(72) Inventors:
• Bloch, Jocelyne
1094 PAUDEX (CH)
• Milekovic, Tomislav
1700 FRIBOURG (CH)
• Martin Moraud, Eduardo
1003 LAUSANNE (CH)
• Courtine, Grègoire
1003 Lausanne (CH)

• Newton, Cho
Toronto, M5B 1L3 (CA)
• Squair, Jordan
1003 Lausanne (CH)
• Demesmaeker, Robin
1110 Morges (CH)
• Asboth, Léonie
1006 Lausanne (CH)
• Bole-Feysot, Léa
1020 Renens (CH)

(74) Representative: WENDE IP GbR
Hopfenstraße 8
80335 München (DE)

Remarks:
•This application was filed on 10-03-2026 as a divisional application to the application mentioned under INID code 62.
•Claims filed after the date of receipt of the divisional application (Rule 68(4) EPC).

(54) NEUROMODULATION/NEUROSTIMULATION SYSTEM FOR MITIGATING LOCOMOTOR DEFICITS OF PARKINSON'S DISEASE, SPINAL CORD INJURY (SCI), STROKE AND/OR OTHER NEUROLOGICAL DISORDERS

(57) The present invention relates to a combined neuromodulation and/or neurostimulation system (10) for mitigating locomotor deficits of/or neuronal disorders, especially Parkinson's disease, said system (10) comprising:
- at least one Deep Brain Stimulation (DBS) System for providing Deep Brain Stimulation to brain tissue of a subject (P),
- at least one control unit, configured and adapted to provide stimulation data,
- at least one stimulation unit (12), operatively connected to the at least one control unit, the at least one stimulation unit (12) being configured and adapted to deliver epidural electrical stimulation (EES) to the dorsal side of the spinal cord (S) of said subject (P), and
- at least one implantable pulse generator (IPG) (13), wherein the at least one stimulation unit (12) includes one or more electrodes (14) configured to be implanted epidurally, the one or more electrodes (14) being operatively connected to the at least one IPG (13).

Fig. 1

**Description**

The present invention belongs to the technical field of spinal cord stimulation for mitigating motor deficits in a mammal, in particular a human.

More specifically, the present invention relates to a combined neuromodulation/neurostimulation system targeted at mitigating locomotor deficits of/or neuronal disorders, especially Parkinson's disease.

Parkinson's disease (PD) is one of the most prevalent neurodegenerative disorders, presently affecting more than ten million people worldwide. More than 90% subjects affected by PD suffer from motor, in particular locomotor, disturbances that significantly affect their quality of life and increase comorbid conditions **(1),** including gait and balance locomotor deficits as well as freezing-of-gait (FOG).

Contrary to upper-limb motor deficits of PD, locomotor deficits of PD tend to poorly respond to commonly available therapies such as dopamine replacement strategies and deep brain stimulation (DBS), especially at the late-stage of the disease **(2-5).**

Gait and balance deficits are in part due to the disruption of the communication between the brain and spinal cord resulting from the depletion of dopaminergic and cholinergic circuits located in the basal ganglia and brainstem **(2).**

In past experiments, the inventors engineered a wireless brain-spine interface (BSI) that restored this communication after spinal cord injury (SCI) **(3).**

The inventors have found that the divergence in the nature and dynamics of the circuits that control manual dexterity versus locomotion may explain why locomotor deficits of PD are resistant to treatments optimized for upper-limb motor symptoms.

Previous studies reported gait improvements during continuous stimulation of the dorsal columns at the thoracic level of the spinal cord.

However, the results have proven inconsistent and variable.

Thus, there is the need to provide a new clinical approach that allows to consistently and reliably mitigate locomotor deficits in subjects with PD.

Epidural stimulation in combination with Deep Brain Stimulation are described for the treatment after spinal cord injury (Noga, et. al, Combined neuromodulatory approaches in the central nervous system for treatment of spinal cord injury, Current Opinion in Neurology, Volume 34, Number 6, December 2021, pp. 804-811(4)).

Low-frequency Deep Brain Stimulation (DBS) is also known (Baizabal-Carvallo, JF, Low-frequency deep brain stimulation for movement disorders, Parkinsonism & Related Disorders, Vol. 31, Oct. 2016, p.14-22).

It has been described that the lateral hypothalamus (LH) is a functionally and anatomically dynamic region, which plays a role in recovery after spinal cord injury (Cho, et al, Neurosurgery, unbiased interrogation of whole brain circuits identifies neurons that restore walking after spinal cord injury, April 2022, Vol. 68, p. 61).

Further, in combination with rats the combination of Deep Brain Stimulation (DBS) of the midbrain locomotor region and epidural electrical stimulation of the lumbar spinal cord (EES) has been described to improve locomotor function (Bonizzato, et al., Multi-pronged neuromodulation intervention engages the residual motor circuitry to facilitate walking in a rat model of spinal cord injury, Nature Communications 12, Article No. 1925 (2021).

It is therefore an object of the present invention and disclosure, to provide and describe a neuromodulation and/or neurostimulation system for mitigating locomotor deficits of Parkinson's disease, especially in that the system can provide an enhanced rehabilitation and recovery for locomotor and movement disorders, but also for other functions like autonomous function.

This object is achieved by the provision of a neuromodulation/neurostimulation system according to claim 1.

In particular, the present invention provides a combined neuromodulation and/or neurostimulation system for mitigating locomotor deficits of/or neuronal disorders, especially of Parkinson's disease, said system comprising:

- at least one Deep Brain Stimulation (DBS) System for providing Deep Brain Stimulation to brain tissue of a subject,

- at least one control unit, configured and adapted to provide stimulation data,

- at least one stimulation unit, operatively connected to the at least one control unit, the at least one stimulation unit being configured and adapted to deliver epidural electrical stimulation (EES) to the dorsal side of the spinal cord (S) of said subject (P), and

- at least one implantable pulse generator (IPG),

wherein the at least one stimulation unit includes one or more electrodes configured to be implanted epidurally, the one or more electrodes being operatively connected to the at least one IPG.

The neuronal disorders can be neuronal disorders of the brain. For example, such a disorder can be Parkinson's disease. Further disorders that can be treated (but not limited to) epilepsy, chronic pain (like (cluster) headache or migraine), depression, Alzheimer's disease, obsessive compulsive disorders, obesity, and/or other locomotor deficits and/or neuronal disorders after spinal cord injury (SCI), stroke, and/or other neurological disorders.

The system includes at least one control unit.

For instance, the system may include a single control unit.

At least one control unit is configured and arranged to provide stimulation data.

The system further includes at least one stimulation unit, operatively connected to the at least one control unit.

For instance, the system may include a single stimulation unit.

At least one stimulation unit is being configured and adapted to deliver epidural electrical stimulation (EES) to the dorsal side of the spinal cord of a subject.

The system further includes at least one implantable pulse generator (IPG).

For instance, the system may include a single IPG.

At least one stimulation unit includes one or more electrodes, configured to be implanted epidurally.

The epidurally implanted electrodes are configured and arranged to deliver EES over the dorsal side of the spinal cord of the subject.

One or more electrodes are operatively connected to the at least one IPG.

Preferably, at least one stimulation unit is configured and adapted to deliver EES to the lumbosacral region of the spinal cord.

The invention is based on the basic idea that, by modulating the neural circuits within the spinal cord, which is directly responsible for determining and maintaining locomotion, it is possible to significantly and reliably mitigate locomotor deficits (such as gait and balance deficits) of PD. The combination of DBS treatment and EES stimulation for restoring and facilitating locomotion and gait restoration is much more effective than DBS alone, which has been shown in extensive trials. In particular, this neuromodulation leverages spinal-cord stimulation technologies that can recruit individual posterior roots of the spinal cord in order to modulate the spinal circuits involved in the control of leg and torso movements. The recruitment of the spinal posterior roots that coincide with the ongoing movement intentions of the subject reinstates the natural dynamics of the spinal motor circuits that, in turn, leads to reduction of gait and balance deficits of PD.

Preferably, at least one stimulation unit further includes means for delivery of deep brain stimulation (DBS).

In particular, at least one stimulation unit may be controlled to deliver low frequency waves of DBS.

More preferably, at least one stimulation unit is configured and adapted to deliver EES at spinal cord segments from L2 to S1. This section has been investigated and shown to be effective for EES stimulation to facilitate locomotion and gait restoration.

The tissue to be stimulated with EES is especially the area of the dorsal roots of the spinal cord.

Furthermore, it is possible that the Deep Brain Stimulation (DBS) System is configured to provide low frequency stimulation to the brain tissue. For example, this can be in the range between 0-150Hz, especially in the range of 20-100Hz, more especially in the range of 20-70 Hz, preferably around 20-45 Hz or 20-60 Hz. Reference is made to the below examples and also to Baizabal-Carvallo et al, Low-Frequency Deep Brain Stimulation for Movement Disorders, Parkinsonism and Related Disorders, Vol. 31, October 2016, p. 14-22.

Additionally, the Deep Brain Stimulation (DBS) System is configured to provide low frequency stimulation to the brain tissue by means of charge balanced biphasic stimulation pulses. These pulses have been shown to be effective in DBS and can be easily adjusted to finetune the DBS treatment (cf. below examples).

The Deep Brain Stimulation (DBS) System can be configured to provide stimulation pulses with a pulse width of approx. 90 $\mu$s. This range can be e.g. set around 90 $\mu$s, e.g. from 80 $\mu$s to 100 $\mu$s. This pulse width has been successfully used in trials as shown below in the examples.

The Deep Brain Stimulation (DBS) System can be configured to provide stimulation to the subthalamic nucleus (STN). This brain area has been used for the delivery of DBS neurostimulation and the results have shown great effectivity, please see examples and data below.

Additionally, the stimulation unit can be configured to provide stimulation to the spinal cord tissue and/or the tissue of the spinal cord dorsal roots, for example in the range between 0-150 Hz, especially in the range of 20-100 Hz, more especially in the range of 20-70 Hz, preferably around 20-60 Hz, more preferably at a frequency of around 60 Hz, i.e. in the range of 50-70 Hz. Reference is made to the below examples, where these frequencies have been used and shown to be surprisingly effective.

Moreover, the stimulation unit can be configured to provide stimulation to spinal cord tissue and/or the tissue of the spinal cord dorsal roots at currents in the range of approx. 0.4-1.7mA. Again, reference is made to the below examples, where these amplitudes have been used and shown to be surprisingly effective.

The stimulation unit can be further configured provide stimulation to spinal cord tissue and/or the tissue of the spinal cord dorsal roots with a pulse width of approx. 200-300 $\mu$s. Also here, reference is made to the below examples, where these pulse widths have been used and shown to be surprisingly effective.

Moreover, it is possible that the stimulation unit comprises at least a lead paddle with an electrode array and is configured to provide stimulation to the spinal cord tissue and/or the tissue of the spinal cord dorsal roots with the electrodes of the lead paddle, where the cathode(s) is are provided by the electrodes of the lead paddle and/or the anode(s) is/are provided at least partially by either the electrodes of the lead paddle or the IPG case. Also here, reference is made to the below examples, where this setup has been shown to be effective.

Furthermore, it is possible that the system further comprises at least one module for administering a pharmacological agent to the subject.

For instance, the pharmacological agent may include Levodopa.

Additionally, it is possible that the system comprises a Brain Spinal Interface (BSI) with microelectrode arrays, which are configured to be placed into the cortex, preferably into the motor cortex, preferably into the primary motor cortex, preferably into the leg primary motor cortex, preferably into the left and right leg primary motor cortex. Surprisingly, it has been shown that a BSI instantly reduced freezing of gait and improved the walking speed, gait quality, posture and locomotor dexterity. Complementing the BSI with DBS in the subthalamic nucleus (STN) improved both locomotor performance and alertness, cf. also the results of the trials below.

A clinical trial on a PD subject (cf. Clinical trial on a human PD subject described below) showed that the combination of EES with DBS, preferably low frequency waves of DBS, provides for a synergistic effect that allows to obtain the most encouraging results in terms of locomotor improvements (e.g., fastest walking) in subjects with PD symptoms.

Advantageously, the delivered stimulation may be also combined with pharmacology, such as known neuromodulator drugs.

Advantageously, the delivered stimulation may be also combined with other medical therapies such as dopaminergic and cholinergic replacement therapies, sensory cuing devices and spinal and cortical stem cell implants.

The combination of electrical stimulation with pharmacology and/or other medical therapies allows to obtain a synergistic effect that potentiates effectiveness of the delivered stimulation.

Advantageously, the one or more electrodes may be connected to at least one IPG through one or more cables.

In particular, at least one IPG may be configured and adapted to generate a temporal sequence of electric currents that are transmitted to one or more electrodes through one or more cables.

The IPG may work independently until its batteries have been discharged.

Advantageously, the IPG may be rechargeable.

The IPG may be controlled by another device that can modify the generated sequence of electric currents, including stopping the delivery of the current.

At least one control unit may be configured and adapted to control at least one stimulation unit to deliver stimulation according to a predefined stimulation sequence.

In particular, the predefined stimulation sequence may be set to modulate over time.

Additionally or alternatively, the predefined stimulation sequence may be set to modulate over different stimulation locations.

The predefined stimulation sequence cannot be controller by the subject during delivery of electrical stimulation.

The stimulation parameters may be set to modulate over time.

Alternatively, the stimulation parameters may be fixed.

The system may further include a means for providing a feedback on the delivered stimulation to the subject.

Preferably, said feedback may include an acoustic feedback (e.g., a sound).

Additionally or alternatively said feedback may include a haptic feedback (e.g., a vibration).

Additionally or alternatively, said feedback may include visual feedback (e.g., a visual stimulus).

**In** one embodiment, the system may further include one or sensors.

**In** particular, said one or more sensors may include a cortical neurosensor for recording a cortical activity of a subject.

Additionally or alternatively, said one or more sensors may include a motion sensor.

Additionally or alternatively, said one or more sensors may include a haptic sensor.

Additionally or alternatively, said one or more sensors may include a physiological sensor.

The one or more sensors, e.g. one or more cortical neurosensors, may be non-invasive.

Non-invasive cortical neurosensors for use in the system of the invention may include one or more among electroencephalography (EEG) electrode sets, sets of functional near-infrared (fNIR) sensors, sets of magnetoencephalography (MEG) sensors and/or functional magnetic resonance imagining (fMRI) sensors.

Alternatively, the one or more sensors, e.g. one or more cortical neurosensors, may be invasive and thus implanted through surgery.

Invasive cortical neurosensors for use in the system of the invention may include one or more among stereotactic EEG, epidural and subdural electrocorticography (ECoG), penetrating cortical glass electrodes, and/or penetrating cortical microelectrodes.

In this embodiment, the system further includes one or more controller devices.

In particular, the one or more controller devices are configured and adapted to:

receive signals from the one or more sensors;

process the received signals to determine one or more parameters regarding movement intentions of the subject, and

generate one or more stimulation parameters based on the determined one or more movement intentions parameters.

The one or more controller devices may be implantable.

Alternatively, the one or more controller devices may be non-implantable and thus external to the body of the subject.

In this embodiment, the system further incudes one or more communicator devices, configured and adapted to communicate the generated one or more stimulation parameters to at least one IPG.

The one or more communicator devices may be implantable.

Alternatively, the one or more communicator devices may be non-implantable and thus external to the body of the subject.

Advantageously, the one or more sensors, e.g. one or more cortical neurosensors, the one or more controller devices and/or the one or more communicator devices may be integrated in a single unit.

This allows to reduce the overall dimensions of the system.

**In** another embodiment, the system may further include one or more muscle activity sensors for recording muscle activity of the subject.

For instance, muscle activity of the subject may include movements of the limbs and/or torso and/or head.

The one or more muscle activity sensors may be non-invasive.

Alternatively, the one or more muscle activity sensors may be invasive and thus implanted through surgery.

Additionally or alternatively, the system may further include one or more behavioral sensors (for instance, motion tracking sensors) for recording a behavior of the subject.

The one or more behavioral sensors may be non-invasive.

Alternatively, the one or more behavioral sensors may be invasive and thus implanted through surgery.

**In** this embodiment, the system further includes one or more controller devices.

**In** particular, the one or more controller devices are configured and adapted to:

receive signals from the one or more muscle activity sensors and/or one or more behavioral sensors;

process the received signals to determine one or more parameters regarding movement intentions of the subject, and

generate one or more stimulation parameters based on the one or more determined movement intentions parameters.

One or more controller devices may be implantable.

Alternatively, one or more controller devices may be non-implantable and thus external to the body of the subject.

In this embodiment, the system further includes one or more communicator devices, configured and adapted to communicate the generated stimulation parameters to at least one IPG.

One or more communicator devices may be implantable.

Alternatively, one or more communicator devices may be non-implantable and thus external to the body of the subject.

Advantageously, one or more muscle activity sensors and/or one or more behavioral sensors, one or more controller devices and/or the one or more communicator devices may be integrated in a single unit.

This allows to reduce the overall dimensions of the system.

In yet another embodiment, the system may further include a user input means for providing a user's command.

For instance, the user input means may include a microphone for providing a vocal command.

Additionally or alternatively, the user input means may include one or more buttons.

The user input means is operatively connected to one or more control sensors for collecting a user's command provided through the user input means.

One or more control sensors may be non-invasive.

Alternatively, one or more control sensors may be invasive and thus implanted through surgery.

In this embodiment, the system further includes one or more controller devices.

In particular, one or more controller devices are configured and adapted to generate one or more stimulation parameters based on the received user's command.

One or more controller devices may be implantable.

Alternatively, one or more controller devices may be non-implantable and thus external to the body of the subject.

In this embodiment, the system further includes one or more communicator devices, configured and adapted to communicate generated stimulation parameters to at least one IPG.

One or more communicator devices may be implantable.

Alternatively, one or more communicator devices may be non-implantable and thus external to the body of the subject.

Advantageously, user input means, one or more control sensors, one or more controller devices and/or one or more communicator devices may be integrated in a single unit.

This allows to reduce the overall dimensions of the system.

One or more features of the above-described embodiments may be as well combined in a single embodiment.

Explicitly disclosed herein is a method for mitigating locomotor deficits of Parkinson's disease with a combined neuromodulation and/or neurostimulation comprising at least the following steps:

- Deep Brain Stimulation (DBS) is provided to brain tissue of a subject;

- epidural electrical stimulation (EES) to the dorsal side of the spinal cord of said subject.

More preferably, the EES stimulation is delivered at spinal cord segments from L2 to S1. This section has been investigated and shown to be effective for EES stimulation to facilitate locomotion and gait restoration.

The tissue to be stimulated with EES is especially the area of the dorsal roots of the spinal cord.

Furthermore, it is possible that the Deep Brain Stimulation (DBS) is provided at low frequency stimulation to the brain tissue. For example, this can be in the range between 0-150Hz, especially in the range of 20-100Hz, more especially in the range of 20-70 Hz, preferably around 20-45 Hz or 20-60 Hz. Reference is made to the below examples and also to Baizabal-Carvallo et al, Low-Frequency Deep Brain Stimulation for Movement Disorders, Parkinsonism and Related Disorders, Vol. 31, October 2016, p. 14-22.

Additionally, the Deep Brain Stimulation (DBS) System can be provided as low frequency stimulation to the brain tissue by means of charge balanced biphasic stimulation pulses. These pulses have been shown to be effective in DBS and can be easily adjusted to finetune the DBS treatment (cf. below examples).

The Deep Brain Stimulation (DBS) provided with stimulation pulses with a pulse width of approx. 90 $\mu$s. This range can be e.g. set around 90 $\mu$s, e.g. from 80 $\mu$s to 100 $\mu$s. This pulse width has been successfully used in trials as shown below in the examples.

The Deep Brain Stimulation (DBS) can be provided to the subthalamic nucleus (STN). This brain area has been used for the delivery of DBS neurostimulation and the results have shown great effectivity, please see examples and data below.

Additionally, the stimulation to spinal cord tissue and/or the tissue of the spinal cord dorsal roots is for example provided at frequencies in the range between 0-150 Hz, especially in the range of 20-100 Hz, more especially in the range of 20-70 Hz, preferably around 20-60 Hz, more preferably at a frequency of around 60 Hz, i.e. in the range of 50-70 Hz. Reference is made to the below examples, where these frequencies have been used and shown to be surprisingly effective.

Moreover, the stimulation currents for the method can be in the range of approx. 0.4-1.7mA. Again, reference is made to the below examples, where these frequencies have been used and shown to be surprisingly effective.

The stimulation can be further provided to spinal cord tissue and/or the tissue of the spinal cord dorsal roots with a pulse width of approx. 200-300 $\mu$s. Also here, reference is made to the below examples, where these frequencies have been used and shown to be surprisingly effective.

Furthermore, it is possible that the method further comprises the step of administering a pharmacological agent to the subject.

For instance, the pharmacological agent may include Levodopa.

The neuromodulation/neurostimulation system of the invention may be used in a method for mitigating locomotor deficits in a subject affected by PD.

In particular, the exemplary method includes the following steps of:

equipping the subject with

- at least one control unit, configured and arranged to provide stimulation data,

- at least one stimulation unit being configured and adapted to deliver epidural electrical stimulation (EES) to the dorsal side of the spinal cord of a subject, and operatively connected to the at least one control unit

- at least one implantable pulse generator (IPG),

wherein at least one stimulation unit includes one or more electrodes configured to be implanted epidurally, one or more electrodes being operatively connected to at least one IPG, and

delivering electrical stimulation over the dorsal side of the spinal cord of the subject through one or more epidurally implanted electrodes.

Preferably, electrical stimulation is delivered to the lumbosacral region of the spinal cord.

More preferably, electrical stimulation is delivered at spinal cord segments from L2 to S1.

Preferably, the method further includes delivering deep brain stimulation (DBS) along with EES.

A clinical trial on a PD subject (cf. Clinical trial on a human PD subject described below) showed that the combination of EES with DBS allows to obtain a synergistic effect that allows to obtain the most encouraging results in terms of locomotor improvements (e.g., fastest walking) in subjects with PD symptoms.

Additionally or alternatively, the method may include administering pharmacology in combination with the delivered electrical stimulation.

Additionally or alternatively, the method may include administering other medical therapies in combination with the delivered electrical stimulation.

Said medical therapies may include dopaminergic and cholinergic replacement therapies, sensory cuing devices and spinal and cortical stem cell implants.

The combination of electrical stimulation with pharmacology and/or other medical therapies allows to obtain a synergistic effect that potentiates effectiveness of the delivered stimulation.

Stimulation may be delivered according to a predefined stimulation sequence.

In such a case, the predefined stimulation sequence may be set to modulate over time.

Additionally or alternatively, the predefined stimulation sequence may be set to modulate over different stimulation locations.

The predefined stimulation sequence cannot be controlled by the subject during delivery of electrical stimulation.

Alternatively, the subject can control some or all of the stimulation sequence parameters during delivery of electrical stimulation.

The stimulation parameters may be set to modulate over time.

Alternatively, the stimulation parameters may be fixed.

The method may further include providing a feedback on the delivered stimulation to the subject.

Preferably, said feedback may include an acoustic feedback (e.g., a sound).

Additionally or alternatively, said feedback may include a haptic feedback (e.g., a vibration).

Additionally or alternatively, said feedback may include visual feedback (e.g., a visual stimulus).

In one example, the method further includes:

equipping the subject with one or more cortical neurosensors for recording a cortical activity of the subject, one or more controller devices and one or more communicator devices;

receiving signals from the one or more cortical neurosensors through one or more controller devices;

processing the received signals to determine one or more parameters regarding movement intentions of the subject;

generating one or more stimulation parameters based on the determined one or more movement intentions parameters, and

communicating the generated one or more stimulation parameters to at least one IPG through the one or more communicator devices.

The one or more cortical neurosensors may be non-invasive cortical neurosensors such as electroencephalography (EEG) electrode sets, sets of functional near-infrared (fNIR) sensors, sets of magnetoencephalography (MEG) sensors and/or functional magnetic resonance imagining (fMRI) sensors.

Alternatively, the one or more cortical neurosensors may be invasive cortical neurosensors such as stereotactic EEG, epidural and subdural electrocorticography (ECoG), penetrating cortical glass electrodes, and/or penetrating cortical microelectrodes.

The one or more controller devices may be implantable.

Alternatively, one or more controller devices may be non-implantable and thus external to the body of the subject.

Similarly, one or more communicator devices may be implantable.

Alternatively, one or more communicator devices may be non-implantable and thus external to the body of the subject.

In another example, the method may further include:

equipping the subject with one or more muscle activity sensors and/or one or more behavioral sensors for recording muscle activity and/or a behavior of the subject, one or more controller devices and one or more communicator devices;

receiving signals from one or more muscle activity sensors and/or one or more behavioral sensors through one or more controller devices;

processing the received signals to determine one or more parameters regarding movement intentions of the subject;

generating one or more stimulation parameters based on the determined one or more movement intentions parameters, and

communicating the generated one or more stimulation parameters to at least one IPG through one or more communicator devices.

For instance, muscle activity of the subject may include movements of the limbs and/or torso and/or head.

The one or more muscle activity sensors and/or one or more behavioral sensors may be non-invasive.

Alternatively, one or more muscle activity sensors and/or one or more behavioral sensors may be invasive and thus implanted through surgery.

One or more controller devices may be implantable.

Alternatively, one or more controller devices may be non-implantable and thus external to the body of the subject.

Similarly, one or more communicator devices may be implantable.

Alternatively, one or more communicator devices may be non-implantable and thus external to the body of the subject.

In yet another example, the method may further include:

equipping the subject with a user input means, one or more control sensors, one or more controller devices and one or more communicator devices;

providing a user command through the user input means;

collecting user commands though one or more control sensors, operatively connected to user input means;

generating one or more stimulation parameters based on received user's commands through one or more controller devices, and

communicating the generated one or more stimulation parameters to at least one IPG through one or more communicator devices.

The user input means may include, e.g., a microphone for providing a vocal command and/or one or more buttons.

One or more control sensors may be non-invasive.

Alternatively, one or more sensors may be invasive and thus implanted through surgery.

One or more controller devices may be implantable.

Alternatively, one or more controller devices may be non-implantable and thus external to the body of the subject.

Similarly, one or more communicator devices may be implantable.

Alternatively, one or more communicator devices may be non-implantable and thus external to the body of the subject.

One or more method steps according to the above-described examples may be combined.

Further details and advantages of the present invention shall now be disclosed in connection with the drawings, where:

**Fig. 1** is a block diagram schematically illustrating a combined neuromodulation/neurostimulation system for mitigating locomotor deficits of/or neuronal disorders, especially Parkinson's disease, according to one embodiment of the invention;

**Fig. 2** is a block diagram schematically illustrating a combined neuromodulation/neurostimulation system for mitigating locomotor deficits of/or neuronal disorders, especially Parkinson's disease, according to another embodiment of the invention;

**Fig. 3** is a block diagram schematically illustrating a combined neuromodulation/neurostimulation system for mitigating locomotor deficits of/or neuronal disorders, especially Parkinson's disease, according to yet another embodiment of the invention;

**Fig. 4** is a block diagram schematically illustrating a combined neuromodulation/neurostimulation system for mitigating locomotor deficits of/or neuronal disorders, especially Parkinson's disease according to a further embodiment of the invention;

**Fig. 5a** schematically shows an example of an IPG which is suitable for use in the system according to the invention;

**Fig. 5b** schematically shows an example of a 5-6-5 spinal lead which is suitable for use in the system according to the invention;

**Fig. 6** is a clinical table showing parametrization of EES protocols used in tonic and closed-loop programs in a clinical trial on a human PD subject;

**Fig. 7** is a clinical table showing characterization of Left Foot Off and Right Foot Off EES sequences used for a closed-loop program in the same clinical trial as **Fig. 6;**

**Fig. 8** is a diagram showing the results obtained in the clinical trial of **Fig. 6,** illustrating that DBS and spinal EES synergistically alleviate gait deficits and facilitate over ground locomotion;

**Fig. 9** provides an overview of experimental procedures on a non-human primate (NHP) model of individuals (monkeys M1-M11) with pharmacologically-induced PD symptoms;

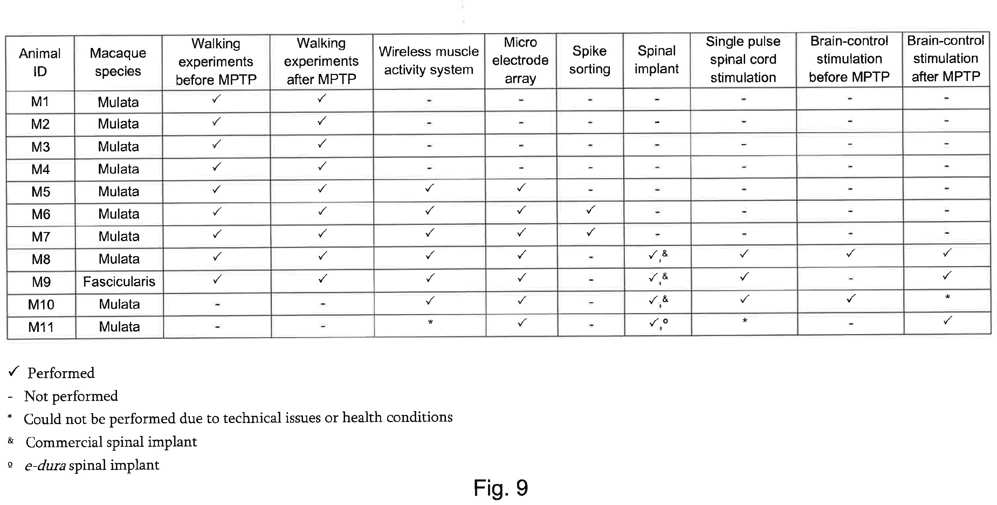

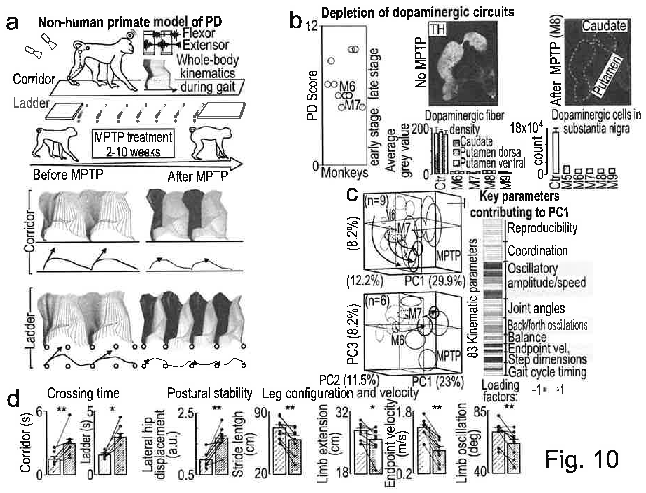

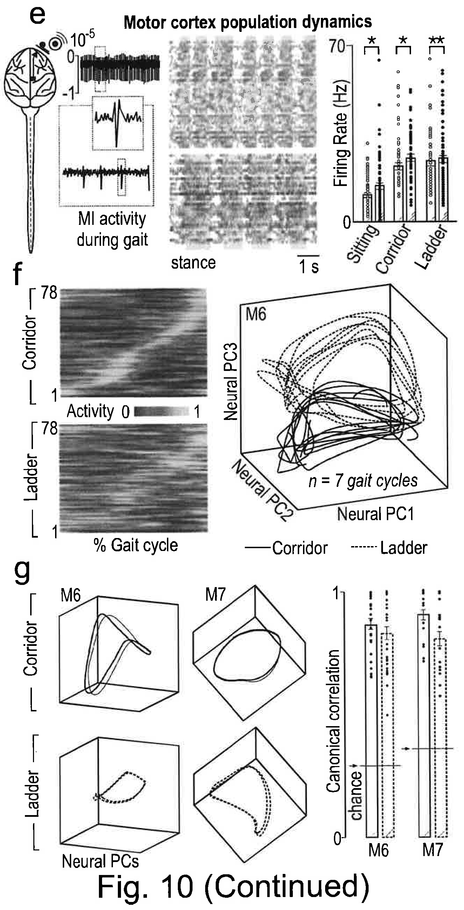

**Figs. 10a-g** are diagrams showing studies in gait and balance deficits based on experiments on the NHP model of PD shown in **Fig. 9.** In detail: **Fig. 10a.** Leg kinematics during basic and skilled locomotion before and after inducing PD symptoms by treating the monkeys with MPTP. **Fig. 10b.** The scatter plot shows the rounded mean of PD scores for each monkey across sessions recorded after MPTP administration. Photographs: dopaminergic projections labelled with tyrosine hydroxylase (TH) in the putamen and caudate of a healthy monkey and in M8 after the MPTP treatment. The bar plots show the density of TH-labeled projections in the putamen and caudate and count of dopaminergic cells in substantia nigra in healthy control (Ctr, n = 3) and each of the monkeys involved in MI recordings. **Fig. 10c.** PC analysis applied on 83 gait parameters. Balloons show mean $\pm$ SD of all gait cycles for each monkey before and after the MPTP treatment. Factor loadings (first PC) identified gait parameters most affected by the MPTP treatment. **Fig. 10d.** Bar plots report the mean values of these parameters. Overlaid dots show values for each monkey. **Fig. 10e.** Spiking activity of MI neurons while M6 and M7 were crossing the corridor or traversing the horizontal ladder. Raster plots of all sorted MI neurons underlying locomotion before and after the MPTP treatment in M7. Bar plots report the average mean spike rate of all sorted neuron (M6-M7) during sitting and walking before (n = 78 neurons) versus after the MPTP treatment (n = 82 neurons). **Fig. 10f.** Leg MI neural dynamics varies according to the locomotor task. Color plots display the average normalized spike rate of each neuron across a gait cycle, sorted according to their peak firing in the corridor and ladder before MPTP (number of gait cycles, corridor: M6: 67, M7: 53; after the MPTP treatment: M6: 104, M7: 190; ladder: M6: 78, M7: 37). The 3D plot shows the activity of all sorted neurons projected into space spanned by the three leading PCs for six corridor and six ladder steps before the MPTP treatment. **Fig. 10g.** Canonical correlation analysis applied on all sorted MI neurons, revealing reproducible neuronal population trajectories before and after the MPTP treatment. Bar plots report the canonical correlations for M6 and M7. *,** p < 0.05 and p < 0.01, respectively. Wilcoxon signed rank test. Error bars, sem.;

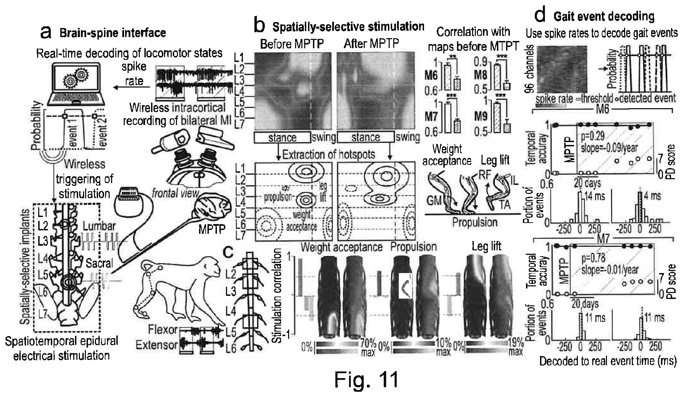

**Figs. 11a-d** are diagrams showing the methodological design of wireless BSI in the NHP model of PD shown in **Fig. 9.** In detail: **Fig. 11a.** Recording devices equipped with wireless modules are screwed onto skull-mounted pedestals to transmit wideband neural data from microelectrode arrays inserted into the leg region of left and right MI, and EMG activity from leg muscles. A control computer uses decoding algorithms to detect gait events from the received neural signals. The computer then wirelessly transmits the stimulation commands to an implanted pulse generator connected to spinal implants targeting the lumbar and sacral posterior roots. **Fig. 11b.** Leg muscle activity underlying locomotion projected onto the location of motor neurons to generate spatiotemporal maps of motor neuron activity, shown before and after MPTP administration (M8, n = 20 and 17 gait cycles before and after MPTP administration, respectively). Bar plots compare the correlation between two maps calculated before MPTP administration (before MPTP) and between a map calculated before MPTP administration and a map calculated after the MPTP administration (after MPTP) in M6, M7, M8 and M9 (number of gait cycles: before MPTP: M6: 12, M7: 10, M8: 20, M9: 32; after MPTP: M6: 55, M7: 44, M8: 17, M9: 11). Error bars, s.e.m. calculated using bootstrapping **(6).** The contour plots show hotspots of motor neuron activation extracted using Gaussian Mixture Modeling. **Fig. 11c.** The bars plots show the correlations between motor neuron activations achieved by spinal stimulation and the activation of each hotspot. **Fig. 11d.** The control computer implements a Regularized Linear Discriminant Analysis **(3,42)** algorithm to calculate the probability of hotspot initiation events from MI activity. The plots show temporal accuracy of detected hotspots and PD score for M6 (9 sessions) and M7 (8 sessions) before (white) and during (grey) the development of MPTP-induced gait and balance deficits. The histogram plots show the distributions of times between hotspot initiation events measured from kinematic recordings (ground truth) and the decoded hotspot initiation events;

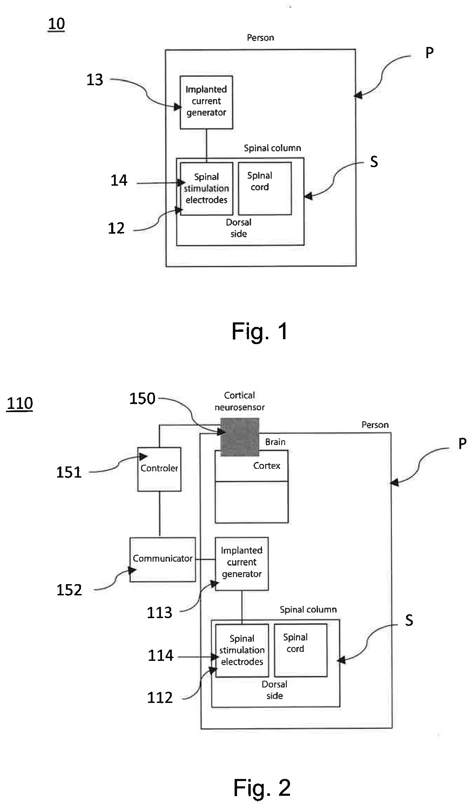

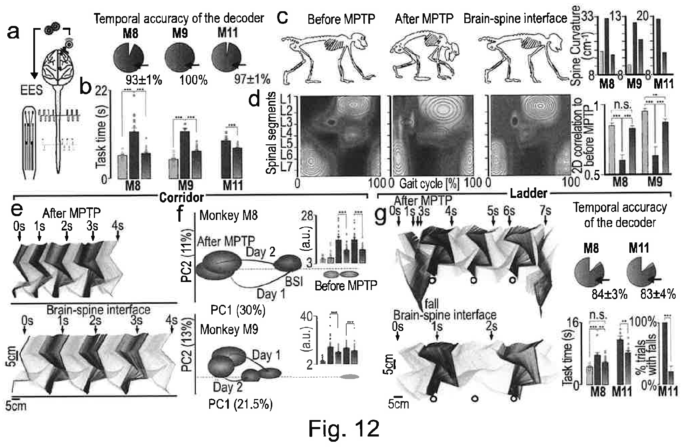

**Figs. 12a-g** are diagrams showing how PD symptoms are alleviated by using BSI in the NHP model of PD shown in **Fig. 9.** In detail: **Fig. 12a.** The scheme illustrates the BSI system. Pie charts report the average temporal accuracy of the decoder (n = 516, 618 and 612 events for M8, M9 and M11, respectively). **Fig. 12b.** Bar plots report the average task time (n = 16, 21, 8 trials for M8, 11, 12, 7 trials for M9, and 15, 22 trials for M11 across conditions from left to right). c. Body posture reconstructed from body kinematics using a whole-body skeletal model. Bar plots show the spine curvature measured from these reconstructions. **Fig. 12d.** Average spatiotemporal maps of motor neuron activity for M8, same experimental conditions as (c). Hotspots were extracted as explained in **Fig. 2.** Bar plots compare the surface correlation between two maps calculated before MPTP administration (before MPTP) to surface correlation calculated between a map calculated before MPTP administration and a map calculated after the MPTP administration when the stimulation is off (Stimulation OFF) and when using the BSI. For the before MPTP condition, the surface correlation was calculated between two healthy maps, each derived from a separate day (M8), or two healthy maps, each derived from one half of the steps of a single day (M9) (n = 17, 39 and 33 steps for M8, and 11, 26 and 34 steps for M9 across conditions from left to right). S.e.m. calculated using bootstrapping **(41). Fig. 12e.** Leg kinematics during locomotion (M9). **Fig. 12f.** Principal component analysis of leg kinematics. Bar plots report the Euclidean distance in the full 83-dimensional gait space between each gait cycle and the mean values across all the gait cycles recorded before MPTP administration (n = 26, 51, 27, 81, 50, 45 gait cycles for M8, 63, 62, 45, 140 and 55 gait cycles for M9 across conditions from left to right). **Fig. 12g.** Leg kinematics during walking along the horizontal ladder. Pie charts report the temporal accuracy of the decoder (n = 135 and 103 events for M8 and M11 respectively). Bar plots repot the occurrence of fall (n = 30 trials), and the time needed to complete the task (n = 9, 30, 11 trials for M8 and 6, 14 trials for M11 across conditions from left to right). *,**,*** significant difference at p < 0.05, p < 0.01 and p < 0.001, respectively using Wilcoxon rank sum test or the Monte Carlo permutation test. Error bars, s.e.m.;

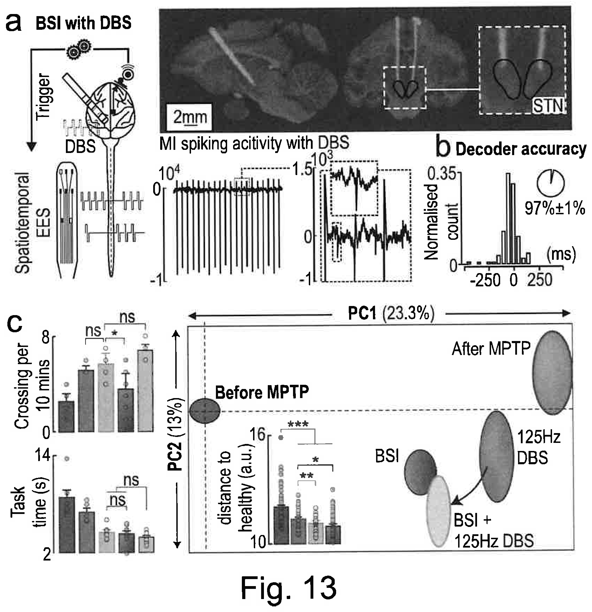

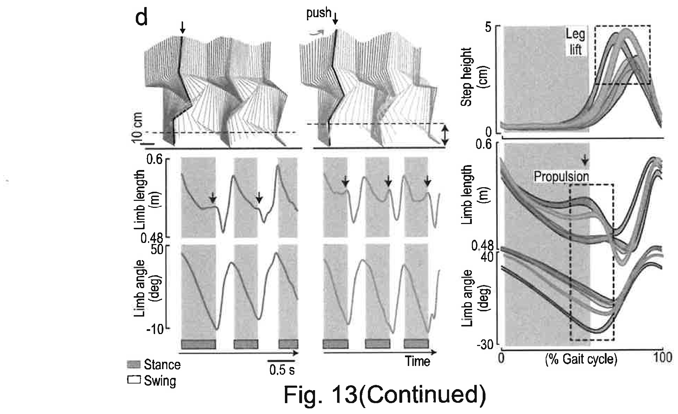

**Figs. 13a-d** are diagrams showing how BSI complements STN DBS by alleviating non-responsive gait and balance deficits in the NHP experiment of **Fig. 9.** In detail: **Fig. 13a.** Methods to study interactions between BSI and DBS (125 Hz) delivered in the STN. Post-mortem MRI confirming the location of DBS leads in each STN. The plots show MI recording during DBS, highlighting neuronal spikes interleaved with DBS-induced artefacts. **Fig. 13b.** The histogram plots show the distributions of times between hotspot initiation events measured from kinematic recordings (ground truth) and the detected hotspot initiation events while using the BSI + DBS therapy (n = 145 events). Pie chart reports the average temporal accuracy of the decoder. **Fig. 13c.** Locomotor performance measured before MPTP (grey) and after MPTP treatment with stimulation off (red), using DBS (green), using BSI (red to blue transition), and BSI + DBS (orange) (number of gait cycles: before MPTP: 39; after MPTP: 47; DBS: 37; BSI: 60; BSI + DBS: 43). The bar plot inset reports the Euclidean distance in the full 83-dimensional gait space between each gait cycle and the mean values across all the gait cycles recorded before MPTP administration. The bar plots report the number of corridor crossings within 10 minutes (before MPTP: 24; after MPTP: 19; DBS: 17; BSI: 19; BSI + DBS: 22) and task time (number of trials: before MPTP: 11; after MPTP: 7; DBS: 5; BSI: 9; BSI + DBS: 7). **Fig. 13d.** Gait cycles under DBS (left) and BSI + DBS (right) illustrate the enhanced propulsion achieved by adding BSI. From top to bottom: stick diagram decomposition of left leg movement; limb length of the left leg; and left leg limb angle. The light grey and white backgrounds correspond to the left stance and swing gait phases, respectively. The plots show mean ± standard deviation of left leg step height, limb length and limb angle across the gait cycle. *, **, *** significant difference at p < 0.05, p < 0.01, p < 0.001, respectively, using Wilcoxon rank sum test or the Monte Carlo permutation test. Error bars show s.e.m.;

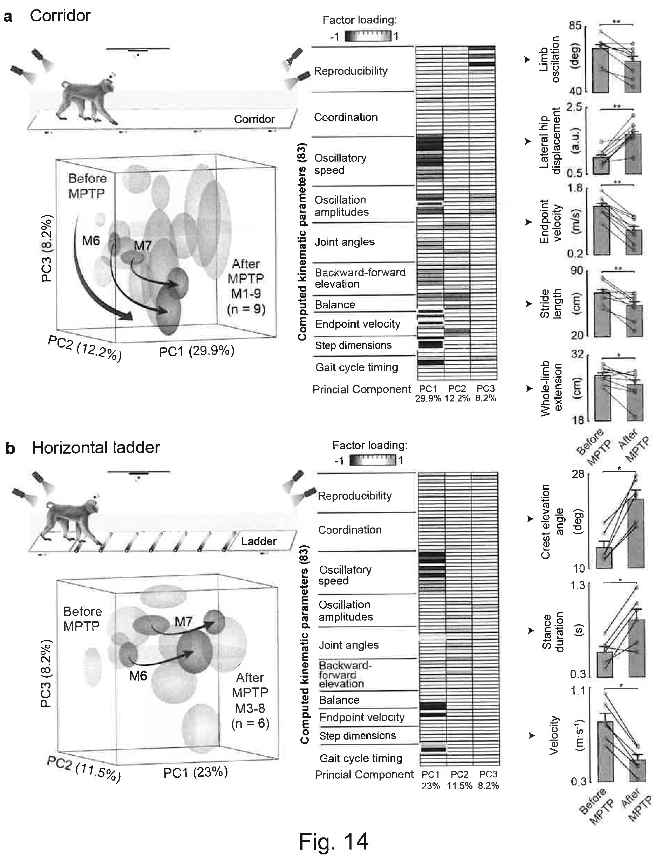

**Fig. 14** shows diagrams illustrating the objective quantification of gait and balance deficits following administration of MPTP in the NHPs in the NHP experiment of **Fig. 9.** In detail: The NHPs were trained to walk across a corridor (M1-9, n = 9) and along the rungs of a horizontal ladder (M3-8, n = 6). Both runways were embedded within Plexiglass enclosures that allowed the NHPs to behave freely and untethered. White marks were painted on the leg joints and other anatomical landmarks. As the monkey walked across the runways, said white marks were filmed using 4 or 6 cameras. Afterwards, these videos were processed to reconstruct whole-body kinematics in 3D. These kinematic recordings were used to compute 83 variables from each gait cycle that quantified kinematic features of monkeys' locomotor patterns (see also **Supplementary Table 2** below). This dataset was arranged in a matrix with variables as the matrix columns and each row representing one gait cycle. Data collected from different conditions (before and after MPTP administration) and different monkeys were pooled together in a single matrix and z-scored across columns. Two different tasks (corridor and ladder) were analyzed separately. Then, PCA was applied on this dataset and visualized the outcome by plotting the dataset in a new space spanned by the three leading PCs. The data for each monkey and condition is represented by balloons - ellipsoids with the center and principal semi-axis as the mean and standard deviation calculated across all the gait cycles for that condition and monkey (number of gait cycles in corridor: before MPTP: M1: 4, M2: 6, M3: 18, M4: 13, M5: 14, M6: 22, M7: 14, M8: 95, M9: 49; after MPTP: M1: 28, M2: 8, M3: 16, M4: 22, M5: 11, M6: 6, M7:9, M8: 48, M9: 59; ladder: before MPTP: M3: 12, M4: 25, M5: 23, M6: 19, M7: 9, M8: 40; after MPTP: M3: 18, M4: 7, M5: 24, M6: 31, M7: 24, M8: 14). To identify the parameters that best captured gait and balance deficits after MPTP administration, the variables were extracted with the highest loading factors on PC1 and PC2, here displayed with a color coding. Loading factors correspond to the correlation between each kinematic variable and a PC. The bar plots report the mean values of the parameters with the highest factor loadings. These parameters reflect gait and balance deficits commonly observed in people with PD. *,** $p < 0.05$ and $p < 0.01$, respectively. Wilcoxon signed rank test. Error bars, sem.;

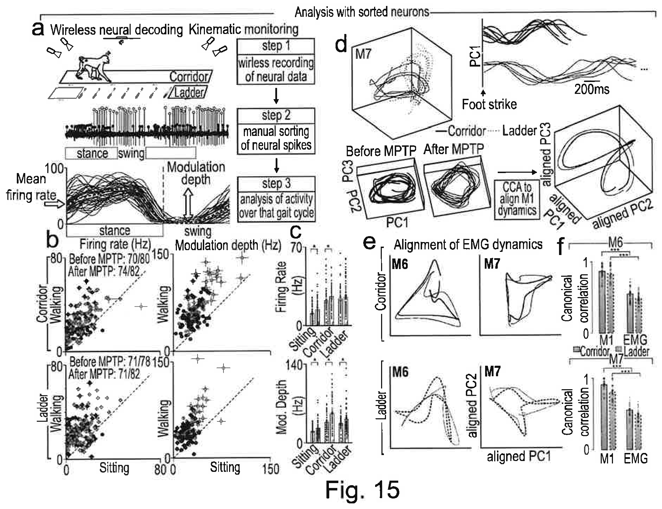

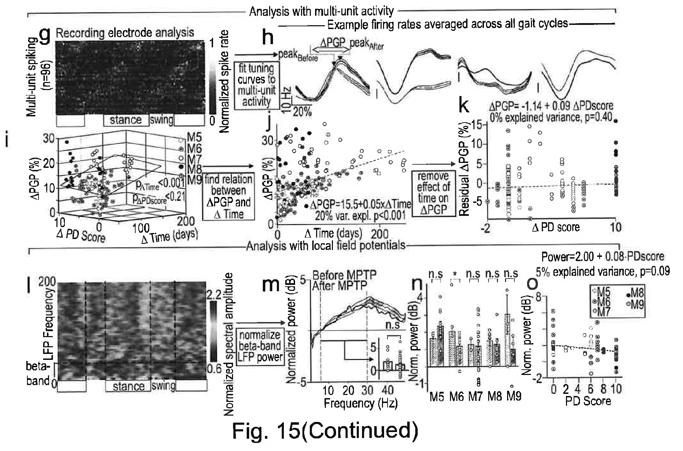

**Figs. 15a-o** show diagrams illustrating how MI population dynamics and LFP β-band activity remain similar despite gait and balance deficits in the NHP experiment of **Fig. 9.** In detail: **Fig. 15a.** Wireless recording of neural activity from MI neurons while M6 and M7 are crossing the corridor or traversing the horizontal ladder. The spikes were sorted from individual neurons and converted them into spike rates, as shown for one electrode, including identified action potentials marked with dots. The plot shows the firing rate of a representative neuron over the normalized duration of several gait cycles (grey, individual cycle; black average). **Fig. 15b.** Scatter plots showing the average firing rate of each sorted neuron during sitting (horizontal axes) and walking (vertical axes) before (gray) and after MPTP (red) administration for the corridor (top) and ladder (bottom) tasks. The number of sorted neurons that showed a significant increase in firing rate during walking is reported in each plot (number of gait cycles: corridor: before MPTP: M6: 67, M7: 53; after MPTP: M6:104, M7:190; ladder: before MPTP: M6:78, M7:37; after MPTP: M6:160, M7:90). **Fig. 15c.** Bar plots reporting the average population firing rate and modulation depth across the whole population of sorted neurons before and after MPTP administration (gait cycles as in **b**). **Fig. 15d.** Neural trajectories during corridor and ladder walking. The example shows a trial from M7 (top left). Corridor walking both before and after MPTP showed similar neural dynamics (bottom left), although the dynamics evolved at a slower pace after MPTP due to the slower walking speed (top right). However, the alignment process could compensate for any apparent differences and unveil a close correspondence between the neural trajectories (bottom right). **Fig. 15e.** Canonical correlation analysis applied on activity of all sorted MI neurons and on all the implanted muscles spanning four joints of the right leg (n = 8 muscles) for M6 (left) and M7 (right) in both the corridor (top) and ladder (bottom) tasks. The neuronal population trajectories differ between both tasks but are remarkably similar within each task regardless of the neurological status of the NHP. In contrast, the muscle activity trajectories were uncorrelated both between conditions and tasks. **Fig. 15f.** Bar plots report the mean canonical correlations of neuronal and muscle activity dynamics for M6 (top) and M7 (bottom) across all the pairs of sessions. Dots show the canonical correlation for each pair of sessions. **Fig. 15g.** Modulation of multiunit spike rates over successive gait cycles. Recordings are displayed for all the electrodes of the arrays, ordered vertically based on the timing of peak firing. **Fig. 15h.** The plots show four examples of average multiunit spike rates over the normalized gait cycle duration recorded on the same electrode before (blue) and after MPTP administration (red). To quantify the change in the modulation of multiunit activity over time, we calculated the absolute Preferred Gait Phase Change (ΔPGP), as illustrated in the first example. **Fig. 15i.** 3D plot showing the relationships between ΔPGP, the number of days between sessions and the change in PD score for each pair of sessions (n = 78, 55, 66, 28 and 8 sessions for M5, M6, M7, M8 and M9, respectively). **Fig. 15j.** Fitting a linear function (grey plain) revealed the significant influence of the time elapsed between sessions on the ΔPGP. **Fig. 15k.** Removing the contribution of the time difference on ΔPGP, as modelled by the linear fit, revealed no relationship between ΔPGP and neurological deficits, as measured by the PD score. Note that the relationship between ΔPGP and PD score is not significant. n.s., non-significant difference. **Fig. 15l.** Modulation of spectral amplitudes of LFPs over successive gait cycles. **Fig. 15m.** The LFP power spectrum was normalized by the pink noise estimated from LFPs recorded over the entire session (see methods). The plot shows the mean (line) and standard deviation (shaded area) of this pink-normalized LFP power calculated over all the sessions (n = 2, 2, 4, 6 and 3 sessions before MPTP administration and 11, 25, 11, 8 and 4 sessions after MPTP administration for M5, M6, M7, M8 and M9, respectively). The vertical dotted lines highlight the 6-30Hz band during which LFP power was calculated for each session and subject. **Fig. 15n.** Bar plots report, for each subject independently, the mean of the average power in the 6-30Hz band calculated over all the sessions (single dots) before and after MPTP administration. **Fig. 15o.** Relationship between the mean average power in the 6-30Hz band and PD score, reported for each session and subject. The broken line shows the linear fit, which describes only 5% of the variance in the data and is not significantly different from a fit to the mean value. *,** $p < 0.05$ and $p < 0.01$, respectively using non-parametric Wilcoxon rank sum test or Wilcoxon signed rank test. Error bars show s.e.m.;

**Figs. 16a-c** show diagrams illustrating the design of EES protocols based on spatiotemporal maps of motor neuron activity in the NHP experiment of **Fig. 9.** In detail:

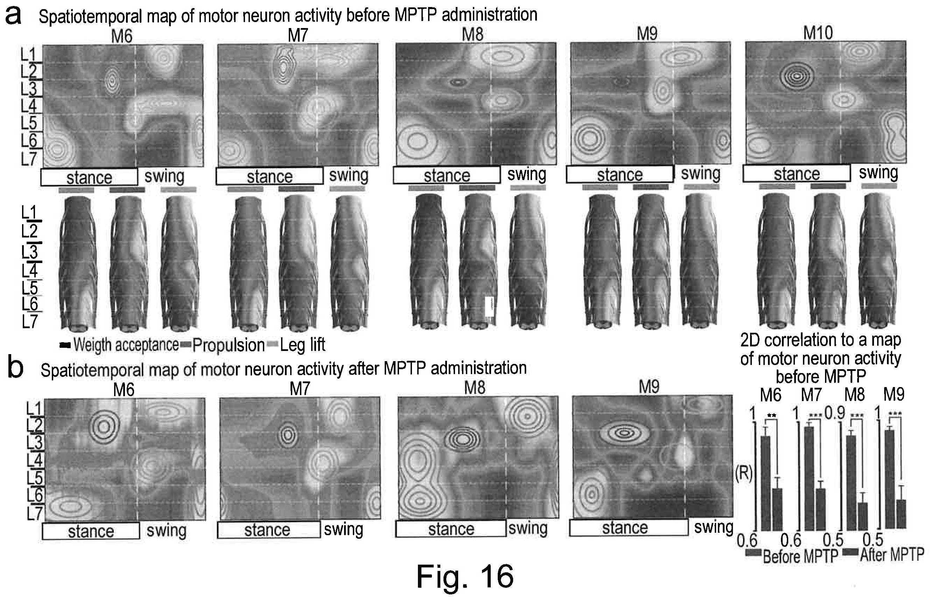

**Fig. 16a.** Colorplots showing the average spatiotemporal map of motor neuron activity underlying locomotion in M6 (n = 12 gait cycles), M7 (n = 10), M8 (n = 20), M9 (n = 32) and M10 (n = 13) before MPTP administration (healthy). The contours highlight the hotspots of motor neuron activity, which were identified using Gaussian Mixture Modeling. The spatial maps of motor neuron activity corresponding to the time at which each hotspot reached a maximum (center) are laid over the schematics of the spinal cord. **Fig. 16b.** Colorplots show the spatiotemporal maps of motor neuron activity underlying locomotion in M6 (n = 55 gait cycles), M7 (n = 44), M8 (n = 17) and M9 (n = 11) after MPTP administration. Bar plots compare the surface correlation between two maps calculated before MPTP administration and between a map calculated before MPTP administration and a map calculated after the MPTP administration. **16c**. Circular plots reporting the amplitude (grey scale) of responses recorded into leg muscles when delivering single-pulse of EES at increasing amplitudes (radial axis). White circles highlight the optimal amplitude while the polygon quantifies the muscular selectivity at this amplitude. Spatial map of motor neuron activity corresponding to optimal EES amplitudes are displayed for each hotspot (black traces). Bar plots report the correlation between each spatial map of motor neuron activity and the spatial map corresponding to the targeted hotspot. Muscle responses were normalized to the maximum amplitudes observed across all the recording sessions. * $p < 0.05$ using non-parametric Monte Carlo permutation test. Error bars show s.e.m.;

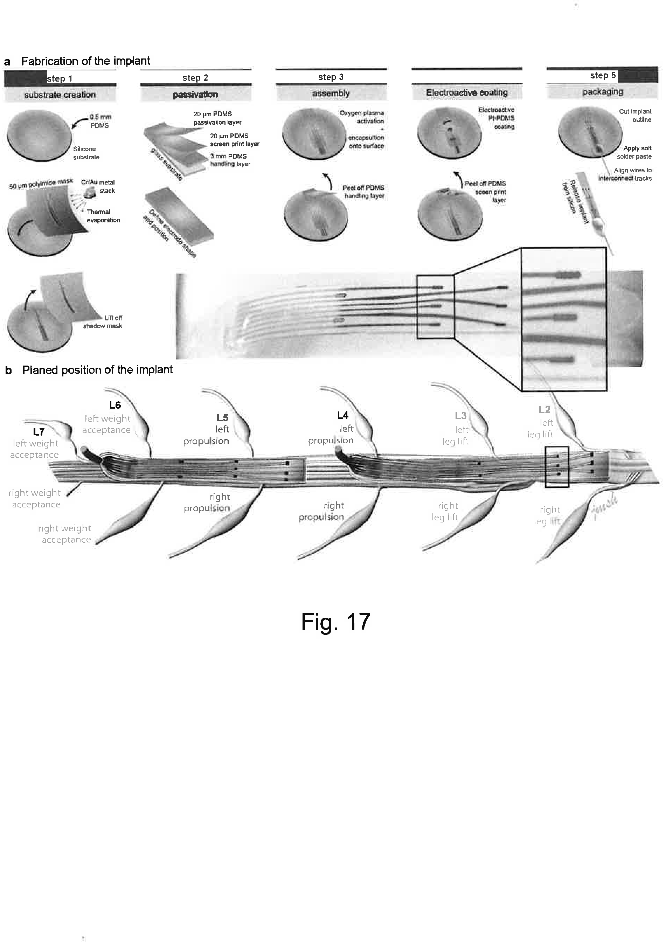

**Figs. 17a-b** show diagrams illustrating a detail of design and fabrication of a spinal implant for the NHP experiment of **Fig. 9**. In detail, a fabrication process was developed to manufacture the spinal implants used in M11, including the following steps **(Fig. 17a): Step 1: Substrate creation.** 4" silicon wafers were used as substrate to prepare the implants. (top) A polystyrene sulfonic acid layer is spin coated on the carrier to provide a water-release layer for the substrate stack. A PDMS layer is subsequently cast on the substrate until reaching a thickness of approximately 500 $\mu$m. (middle) A laser-machined, 50 $\mu$m thick polyimide mask is then manually laminated on the PDMS surface, and the carriers are then mounted in a thermal evaporation chamber. A stack of chromium and gold is thermally evaporated on the carriers through the polyimide masks, at a thickness of 5 nm (Cr) and 55 nm (Au). The chromium acts as adhesion promoter for the gold interconnect on PDMS. (bottom) The polyimide shadow mask is then peeled off the surface, revealing the interconnect design patterned in the metal stack. **Step 2: Passivation.** (top) A 3 mm thick PDMS handling layer is cast in a Petri dish. Once cured, the top surface is exposed to an oxygen plasma and a vapour-phase 1H, 1H,2H,2H-perfluorooctyltriethoxysilane layer is applied in a vacuum chamber. This process inhibits the adhesion of the silicone to subsequent PDMS layers deposited on the surface. Two subsequent 20 $\mu$m thick PDMS layers are spin-coated and cured on the thick PDMS, separated by the same adhesion inhibiting layer. A slab of this triple PDMS stack (3 mm, 20 $\mu$m, 20 $\mu$m in cross section) is then cut with a blade and mounted on a glass slide. (bottom) A mechanical catheter puncher is used to make holes through the two thin PDMS layers and into the thick handling layer, in order to machine the passivation stack with through-vias. Each via is created by punching a series of 4 round holes of 690 $\mu$m diameter with 400 $\mu$m centre-to-centre spacing. **Step 3: Assembly.** (top) The top surfaces of the substrate and triple stack encapsulation are exposed to oxygen plasma, then mounted on an alignment rack, with the two treated surfaces facing one another. The vias machined in the encapsulation are aligned with the interconnect patterned on the substrate, and the two parts are then put into contact in order to form a covalent bond between the silicone layers. (bottom) Once bonded, the thick PDMS handling layer is peeled off the substrate, leaving the interconnect encapsulated by two 20 $\mu$m thick PDMS layers with openings corresponding to the position of the electrodes. **Step 4: Electroactive coating.** (top) The electroactive coating is prepared as a composite material obtained by dispersing microscale platinum particles (3.5 $\mu$m maximum particle size) within a polydimethylsiloxane (PDMS) matrix. This creates a conductive paste that offers a balance between the charge injection properties of platinum and the mechanical properties of PDMS. The composite paste is applied on the encapsulation through the screen print PDMS layer, filling the openings to make an electrical contact with the interconnect. (bottom) The screen print layer is then peeled off to remove the excess coating and define the active stimulation sites. **Step 5: Packaging.** (top) The assembled implant is manually cut while still on wafer to the desired shape using a blade. Electrical wires are threaded in a PDMS guiding piece through holes that are machined at the same pitch as the gold tracks on the substrate. This soft connector is aligned and placed onto the interconnect with wires close to the ends of the gold tracks. Conductive Silver paste is then pressure dispensed to form individual electrical connections between the wires and the gold tracks. Once all electrical connections are made, a bolus of room temperature vulcanisation sealant (one component silicone sealant 734, Dow Corning) is applied over the connector to mechanically secure the assembly. (bottom) After the sealant is cured, the implant is released from the silicon carrier by dissolving the PSS layer under the PDMS substrate with DI water. All silicone layers are prepared by mixing polydimethylsiloxane using a weight ratio of 10:1 between pre-polymer and cross-linker. The deposited layers are cured for a minimum of 3 hours in a temperature-controlled oven set to 80°C. This fabrication process was exploited to produce spinal implants that embedded laterally-located electrodes targeting the left and right posterior roots of the lumbar spinal cord, as well as midline-located electrodes targeting the ascending fibers within the dorsal column **(Fig. 17b).** The spinal cord was reconstructed from a magnetic resonance imaging scan of a rhesus macaque onto which we represented the planed locations of the two fabricated spinal implants;

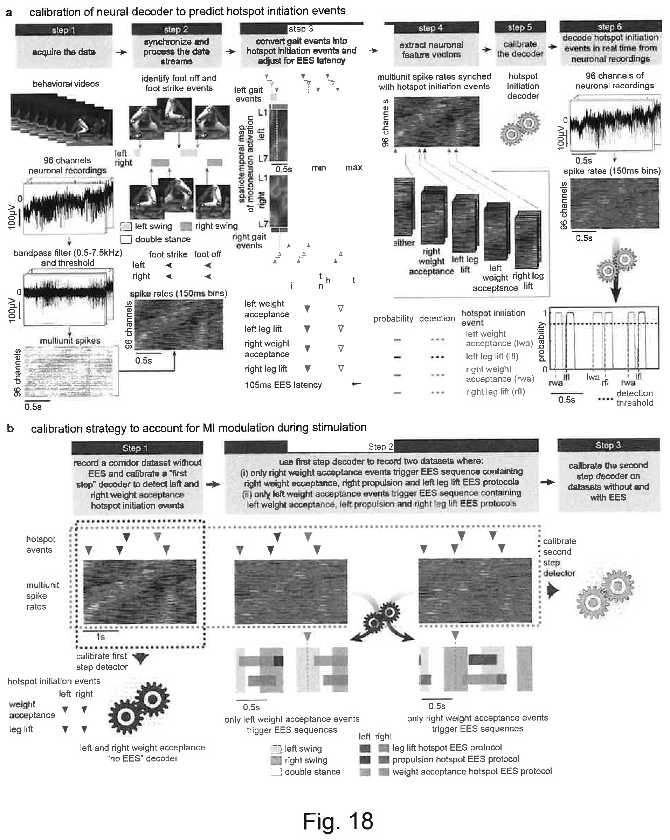

**Figs. 18a-b** show diagrams illustrating a calibration and validation process of decoders for real-time detection of hotspot events in the NHP experiment of **Fig. 9.** In detail, **Fig. 18a.** shows the following steps: **Step 1:** Hindlimb kinematics and MI activity were recorded during locomotion. The neural signals were band-pass filtered (0.5-7.5kHz), and multiunit spike events were collected based on a threshold set at 3.5 times the standard deviation. **Step 2:** Video frames containing left and right foot off and foot strike events were marked. Multiunit spike rates were estimated from overlapping 150 ms bins that were updated every 0.5 ms. **Step 3:** The right weight acceptance and right leg lift hotspots initiation events were estimated from the spatiotemporal map of motor neuron activity by aligning the gait events to the derived map of spatiotemporal motor neuron activity. The left hotspot initiation events were derived using the same process, assuming symmetry between both legs. The hotspot events were adjusted to account for the stimulation latency of 105 ms. **Step 4:** Feature vectors that originated at hotspot events were extracted and assigned to respective hotspot classes. All other feature vectors were assigned to the "neither" class. **Step 5:** These feature vector classes were used to calibrate a regularized linear discriminant analysis decoder. **Step 6:** The decoder was uploaded into the real-time analysis software application running on the control computer. Neural data was collected in real-time, processed into multiunit spike rates, and passed through the decoder that calculated the probabilities of hotspot events. When one of the hotspot event probabilities crossed a threshold of 0.8, a wireless command was sent to the implanted pulse generator to trigger the respective stimulation sequences. These sequences were composed of one or more stimulation protocols, each designed to reinforce one of six hotspots: left and right weight acceptance, propulsion and leg lift hotspots. **Fig. 18b.** shows a two-step decoder calibration including the following steps: **Step 1:** Data are acquired without EES to calibrate the first-step decoder as shown in a. **Step 2:** An additional set of data is acquired during which the first step decoder trigger composite stimulation sequences once per gait cycle. This sparse triggering mitigates the ability of EES to influence the neural activity used to detect the hotspot events that trigger EES. The composite EES sequences contain either left weight acceptance, left propulsion and right leg lift; or right weight acceptance, right propulsion and left leg lift EES protocols. A second step decoder is then calibrated using all the acquired datasets. Since the decoder is calibrated on neural activity that is non-affected and affected by EES, the decoder maintains high accuracy regardless of the presence or absence of EES;

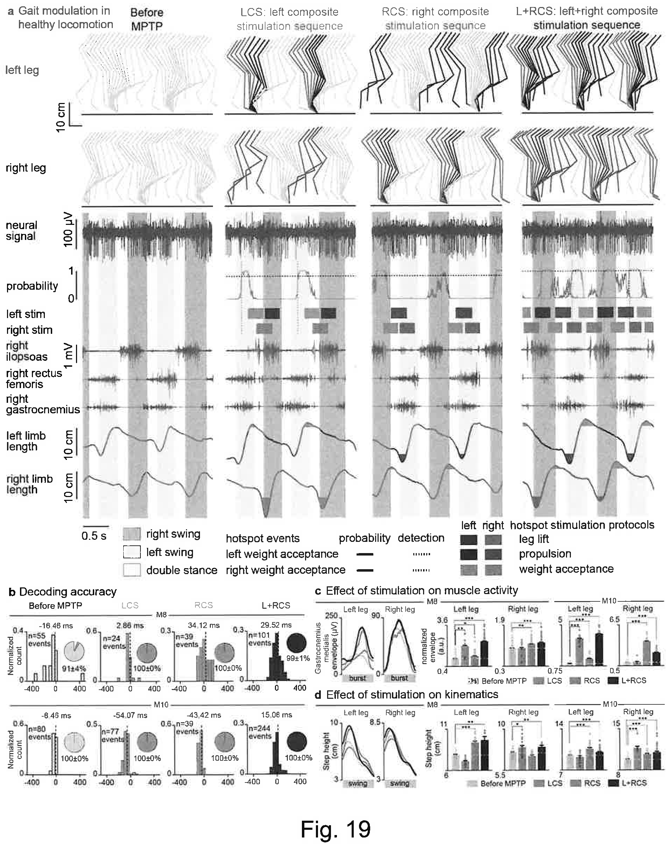

**Figs. 19a-d** show diagrams illustrating how BSI modulates leg kinematics and muscle activity during locomotion in the NHP experiment of **Fig. 9.** In detail: **Fig. 19a.** Examples of two successive gait cycles recorded during locomotion along a corridor without EES (left column), during brain-controlled left composite EES sequences (LCS), during brain-controlled right composite EES sequences (RCS), and during brain-controlled left and right composite EES sequences (L+RCS), from left to right. A composite EES sequence targets the weight acceptance and propulsion hotspot from one side, and the leg lift on the other side. From top to bottom: stick diagram decompositions of left and right leg movements; neural recording from a single channel; probability of left and right weight acceptance events; detected hotspot events (broken vertical lines), periods of stimulation through the electrodes targeting the left and right weight acceptance, propulsion and leg lift hotspots; electromyographic signals; whole-limb extension calculated as distance from the hip to the ankle joint. The white, light grey and dark grey backgrounds correspond to double stance, left and right swing gait phases, respectively. **Fig. 19b.** Decoding remains accurate when using increasingly complex and overlapping EES sequences. Histogram plots show the distribution of the temporal differences between real and detected events. Pie charts display the temporal accuracy, defined as the portion of real events that have the same type of event detected in their $\pm$ 200 ms neighborhood. From left to right: Offline decoding in the absence of stimulation (n = number of events 55 and 80 for M8 and M10, respectively), online decoding used to trigger LCS (n = 24 and 77), online decoding used to trigger RCS (n = 39 and 39), online decoding used to trigger L+RCS (n = 101 and 244). **Fig. 19c.** Brain-controlled composite EES sequences selectively strengthen the activity of the muscles involved in the gait period associated with the targeted weight acceptance hotspot. The plots show the average envelope of the gastrocnemius medialis muscle, which is the main muscle activated during weight acceptance. The bar plot reports the relative increase in the average peak envelope of the gastrocnemius medialis muscle during EES compared to locomotion without EES, for each of the conditions shown in a (n = number of gait cycles for each leg: M8: 73, 9, 36, 37; M10: 23, 18, 19, 21; Intact, LCS, RCS, and L+RCS, respectively). **Fig. 19d.** The plots show the vertical trajectory of the left and right feet during the swing phase under the same conditions as in a (n = number of gait cycles: M8: left leg: 26, 21, 17, 8; right leg: 24 20 17 8; M10: left leg: 26, 12, 30, 59; right leg: 19, 10, 22, 51; Intact, LCS, RCS, and L+RCS, respectively). Bar plots report the average maximum foot height for each experimental condition. *,**,*** significant difference at p < 0.05, p < 0.01 and p < 0.001, respectively using Wilcoxon rank sum test. Error bars show s.e.m.;

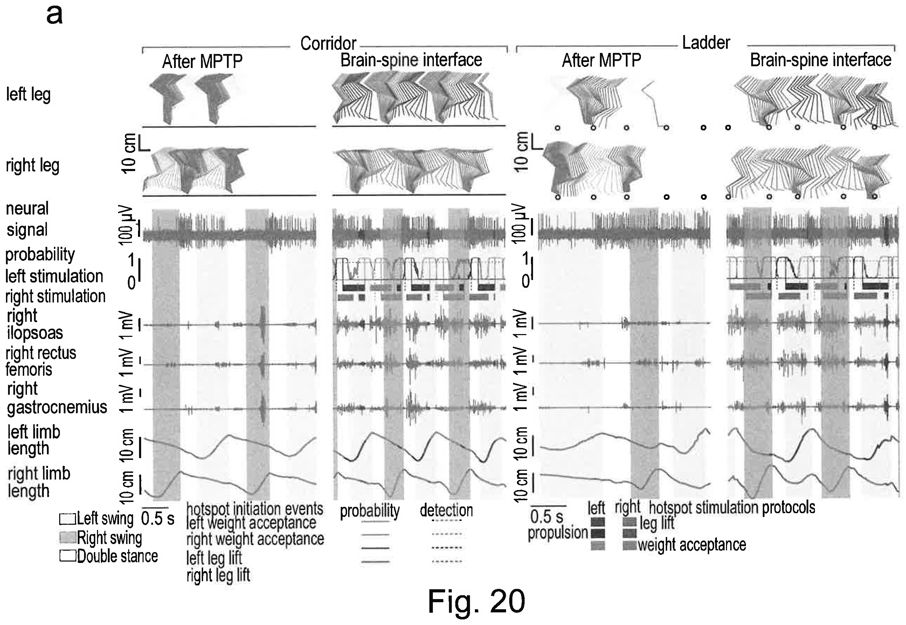

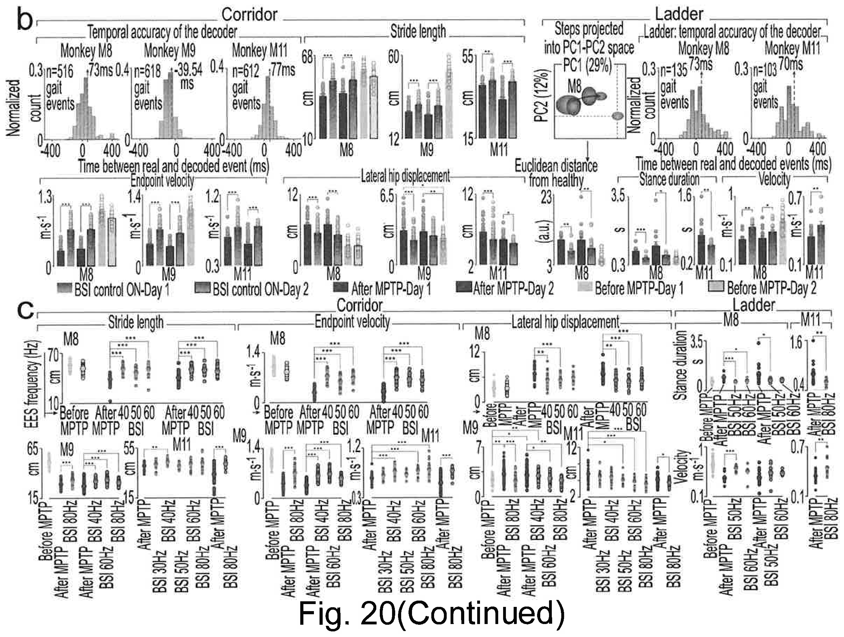

**Figs. 20a-c** show diagrams illustrating how BSI improves basic and skilled locomotion after MPTP administration in the NHP experiment of **Fig. 9.** In detail: **Fig. 20a.** Examples of locomotor execution along the corridor (3.3s) and ladder (2.4s) without stimulation (left columns) and when using the BSI in M8 after MPTP administration. Conventions as in **Fig. 19a. Fig. 20b.** PC analysis applied on 83 gait parameters for all the gait cycles recorded in each experimental condition. Balloons show mean ± standard deviation of all gait cycles for a given condition in space defined by PC1 and PC2, which explained 41% of all the variance. Bar plots report the Euclidean distance in the full 83-dimensional space of gait parameters between each gait cycle and the mean values across all the gait cycles recorded during two independent sessions before MPTP administration; as well as key parameters associated with gait and balance deficits commonly observed in people with PD (Corridor: n = 26, 51, 27, 81, 50 and 45 steps for M8, 63, 62, 45, 140 and 55 steps for M9, and 25, 87, 33 and 19 steps for M11 across conditions from left to right; Ladder: n = 10, 13, 14, 19 and 40 steps for M8, and 20 and 12 steps for M11 across conditions from left to right). The histogram plots show the distributions between hotspot initiation events measured from kinematic recordings (ground truth) and hotspot initiation events decoded during locomotion with the BSI (n = 516, 618 and 612 events for corridor for M8, M9 and M11, and 135 and 103 events for ladder for M8 and M11, respectively). Conventions as in **Fig. 13. Fig. 20c.** Changes in EES frequency between 30 and 80 Hz modulates gait parameters but has minimal impact on the efficacy of the therapy. The plots report the mean stride length, endpoint velocity and lateral hip displacement during locomotion along the corridor (n = 50, 45, 26, 16, 17, 18, 27, 26, 22 and 33 steps for M8, 55, 63, 62, 45, 58, 43 and 39 steps for M9, and 25, 11, 29, 10, 19, 26, 33 and 19 steps for M11 across conditions from left to right), and the stance duration and velocity during locomotion along the ladder (n = 40, 10, 8, 5, 14, 14 and 5 steps for M8, and 20 and 12 steps for M11 across conditions from left to right) under different EES frequencies with the BSI. Small circles, individual gait cycles; large circles, mean across all gait cycle for each condition. *,**,*** significant difference at p < 0.05, p < 0.01 and p < 0.001, respectively using Wilcoxon rank sum test. Error bars show s.e.m.;

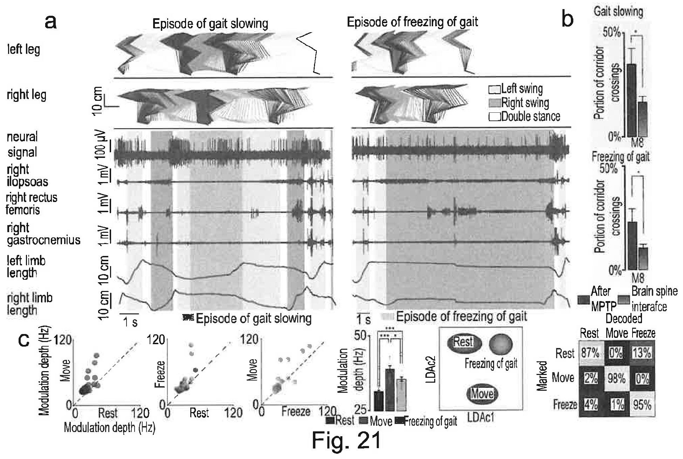

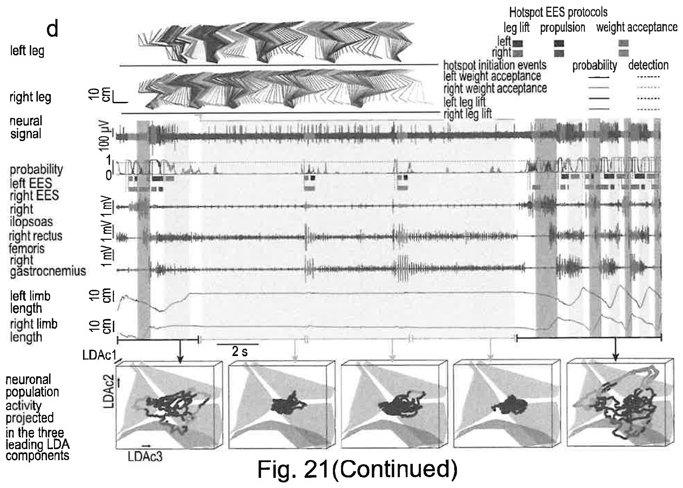

**Figs. 21a-d** show diagrams illustrating how BSI reduces the frequency of episodes of slowing and freezing of gait in the NHP experiment of **Fig. 9.** In detail: **Fig. 21a.** Examples of a slowing (11.45s) and freezing of gait (17.11s) episodes during locomotion along the corridor. Conventions are the same as **Fig. 19a. Fig. 21b.** Bar plots show the relative number of trials during which episodes of slowing or freezing of gait occurred without stimulation (n = 30 trials) and with the BSI (n = 129 trials). **Fig. 21c.** The scatter plots show the relationships between the modulation depths of multiunit spike rates (n = 48 multiunits) in the conditions move, rest and freezing of gait. Bar plots report the average modulation depth associated with these three conditions. To evaluate the separation between these three conditions, an rLDA classifier was calibrated on the neuronal population activity during rest, move and freezing of gait episodes (n = 23 trials). The balloons show mean ± standard deviation of neural population activity in the plane spanned by the two leading LDA components (LDAc1-2). The confusion matrix shows the classification performance. **Fig. 21d.** Example of a trial across the corridor during which a freezing of gait occurred while using the BSI. The 3D plots at the bottom report the projection of the neuronal population activity in the 3D space spanned by the three leading LDA components (LDAc1-3) during five periods of the trial, ordered temporally from left to right. The colored surfaces show the borders of spaces where probability of detecting one of the hotspot initiation events is higher than the threshold probability of 0.8. The neural trajectories are colored according to the space in which they located at a given moment. The decoder does not distinguish freezing of gait from rest. *, *** significant difference at p < 0.05 and p < 0.001, respectively, using Monte Carlo permutation test with bootstrapping. Error bars show s.e.m.;

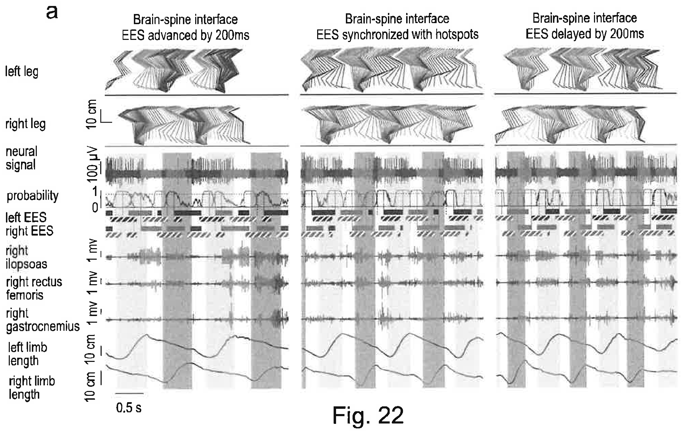

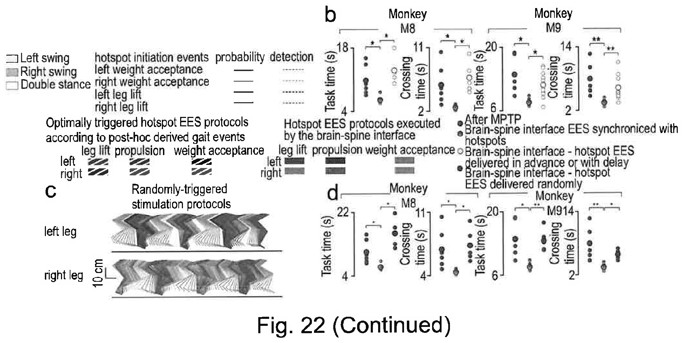

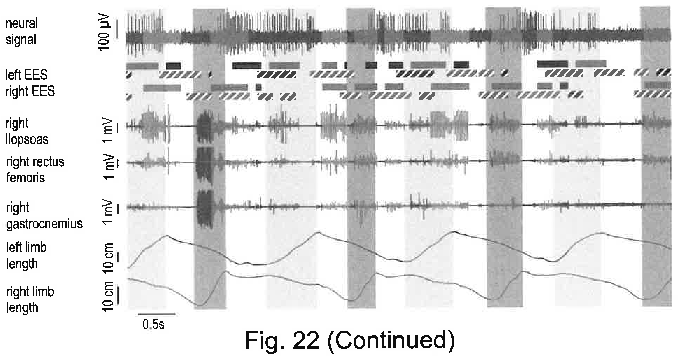

**Figs. 22a-c** show diagrams demonstrating that, with reference to the NHP experiment of **Fig. 9,** EES protocols must be synchronized precisely with hotspot activation events. In detail: **Fig. 22a.** Examples showing 3.3s of locomotion across the corridor using the BSI with three different decoding models (M8). From left to right: hotspot stimulation protocols initiated 200 ms before their actual initiation, hotspot stimulation protocols synchronized with hotspot activation, hotspot stimulation protocols initiated 200 ms after their actual initiation. Conventions are the same as in **Fig. 20a. Fig. 22b.** Dot plots showing the task time and crossing time without stimulation and when using the BSI delivering synchronized EES or EES advanced or with delay (task time: n = 7, 4 and 4 trials for M8 and 5, 5 and 11 trials for M9; crossing time: n = 7, 4 and 5 trials for M8 and 5, 5 and 11 trials for M9 for conditions from left to right, respectively). **Fig. 22c.** Example showing 7.16s of locomotion across the corridor during the randomly triggered stimulation protocols. Conventions are the same as in **Fig. 20a. Fig. 22d.** Dot plots showing the task time and crossing time without stimulation, when using the BSI to deliver synchronized EES, and random delivery of EES (task time: n = 7, 4 and 5 trials for M8 and 5, 5 and 5 trials for M9; crossing time: n = 7, 4 and 5 trials for M8 and 5, 5 and 5 trials for M9 for conditions from left to right, respectively). *, ** significant difference at p < 0.05 and p < 0.01, respectively, using Wilcoxon rank sum test;

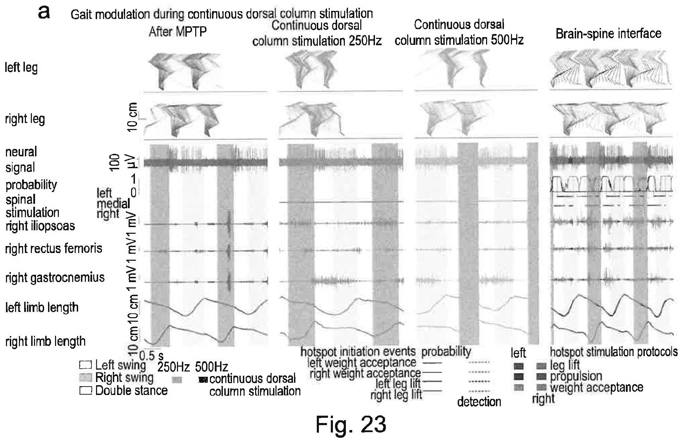

**Figs. 23a-c** show diagrams demonstrating that, with reference to the NHP experiment of **Fig. 9,** continuous dorsal column stimulation is less effective than the BSI. In detail:

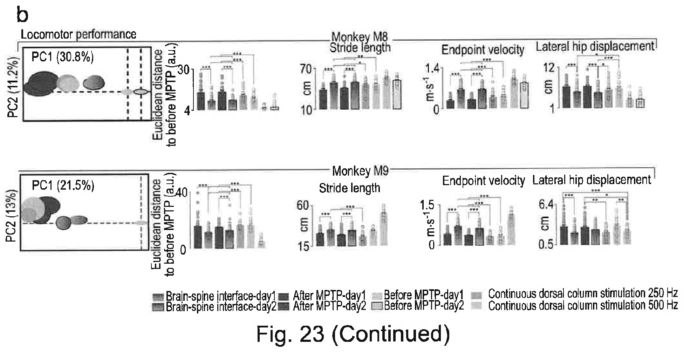

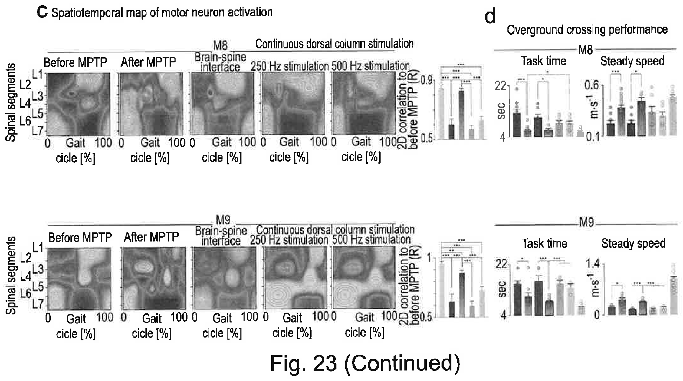

**Fig. 23a.** Examples showing 3.3s of locomotion across a corridor without stimulation, during continuous stimulation targeting the dorsal column at 250Hz or 500Hz, and using the BSI (M8 and M9). Conventions are the same in **Fig. 20a. Fig. 23b.** A PC analysis was applied to 83 gait parameters computed from all the gait cycles. Balloons show mean ± standard deviation of all the gait cycles in a plane spanned by the two leading PC. The bar plots report the Euclidean distance in the full 83-dimensional space of gait parameters and average values of gait parameters commonly affected in people with PD for the different experimental conditions, which were repeated on two distinct days (from left to right, n = 26, 51, 27, 81, 27, 29, 50 and 45 gait cycles for M8 and 63, 62, 45, 140, 46, 32 and 55 gait cycles for M9). **Fig. 23c.** Spatiotemporal maps of motor neuron activity for the same experimental conditions as in a (n = 20, 17, 39, 18 and 15 steps for M8 and 13, 11, 26, 16 and 18 steps for M9, from left to right). Conventions are the same as in **Fig. 13b.** The bar plot reports the spatial correlation between the spatiotemporal maps, using the same conventions as in **Fig. 13b.** **Fig. 23d.** Bar plots reporting the task time and steady speed for the conditions shown in a (n = 9, 21, 7, 4, 7, 6 and 8 trials for M8 and 7, 10, 5, 16, 5, 3 and 11 trials for M9, from left to right). *,**,*** significant difference at p < 0.05, p < 0.01 and p < 0.001, respectively using Wilcoxon rank sum test or the Monte Carlo permutation test. Error bars show s.e.m.;

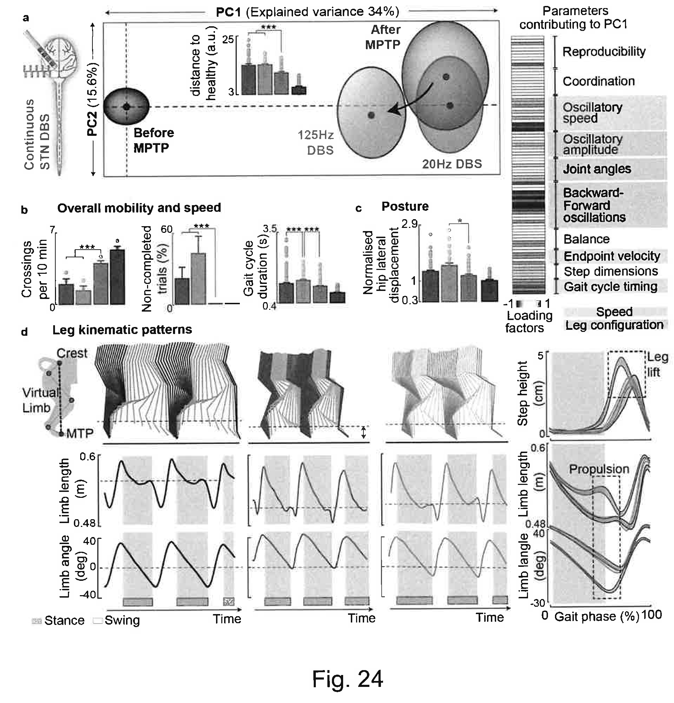

**Figs. 24a-d** show diagrams demonstrating that, with reference to the NHP experiment of **Fig. 9,** DBS reduces bradykinesia in an NHP MPTP model of PD. In detail: **Fig. 24a.** The monkey M9 was implanted with mini-DBS electrodes in the left and right subthalamic nucleus (STN) after the MPTP treatment to test the effect of low frequency (20Hz) and high frequency (125Hz) DBS during corridor walking. We evaluated the locomotor performance for the following conditions: before MPTP administration (grey) and after MPTP administration with stimulation off (red), using 20Hz DBS (purple), and using 125Hz DBS (green). Balloons show mean ± SD of all gait cycles for each condition in the space spanned by two leading PCs (number of gait cycles: before MPTP: 39; after MPTP: 47; 20Hz DBS: 27; 125Hz DBS: 37). The bar plot inset reports the Euclidean distance in the full 83-dimensional gait space between each gait cycle and the mean values across all the gait cycles recorded before MPTP administration. To identify the MPTP-induced locomotor deficits affected the most by the DBS, we identified the parameters with the highest loading factors on PC1. This analysis revealed a strong influence of DBS on parameters related to gait velocity and size, but reduced impact of limb configuration values (flexion, propulsion) during gait. **Fig. 24b.** As reported in Parkinson's disease patients, high frequency DBS increases the overall mobility and mediates a moderate increase on gait speed, while the low frequency DBS fails to improve locomotion and impairs awareness. The bar plots show the number of corridor crossings within 10 minutes (before MPTP: 24; after MPTP: 19; 20Hz DBS: 12; 125Hz DBS: 17), percentage of uncompleted trials, and gait cycle duration (number of gait cycles: before MPTP: 50; after MPTP: 160; 20Hz DBS: 49; 125Hz DBS: 137). **Fig. 24c.** High frequency DBS moderately improves the balance locomotor deficits. The bar plot shows the mean lateral displacement of the hip during gait (number of gait cycles same as in a). **Fig. 24d.** DBS failed to correct for the lack of propulsion and flexion induced by MPTP. The plots show examples of three successive gait cycles recorded before MPTP (left column), and after MPTP using 125Hz DBS (middle column) or without using stimulation (right column). Conventions as in **Fig. 13.** The plots show mean ± SD of left leg step height, limb length and limb angle across the gait cycle (number of gait cycles same as in a). *, **, *** reflect a significant difference at p < 0.05, p < 0.01, p < 0.001 respectively, using Wilcoxon ranksum test or the Monte Carlo permutation test. Error bars show s.e.m.

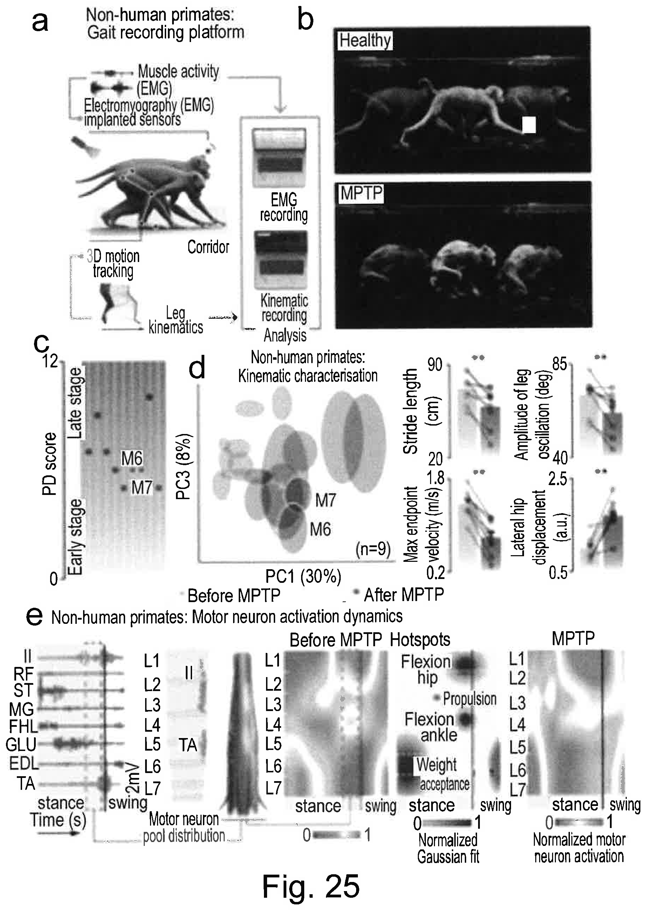

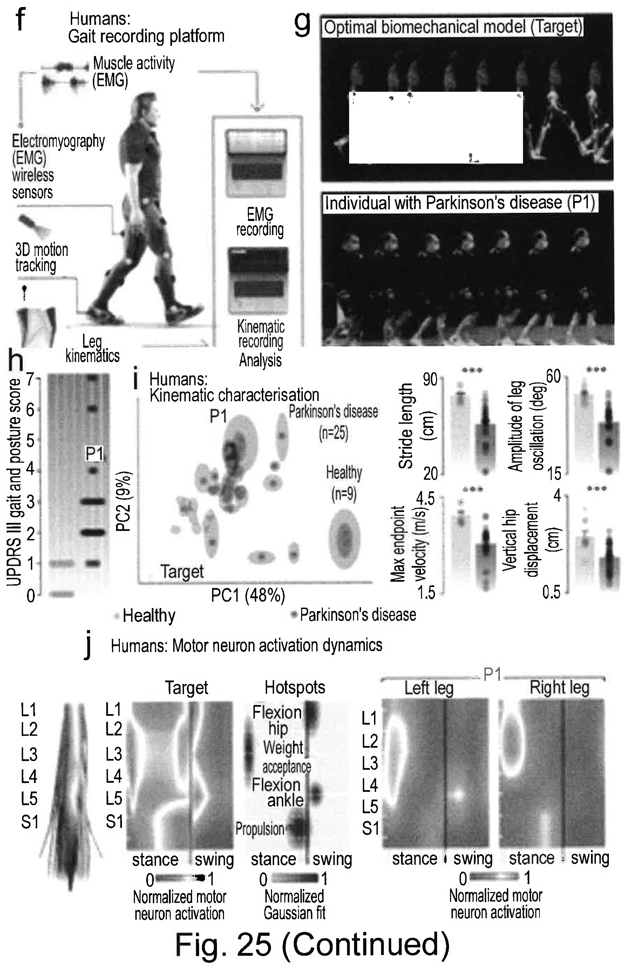

**Figs. 25a-j** show diagrams demonstrating that the NHP MPTP model of PD accurately replicates gait and balance deficits of people with PD. In detail: **Fig. 25a.** Recording platform for NHPs. **Fig. 25b.** Chronophotographies illustrating gait deficits in MPTP-treated NHPs. **Fig. 25c.** Distribution of PD scores for the 9 MPTP-treated NHPs. **Fig. 25d.** A PC analysis was applied on 83 parameters calculated for each gait cycle. Balloons show mean ±SD of all gait cycles projected in the space spanned by two of the three leading PCs. Bar plots report the mean values for 4 gait parameters highly correlated with PC1. **Fig. 25e.** From left to right: EMG recordings of right iliopsoas (IPS), rectus femoris (RF), semitendinosus (ST), gastrocnemius medialis (MG), flexor hallucis longus (FHL), gluteus (GLU), extensor digitorum longus (EDL) and tibialis anterior (TA) muscles during one gait cycle, location of IL and TA motor pools **(14);** projection of EMG signals onto these locations to elaborate a spatial map of motor neuron activation during the highlighted period of the stance phase; extension of this map across the entire duration of the gait cycle (n = 20 gait cycles); 3 hotspots of the right hemicord extracted by fitting a Gaussian model: weight acceptance, propulsion and leg lift that comprises anatomically separated ankle and hip flexion motor neuron activations; and changes in the map after MPTP (n = 17 gait cycles). **Fig. 25f.** Recording platform for human. **Fig. 25g.** Chronophotographies illustrating an optimal gait for participant P1 based on a personalized OpenSim neurobiomechanical model (target gait), and gait deficits of P1. **Fig. 25h.** UPDRS scores (gait) of 9 healthy people and 25 people with PD. **Fig. 25i.** Same analysis as in d for human, including for P1 and its target gait (yellow). **Fig. 25j.** Same analysis as in e for human: from left to right: projection of EMG signals during leg light from the target gait onto the anatomical model of P1; Target map (n = 168 gait cycles), extracted hotspots, and maps for the left leg (n = 130 gait cycles) and right leg (n = 131 gait cycles) of P1. **,*** p < 0.01 and p < 0.001, respectively, using Wilcoxon signed rank or rank sum tests. Error bars, sem.

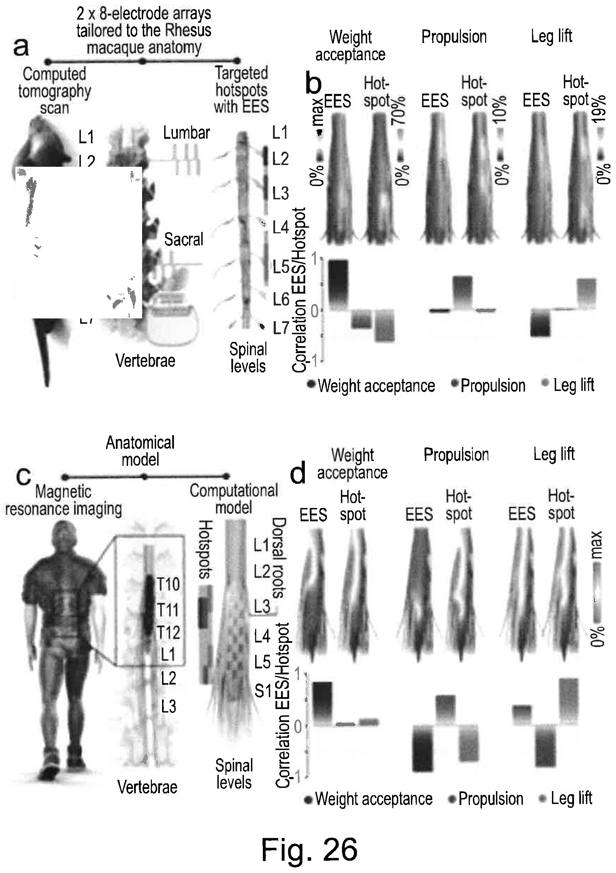

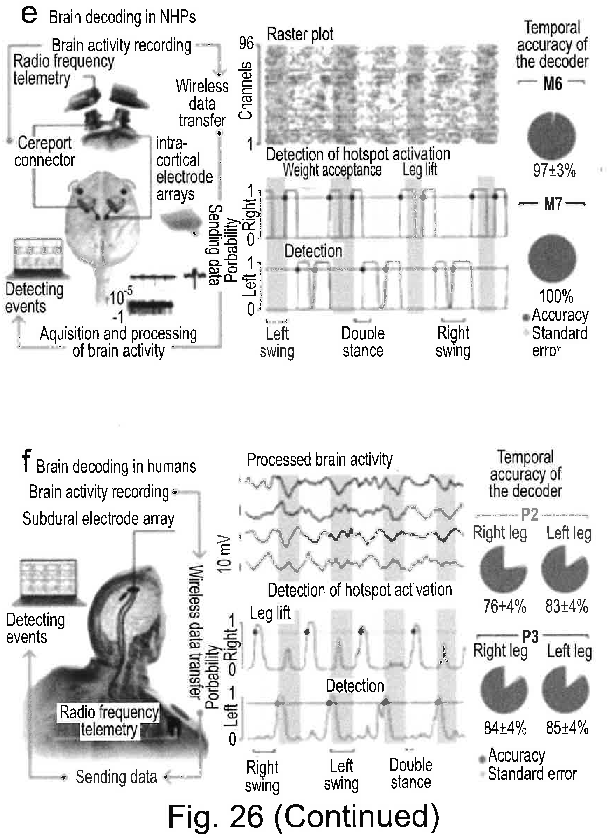

**Figs. 26a-f** show diagrams demonstrating the design of the BSI to alleviate gait deficits of PD for both NHPs and people with PD. In detail: **Fig. 26a.** CT scan of the spine determining the design of two 8-electrode arrays that target the 3 hotspots from each side, highlighted by specific colors in the vertical bars near the spinal cord. **Fig. 26b.** Spatial maps evoked by EES (left) and of the targeted hotspot (right). Bars plots reporting the correlations between the maps evoked by EES and the map associated of the 3 targeted hotspots. **Fig. 26c.** Anatomical model of P1's spine reconstructed from MRI and CT, and determining the surgical placement of a 16-electrode array. **Fig. 26d.** Same analysis as in b. **Fig. 26e.** Devices equipped with wireless modules are screwed onto skull-mounted pedestals to record and transmit wideband neural data from microelectrode arrays inserted into the leg region of left and right motor cortex in NHPs. Raster plots of neural activity and concomitant probability of activation of weight acceptance and leg lift hotspots over successive gait cycles from M6 after MPTP administration. Dots indicate when the probability crosses the detection threshold. The pie charts show the mean $\pm$ sem accuracy of the detections for M6 (178 events) and M7 (256 events). **Fig. 26f.** Quadripolar cortical paddle inserted subdurally over the motor cortex, and connected to the Medtronic Summit RC+S device to acquire epicortical signals wirelessly. The neural signals and probability of leg lifts are shown while participant P2 walks freely along a corridor. The pie charts show the mean $\pm$ sem accuracy of the detections for P2 (events: left: 64; right: 62) and participant P3 (events: left: 70; right: 74).

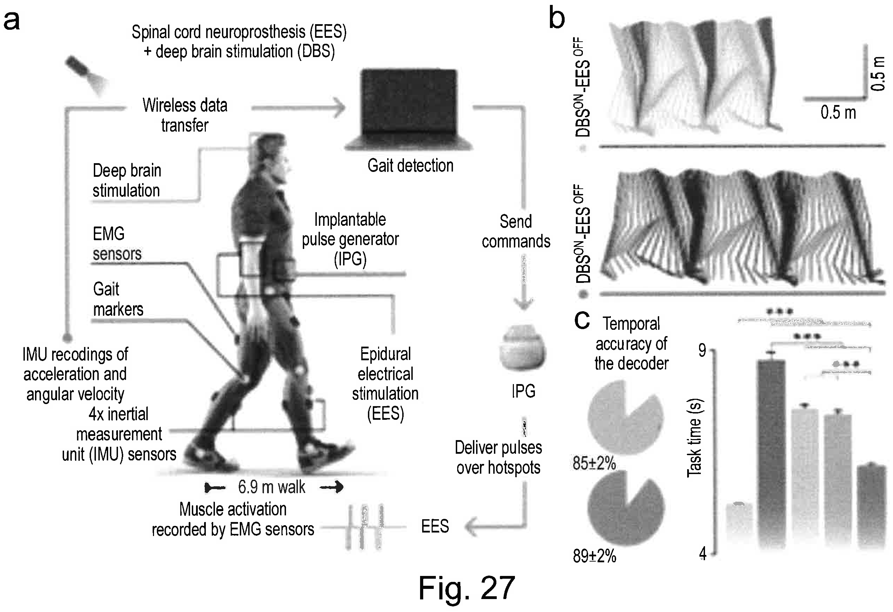

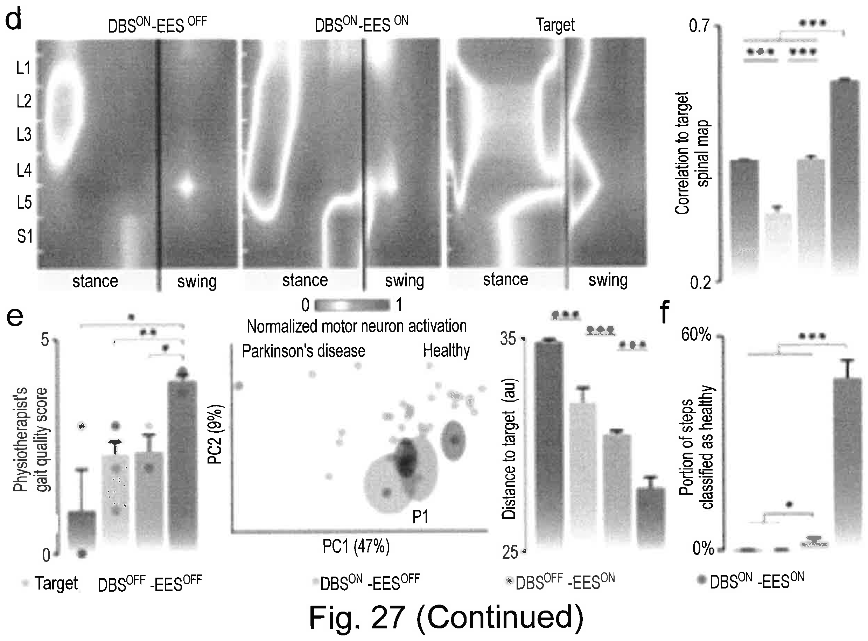

**Figs. 27a-f** show diagrams demonstrating that the spinal cord neuroprosthesis controlled by the behavioral IMU sensors alleviates gait deficits in a person with PD. In detail: **Fig. 27a.** Scheme illustrating the neuroprosthesis combined with DBS of the STN in participant P1. **Fig. 27b.** Stick diagram decomposition of leg kinematics during 3s of locomotion with DBSON - EESOFF and DBSON - EESON. **Fig. 27c.** Pie charts report the accuracy of the decoder during DBSOFF - EESON (n =462 events) and DBSON - EESON (n = 328 events) while P1 walks overground. Bar plot report task time (number of trials: DBSOFF - EESOFF: 25; DBSON - EESOFF: 24; DBSOFF - EESON: 38; DBSON - EESON: 32). **Fig. 27d.** Spatiotemporal maps of motor neuron activation and bar plots reporting the surface correlation between motor neuron activation maps during DBSON - EESOFF (n = 66 gait cycles) and DBSON - EESON (n = 91 gait cycles) compared to the map from the target gait (n = 168 gait cycles). Sem, calculated using bootstrapping. **Fig. 27e.** Bar plots report gait quality score evaluated by physiotherapists (number of trials: DBSOFF - EESOFF: 2; DBSON - EESOFF: 2; DBSOFF - EESON: 5; DBSON - EESON: 5). PC analysis of gait kinematics applied on 39 parameters, represented as in **Fig. 25i** (number of gait cycles: DBSOFF - EESOFF: 255; DBSON - EESOFF: 105; DBSOFF - EESON: 317; DBSON - EESON: 172). Bar plots report the mean Euclidean distance in the full 39-dimensional space between each gait cycle in a given condition and the mean gait cycle of the target gait. **Fig. 27f.** Bar plots report the portion of steps classified as healthy using a linear discriminant analysis decoder calibrated on all the steps from 25 people with PD and 9 healthy age-matched people (data reported in **Fig. 27e**). *, **, *** significant difference at p < 0.05, p < 0.01, p < 0.001, respectively, using Wilcoxon rank sum test or the Monte Carlo permutation test. Error bars, sem.

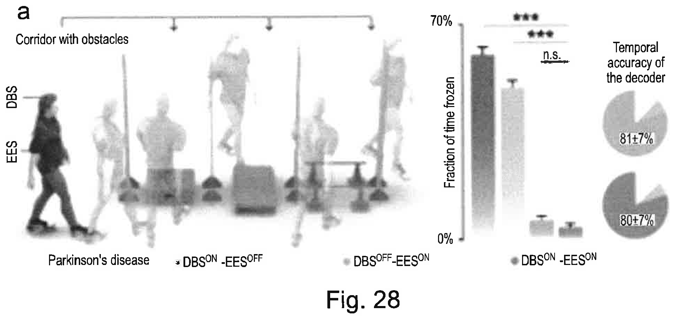

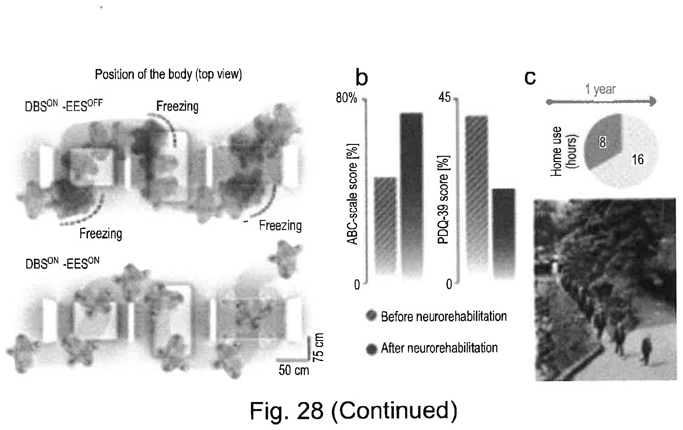

**Figs. 28a-c** show diagrams demonstrating that the spinal cord neuroprosthesis controlled by the behavioral IMU sensors reduces the frequency of freezing, improves balance, increases quality of life and supports mobility in community settings. In detail: **Fig. 28a.** Scheme illustrating a circuit that comprises three narrow passages with obstacles that provoked freezing of gait in P1. Below is shown the successive position of the body while P1 navigates along this circuit with DBS$^{ON}$ - EES$^{OFF}$ and DBS$^{ON}$ - EES$^{ON}$. Broken lines denote the occurrence of freezing of gait events. The bar plots report the fraction of time during which P1 experienced freezing (measurement duration: DBS$^{OFF}$ - EES$^{OFF}$: 39.0s; DBS$^{ON}$ - EES$^{OFF}$: 51.3s; DBS$^{OFF}$ - EES$^{ON}$: 27.7s; DBS$^{ON}$ - EES$^{ON}$: 23.3s). The pie charts report the accuracy of the decoder during DBS$^{OFF}$ - EES$^{ON}$ (n = 26 events) and DBS$^{ON}$ - EES$^{ON}$ (n = 29 events) while navigating the circuit. **Fig. 28b.** Bar plots reporting gains in balance measured using ABC scale questionnaire, and the quality of life measured using the PDQ-39 questionnaire after neurorehabilitation supported by the neuroprosthesis. **Fig. 28c.** Pie chart shows the daily independent use of the neuroprosthesis at home reported by P1 at his follow-up one year after the implantation. The chronophotograpy shows P1 using the neuroprosthesis for recreational walks in the nature. *** significant difference at p < 0.001 using Monte Carlo permutation test. Error bars, sem.

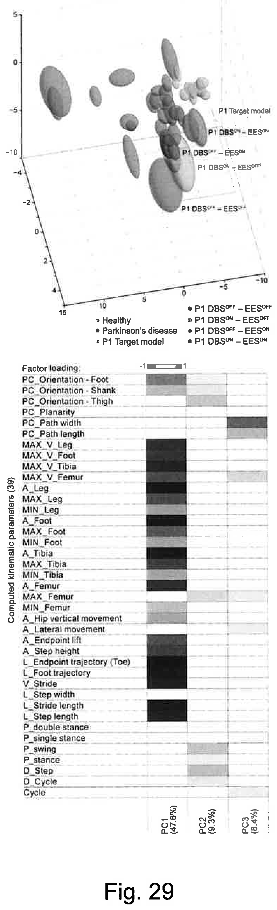

**Fig. 29** shows diagrams demonstrating that people with PD exhibit gait and balance deficits, which are accurately replicated by an NHP MPTP model of PD. In detail: Healthy subjects (H; n = 9) and subjects with PD (PD; n=25) walked straight overground as we recorded their full-body kinematics in 3D using the Vicon multi-camera system. We used these kinematic recordings to compute 35 variables from each gait cycle that quantified kinematic features of human locomotor patterns **(Supplementary Table 3).** As for the NHPs, we arranged this dataset in a gait parameters x gait cycles matrix, applied Principal Component Analysis on this dataset and visualized the outcome by plotting the distribution balloons for each subject in a space spanned by the three leading principal components (number of gait cycles: H1: 37, H2: 36, H3: 33, H4: 44, H5:42, H6: 38, H7: 45, H8: 33, H9: 39; PD1: 28, PD2: 30, PD3: 54, PD4: 53, PD5: 81, PD6: 47, PD7: 69, PD8: 37, PD9: 8, PD10: 100, PD11: 32, PD12: 22, PD13: 48, PD14: 25, PD15: 69, PD16: 70, PD17: 33, PD18: 61, PD19: 40, PD20: 29, PD21: 82, PD22: 8, PD23: 66, PD24: 33, PD25: 29). The colorplot shows the loading factors for the three leading PCs. *, ** $p < 0.05$ and $p < 0.01$, respectively, using Wilcoxon signed rank test. Error bars, sem.

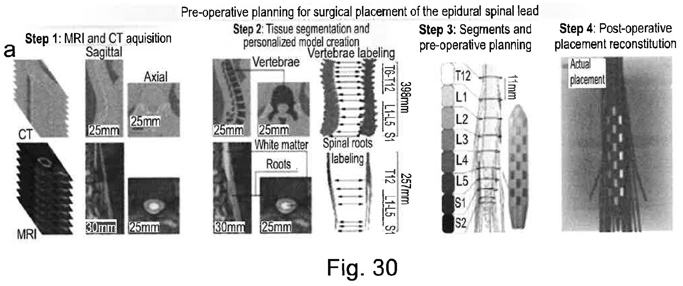

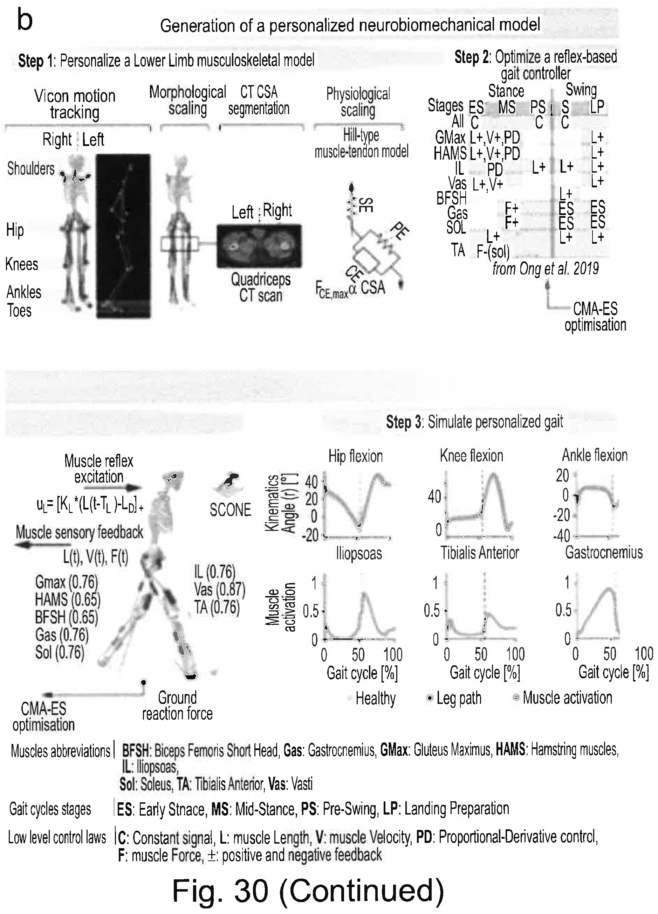

**Figs. 30a-b** show diagrams demonstrating procedures to achieve spinal cord neuroprosthesis in human. In detail: **Fig. 30a.** Clinical trial participant P1's CT and MRI scans were used to generate a three-dimensional anatomical model of the spine, which was then used to plan the surgical placement of the epidural spinal lead over the entire lumbar spinal cord. **Step1:** P1 underwent a CT and a structural 1.5T MRI scan of his spine. **Step 2:** The vertebral bones and disks from the CT scan and the spinal cord tissues (spinal cord, spinal cord roots and cerebrospinal fluid) were segmented from the MRI scan. The tissues segmented from the two different scans were co-registered, and combined into a 3D anatomical model of the P1's spine. **Step 3:** A 3D model of the spinal lead was loaded and placed centred over the dorsal side of the spinal cord covering the L1-L5 spinal segments. Position of the lead with respect to the segmented vertebral column determined the insertion point of the lead to be between L1 and T12 vertebra. During the surgery, the access to the surface of the dura was opened by small incisions and a T12/L1 flavectomy, and the tip of the lead was placed over the midline of the exposed dura and advanced the lead rostrally to the target location. The medial and segmental position of the paddle lead were accurately adjusted by monitoring the muscle responses to single-pulse EES delivered by different lead electrodes. **Step 4:** After the surgery, a post-operative CT scan was performed to reconstruct the position of the spinal lead with respect to the patient spine. The actual placement of the lead was within 1 cm of the preoperative plan, as expected due to segmentation and co-registration inaccuracies. **Fig. 30b.** In order to estimate the target for the immediate effects of the therapy, it was sought to simulate the gait of P1 given his anatomy but in the absence of neurodegeneration. To this end, a personalized neurobiomechanical model of P1 was generated. **Step1:** The Lower Limb model was personalised to P1's anatomy using morphological and physiological scaling. The morphological scaling was performed based on full-body motion tracking using the Vicon system. Then, the physiological scaling was implemented based on segmentation of muscles' cross-sectional area (CSA) from CT images. **Step2:** The reflex-based gait controller was optimized using the SCONE software. This controller is composed of phase dependent reflexes providing muscle excitation based on muscle length, velocity or force feedback. **Step3:** P1 gait was simulated in the absence of neurodegeneration using Covariance Matrix Adaptation Evolutionary Strategy (CMA-ES) to optimize controller parameters. About 500 generations of CMA-ES were necessary to reach a stable gait from initialization. Once the parameters of the controller have converged, 200 steps of this model were generated and full kinematics of lower limbs and muscle activity were extracted.

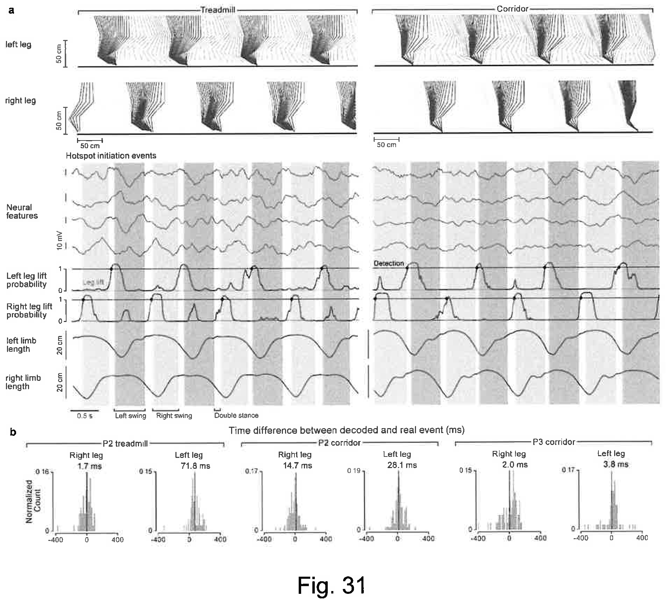

**Figs. 31a-b** show diagrams demonstrating that ECoG signals collected from the surface of the motor cortex of people with Parkinson's disease enable accurate detection of hotspot initiation events during locomotion. In detail: **Fig. 31a.** Examples of locomotor execution along the treadmill (5s) and corridor (5s) of participant P2. From top to bottom: stick diagram decompositions of left and right leg movements; neural features (low-pass filtered ECoG signal) from all four recorded channels; probability of left and right weight acceptance events with detected hotspot events (black dots); left and right limb length calculated as distance from the hip to the ankle joint. The white, light grey and dark grey backgrounds correspond to double stance, left and right swing gait phases, respectively. **Fig. 31b.** Decoding remains accurate for both tasks in P2 and for P3. Histogram plots show the distribution of the temporal differences between real and detected events (events: P1 treadmill: left: 92; right: 118; P1 corridor: left: 64; right: 62; P2 corridor: left: 70; right: 74). Median temporal difference is provided and marked by a vertical blue line.

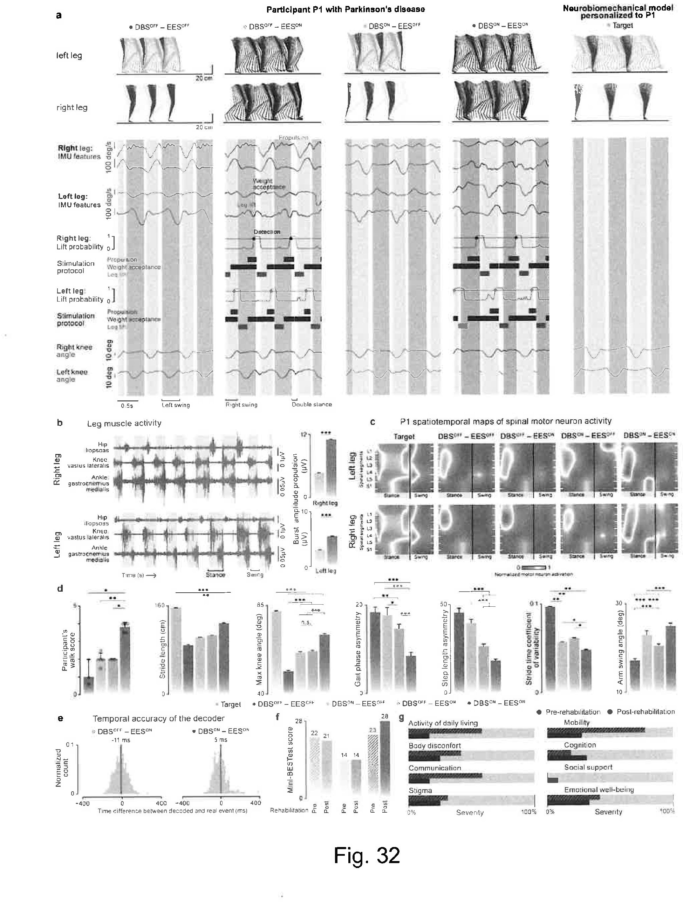

**Figs. 32a-g** show diagrams demonstrating that the Spinal cord neuroprosthesis delivering spinal EES in synchrony with attempted movements alleviates gait deficits of Parkinson's disease alone and synergistically with STN DBS. In detail: **Fig. 32a.** Examples show 3s of P1's locomotor execution along the corridor in four combinations of using or not the kinematically-controlled spinal cord neuroprosthesis (marked "EES") and STN DBS, and of the personalized neurobiomechanical model of P1. From top to bottom: stick diagram decompositions of left and right leg movements; four IMU features used to control the EES; probability of left and right weight acceptance events; detected hotspot events (broken vertical lines), periods of stimulation using a combination of 10 EES protocols targeting the left and right weight acceptance, propulsion and leg lift hotspots; and left and right knee angles. The white, light grey and dark grey backgrounds correspond to double stance, left swing and right swing gait phases, respectively. **Fig. 32b.** The plots showing EMG of left and right hip (iliopsoas), knee (gastrocnemius medialis) and ankle (vastus lateralis) leg muscles when transitioning from DBS$^{ON}$ - EES$^{OFF}$ into DBS$^{ON}$ - EES$^{ON}$ conditions illustrate the change in muscle activation. Bar plots show the burst amplitude of left and right gastrocnemius medialis muscle during the propulsion phase of gait in DBS$^{ON}$ - EES$^{OFF}$ (gait cycles: left: 79; right: 72) and DBS$^{ON}$ - EES$^{ON}$ (gait cycles: left: 136; right: 134) conditions. **Fig. 32c.** With the progressive use of spinal cord neuroprosthesis and DBS therapies, the motor neuron activation dynamics became more similar to that of the P1 neurobiomechanical model. The colorplots show the Target spatiotemporal spinal map derived from the P1 neurobiomechanical model, and the left and right leg spatiotemporal spinal maps of P1 in four combinations of using or not the kinematically-controlled spinal cord neuroprosthesis and STN DBS (number of gait cycles: Target: 168; left leg: DBS$^{OFF}$ - EES$^{OFF}$: 130; DBS$^{ON}$ - EES$^{OFF}$: 67; DBS$^{OFF}$ - EES$^{ON}$: 119; DBS$^{ON}$ - EES$^{ON}$: 95; right leg: DBS$^{OFF}$ - EES$^{OFF}$: 131; DBS$^{ON}$ - EES$^{OFF}$: 66; DBS$^{OFF}$ - EES$^{ON}$: 118; DBS$^{ON}$ - EES$^{ON}$: 91). Surface correlation between the Target and therapy spatiotemporal spinal maps are shown on **Fig. 27d. Fig. 32d.** Bar plots show measures of gait quality, efficacy and symmetry, as well as balance: participants walk score (number of trials: DBS$^{OFF}$ - EES$^{OFF}$: 2; DBS$^{ON}$ - EES$^{OFF}$: 2; DBS$^{OFF}$ - EES$^{ON}$: 5; DBS$^{ON}$ - EES$^{ON}$: 5), stride length (number of gait cycles: P1 target model: 335, DBS$^{OFF}$ - EES$^{OFF}$: 239; DBS$^{ON}$ - EES$^{OFF}$: 120; DBS$^{OFF}$ - EES$^{ON}$: 297; DBS$^{ON}$ - EES$^{ON}$: 209), max knee angle (number of gait cycles: Target: 336; DBS$^{OFF}$ - EES$^{OFF}$: 328; DBS$^{ON}$ - EES$^{OFF}$: 175; DBS$^{OFF}$ - EES$^{ON}$: 431; DBS$^{ON}$ - EES$^{ON}$: 339), gait phase asymmetry (number of gait cycles: DBS$^{OFF}$ - EES$^{OFF}$: 119; DBS$^{ON}$ - EES$^{OFF}$: 60; DBS$^{OFF}$ - EES$^{ON}$: 148; DBS$^{ON}$ - EES$^{ON}$: 103), step length asymmetry as measured by the ratio between lengths of the left and right steps (number of gait cycles: P1 Target model: 167; DBS$^{OFF}$ - EES$^{OFF}$: 119; DBS$^{ON}$ - EES$^{OFF}$: 60; DBS$^{OFF}$ - EES$^{ON}$: 148; DBS$^{ON}$ - EES$^{ON}$: 103), stride time coefficient of variability (number of gait cycles same as for stride length), and arm swing angle (number of gait cycles: DBS$^{OFF}$ - EES$^{OFF}$: 328; DBS$^{ON}$ - EES$^{OFF}$: 175; DBS$^{OFF}$ - EES$^{ON}$: 431; DBS$^{ON}$ - EES$^{ON}$: 339). **Fig. 32e.** Decoding of hotspot initiation events from IMU signals to control the spinal cord neuroprosthesis remains accurate both when DBS is on or off. Histogram plots show the distribution of the temporal differences between real and detected events (events: DBS$^{OFF}$ - EES$^{ON}$: 462; DBS$^{ON}$ - EES$^{ON}$: 328). Median temporal difference is provided and marked by a vertical line. **Fig. 32f.** The bar plots show the gains in balance, as measured using the Mini-BESTest, after the three-month rehabilitation supported by the spinal cord EES. **Fig. 32g.** The bar plots show the subcathegories of the quality of life PDQ-39 questionnaire scores before and after the three-month rehabilitation supported by the spinal cord EES. *, **, *** significant difference at $p < 0.05$, $p < 0.01$, $p < 0.001$, respectively, using Wilcoxon rank sum test or the Monte Carlo permutation test. Error bars show sem.

**Fig. 1** provides a schematic overview of a combined neuromodulation/neurostimulation system 10 for mitigating locomotor deficits of/or neuronal disorders, especially Parkinson's disease, according to one embodiment of the invention.

The neuronal disorders can be neuronal disorders of the brain. For example, such a disorder can be Parkinson's disease. Further disorders that can be treated (but not limited to) epilepsy, chronic pain (like (cluster) headache or migraine), depression, Alzheimer's disease, obsessive compulsive disorders, obesity, and/or other locomotor deficits and/or neuronal disorders after spinal cord injury (SCI), stroke, and/or other neurological disorders.

Not shown is that the system 10 comprises at least one control unit configured and arranged to provide stimulation data.

In the present embodiment, the system 10 comprises a single control unit.

The system 10 further comprises at least one stimulation unit 12.

In the present embodiment, the system 10 includes a single stimulation unit 12.

The stimulation unit 12 is operatively connected to the control unit.

The stimulation unit 12, is configured and adapted to deliver epidural electrical stimulation (EES) to the dorsal side of the spinal cord S of a subject P.

The system 10 further comprises at least one implantable pulse generator (IPG) 13.

In the present embodiment, the system 10 comprises a single IPG 13.

The stimulation unit 12 includes one or more electrodes 14, configured to be implanted epidurally.

The one or more electrodes are operatively connected to the IPG 13.

In the present embodiment, the stimulation unit 12 is configured and adapted to deliver electrical stimulation to the lumbosacral region of the spinal cord S, preferably at spinal cord segments from L2 to S1.

The recruitment of the spinal posterior roots that coincides with the ongoing movement intentions of the subject allows to reinstate the natural dynamics of the spinal motor circuits that, in turn, leads to reduction of gait and balance deficits of PD.

Not shown is that the at least one stimulation unit 12 further includes a means for delivery of deep brain stimulation (DBS).

Preferably, the at least one stimulation unit is configured and adapted to deliver low frequency waves of DBS.

The Deep Brain Stimulation (DBS) System is configured to provide low frequency stimulation to the brain tissue, especially at around 60 Hz. For example, this can be in the range between 0-150Hz, especially in the range of 20-100Hz, more especially in the range of 20-70 Hz, preferably around 20-45 Hz or 20-60 Hz.

For the trials, the usual frequency of 125 Hz was used, see below.

The Deep Brain Stimulation (DBS) System is configured to provide low frequency stimulation to the brain tissue by means of charge balanced biphasic stimulation pulses.

The Deep Brain Stimulation (DBS) System can be configured to provide stimulation pulses with a pulse width of approx. 90 μs. This range can be e.g. set around 90 μs, e.g. from 80 μs to 100 μs. This pulse width has been successfully used in trials as shown below in the examples.

The Deep Brain Stimulation (DBS) System can be configured to provide to the subthalamic nucleus (STN). This brain area has been used for the delivery of DBS neurostimulation and the results have shown great effectivity, please see examples and data below.

Additionally, the stimulation unit can be configured provide stimulation to spinal cord tissue and/or the tissue of the spinal cord dorsal roots, for example in the range between 0-150 Hz, especially in the range of 20-100 Hz, more especially in the range of 20-70 Hz, preferably around 20-60 Hz, more preferably at a frequency of around 60 Hz, i.e. in the range of 50-70 Hz. Reference is made to the below examples, where these frequencies have been used and shown to be surprisingly effective.

Moreover, the stimulation unit can be configured provide stimulation to spinal cord tissue and/or the tissue of the spinal cord dorsal roots at currents in the range of approx. 0.4-1.7mA. Again, reference is made to the below examples, where these frequencies have been used and shown to be surprisingly effective.

The stimulation unit can be further configured to provide stimulation to spinal cord tissue and/or the tissue of the spinal cord dorsal roots with a pulse width of approx. 200-300 μs. Also here, reference is made to the below examples, where these frequencies have been used and shown to be surprisingly effective.

Moreover, it is possible that the stimulation unit comprises at least a paddle lead with an electrode array and is configured to provide stimulation to spinal cord tissue and/or the tissue of the spinal cord dorsal roots with the electrodes of the lead paddle, where the cathode(s) is (are) provided by the electrodes of the paddle lead, and the anode(s) is/are provided at least partially by either the electrodes of the lead paddle or the IPG case. Also here, reference is made to the below examples, where this setup has been shown to be effective.

Furthermore, it is possible that the system further comprises at least one module for administering a pharmacological agent to the subject

For instance, the pharmacological agent can be Levodopa.

A clinical trial on a PD subject (cf. Clinical trial on a human PD subject described below) showed that a constant open-loop EES alleviated some deficits. Nonetheless, the combination of EES delivered synchronously with the subject's movement intentions, and DBS, preferably low frequency waves of DBS, provides for a synergistic effect that allows to obtain the most encouraging results in terms of locomotor improvements (e.g., fastest walking) in subjects with PD symptoms.

The delivered stimulation may be also combined with administration of pharmacology, such as known neuromodulator drugs.

Additionally or alternatively, the delivered stimulation may be also combined with other medical therapies such as dopaminergic and cholinergic replacement therapies, sensory cuing devices and spinal and cortical stem cell implants.

The combination of electrical stimulation with pharmacology and/or other medical therapies allows to obtain a synergistic effect that potentiates effectiveness of the delivered stimulation.

The electrodes 14 may be arranged into one or more electrode arrays (not shown) and epidurally implanted into the spinal cord S of the subject P.

The one or more electrodes 14 are connected to the at least one IPG 13 through one or more cables.

In particular, the IPG 13 can be configured to generate a continuous stimulation.

Alternatively and advantageously, the IPG 13 can also be configured and adapted to generate a temporal sequence of electric currents that are transmitted to the one or more electrodes 14 through the one or more cables.

The IPG 13 may work independently until its batteries have been discharged.

In the present embodiment, the IPG 13 is rechargeable.

In the present embodiment, the at least one control unit is configured and adapted to control the at least one stimulation unit 12 according to a predefined stimulation sequence.

In particular, the predefined stimulation sequence may be set to modulate over time and/or over different stimulation locations.

The predefined stimulation sequence cannot be controller by the subject P during delivery of electrical stimulation.

The stimulation parameters may be set to modulate over time.

Alternatively, the stimulation parameters may be fixed.

Advantageously, the system 10 may further include a means for providing a feedback on the delivered stimulation to the subject P.

In particular, said feedback may include an acoustic feedback (e.g., sound).

Additionally or alternatively, said feedback may include a haptic feedback (e.g., vibration).

Additionally or alternatively, said feedback may include a visual feedback (e.g., a visual stimulus).

**Fig. 2** provides a schematic overview of a combined neuromodulation/neurostimulation system 110 for mitigating locomotor deficits of/or neuronal disorders, especially Parkinson's disease according to another embodiment of the invention.

The neuronal disorders can be neuronal disorders of the brain. For example, such a disorder can be Parkinson's disease. Further disorders that can be treated (but not limited to) epilepsy, chronic pain (like (cluster) headache or migraine), depression, Alzheimer's disease, obsessive compulsive disorders, obesity, and/or other locomotor deficits and/or neuronal disorders after spinal cord injury (SCI), stroke, and/or other neurological disorders.

The system 110 comprises at least one control unit, at least one stimulation unit 112 including one or more epidurally implantable electrodes 114, and at least one IPG 113 **(Fig. 2)**. These components have the same structure and function of the corresponding components described above with respect to the system 10 of the first embodiment, and are therefore not further described for the sake of brevity.

In the present embodiment, the system 110 further includes one or more sensors.

In particular, in the shown embodiment, said one or more sensors include one or more cortical neurosensors 150 for recording a cortical activity of a subject P **(Fig. 2).**

The one or more cortical neurosensors 150 may be non-invasive.

Non-invasive cortical neurosensors 150 for use in the system 110 of the present embodiment may include one or more among electroencephalography (EEG) electrode sets, sets of functional near-infrared (fNIR) sensors, sets of magnetoencephalography (MEG) sensors and/or functional magnetic resonance imagining (fMRI) sensors.

Alternatively, the one or more cortical neurosensors 150 may be invasive and thus implanted through surgery.

Invasive cortical neurosensors for use in the system 110 of the present embodiment may include one or more among stereotactic EEG, epidural and subdural electrocorticography (ECoG), penetrating cortical glass electrodes, and/or penetrating cortical microelectrodes.

The system 110 further includes one or more controller devices 151, configured and adapted to

- receive signals from the one or more cortical neurosensors 150;

- process the received signals to determine one or more parameters regarding movement intentions of the subject P, and

- generate one or more stimulation parameters based on the determined one or more movement intentions parameters.

The one or more controller devices 151 may be implantable.

Alternatively, the one or more controller devices 151 may be non-implantable and thus external to the body of the subject.

The system 110 further includes one or more communicator devices 152, configured and adapted to communicate the generated one or more stimulation parameters to the at least one IPG 113.

The one or more communicator devices 152 may be implantable.

Alternatively, the one or more communicator devices 152 may be non-implantable and thus external to the body of the subject.

Advantageously, the one or more cortical neurosensors 150, the one or more controller devices 151 and/or the one or more communicator devices 152 may integrated in a single unit (not shown).

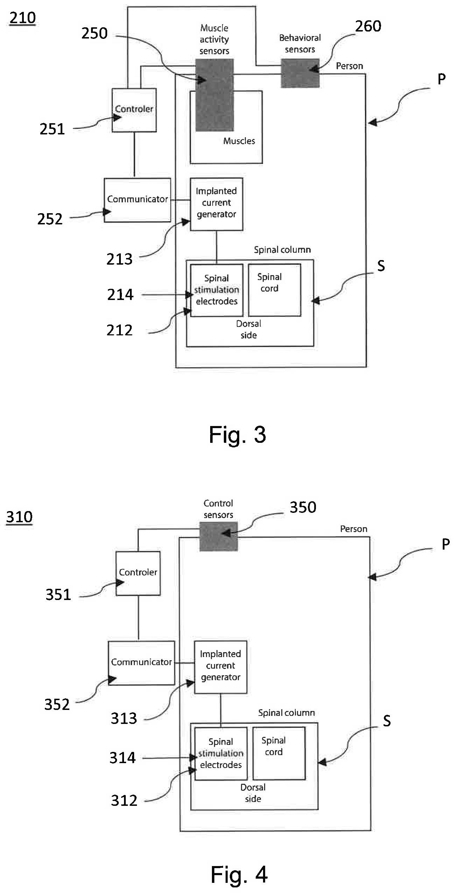

**Fig. 3** provides a schematic overview of a combined neuromodulation/neurostimulation system 210 for mitigating locomotor deficits of/or neuronal disorders, especially Parkinson's disease according to yet another embodiment of the invention.

The neuronal disorders can be neuronal disorders of the brain. For example, such a disorder can be Parkinson's disease. Further disorders that can be treated (but not limited to) epilepsy, chronic pain (like (cluster) headache or migraine), depression, Alzheimer's disease, obsessive compulsive disorders, obesity, and/or other locomotor deficits and/or neuronal disorders after spinal cord injury (SCI), stroke, and/or other neurological disorders.

The system 210 comprises at least one control unit, at least one stimulation unit 212 including one or more epidurally implantable electrodes 214, and at least one IPG 213 **(Fig. 3)**. These components have the same structure and function of the corresponding components described above with respect to the system 10 of the first embodiment, and are therefore not further described for the sake of brevity.

In the present embodiment, the system 210 further includes one or more muscle activity sensors 250 and/or one or more behavioral sensors 260 for recording muscle activity and/or a behavior of a subject P **(Fig. 3).**

The one or more muscle activity sensors 250 and/or the one or more behavioral sensors 260 may be non-invasive.

Alternatively, the one or more muscle activity sensors 250 and/or the one or more behavioral sensors 260 may be invasive and thus implanted through surgery.

The system 210 further includes one or more controller devices 251, configured and adapted to

- receive signals from the one or more muscle activity sensors 250 and/or one or more behavioral sensors 260;

- process the received signals to determine one or more parameters regarding movement intentions of the subject P, and

- generate one or more stimulation parameters based on the one or more determined movement intentions parameters.

The one or more controller devices 251 may be implantable.

Alternatively, the one or more controller devices 251 may be non-implantable and thus external to the body of the subject.

The system 110 further includes one or more communicator devices 252, configured and adapted to communicate the generated stimulation parameters to the at least one IPG 213.

The one or more communicator devices 252 may be implantable.

Alternatively, the one or more communicator devices 252 may be non-implantable and thus external to the body of the subject.

Advantageously, the one or more muscle activity sensors 250 and/or the one or more behavioral sensors 260, the one or more controller devices 251 and/or the one or more communicator devices 252 may be integrated in a single unit (not shown).

**Fig. 4** provides a schematic overview of a combined neuromodulation/neurostimulation system 310 for mitigating locomotor deficits of/or neuronal disorders, especially Parkinson's disease according to yet another embodiment of the invention.

The neuronal disorders can be neuronal disorders of the brain. For example, such a disorder can be Parkinson's disease. Further disorders that can be treated (but not limited to) epilepsy, chronic pain (like (cluster) headache or migraine), depression, Alzheimer's disease, obsessive compulsive disorders, obesity, and/or other locomotor deficits and/or neuronal disorders after spinal cord injury (SCI), stroke, and/or other neurological disorders.

The system 310 comprises at least one control unit, at least one stimulation unit 312 including one or more epidurally implantable electrodes 314, and at least one IPG 313 **(Fig. 4).** These components have the same structure and function of the corresponding components described above with respect to the system 10 of the first embodiment, and are therefore not further described for the sake of brevity.

In the present embodiment, the system 310 further includes a user input means, operatively connected to one or more control sensors 350 for collecting a user's command provided through the user input means **(Fig. 4).**

The user input means may include a microphone for providing a vocal command.

Additionally or alternatively, the user input means may include a button.

The one or more control sensors 350 may be non-invasive.

Alternatively, the one or more control sensors 350 may be invasive and thus implanted through surgery.

The system 310 further includes one or more controller devices 351, configured and adapted to generate one or more stimulation parameters based on the received user's command.

The one or more controller devices 351 may be implantable.

Alternatively, the one or more controller devices 351 may be non-implantable and thus external to the body of the subject.

The system 310 further includes one or more communicator devices 352, configured and adapted to communicate the generated stimulation parameters to the at least one IPG 313.

The one or more communicator devices 352 may be implantable.

Alternatively, the one or more communicator devices 352 may be non-implantable and thus external to the body of the subject.

Advantageously, the user input means, the one or more control sensors 350, the one or more controller devices 351 and/or the one or more communicator devices 352 may be integrated in a single unit.

The one or more controller devices 151, 251, 351 as well as the one or more communicator devices 152, 252, 352 according to the above-described embodiments may be similar in structure and only differ from one another in their respective specific settings.

One or more features of the above-described embodiments may as well be combined in a single embodiment.

**Exemplary setups and applications**

Example 1

The present example involves delivery of EES using tonic programming combined with a dopaminergic replacement therapy, e.g. as described above with reference to **Fig.** 1, optionally in further combination with DBS.

The example involves the use of muscle activity sensors such as the muscle activity sensors 250 described above with reference to **Fig. 3.**

a. Subjects are equipped with:

1. An IPG - Activa RC IPG (Medtronic).

2. Spinal electrodes - one 16-electrode Specify 5-6-5 lead (Medtronic) implanted over the dorsal side of the lumbosacral spinal cord.

3. DBS system - one Intellis DBS system (Medtronic)

b. The implanted current stimulator is programmed with a set of EES protocols. Each protocol is defined by the set of spinal electrodes that delivers the stimulation current, current amplitude, pulse waveform, and stimulation frequency. In particular, these protocols are defined as follows:

1. The user is equipped by a set of surface muscle activity sensors and then lies down on a bed in a supine position. The system then iteratively triggers EES pulses using different sets of EES parameters (stimulation amplitude, frequency, electrode configuration). The EMG responses are analyzed to construct the map between the EES parameters and the muscle responses. Using prior recordings on healthy people, the gait has been decomposed into periods of activity of muscle synergies. From the constructed map, sets of EES parameters, i.e. EES protocols, are identified that selectively evoke those muscle synergies.

2. The user is asked to walk overground while one of these EES protocols is continuously active. During this process, the quality of the gait is examined. This is done as the amplitude and/or the frequency of stimulation is increased or decreased. Following this tuning using individual protocols, the protocols are combined and re-tuned to maximize the gait and balance quality. At the end of the process, the set of selected EES protocols form the tonic EES program. This process is performed while the patient is using a standard PD therapy (e.g. while on a normal L-dopa regime and with DBS on) or with parts of his therapy diminished (e.g. with the DBS off). If the tuning is performed in several conditions (e.g. with DBS on and, separately, with DBS off), the process will result with multiple tonic EES programs, one for each set of therapies EES is combined with.

c. The tonic EES program is loaded into the firmware of the IPG. The EES is then activated and runs continuously in conjunction with the patient's standard PD therapy. The EES protocols may be modified during subsequent re-calibration.

Example 2

The present example provides a brain-spinal interface to alleviate motor deficits of PD.

In the present example, EES of the lumbosacral spinal cord is triggered by events detected from the neural activity recorded from the motor cortex, to alleviate parkinsonian movement deficits.

The present example may be implemented by using the neuromodulation/neurostimulation system 110 described above with reference to **Fig. 2.**

a. Subjects are equipped with:

1. One or two cortical neurosensors - one or two intracortically implanted microelectrode arrays wired to a skull-mounted pedestal that is equipped with a wireless transmitter.

2. A Controller - a wireless receiver connected to a data acquisition system that is wired to a computer equipped with a Bluetooth wireless transmitter.

3. A Communicator - a Patient Programmer that receives the stimulation commands via Bluetooth and relays them to the IPG via wireless telemetry.

4. An IPG - Activa RC IPG (Medtronic).

5. Spinal electrodes - two arrays of 8 electrodes implanted over the dorsal side of the lumbosacral spinal cord.

b. The Controller runs a custom software application that that acquires neural signals, processes them into action potential rates, feeds the rates into a regularized Gaussian Mixture Model decoding algorithm to obtain the current subject's motor intention (one of: Left Foot Off, Left Foot Strike, Right Foot Off, Right Foot Strike, neither of the other four) and, based on the decoded outcome, sends the stimulation commands via Bluetooth to the Communicator.

c. The implanted current stimulator is programmed with a set of spinal cord stimulation sequences. Each sequence is composed of one or more stimulation protocols. Each protocol is defined by the spinal electrode that delivers the stimulation current, current amplitude, stimulation duration, pulse waveform, and stimulation frequency. These protocols are designed as follows:

1. Spatiotemporal maps of motor neuron activity are used to identify hotspots of spinal motor neuron activity. First, behavioral recordings are used to mark the right foot strike and right foot off gait events. The events are used to calculate the average proportion of the stance and swing phases over all collected gait cycles. Muscle activity recordings are transferred into activity of motor neuron pools. The activity of motor neurons during each gait cycle is converted from time to gait phase coordinates. The spatiotemporal maps of motor neuron activity is then generated by averaging the activity of motor neurons over all the recorded gait cycles. Finally, Gaussian Mixture Model is applied to these maps to identify six hotspots associated with weight acceptance, propulsion and leg lift of left and right leg. In turn, each hotspot is defined by its corresponding spinal map of motor neuron activity defined at the center of each hotspot ("hotspot map"). A library of spatiotemporal maps of motor neuron activity and the corresponding hotspot maps that describe walking without motor deficits is generated before applying the therapy on individuals with motor deficits. A spatiotemporal maps of motor neuron activity of an individual that best corresponds to the subject (size, age, sex) is selected from the library for the following steps.

2. Activity of dominant muscles of the leg of the subject is recorded in response to single-pulse EES that is varied across a broad range of stimulation amplitudes, ranging from sub-threshold to saturation. This process is performed for each spinal array electrode. EES pulse waveform is designed to maximize the recruitment of proprioceptive fibers. The same pulse waveform is used across all the electrodes and, eventually for all the EES protocols. These responses are transferred into "response maps" between each spinal array electrode and EES amplitude as inputs; and the corresponding spatial maps of spinal motor neuron activity obtained by transferring the muscle responses into activity of motor neurons.

3. Each of these response maps is correlated with the spatial maps of hotspots. This process identifies six response maps, each having the maximum correlation with one of six hotspot maps. In turn, this six response maps provided six combinations of electrodes and amplitudes that reproduced spinal cord activation similar to the weight acceptance, propulsion and leg lift hotspots maps. Each of six obtained electrodes provide the electrodes that are used for each of six stimulation protocols. The obtained EES amplitudes are used to seed the amplitude of each stimulation protocol.

4. The duration of each protocol was set to the duration of each hotspot from the spatiotemporal map of motor neuron activity derived from (c.1).

5. The final EES amplitudes and EES frequencies are obtained during the decoder calibration phase (see below phase e.4) by observing the muscle responses as the subjects walked in the presence of triggered stimulation.

6. Sequences of protocols are built to reproduce the sections of the spatiotemporal maps that follow the Left Foot Off, Left Foot Strike, Right Foot Off and Right Foot Strike gait events.

d. The stimulation protocols and sequences are loaded into the firmware of the IPG. The protocols and sequences may be modified during the calibration of the decoding algorithm and afterwards to account for fine-tuning.

e. The decoding algorithm is calibrated though a procedure that includes:

1. Recording neural activity and behavior as the subjects walk in the absence of stimulation.

2. Behavioral recordings of the dataset in (e.1) are used to extract the Left Foot Off and Right Foot Off kinematic events. These events are synchronized with the neural signals.

3. Calibration of the decoding algorithms that detect only the Left Foot Off events and only the Right Foot Off events on data recorded in (e.1).

4. Recording neural activity and behavior while triggering stimulation that supports the right stance and left swing gait phase using only the decoded Left Foot Off events.

5. Recording neural activity and behavior while triggering stimulation that supports the left stance and right swing gait phase using only the decoded Right Foot Off events.

6. Behavioral recordings of the datasets in (e.1), (e.4) and (e.5) are used to extract the Left Foot Off, Left Foot Strike, Right Foot Off and Right Foot Strike kinematic events. These events are synchronized with the neural signals.

7. Calibration of the decoding algorithm that detect all four kinematic events on data recorded in (e.1), (e.4) and (e.5).

f. The decoding algorithm is loaded into the Controller software. The brain-spine interface is now calibrated and ready to be used to alleviate subjects' motor deficits.

g. As the subjects attempt to walk, their cortical activity represents their locomotor intentions. This activity is sampled by the cortical neurosensors and passed to the Controller. The controller software processes the activity into action potential rates and input these into its decoding algorithm. The decoding algorithm makes a decision on whether the current cortical activity corresponds to one of Left Foot Strike, Left Foot Off, Right Foot Strike or Right Foot Strike, or to neither of these relevant gait events. On detection of one of these four gait events, the Controller sends a command to the Communicator to initiate one of the four stimulation sequences. Each sequence is composed of some temporal combination of the six stimulation protocols designed to support one of weight acceptance, propulsion and leg lift of left or right leg. The Communicator relays the commands to the IPG. On receiving the command, the IPG stops its current sequence if one is currently running, and initiates the new sequence of current stimulation. The current is delivered through the spinal electrodes. The delivered stimulation promotes the intended movements and, therefore, alleviates the motor deficits.

Example 3

The present example involves delivery of EES using tonic programming combined with a dopaminergic replacement therapy, optionally in further combination with DBS.
The present example may be implemented by using the neuromodulation/neurostimulation system 310 described above with reference to **Fig. 3.**

a. Subjects are equipped with:

1. Behavioral sensors - a set of up to 16 IMU sensors, each equipped with a wireless transmitter (Delsys), placed across different joints or muscles of both feet.

2. A Controller - a wireless receiver connected to a computer equipped with a Bluetooth wireless transmitter.

3. A Communicator - a Patient Programmer that receives the stimulation commands via Bluetooth and relays them to the IPG via wireless telemetry.

4. An IPG - Activa RC IPG (Medtronic).

5. Spinal electrodes - one 16-electrode Specify 5-6-5 lead (Medtronic) implanted over the dorsal side of the lumbosacral spinal cord.

6. DBS system - one Intellis DBS system (Medtronic).

b. The Controller runs a custom software application that acquires the IMU signals, processes them into different kinematic variables, and feeds these variables into a regularized Gaussian Mixture Model decoding algorithm to obtain the current subject's motor intention (one of: Left Foot Off, Right Foot Off, neither of the other two) and, based on the decoded outcome, sends the stimulation commands via Bluetooth to the Communicator.

c. The IPG is programmed with a set of spinal cord stimulation sequences. Each sequence is composed of one or more stimulation protocols. Each protocol is defined by the spinal electrode that delivers the stimulation current, current amplitude, stimulation duration, pulse waveform, and stimulation frequency. These protocols are designed as follows:

1. The user is equipped by a set of surface muscle activity sensors and then lies down on a bed in a supine position. The system then iteratively triggers EES pulses using different sets of EES parameters (stimulation amplitude, frequency, electrode configuration). The EMG responses are analyzed to construct the map between the EES parameters and the muscle responses. Using prior recordings on healthy people, the gait was decomposed into periods of activity of muscle synergies. From the constructed map, sets of EES parameters, i.e. EES protocols, are identified that selectively evoke those muscle synergies.

2. The user is asked to walk overground while one of these EES protocols is triggered to match the natural activation of this synergy during the normal healthy gait. During this process, the quality of the gait is examined. This is done as we the amplitude and/or the frequency of stimulation is increased or decreased, and the duration of the activity. Following this tuning using individual protocols, the protocols are combined into one or more sequences triggered at specific salient gait events (e.g. Left Foot Off and Right Foot Off events) that cover the entire gait cycle. The parameters of EES protocol that form the sequences are re-tuned to maximize the gait and balance quality. At the end of the process, the set of selected EES sequences form the closed-loop EES program. This process is performed while the patient is using a standard PD therapy (e.g. while on a normal L-dopa regime and with DBS on) or with parts of his therapy diminished (e.g. with the DBS off). If the tuning is performed in several conditions (e.g. with DBS on and, separately, with DBS off), the process will result with multiple closed-loop EES programs, one for each set of therapies EES is combined with.

d. The EES protocols and sequences are loaded into the firmware of the IPG. These EES protocols and sequences may be modified during subsequent re-calibration.

e. The decoding algorithm is calibrated though a procedure that includes:

1. Recording neural activity and behavior as the subjects walk in the absence of stimulation.

2. Behavioral recordings of the dataset in (e.1) are used to extract the Left Foot Off and Right Foot Off kinematic events. These events are synchronized with the IMU signals.

3. Calibration of the decoding algorithms that detect the Left Foot Off and Right Foot Off events on data recorded in (e.1).

4. Recording neural activity and behavior while triggering stimulation that supports the right stance and left swing gait phase using only the decoded Left Foot Off events.

5. Recording neural activity and behavior while triggering stimulation that supports the left stance and right swing gait phase using only the decoded Right Foot Off events.

6. Behavioral recordings of the datasets in (e.1), (e.4) and (e.5) are used to extract the Left Foot Off and Right Foot Off kinematic events. These events are synchronized with the IMU signals.

7. Calibration of the decoding algorithm that detect the Left Foot Off and Right Foot Off kinematic events on data recorded in (e.1), (e.4) and (e.5).

f. The decoding algorithm is loaded into the controller software. The closed-loop EES therapy is now calibrated and ready to be used to alleviate subjects' motor deficits.

g. As the subjects walk, the IMU sensors record their current body kinematics. Due to correlation between different body parts during the whole body movements, the body kinematics is highly predictive of persons' locomotor intentions. The recordings from IMU sensors are wirelessly passed to the Controller. The Controller software processes the activity into kinematic variables and input these into its decoding algorithm. The decoding algorithm makes a decision on whether the current kinematics corresponds to one of Left Foot Off, Right Foot Off, or to neither of these two gait events. On detection of one of these two gait events, the Controller sends a command to the Communicator to initiate one of the two EES sequences. Each sequence is composed of some temporal combination of the EES protocols designed to generate or reinforce different muscle synergies. The Communicator relays the commands to the IPG. On receiving the command, the IPG stops its current sequence if one is currently running, and initiates the new sequence of current stimulation. The current is delivered through the spinal electrodes. The delivered stimulation promotes the intended movements and, therefore, alleviates the motor deficits.

## **Clinical trial on a human PD subject**

A clinical trial has been carried out by the inventors, involving a human participant with Parkinson's disease, hereinafter referred to as P1.

P1 was equipped with the Medtronic Intellis DBS system.

P1 finished the main phase of the present clinical trial.

The subject exhibited clear gait and balance motor deficits while using DBS and taking L-dopa (in a DBS on condition). These deficits became even more severe once DBS therapy was deactivated (about 30 minutes after turning off the DBS, DBS off condition).

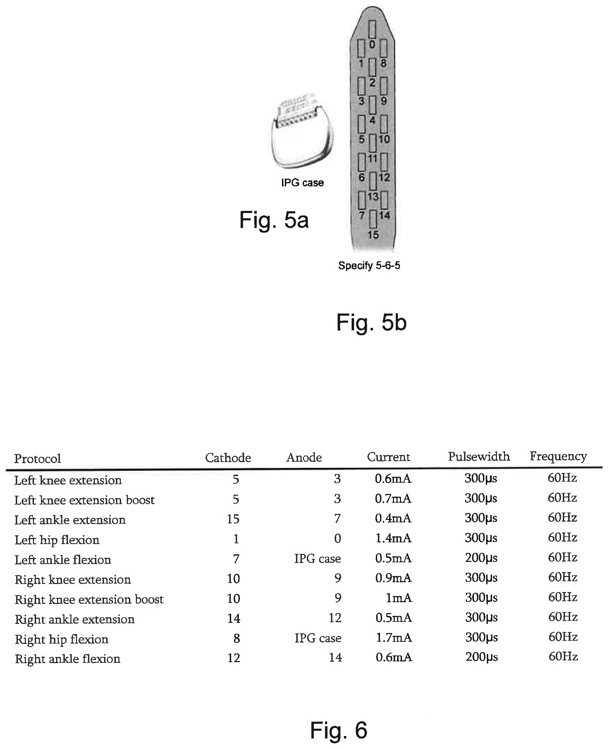

Following enrolment, the subject was implanted with a Specify 5-6-5 spinal lead (Medtronic Inc., schematically shown in **Fig. 5a)** into the dorsal epidural space above the spinal segments from L2 to S1. The lead was then connected by cables to the Activa RC implantable pulse generator (IPG), schematically shown in **Fig. 5b,** which was then implanted in the abdominal muscles of the subject. A custom-developed G-Drive+ software suite enabled wireless real-time communication with the IPG.

Following two weeks of the post-surgical recovery, several sessions were performed to tune the stimulation protocols used to selectively activate different muscle synergies. Each protocol was characterized by the set of cathodes and anodes used to deliver stimulation, stimulation current divided across cathodes and anodes, duration of the stimulation pulse (i.e. pulse width) and the stimulation frequency. Eight different stimulation protocols have been tuned, as shown in **Fig. 6.**

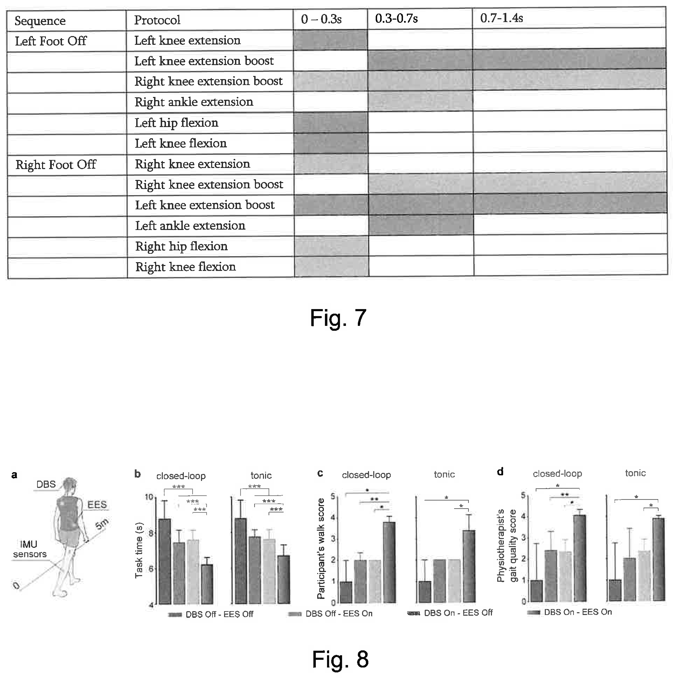

Then, two stimulation programs were defined. The "tonic" program continuously stimulated with both the left and the right knee extension protocols. The tonic program was turned on and then left running until the experimental trial has finished. The "closed loop" program used two stimulation sequences, "Left Foot Off" and "Right Foot Off', each triggered in real-time by the G-Drive+ software. Each of these sequences were 1.4s long. Once triggered, the sequence was executed until it expired (i.e., when the stimulation was turned off) or until it was replaced by another sequence. The gait cycle of the subject typically ranged between 0.8s and 1.2s, therefore resulting with Left Foot Off and Right Foot Off sequences interrupting each other after 0.4s to 0.6s (cf. **Fig. 7).**

During two sessions, the subject walked from one to the other side of the gait laboratory in a straight line. Two pieces of red tape marked the distance of five meters and the subject was instructed to walk past one, keep walking until they crossed the other, turn around and walk back across the same path, thereby repeatedly crossing the 5m corridor four to eight times. He would then stop, sit down to rest and give his assessment of the gait quality as a score from 0 to 5. Then, the physiotherapist that observed the gait of the subject also gave their score from 0 to 5. The period between passing the two markers on the floor was defined as a single trial, and each period of walking between two rest periods as a trial set. Across the two sessions, both the time of each trial and the scores were measured in one of the following six conditions:

1) "DBS off - EES off";

2) "DBS off - EES tonic";

3) "DBS off - EES closed-loop";

4) "DBS on - EES off";

5) "DBS on - EES tonic";

6) "DBS on - EES closed-loop".

Both the subject and the physiotherapist were blinded to the EES setting (off, tonic, closed-loop) during each trial set. Then, these measurements were grouped for tonic and closed-loop stimulation programs separately across the four conditions:

1) "DBS off - EES off";

2) "DBS off - EES on";

3) "DBS on - EES off";

4) "DBS on - EES on".

Based on the above, the inventors observed synergistic effects between the DBS and EES for both stimulation programs (cf. **Fig. 8).** For EES off, the DBS on trial time was substantially lower than the DBS off time. In fact, DBS on - EES off trial time was similar to the DBS off - EES on for both the closed-loop and tonic EES programs. For both programs, synergistical DBS on - EES on conditions resulted in fastest walking. Both the subject's and physiotherapist's score followed the same relationship: DBS off - EES off trial sets received the lowest scores, scores with DBS off - EES on and DBS on - EES off were substantially higher, but similar between them, and the DBS on - EES on had the highest scores.

The inventors then examined the outcomes related to the synergistic use of dopamine replacement pharmaceuticals, DBS and closed-loop EES. They first quantified the impact of this combined therapy on gait quality for P1 **(Fig. 27a-c).** This therapy immediately restored the natural spatiotemporal activation of leg motor neurons during walking, which translated into pronounced improvements in gait and balance, including longer stride, improved gait symmetry, better-coordinated arm swings, and faster walking **(Fig. 27d, Fig. 32d).** These improvements resulted in gait patterns comparable to those derived from P1's neurobiomechanical model **(Fig. 27e, Fig. 30b, Fig. 32a,b,d).** The inventors also implemented a classifier to assess whether gait patterns of P1 when using this combined therapy resembled those from the cohort of people with PD versus healthy people **(Fig. 25i, Fig. 29).** The majority of steps from P1 were classified as healthy **(Fig. 27f).**

Improvements were also observed when the DBS was turned off, albeit to a lesser extent **(Fig. 27, Extended Data Fig. 32a-d).** Neurological assessments using Unified PD Rating Scores (UPDRS) confirmed the complementarity between the EES and DBS to alleviate gait impairments in P1, when combined with Levedopa pharmacological dopamine replacement (MDS UPDRS III score: $DBS^{OFF}$ - $EES^{OFF}$: 65; $DBS^{ON}$ - $EES^{OFF}$: 29; $DBS^{ON}$ - $EES^{ON}$: 24, all measured while P1 was on a well-tuned Levedopa regime). Subjective assessments conducted by physical therapists using gait quality scores reinforced this conclusion **(Fig. 27e).** The detection of gait events, which was used to synchronize EES with P1's movement intentions, remained highly accuracy both with or without the DBS **(Fig. 27c, Extended Data Fig. 32e).**

Freezing-of-gait refers to the episodically hampered ability to move the legs. Freezing-of-gait is one of the most debilitating locomotor deficits of PD, for which current treatment strategies are poorly effective. Despite finely-tuned dopaminergic replacement and DBS therapies, P1 exhibited frequent freezing-of-gait episodes that occurred when turning and when passing through narrow paths. These episodes led to multiple daily falls that severely affected his quality of life.

When using closed-loop EES, P1 experienced a pronounced reduction in the frequency of freezing-of-gait episodes. To quantify this observation, we designed a circuit that involved frequent turns, crossing of obstacles, and passing through narrow spaces **(Fig. 28a).** P1 consistently experienced freezing-of-gait when navigating this environment. The combination of the Lavedopa and closed-loop EES nearly suppressed the occurrence of freezing-of-gait episodes in this task, both when using DBS and when DBS was turned off **(Fig. 28a).** The detection of gait events, which was used to synchronize EES with P1's movement intentions, remained highly accurate despite the navigation through the complex environment of this task **(Fig. 28a).**

Many studies suggested that gait neurorehabilitation reduces locomotor deficits in people with PD, and that the additional support of neuromodulation therapies, such as the EES of the spinal cord, may further enhance the impact of neurorehabilitation. The inventors tested this by having P1 follow a neurorehabilitation program supported by the combined EES, DBS and Levedopa therapy. This neurorehabilitation protocol involved a variety of exercises, including walking on basic and complex terrains, navigating outdoors in community settings, balance training, and basic physical therapy. Neurorehabilitation sessions were conducted 2 times per week for a duration of 3 months. Quantifications of locomotor deficits using well-established clinical scores revealed substantial improvement in balance **(Fig. 28b, Fig. 32f).** Frequent use of the neuroprosthesis during neurorehabilitation translated into a pronounced increase in quality of life **(Fig. 28b, Fig. 32g).**

P1 was eager to integrate the EES therapy into his daily life to support his mobility. To enable the transition to independent use in community settings, the inventors designed a user-friendly interface that allowed P1 to configure and operate EES therapy independently. During the neurorehabilitation phase, P1 used the EES therapy, together with the DBS and Levedopa therapies, for most of the day, only switching EES off when sitting for long periods of time or while sleeping. P1 continued using this combined therapy at home after the end of the neurorehabilitation phase. Over the year that followed, he reported utilizing EES for 8 hours per day **(Fig. 28c).** The EES enabled P1 to enjoy recreational walks in nature over several kilometers without any additional assistance.

## Decoding of events from motor cortex activity in human PD subjects

PD involves a widespread, slow-evolving alteration of neural networks that may not be entirely reproduced using pharmacological treatments delivered over restricted time windows in NHPs. Moreover, detection of motor intentions from neural activity in ambulatory settings remains a challenge in people. Therefore, we sought to verify the conceptual and technological feasibility of detecting events from motor cortex activity in people with PD to synchronize EES protocols with the ongoing movements.

Under the framework of a physician-sponsored clinical study, two participants (P2 and P3) with idiopathic PD and motor fluctuations received bilateral subdural electrode arrays over the primary motor cortex. The arrays were interfaced to the Summit RC+S implanted pulse generators, which enabled wireless streaming of electrocorticogram (ECoG) signals to an external computer.

The inventors implemented an event-detection strategy to detect events associated with the activation of hotspots from ECoG signals **(Fig. 26f, Fig. 31a).** In both participants P2 and P3, the algorithm detected the events linked to leg lift with high accuracy **(Fig. 26f, Fig. 31b).** These results demonstrate the conceptual and technological feasibility of decoding events from primary motor cortex activity to synchronize EES protocols to the ongoing movements in people with PD.

## Experimental data in non-human primate model

An experimental study has been conducted by the inventors on a non-human primate model of monkeys having 1-methyl-4-phenyl-1,2,3,6-tetrahydropyridine (MPTP)-induced symptoms of PD **(Fig. 9).**

As known from other studies from the Courtine Lab at EPFL, the stimulation parameters used for non-human primate model of monkeys can be also similarly used in other mammals, especially in humans.

BSI for PD was optimised and shows this neurotechnology alleviates gait and balance deficits in the 1-methyl-4-phenyl-1,2,3,6-tetrahydropyridine (MPTP) nonhuman primate model of PD **(4).** Macaque monkeys were implanted with intracortical microelectrode arrays into the left and right leg primary motor cortex (MI) to record neural activity, and bipolar electrodes into leg muscles to monitor electromyographic (EMG) signals. The severe loss of midbrain dopaminergic circuits induced pronounced gait and balance impairments that replicated the axial deficits and bradykinesia observed in humans with late-stage PD. However, these impairments did not disrupt the encoding of basic and skilled locomotion in MI neuronal ensembles. Preservation of cortical dynamics allowed highly accurate decoding of locomotor intents from MI activity. Projection of muscle activity onto the location of motor neuron pools in the spinal cord showed that walking involves the sequential activation of spatially-restricted regions, and that this activation is altered when PD symptoms appear. To restore the natural dynamics of motor neurons, spinal implants that targeted the posterior roots projecting to the spinal cord regions containing these motor neurons were designed. A multi-class asynchronous decoder that linked MI modulation to electrical spinal cord stimulation protocols reproducing the spatiotemporal sequence of motor neuron activity during walking was then developed. This BSI instantly reduced freezing of gait and improved the walking speed, gait quality, posture and locomotor dexterity. Complementing the BSI with DBS in the subthalamic nucleus (STN) improved both locomotor performance and alertness. The therapeutic efficacy of this combined strategy will now be tested in people with PD using clinically validated neurotechnologies **(5-7).**

It was first asked whether MPTP-treated macaque monkeys **(4,8)** exhibit the gait and balance deficits commonly observed in people with PD. These deficits encompass freezing of gait, reduced stride length, slow movements and an excessively flexed posture associated with balance instability **(9,10).** Nine macaque monkeys were trained to traverse corridors with flat surfaces or horizontal ladders requiring precise paw placement **Figs. 9** and **10a.** High-resolution kinematic recordings were collected before and after they had reached stable symptoms of PD following repeated MPTP administration. The MPTP intoxication protocols was calibrated to model a late-stage of Parkinsonism, which was assessed by two blind observers using a validated scoring system **(11) (Figure 10b,** see also Supplementary Table 1 below). This neurological stage coincided with the severe depletion of striatal dopaminergic terminals and nigral neurons **(Figure 10b).**

To identify gait and balance deficits, a principal component (PC) analysis **(12,13)** was applied on a comprehensive set of variables calculated from kinematic recordings (n = 83 variables, see also Supplementary Information Table 2 below). PC1 segregated gait patterns recorded before versus after MPTP administration **(Figure 10c).** Extraction of variables correlating with PC1 ($| r | > 0.5$) revealed that the loss of midbrain dopaminergic circuits led to a significant decrease in stride length, slow movements and more flexed posture **(Figs. 10b** and **14;** $p < 0.05$). A significant increase in trunk movement variability and larger pelvis oscillations was detected, which both reflected balance deficits ($p < 0.01$; **Fig. 14**). These results show that MPTP-treated macaque monkeys classified with late-stage Parkinsonism exhibit the common axial symptoms and bradykinesia observed in humans with PD **(10)**.

PD alters the interactions between cortical and subcortical regions, especially between MI and basal ganglia circuits that generate abnormally synchronized beta (6 to 30 Hz) frequency oscillations **(14-21)**. However, it remains controversial whether these excessive beta oscillations translate into changes in the activity of MI neurons **(22,23)** or neuronal ensembles **(24,25),** notably during locomotion. Elucidating locomotor-related MI activity in PD was critical, since the aim was to establish a direct connection between MI and spinal circuits to bypass basal ganglia circuitopathies **(14)**.

To address this question, longitudinal recordings of MI activity underlying basic and skilled locomotion was conducted before and after MPTP administration. Five macaque monkeys (M5-9) were implanted with microelectrode arrays into the leg area of the left MI to record local field potentials (LFPs) and spiking activity from neuronal ensembles, and bipolar electrodes into leg muscles to record EMG signals. Wireless modules enabled transmission of neural (20 kHz) and EMG (2 kHz) signals to external receivers that were synchronized with video recordings (100 Hz) to reconstruct whole-body kinematics **(26)**.

Before MPTP administration, the activity of MI neuronal ensemble (n = 78 cells) during locomotion exhibited remarkably reproducible patterns **(Figs. 10e-f** and **15d)**. Compared to sitting, the majority (74/78, 95%) of recorded neurons showed a pronounced increase in the averaged firing rate during locomotion (10 vs. 21 spikes/s, $p < 0.001$, **Figs. 10e** and **15b-c)**. Compared to locomotion along a flat surface, precise foot placement along the rungs of the ladder triggered an extensive reorganization in the timing of firing rates **(Fig. 10f)** but did not involve changes in the mean firing rates of the recorded neurons **(Figure 10e** and **15b-c)**.

In comparison, the neurons recorded after MPTP administration (n = 82 cells) exhibited a small increase in average firing rate (sitting: 10 vs. 14 spikes/s, $p < 0.05$; corridor: 21 vs. 25 spikes/s, $p < 0.05$; ladder: 24 vs. 25 spikes/s, **Figs. 1e** and **15b-c)**. Various studies reported MI hyperactivity in PD **(27-29)**, particularly in patients who exhibit gait deficits, but other works described hypoactivity **(30,31)**. The origins and implications of these variable and often marginal changes in MI activity remain unclear **(21)**.

It was then asked whether the development of Parkinsonism led to changes in the encoding of gait phases in MI neurons. For this purpose, the activity of multi-units over the course of MPTP administration was analyzed. In all 5 monkeys, the relationships between the activity of multi-units and the timing of gait phases underwent small changes over time, but these changes were unrelated to the development and severity of Parkinsonian symptoms **(Figs. 15g-k)**.

Analysis of local field potentials (LFPs) led to similar conclusions. The 5 monkeys displayed the expected desynchronization in the β-band of LFPs (6-30 Hz) when transitioning from sitting to walking. Contrary to previous observations in rodent **(32)** and marmoset **(15)** models of PD, however, the loss of midbrain dopaminergic circuits did not correlate with an increase in the spectral power of β-band MI LFPs during locomotion **(Figs. 15l-o)**.

Finally, canonical correlation analysis **(33)** was applied on the spiking activity of MI neuronal ensemble to study population dynamics. Indeed, the temporal reorganization of firing rates between basic and skilled locomotion **(Fig. 10f)** suggested that methods for analysis of population dynamics may reveal alterations in the encoding of leg movements in PD. Neural trajectories traced by MI population dynamics followed highly reproducible cyclical paths from step to step, both during locomotion along flat surfaces and horizontal ladders **(Figs. 10f** and **15d)**. The geometry of neural trajectories depended on the task, as expected based on the temporal reorganization of neuronal responses when traversing flat surfaces versus ladders. Strikingly, the loss of midbrain dopaminergic circuits after MPTP administration did not alter neural trajectories. MI population dynamics evolved at a slower velocity that coincided with the reduced walking speed **(Fig. 15d)**. However, the geometry of task-encoding neural trajectories remained preserved, despite pronounced gait deficits and altered muscle activity dynamics **(Figs. 15e-f)**.

These combined analyses conducted in 5 monkeys show that gait and balance deficits resulting from the severe loss of midbrain dopaminergic circuits are unlikely caused by changes in the encoding of locomotor movements in MI. In turn, decoding of motor states from MI could efficiently drive a prosthetic system to alleviate these deficits.

The aim was to link cortical activity to electrical spinal cord stimulation protocols that promote the natural motor neuron dynamics during walking **(Fig. 11a)**. To inform the design of this BSI, the changes in leg muscle activity that caused gait and balance deficits following MPTP administration (M6, M7, M8 and M9) was studied. The recorded EMG signals were projected onto the anatomical location of motor neuron pools within the lumbosacral spinal cord **(34) (Fig. 11b)**. Thus, spatiotemporal maps of motor neuron activity underlying walking was constructed that revealed the sequential activation of six well-defined hotspots located in specific regions of the left and right hemicords. The migration of these hotspots within the spinal cord reflects body mechanics **(35),** successively ensuring weight acceptance, propulsion and leg lift **(Figs. 11b** and **16a)**.

Comparisons of maps before and after MPTP administration showed that gait and balance deficits resulted from diverse alterations in the timing, duration and amplitude of leg muscles associated with each hotspot **(Figs. 11b** and **16b)**. It was thus reasoned that the six hotspots had to be targeted to alleviate gait and balance deficits.

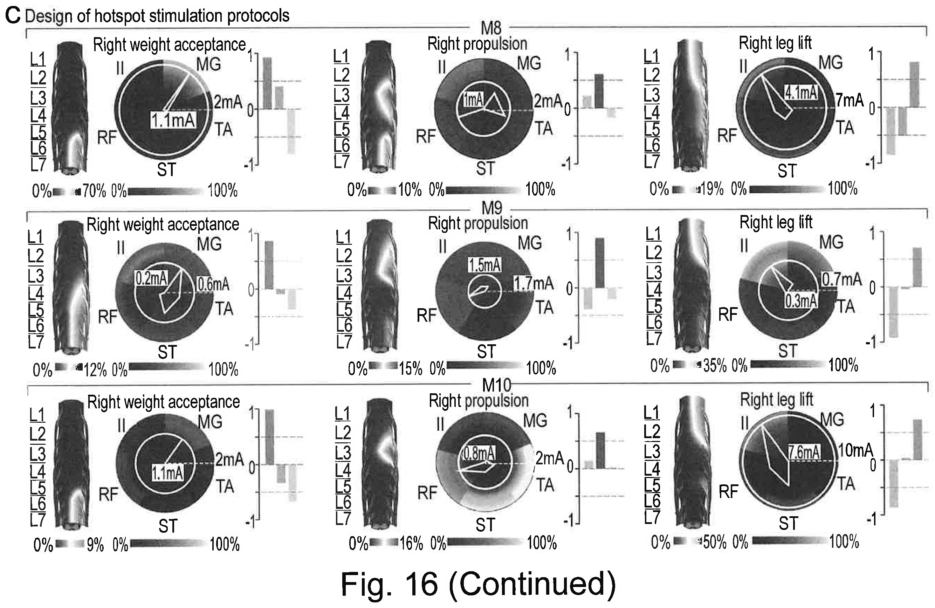

Motor neuron activation hotspots can be modulated with the application of epidural electrical stimulation (EES) targeting the posterior roots projecting to the spinal cord regions containing these hotspots **(36).** Indeed, EES activates muscles through the recruitment of proprioceptive feedback circuits **(37).** Spinal implants were thus designed with electrode configurations targeting the posterior roots projecting to the spinal cord regions embedding the motor neuron pools associated with each hotspot **(Figs. 11c** and **16c).** The implants were designed using AirRay **(38)** and electronic dura mater **(39)** technologies that had been scaled up and adapted to the primate spinal cord **(Fig. 17).**

It was previously showed that the delivery of EES with a spatiotemporal sequence that coincides with the intended movement can reproduce the natural dynamics of motor neuron activation after SCI. This spatiotemporal stimulation immediately reestablished walking after paralysis in rats **(40),** monkeys **(3)** and humans **(5).** It was hypothesized that the same strategy could be used to alleviate gait and balance deficits in PD. For this purpose, EES protocols must be synchronized with locomotor-related neural states.

A decoder that linked the preserved dynamics of MI to spatiotemporal stimulation protocols that targeted the hotspots underlying locomotion of healthy monkeys was developed. While walking involves the successive activation of weight acceptance, propulsion and leg lift hotspots within each hemicord, the maintenance of the symmetry between the locomotor movements of the left and right legs require the synchronized activation of the propulsion hotspot on one side and of the leg lift hotspot on the contralateral side. Consequently, it was focused on the decoding of the initiation of weight acceptance and leg lift hotspots and the propulsion hotspot was linked to the leg lift hotspot of the contralateral side.

To detect hotspot initiation events, regularized linear discriminant analysis decoders was calibrated that detected the initiation of weight acceptance and leg lift hotspots using neural activity from the contralateral MI **(Figs. 11d** and **18a).** This approach was validated in the two monkeys that were implanted with intracortical arrays in the left MI. In the healthy state, the decoders detected the initiation of both weight acceptance and leg lift hotspots with near 100% accuracy over periods ranging from one to four minutes of uninterrupted locomotion. Despite the emergence of substantial gait and balance deficits, the accuracy of the decoded hotspots remained unchanged throughout the development of PD symptoms **(Fig.11d).**

These developments were then leveraged to implement a wireless BSI optimized for PD: a system wherein hotspot initiation events detected from both left and right MI triggered spatiotemporal EES protocols promoting weight acceptance, propulsion and leg lift of the left and right legs. The spinal implants **(39)** were interfaced with an implantable pulse generator commonly used to deliver DBS therapies in humans. This pulse generator was upgraded with wireless communication modules **(3)** that enabled real-time control over bursts of EES with latencies of about 105ms. To control the delivery of EES protocols, intracortical arrays were implanted in the leg region of the left and right MI and extended the decoding algorithm to detect weight acceptance and leg lift motor states from the left and right legs **(Fig. 18a).**

Four macaque monkeys (M8, M9, M10 and M11) were implanted with this wireless BSI. Prior to MPTP administration, the ability of the BSI were verified to target the six hotspots during walking (M8, M9, M10). Recordings of muscle responses when delivering single-pulses of EES revealed that the electrodes were able to recruit the targeted posterior roots associated with the six hotspots **(Figs. 11c** and **16c).** Then sequences of EES bursts were configured that activated these hotspots to reproduce the natural dynamics of motor neuron activation **(3,5,36,40).** For this purpose, the decoder was calibrated to detect the onset of weight acceptance and leg lift hotspots, while taking the wireless communication latency into account.

The delivery of EES led to responses in MI. To mitigate their impact on decoding performance, the decoders were calibrated using less than two minutes of neural activity without and with EES **(Fig. 18b).** This strategy maintained a high decoding accuracy during walking with the BSI **(Figs. 19a-b).**

Without prior exposure to the BSI, brain-controlled triggering of EES bursts targeting weight acceptance, propulsion and leg lift hotspots led to the modulation of gait parameters related to the function of these hotspots **(Figs. 19c-d).**

It was then tested whether the BSI could alleviate the pronounced gait and balance deficits that resulted from the severe depletion of midbrain dopaminergic circuits in M8, M9 and M11 **(Fig. 10b).** The BSI mediated an immediate improvement of locomotor performance **(Figs. 12** and **20).** With the BSI, all three monkeys displayed a significant increase in walking speed (p < 0.001), allowing them to execute the locomotor tasks within a similar duration as in the healthy state **(Figs. 12b** and **20a-c).** As intended, the BSI restored the natural dynamics of motor neuron activity, which was captured in the high spatial correlation between spatiotemporal maps of motor neuron activity measured before MPTP treatment and after the treatment during walking with the BSI **(Fig. 12d).** Kinematic analyses revealed that the improved task execution was associated with significant improvements of gait quality (p < 0.001, **Figs. 12e-f** and **20b).** The BSI also improved posture. To capture postural corrections, computerized tomography scans were transformed into a tridimensional biomechanical model of macaque monkeys onto which whole-body kinematics were morphed. This morphing procedure revealed a decrease in spine curvature with the BSI **(Fig. 12c),** suggesting an increase in postural tone.

It was then tested whether the BSI could improve skilled locomotion. The two tested monkeys (M8, M11; M9 lost MI implants) displayed considerable deficits when traversing the ladder, including very slow progression and even falls resulting from unbalance and foot misplacements onto the rungs. The BSI immediately restored a natural progression along the ladder, reducing falls to nearly zero **(Figure 12g** and **20a-c).**

All 10 MPTP-treated monkeys showed the motor behaviors associated with freezing of gait **(43),** but only M8 displayed complete stops during locomotion **(Fig. 21a).** During these episodes, MI population dynamics remained confined within a specific state that differed significantly from rest and walking (p < 0.001, **Fig. 21c).** Since the decoder was calibrated to detect locomotor events, this state was interpreted as rest **(Fig. 21d).** Yet, the BSI significantly reduced the occurrence of freezing of gait episodes (p < 0.05, **Fig. 21b).**

These results were reproduced over successive days of recordings, during which the same decoder and stimulation protocols could be used without recalibration **(Figs. 12a, 12g,** and **20b).**

The location, timing and frequency of stimulation were critical to obtain optimal therapeutic outcomes. Advancing or delaying the onset of EES bursts by 200 ms reduced locomotor performance, reaching values that did not differ from baseline **(Figs. 22a-b).** Similarly, inaccurate triggering of stimulation that resulted in semi-random electrical burst sequences failed to improve locomotion **(Figs. 22c-d).**

Previous work in animal models **(15,32)** and patients with PD **(44-46)** reported improvement of locomotion when applying continuous stimulation that targets the dorsal columns within the thoracic spinal cord in order to modulate basal ganglia circuits. This strategy was thus compared to the BSI that instead targets the lumbosacral posterior roots to modulate spinal motor circuits. The most rostral electrodes were used to configure an electrical field that targeted low thoracic dorsal column fibers. Stimulation was delivered with frequencies of 250 and 500 Hz using sub-threshold amplitudes to avoid muscle contractions, as recommended in previous studies **(32).** This strategy mediated minimal (M9) or modest (M8) improvement of locomotion, but to an extent that was significantly inferior compared to the BSI (p < 0.05; **Fig. 23).**

DBS is the primary surgical intervention proposed to patients to alleviate the multifaceted symptoms of PD, but benefits on locomotion vary from modest or non-existent **(14,47).** The BSI would thus complement DBS in case of persistent gait and balance deficits. Consequently, it was asked whether the BSI is superior to DBS in alleviating locomotor impairments, and whether both interventions can operate concurrently.

In addition to the BSI, DBS electrodes were inserted in the left and right STN, as verified with magnetic resonance imaging (M9, **Fig. 13a).** DBS delivered at the known therapeutic frequency of 125 Hz (but not 20 Hz, **Fig. 24)** improved alertness, which translated into a markedly higher number of completed trials **(Fig. 13c).** However, the legs remained exaggeratedly flexed during the stance phase, thereby lacking the extension required for propulsion **(Fig. 24d).** Turning on the BSI together with DBS substantially increased the walking speed **(Fig. 13c),** improved locomotor performance **(Fig. 13c)** and led to marked recovery of propulsion components **(Fig. 14d).**

## Experimental methods

### Animal husbandry

Experiments were approved by the Institutional Animal Care and Use Committee of Bordeaux (CE50, France) under the license number 50120102-A and performed in accordance with the European Union directive of 22 September 2010 (2010/63/EU) on the protection of animals used for scientific purposes in an AAALAC-accredited facility (Chinese Academy of Science, Beijing, China). Eleven macaque monkeys M1-11 (10 *macaca mulatta, 1 macaca fascicularis,* China, **Fig. 9)** aged between 4 and 6 years old, and weighing between 4.1 kg and 7.1 kg (5.88 ± 0.72 kg) were housed individually in cages designed according to European guidelines (2 m × 1.6 m × 1.26 m). Environmental enrichment included toys and soothing music.

### Behavioural training of NHPs

The animals were trained to walk on a motorized treadmill, across a straight corridor, and across a horizontal ladder course that consisted of a platform at the beginning and at the end of the course and seven equally spaced rungs in-between. Plexiglas enclosures (treadmill: 110 cm × 40 cm × 70 cm; corridor/ladder: 300 cm × 35 cm × 70 cm) were used to keep the animals within the field of view of the cameras. Food pellets and fruits rewarded appropriate behaviour. Additional food to complete daily dietary requirements was provided after training.

### Preparation and assessment of the NHP model of PD

1-methyl-4-phenyl-1,2,3,6-tetrahyd-ropyridine (MPTP) was administered in M1-11 according to a previously published protocol **(48-54).** Animals were treated daily (7:00 A.M.) with MPTP hydrochloride (0.2 mg/kg, i.v.; Sigma, St. Louis, MO) dissolved in saline according to a predefined protocol **(8).** This protocol describes a reproducible MPTP cumulative dosing regimen that leads to the first appearance of Parkinsonian clinical signs after 15 ± 1 injections (i.e., a cumulative dose of 3.0 ± 0.2 mg/kg). Neurological deficits were evaluated every morning at 9 am in home cages for 30 min. Two blinded observers who were not involved in the study quantified these deficits using a validated Parkinsonian Disability (PD) score **(8)** assessing tremor, general level of activity, body posture, vocalization, freezing and frequency/rigidity of arm movements. The injections were stopped when the deficits corresponded to late-stage Parkinsonism (defined by PD score of 5 or higher). Injections were repeated if the monkeys displayed recovery of gait and balance. The kinetics of nigrostriatal degeneration in this model and the critical thresholds associated with the symptom appearance have been thoroughly investigated using a number of in vivo and post-mortem endpoints **(8, 55).**

NHP surgical procedures

All the surgical procedures have been described in detail previously **(10,11).** Surgical interventions were all performed under full anaesthesia induced by atropine (0.04 mg kg$^{-1}$) and ketamine (10 mg kg$^{-1}$, intramuscular injection) and maintained under 1%-3% isofluorane after intubation. A certified functional neurosurgeon supervised all the surgical procedures. Surgical implantations were performed during one or two surgeries. M5-7 were implanted with a 96-channel microelectrode array (Blackrock Microsystems, 1.5 mm pitch) into the leg area of the left MI, and a wireless system **(3,56)** to record electromyographic signals (T33F-4, Konigsberg Instruments, USA) from the following leg muscles: gluteus medius (GLU), iliopsoas (IPS), rectus femoris (RF), semitendinosus (ST), gastrocnemius medialis (GM), tibialis anterior (TA), extensor digitorum longus (EDL), and flexor hallucis longus (FHL). M8-11 were implanted two 48-channel microelectrode arrays in the leg area of left and right MI, and a custom system that wired the electromyographic signals from right IPS, RF, ST, GM and TA; and left GM and TA muscles to a skull-mounted titanium pedestal. In a second surgery, two spinal implants were inserted into the epidural space under the L2-L3 and L5-L6 vertebrae according to previously described methods **(3,11).** The rostrocaudal and mediolateral position of the electrodes was finely adjusted using intraoperative electrophysiological monitoring **(11).** The implants were anchored to the spinous process of L3 and L6 vertebra with a suture. The wires of each spinal implant were routed subcutaneously to an implantable pulse generator (Activa RC, Medtronic Inc., USA) inserted between the intercostal muscles (see laso Supplementary Information section below). All the implantable devices were acquired from Medtronic, and research software reused from prior work. The proper location of the intracortical arrays and spinal implants with respect to gross anatomical landmarks was verified post-mortem in all the monkeys.

M9 was additionally implanted with two DBS mini-leads (4 contacts per lead, 0.5mm contact length, 0.5mm inter-contact distance, diameter 0.625mm, NuMED; USA) in the right and left subthalamic nucleus. Stereotactic coordinates of each subthalamic nuclei were calculated based on a ventriculography, as detailed previously **(57).** First, a hollow cannula (inner diameter 0.625mm) was attached to a stereotactic frame in order to insert the probe into the brain until the tip was located at the computed coordinates. Then, the leads were descended into the brain through the cannula. After the descent, the proper location of each implant was verified using X-ray. Then, the cannula was removed and the lead was fixed to the skull using surgical cement. The location of each lead after fixation was again confirmed using X-ray, and then d the leads were connected with an implanted pulse generator (Activa RC, Medtronic, USA) using quadripolar extension cables (Medtronic, USA). This connection was validated by recording stimulation artifacts following stimulation through each DBS contact. The placement of DBS leads was confirmed post-mortem using an MRI of the explanted brain.

The veterinary team continuously monitored the animals during the first hours after surgery, and several times daily during the seven following days. A few hours after surgery, the animals were able to move around and feed themselves unaided. Clinical rating and monitoring scales were used to assess post-operative pain. Ketophen (2 mg kg$^{-1}$; subcutaneous) and Metacam (0.2 mg kg$^{-1}$; subcutaneous) were administered once daily. Lidocaine cream was applied to surgical wounds twice per day. The ceftriaxone sodium antibiotic (100 mg kg$^{-1}$; intramuscular) was given immediately following surgery, and then once daily for 7 days.

Design and fabrication of NHP epidural spinal implants

According to an earlier modelling work **(5,36,58,59),** in order to recruit the proprioceptive fibres that access motor neuron pools located in the L2-L7 spinal segments, EES needs to target the dorsal roots projecting to these segments. For this purpose, spinal implants were tailored to the anatomy and physiology of the vertebral and spinal columns, taking into account the electrochemical requirements related to the size of the electrodes and interconnect tracts that are connected to the implantable pulse generator. To satisfy these limitations, two implants were designed: a rostral implant targeting the L2-L4 roots, and a caudal implant targeting the L5-L7 roots. The spinal implants inserted in M8, M9 and M10 were produced by CorTec (Germany) using AirRay Electrode Technology. Also, spinal implants were fabricated using e-dura technology **(39)** (see also Supplementary Information section below providing details on the fabrication process), which was implanted in M11.

Data acquisition in NHPs

Procedures to record kinematics and muscle activity have been detailed previously **(3,56,60).** Whole-body kinematics was capured using the high-speed motion capture SIMI system (Simi Reality Motion Systems, Germany), combining 4-6 video cameras (100 Hz). Reflective white paint was applied on the shaved skin of the animals overlying the following body landmarks: iliac crest (crest), greater trochanter (hip), lateral condyle (knee), lateral malleolus (ankle) and the fifth metatarsophalangeal (foot). The Simi Motion software (Simi Reality Motion Systems, Germany) was used to reconstruct the 3D spatial coordinates of the markers. Joint angles were computed accordingly. In M5-7, we used a custom-built system to receive the wirelessly emitted signals from the implanted T33F-4 devices (Konigsberg Instruments, USA). In M8-10, we used a W16 system (Triangle Biosystems, USA) to acquire the wirelessly transmitted signals. In M11, the implanted electromyographic system failed soon after the surgery. Consequently, all subsequent analyses were performed using kinematic recordings. Neural signals were wirelessly recorded using the Cereplex-W system **(3,26)** (Blackrock Microsystems, USA In M9, the skull-mounted pedestal used to connect the Cerelplex-W system failed before the efficacy of the brain-controlled spinal cord stimulation on the ladder could be tested.

Both electromyographic and neural wireless recording systems were connected to a Blackrock Neural Signal Processor (NSP, Blackrock Microsystems, USA) that synchronized electromyographic and neural signals sampled at 2kHz and 22kHz, respectively. The NSP band-pass filtered (500Hz-7.5kHz) the neural signals and extracted multiunit spikes as times when the signal crossed a threshold set to 3.5 times the root-mean-square of the signal **(3).** This procedure resulted in spike times from each of the 96 electrodes. An additional NSP channel was used to record an analogue trigger of the SIMI system used to synchronize the SIMI video recordings with the physiological signals. The NSP continuously broadcasted Used Datagram Protocol (UDP) packets containing neural, multiunit spiking, electromyographic and trigger channel recordings over the local network. A dedicated storage computer received the UDP packets and saved the recordings on a hard drive.

### Analysis of NHP gait and balance deficits resulting from MPTP treatment

In monkeys M1-9, gait kinematics were recorded before and after MPTP administration. Two metrics were used as aggregate measures of performance: task time and crossing time. Similar to the instrumented Timed-up-and-go (iTUG) assessment used to evaluate motor capacity of human patients with Parkinson's disease **(61,62),** task time is the period from the moment the animal stands up on one side of a corridor or a ladder to the moment it places the food reward in its mouth on the other side of the corridor. Since task time may vary due to differences in the time each animal spent reaching for the food, the crossing time as the period necessary for the animals to cross the central portion of the corridor or ladder was also calculated. To quantify locomotor performance, a PC analysis was applied on 83 gait parameters (see Supplementary Table 2 below) calculated based on previously described methods **(3,36,60).** Two leading PC of the space spanned by these parameters were used to visualize the differences between the gait cycles recorded before and after MPTP administration, or across the experimental conditions. Next, the factor loading was extracted on each PC to identify the parameters that more robustly explained the differences between the conditions.

### Analysis of MI activity before and after MPTP administration

Single units were sorted in M6 and M7. The depth of modulation and mean firing rates during sitting and walking over a corridor or a ladder was then calculated for each identified neuron. Spikes were extracted from the band-pass filtered (0.5-7.5kHz) neural signal by manual sorting based on PC analysis using a custom Matlab program. Only well-isolated neurons were considered. To quantify the single neuron firing during the transition from sitting to movement, the neural firing rates were estimated every 10ms and each corridor trial was separated into periods of sitting and periods of locomotion. Then, calculated the mean firing rate during these periods and the modulation depth were calculated. The modulation depth was defined non-parametrically as the difference between the upper and lower 95th percentile of all firing rates observed within each period. Then, the activity of each neuron was mapped during locomotion to the respective gait phase. The gait phase was defined as a percentage (0 - 100%), with 0% and 100% occurring at the time of foot strike from the right hindlimb. The average activity over all the gait cycles was calculated using PC analysis to project the activity of neuronal population into a space spanned by eight leading PC **(63).** To visualize and quantify putative changes in population dynamics before and after MPTP administration, canonical correlation analyses were applied on all the identified neurons. This method finds a linear transformation between two low-dimensional subspaces of neural activity that maximize the correlation between neural dynamics **(33).** To ensure that correlation values could not be explained trivially by the cyclical motor output, this procedure was repeated using the envelopes of the electromyography signals from the eight recorded leg muscles. To estimate the lower bound of the correlation values, neural trajectories recorded before MPTP administration and applied the same analysis were shuffled.

### Analysis of multiunit spikes

The time course of putative changes in multiunit spiking activity before and after MPTP administration was evaluated in M6, M7, M8, M9 and M10. For each session, the multiunit signal of each electrode to the gait phase was mapped, fitted with a cosine function and the preferred gait phase (PGP) as the time at which the activity peaked was identified. For each pair of sessions, the absolute difference between the PGP of a given electrode was computed, and averaged across all electrodes ($\Delta$PGP). $\Delta$PGP may increase due to the progression of the disease, but also due to the passage of time between sessions as a result of widely observed neural signal instabilities (64-76). To evaluate the impact of these two effects, $\Delta$PGP was regressed to temporal (number of days) and symptomatic (PD score) differences between each pair of sessions. To further test the direct effect of MPTP administration, $\Delta$PGP was regressed to the temporal difference between the sessions alone. Then, the effects of this fit were subtracted and the $\Delta$PGP residuals were regressed to the Parkinson's disease score difference between the sessions.

Analysis of local field potentials (LFPs)

Changes in the $\beta$-band of LFPs were analysed before and after MPTP administration. Broadband activity recorded from each electrode was band-pass filtered between 2 and 300 Hz. Then, Welch's method was used to compute the power spectral density on each electrode during locomotion (77). To facilitate comparison across sessions, the power was normalized on each day by a pink noise spectrum estimated from a 3-100Hz frequency range excluding 6-30Hz band (spectral band of interest) and the 49-51Hz band (line noise) (15). The normalized power in the 6-30Hz spectral band WAS compared between locomotion recorded before and after MPTP administration, and the power in this band was regressed to the Parkinson's disease score measured before each recording session.

Analysis of gait impairments in PD human subjects

To compare gait deficits of people with PD to the gait deficits recorded from our MPTP non-human primate model, gait recordings in people with PD and healthy controls were organized as part of the PREDI-STIM clinical study (NCT 02360683). The PREDI-STIM study was approved by the Nord Ouest-IV Ethical Committee (2013- A00193-42) and was conducted in accordance with the Declaration of Helsinki. The inclusion criteria involved patients with Parkinson's disease receiving a pre-therapeutic assessment and monitoring for one, three and five years as part of the regular monitoring of the subthalamic nucleus deep brain stimulation. The study involved one or more sessions during which we recorded full-body kinematics of the study participants.

Under the framework of the PREDI-STIM study, gait of N = 25 participants diagnosed with Parkinson's disease and N = 9 age-matched healthy controls was recorded and analysed. Gait of participants with PD exhibited debilitating gait deficits with various degrees of severity. Whole-body kinematics was recorded using a Vicon motion-capture system (Vicon, Oxford, UK). The PREDI-STIM participants were equipped with 34 reflective markers (26 on the legs, arms and trunk, and 8 on the head and wrists) on the surface of the skin to cover all key body joints (foot, ankle, knee, hip, shoulder, elbow, hand, neck and head, as well as spinal vertebrae T10 and C7). Nexus biomechanical software (Vicon, Oxford, UK) was employed to triangulate the centroid of each joint in each time point. Putting the joint centroids together provided a full-body kinematics. MATLAB environment software (The MathWorks, Inc., USA) was used to derive the timing of gait events (heel strike / foot off) and calculate the walking parameters from full-body kinematics. This software was used to discretize each gait-cycle into N = 65 kinematic variables, which quantified the amplitude, speed or consistency of each step. To enable comparisons with non-human primate results, analyses were restricted to the 35 kinematic variables related to leg movements **(Supplementary Table 3)**. Principal component analysis was applied on this high-dimensional representation of gait across Parkinsonian and healthy study participants to identify the most relevant variables to explain changes in walking induced by the Parkinson's disease, and potential improvements that can be brought in by a therapy.

Identifying the hotspots from spatiotemporal maps of motor neuron activity

Video recordings were used to mark the right foot strike and right foot off gait events, and thus calculate the average proportion of the stance and swing phases over all collected gait cycles. The electromyographic signals were transformed into activity of motor neuron pools on using previously described methods (3,36). The activity of motor neurons during each gait cycle was converted from time to gait phase coordinates (see also Supplementary Information section below). Then, the spatiotemporal maps of motor neuron activity were generated by averaging the activity of motor neurons over all the recorded gait cycles. A Gaussian Mixture Model was applied to these maps to identify the dominant hotspots of activity **(78)**.

Decoding of hotspot initiation events from MI neural activity of MPTP-treated NHPs

The inventors implemented an algorithm that decoded the initiation of weight acceptance and leg lift hotspots from the neural activity. Datasets with synchronized multiunit spikes and gait events were used to calibrate the decoding algorithm. Spatiotemporal maps of motor neuron activity were used to calculate the average temporal difference between the right foot off and foot strike gait events and the right weight acceptance and leg lift hotspot initiation events, respectively. These latencies were used to transform gait events into weight acceptance and leg lift hotspots initiation events. In the same dataset, multiunit spike rates in each of the 96 channels were estimated by summing up the multiunit spikes with a 150 ms history every 0.5 ms. These multiunit rates and the hotspot initiation events were used to calibrate a multiclass regularized linear discriminant analysis decoder. Based on a 300-500 ms history of neural activity, the decoder returned the probabilities of observing each of category of hotspot initiation events used to calibrate the decoder. The hotspot event types included: (i) left weight acceptance, (ii) left leg lift, (iii) right weight acceptance, and (iv) right leg lift. When the probability of one of the hotspot initiation events crossed a threshold of 0.8, that event was detected. To verify the efficacy of this decoding approach after MPTP administration, the dataset collected from M6 and M7 was used to demonstrate the accurate decoding, as measured by an offline cross-validation approach. Temporal accuracy of the decoder in 9 (M6) and 8 (M7) sessions performed before and after MPTP administration was measured using the decoding approach.

Decoding of hotspot initiation events from epicortical signals in PD human subjects

The inventors analysed data of participants P2 and P3 of the "Motor Network in Parkinson's Disease and Dystonia: Mechanisms of Therapy" clinical trial (clinicaltrials.gov ID: NCT03582891). In this trial, the participants provided written consent in accordance with the IRB and the Declaration of Helsinki. They were recruited from a population referred for implantation of deep brain stimulators for PD. Before recruitment, they were evaluated by a movement disorders neurologist and met diagnostic criteria for PD and by a neuropsychologist to exclude major cognitive impairment or untreated mood disorder. Inclusion criteria included motor fluctuations with prominent rigidity and bradykinesia in the off-medication state, baseline off-medication MDS-UPDRS-III scores between 20 and 80, greater than 30% improvement in MDS-UPDRS-III on medication than off of medication and absence of significant cognitive impairment (score of 20 or above on the Montreal Cognitive Assessment). The full IDE application (G180097) and study protocol have been shared with other researchers via the Open Mind initiative (https://openmind-consortium.github.io).

Study participants underwent bilateral placement of cylindrical quadripolar deep brain stimulator leads into the STN (Medtronic model 3389; 1.5-mm contact length and 2.0-mm intercontact spacing), bilateral placement of quadripolar cortical paddles into the subdural space over the cortical area that included the motor cortex (Medtronic model 0913025; 4-mm contact diameter and 10-mm intercontact spacing) and bilateral placement of investigational sensing IPGs in a pocket over the pectoralis muscle (Medtronic Summit RC+S model B35300R). The IPG on each side was connected to two leads by 60-cm lead extenders (Medtronic model 37087). The Summit RC+S IPG is a 16-channel device that, through the use of its application programming interface, allows researchers to record four bipolar time domain channels (250/500 Hz) or two channels at 1,000 Hz. For all research functions, including configuring and initiating sensing and developing embedded or distributed adaptive DBS, investigators controlled the device by writing software in C# within the device API, accessed using a 'research development kit' (RDK; Medtronic model 4NR013) provided by the manufacturer. A software application was developed to configure and initiate streaming data from two RC+S devices simultaneously (available at https://openmind-consortium.github.io). This software is written in compliance with the FDA code of federal regulation CFR 820.30, which specifies design controls for implantable human devices.

Recordings collected with P2 and P3 during one session of overground walking in a corridor, and with P2 during one session of walking on a treadmill were analysed. In those sessions, the participants were on their regular levodopa therapy and their DBS was kept off. Cortical field potentials were sampled at 500 Hz. Simultaneous recordings of video using a camera and full body kinematics using a set of IMU sensors distributed across major anatomical landmarks were recorded while the participants performed their tasks.

The reconstructed kinematics of the ankle was used to identify left and right leg lift hotspot initiation events. These events match the events used to synchronize the spinal stimulation with the movement intentions of non-human primates. The epicortical signals were processed using a high-pass filter at 0.5Hz and then lowpass filtered with a $2^{nd}$ order 0.25s long Savitzky-Golay filter. The synchronized dataset of processed epicortical signals and gait events was then used to calibrate two decoders targeting respectively left and right leg lift hotspot initiation events. The decoding accuracy was then evaluated for each side separately by performing cross-validation of the decoders.

Decoding performance

To calculate decoding performance, the distribution of temporal differences between the decoded hotspots initiation events and the hotspots initiation events reconstructed from video recordings was calculated. The performance of decoders is then quantified using temporal decoding accuracy, defined as the proportion of temporal differences falling within a window of 200 ms. Standard error was estimated using bootstrapping with 10 000 resamples.

Therapeutic approach

The aim of the experiment was to re-establish the natural dynamics of motor neuron activation using EES protocols targeting the weight acceptance, propulsion and leg lift hotspots associated with the leg and right legs. This concept implied that EES had to (i) be triggered at the appropriate locations with amplitudes that reproduce the activation of these hotspots (ii) at the times when those hotspots should be active and (iii) for the duration over which each hotspot is activated. The following three sections describe how we these three requirements were fulfilled.

Identification of amplitude and duration to target each hotspot

In M8-10, the electromyography from the right leg muscles in response to single-pulses of EES was recorded. For each of the 16 electrodes of the spinal implant, the muscle responses were recorded over a broad range of stimulation amplitude, ranging from sub-threshold to saturation. These recordings were translated into a mapping between each electrode, EES amplitude, and evoked muscle responses. The relative amplitude of the observed responses was transferred into spatial maps of motor neuron activity. Then, the optimal combinations of electrodes and amplitudes were identified that maximized the correlation between the targeted and elicited spatial map of motor neuron activity. Correlations were measured at the centre of each hotspot. Thus, three combinations of electrodes and amplitudes were obtained that targeted the weight acceptance, propulsion and leg lift hotspots. Since only two muscles were recorded on the left side, it has not been possible to follow the same procedure to determine EES protocols for the left hotspots. Consequently, these combinations based were determined on the observed kinematic responses to single-pulse stimulation. This procedure was applied for both sides in M11, since muscle activity could not be recorded in this animal. EES durations were set to the duration of the corresponding hotspots. In M11, the durations were determined by observing kinematic responses to stimulation during locomotion. Thus, a set of "stimulation protocols" were defined - combinations of stimulation location, amplitude and duration designed to reinforce a specific hotspot.

Using detected hotspot initiation events to trigger spinal stimulation

A real-time control system was designed that used the decoding algorithm to trigger EES protocols at appropriate times. The control computer was connected to the local network and continuously received used datagram protocol (UDP) packets containing neural recordings. A C++ software application (Visual Studio 2010, 2015) was developed running on the control computer, which analysed the neural signals in real-time. Every 15 ms, the application used the decoding algorithm to calculate the probabilities. If one of the hotspot initiation events was detected, the application triggered the relevant EES sequence. EES sequences were designed as a composite stimulation protocols that reinforce one or more of the ongoing or soon-to-be-active hotspots. Since these hotspots often overlapped, the sequences executed protocols in parallel to reinforce the natural dynamics of motor neuron activity. The wireless control of the stimulation had a mean transmission latency of 105 ms[3]. This latency was accounted when translating gait events into hotspot initiation events **(Fig. 18a)**.

Tuning of EES sequences using brain-controlled stimulation

Once the decoder was calibrated, the BSI was used to trigger EES sequences that contained only one of the 6 protocols derived from the optimal combinations of electrode and amplitude targeting each hotspot. The animals walked while the BSI while triggered these simple EES sequences. Then, the amplitudes and durations of EES protocols were tuned based on muscle and kinematic responses. This procedure was performed for all six protocols. Then, designed "left" and "right" composite EES sequences were designed, containing either: (i) left weight acceptance, right leg lift and left propulsion protocols, or (ii) right weight acceptance, left leg lift and right propulsion protocols, respectively. Then, the BSI was used to trigger these sequences from the decoding left or right weight acceptance events. To account for interaction of multiple EES protocols, the design of these composite EES sequences was tuned until satisfying behavioural responses were reached. This tuning process typically lasted for about an hour during the first session. Subsequent sessions required only minor tuning that typically lasted 5 to 10 min.

Calibration procedure to account for stimulation-induced changes in neural signals

Due to sparsity of EES bursts using only one hotspot event (once over a 1-2s long gait cycle), the neural activity used to decode hotspot initiation events was not affected by the motor cortical response elicited by bursts of EES **(3).** However, the repeated delivery of EES sequences that reinforce all six hotspots disrupted the decoder. Thus, a procedure was developed to ensure that the decoder would remain reliable during EES despite cortical responses to EES bursts. For this purpose, a two-step calibration procedure (3) was implemented, which was optimized for Parkinson's disease. Briefly, once the composite EES sequences were tuned, several trials were recorded along the corridor while the BSI triggered either left or right composite sequence. Then, a second decoder was calibrated based on the data recorded without and with EES. This two-step decoder successfully compensated for stimulation-induced changes in motor cortex activity.

Evaluation of the BSI in NHPs before MPTP administration

The calibration of the decoder, optimisation of EES protocols, and operations of the BSI in M8 and M10 were tested prior to the administration of MPTP. First, all the procedures described above were applied. The animals then walked along the corridor while the BSI delivered EES sequences in three conditions: (i) using only decoded left weight acceptance events to trigger the left composite EES sequence, (ii) using only decoded right weight acceptance events to trigger the right composite EES sequence, and (iii) using both left and right weight acceptance events to trigger both left and right composite EES sequences. The decoding accuracy as well the modulation of leg kinematics and muscle activity were quantified.

Evaluation of the BSI in NHPs after MPTP administration

The therapeutic efficacy of the BSI to alleviate gait and balance deficits was evaluated in M8, M9 and M11. All the procedures described above were applied to calibrate the decoder and tune EES protocols. In M8, the decoders used in both reported sessions were calibrated using the data recorded one or two days before. The same decoder was used to deliver EES sequences during locomotion along the ladder. Locomotor performance was measured using task and crossing time, as explained above. M8 and M11 showed specific deficits. M8 developed occasional episodes of slowing of gait and freezing of gait. M11 displayed frequent falls when progressing along the ladder. To evaluate improvement of these specific deficits with the BSI, the proportion of trials along the corridor that contained episodes of slowing of gait or freezing of gait in M8, and the proportion of trials along the ladder in which M11 fell were calculated. Also, the impact of the BSI on kinematics and muscle activity was calculated. All the gait cycles with the BSI where all four decoded hotspot initiation events were within a window of 200ms with respect to the reconstructed hotspot initiation events were isolated. In M8 and M9, locomotor performance with and without the BSI was compared for two different days of recordings before the MPTP administration, as a reference to its healthy gait. The effect of the BSI was compared on the variables that were identified as most relevant to account for gait and balance deficits resulting from MPTP administration: stride length, endpoint velocity of the foot and lateral hip displacement in the corridor task; and stance duration and velocity in the ladder task. In M8 and M9, a PC analysis was applied on 83 gait parameters calculated for each gait cycle and the impact of the experimental conditions was visualized in the plane defined by the first two PC, according to previously described methods **(3,36,60).** To quantify gait performance, the mean Euclidean distance between gait cycles in the entire 83-dimensional space of kinematic parameters was calculated as described previously **(3,60)** and above.

Evaluation of the synergistic effects of BSI and DBS therapies in NHPs

Monkey M9 was equipped with both the BSI and DBS to evaluate how BSI complements the effects of the DBS therapy. First, the BSI therapy was tuned and tested as described above, followed up by tuning and testing the effects of the DBS therapy and, finally, testing the two therapies together. To tune the DBS, charge-balanced biphasic stimulation pulses were applied (90 $\mu$s, 125 Hz, constant voltage mode) using each DBS contact at increasing amplitudes in steps of 0.2V. The amplitude was increased until stimulation-induced motor responses (e.g. neck twitches) were observed while the animal was sitting. Then, the amplitude was reduced, typically by 0.1 - 0.4V, until these motor responses disappeared. To evaluate the effects of DBS, the stimulation was turned on at least 30 minutes before the experiments started to account for reported slow dynamics in the effects of DBS on gait **(81).** When evaluating 20Hz DBS, the amplitude of stimulation was increased to compensate for frequency-related changes in induced charge according to previously published guidelines **(82).** The motor changes induced by DBS additionally included observations in free behaviour of the animal, which were quantified in terms of (i) number of corridor crossings within a ten-minute interval, (ii) number of corridor crossings completed without stopping, and (iii) average gait cycle duration during each crossing. These quantifications aimed to account for improvements in mobility and awareness, facility to turn and ambulate, and locomotor speed.

Effect of continuous dorsal column stimulation

Previous studies showed that continuous high-frequency EES (200 to 300 Hz) targeting the dorsal column within upper thoracic segments alleviated gait deficits associated with PD **(15,32).** The aim was to compare the impact of this protocol with respect to the BSI. Since the used spinal implants did not cover the upper thoracic region, the most rostral electrodes, located over upper lumbar segments, were selected, and an electrical field was configured that targeted the dorsal column. Since the used stimulator could not deliver EES at 300Hz, the two closest available frequencies were tested: 250Hz and 500Hz. In a dedicated session with each of M8 and M9, the same approach was used as with the BSI to identify EES amplitude ranges that maximized locomotor performance during locomotion along the corridor. Then, the subjects were recorded during locomotion along the corridor while delivering continuous EES at amplitudes that covered the identified effective range (6.2V, 6.5V and 6.8V for M8 and 0.4V for M9), both at 250Hz and 500Hz. The task time, crossing time, Euclidean distance to healthy kinematics and spatial correlation to the healthy spatiotemporal spinal map were calculated to compare the efficacy of this stimulation protocol with respect to the BSI.

Current study design and objectives (STIMO-PARK study)

The experiments to evaluate the spinal cord neuroprosthesis were carried out as part of the ongoing clinical feasibility study called STIMO-PARK (clinicaltrials.gov ID: NCT04956770) that investigates the effects of lumbosacral EES to improve mobility in people with Parkinson's disease. The STIMO-PARK study was approved by the Swiss ethical authorities (Swissethics protocol number 2021-0047) and the relevant regulatory authorities (Swissmedic protocol 100008) and has been conducted following the Declaration of Helsinki. The inclusion criteria include idiopathic Parkinson's disease with III-IV Hoehn-Yahr stage, exhibiting severe gait difficulties and postural instability, use of the Medtronic DBS implant and receiving medication for Parkinson's disease. The study involves assessments before the implantation surgery for the spinal EES neurostimulation system, the surgical implantation of the neurostimulation system, a 1-month period for configuration of EES protocols and sequences, and a 3-month period of physiotherapist-assisted rehabilitation during 3-hour sessions taking place 2 to 3 times per week. At each of the sessions, P1 reported on the duration of the use of the neuroprosthesis. The rehabilitation program is personalized based on the participants' needs and improvements.

P1 participant of the STIMO-PARK study

P1 was enrolled in the STIMO-PARK study by signing a written informed consent. He provided written informed consent for publication of identifiable images and video. He was a 61-year-old male at time of enrolment. He has been diagnosed with Parkinson's Disease at the age of 36 and implanted with DBS at the age of 44. He is currently in stage 3 of the Hoen-Yahr scale. He experiences fluctuations in his gait pattern and lower limb symptoms typical of later stages of PD, including slowness, asymmetry, rigidity, small steps, flexed posture. Before being implanted with the EES system, during the 6-minute walk test P1 was able to cover 433 meters with DBS turned on and during his regular levodopa intake, and 224 meters with DBS turned off and with the last levodopa intake the evening before. His MDS-UPDRS motor examination scores (part III) in these two states were 20 and 47, respectively. He started to experience freezing-of-gait over the last decade, which greatly impacted his independence and quality of life. Before participation in the study, he reported 4 falls per day on average due to freezing-of-gait. Following the enrolment, a CT scan of P1's torso and a structural MRI scan of P1's spine were performed to generate a personalized anatomical model of P1's spine. Following the pre-surgical assessments, P1 was implanted with the spinal EES neurostimulation system. After the surgery, P1 was transferred to the neurosurgery ward for recovery. P1 then went back to the hotel for a one-week recovery period. After recovery, the EES protocols and sequences for P1 were successfully configured during the study configuration period. This study presents the analysis of seven sessions recorded during the study configuration period after the EES protocols and sequences have been configured. P1 continued with the study procedures afterwards. All surgical and experimental procedures were performed at the Lausanne University Hospital (CHUV). The procedures are described in detail below.

P1 personalized neurobiomechanical model

In order to estimate the target for the immediate effects of the therapy, the gait of P1 was simulated based on his anatomy but in the absence of neurodegeneration. To this end, a personalized musculoskeletal model of P1 was generated by adapting the Lower Limb model to P1's anatomy and by optimizing the reflex-based gait controller used to generate walking. Similar methods have been used to reproduce gait of healthy individuals, gait of elderly affected by the muscle weakness and reduced contraction speed, and gait adaptations due to ankle plantarflexor muscle weakness and contracture. The musculoskeletal model included three planar degrees of freedom at the pelvis and another three for each leg: hip flexion, knee flexion and ankle flexion. These degrees of freedom are actuated by eight Hill-type muscle-tendon units **(69)** per leg corresponding to eight different leg muscles: (1) gluteus maximus, (2) hamstring muscles, (3) iliopsoas, (4) vastus muscles, (5) biceps femoris, (6) gastrocnemius, (7) soleus, and (8) tibialis anterior. This model is scaled to P1 morphology derived from the motion tracking of P1 while standing using the OpenSim scaling tool **(70)**. The muscular properties of the model were scaled using muscles' cross-sectional area segmented from P1's thigh CT scan. As the maximal voluntary force and cross-sectional area are significantly correlated through aging **(71),** the maximal isometric force of each Hill-type muscle-tendon unit were scaled to the ratio of the muscle cross-sectional area over the healthy reference from **(72,73)**. Due to the absence of the calf CT scans, the maximal isometric force of the calf muscles were scaled using the mean of the thigh muscle ratios. A more robust estimate of the muscle strengths was obtained by averaging the scaling over both sides. The resulting scaling factors were 0.65 for the hamstrings group, 0.87 for the quadriceps group and 0.76 for the calf group. The SCONE software was then used to optimize the parameters of the reflex-based gait controller. This controller is composed of phase dependent reflexes providing muscle excitation based on muscle length, velocity or force feedback. P1's gait in the absence of neurodegeneration was simulated using Covariance Matrix Adaptation Evolutionary Strategy to optimize the controller parameters. A cost function penalizing falling, muscles' metabolic cost **(76),** joint angles out of healthy ranges and the head balance was used, with respective weights 100, 0.1, 0.1 and 0.05 balancing the competitive objectives. About 500 generations of CMA-ES were necessary to reach a stable gait initialization. Once the parameters of the controller have converged, 200 steps of this neurobiomechanical model were generated. These steps were used to extract full kinematics of lower limbs and muscle activity. These simulation-generated kinematic and muscle activity signals were compared with the signals recorded during the P1 study sessions.

Planning the surgical placement of the epidural spinal lead in P1 using personalized anatomical model of the spine.

P1's CT and MRI scans were used to generate a three-dimensional anatomical model of his spine. First, the vertebral bones were segmented from the CT scan using a convolutional neural network (CNN) based framework trained on the VerSe datasets. The spinal cord tissues were then segmented from the MRI scan using a nnU-Net **(79)** after pre-processing and with post-processing using the Spinal Cord Toolbox **(80).** Remainder of the lead placement planning was performed in the Sim4Life software. The MRI scan images and the segmented spinal cord tissues were then loaded into the Sim4Life software to identify the locations where spinal cord roots merge with the spinal cord, hereafter termed root merging points. The trajectories of the spinal cord roots were then generated that follow the path from the root merging point to the entry of that root into the spinal canal. The spinal cord segments were defined as spinal cord tissue bounded by the midpoints between the two neighbouring root merging points. The root trajectory leading to the root merging point was defined as the central rootlet and distributing four off-centre rootlets across the segment. The vertebral bones and disks segmented from the CT scan were then loaded into Sim4Life to align the bones and disks to the spinal cord and roots. This combined anatomical model was then used to propose a placement of the lead. A 3D model of the lead was first loaded and placed centred over the dorsal side of the spinal cord covering most of the L1-L5 spinal segments, which control the contraction of leg muscles. Position of the lead with respect to the segmented vertebral column determined the insertion point of the lead to be between L1 and T12 vertebra.

Surgical implantation of the STIMO-PARK investigational spinal EES neurostimulation system in P1.

During the surgery, P1 was implanted with a Specify® SureScan® MRI 5-6-5 16-electrode epidural spinal lead (Medtronic plc, Fridley, MN, USA) in the posterior epidural space covering the lumbar spinal cord segments. This lead is clinically approved for the treatment of chronic pain. The lead was then connected by a cable to an Activa™ RC IPG (Medtronic plc, Fridley, MN, USA), which is clinically approved as an IPG for deep-brain stimulation therapy. The IPG was first inserted into a subcutaneous pocket in the abdomen. The lead cables were then tunnelled from one opening to the other and connected to the IPG. These combined elements and associated firmware constitute an investigational spinal EES neurostimulation system that was tested as part of this clinical study.

The insertion level was identified during surgery using fluoroscopy. To insert the lead, an approximately 5 cm midline skin incision was performed. The fascia was opened and the muscles were retracted bilaterally. Excision of the midline ligamentous structures and T12/L1 flavectomy and partial laminectomy enabled the insertion of the lead into the spinal epidural space. To perform the insertion, the lead was placed over the midline of the exposed dura and advanced rostrally to the target location.

An ISIS XPress monitoring and stimulation system (inomed Medizintechnik GmbH, Emmendingen, Germany) was used to accurately adjust the medial and segmental position of the lead. To this end, EES pulses with a pulse width of $300\mu$s were delivered at 0.5 Hz and at increasing amplitudes to elicit muscle responses that were recorded with subdermal (Neuroline Twisted Pair Subdermal, 12 x 0.4 mm, Ambu A/S, Balle- rup, Denmark) or intramuscular needle electrodes (Inomed SDN electrodes, 40 mm, inomed Medizintechnik GmbH, Emmendingen, Germany). The lateral position of the lead was adjusted so that the bottom electrodes selectively recruit the calf muscles while maintaining good activation of the hip flexor muscles using the top electrodes. The medial position of the lead was adjusted to reach a similar side-specific muscle recruitment with the corner electrodes. The final location of the lead overlaid lumbar and upper sacral segments. The surgery then finished and P1 was transferred to the neurosurgery ward for recovery.

Reconstruction of the electrode position with respect to the patient spine was assessed from the post-operative CT scan. CT images enabled reconstructing the three-dimensional geometry of vertebral and the location of the lead electrodes inside the vertebral column. The resulting 3-dimensional volume reconstruction of the spinal cord of the participant included the lead with its electrodes, vertebral bodies, white matter, trajectory of dorsal spinal roots and rootlets.

P1 spinal cord neuroprosthesis

The EES of the lumbar spinal cord was done by controlling the delivery of current though the 16 IPG header channels, each connected to one of 16 electrodes of the epidural spinal lead, or through the IPG case. The IPG was modified from its clinical version with an investigational firmware that enabled real-time communication with a NEUWalk Research Programmer Application software (NRPA, Model 09103, Medtronic) running on an external computer. The NRPA acted as a relay between the G-Drive Plus control software (described below) and the IPG. The NRPA communicated wirelessly with the IPG through the following communication chain: the NRPA sent commands via a virtual COM port corresponding to a Bluetooth adapter, a custom wireless bridge consisting of a nano computer (Raspberry Pi) received this command and forwarded it to a virtual COM port corresponding to a USB adapter, a USB to infrared adapter (ACT-IR224UN-LN115-LE, ACTiSYS Corporation, Fremont, CA, USA) transformed this command into infrared signals that were then read by a modified Medtronic patient's programmer (Sensing Programmer Telemetry Module SPTM, Medtronic), which finally transmitted the command to the patient's IPG by electromagnetic induction through the skin.

G-Drive Plus software for configuration and control of the spinal cord neuroprosthesis in P1

A custom G-Drive Plus software application was developed to configure and control the spinal cord neuroprosthesis. G-Drive Plus runs on a desktop computer, laptop or tablet and interfaces with the stimulation system (through NRPA) and collects the signals from different sensors that can be used for closed-loop stimulation. The sensors include the wireless Next Generation Inertial Measurement Units (NGIMU; x-io Technologies Limited, UK) and the Trigno® Research+ System utilizing wireless Trigno® Avanti sensors that can record both the IMU and EMG signals simultaneously. G-Drive Plus includes a graphical user interface (GUI) that enables rapid personalization of EES protocols, each of which is parametrized by a set of cathodes and anodes to deliver the stimulation, the amplitude of the stimulation current and the stimulation frequency. G-Drive Plus GUI also includes a stimulation scheduler that enables rapid personalization of sequences of EES protocols. Once defined, these EES protocols and sequences can be uploaded to the IPG. To enable closed-loop controlled stimulation, the execution of EES sequences is linked to specific events detected from data collected by the sensors. On detection of an event, G-Drive Plus sends wireless command to the IPG to execute the linked sequence. After 105ms on average, the currently running stimulation sequence is interrupted and the commanded sequence is initiated. The following command can only be sent after 200ms on average, once the IPG has send back the confirmation that the previous command has been received and executed. G-Drive Plus can also trigger acquisition from video cameras and emit standardized synchronization pulses. During recording sessions, these functionalities allow us to use G-Drive Plus to visualize and immediately assess the effects of the EES protocols and EES sequences on muscle activity and whole-body kinematics. All the acquired data, including the delivery of EES protocols synchronized with the kinematics and muscle activity is saved for offline analysis.

Calibration of EES protocols in P1

In an EES calibration session, single-pulse EES were used to identify electrode configurations that recruit the six targeted hotspots of spinal activity: left and right weight acceptance, propulsion and leg lift hotspots. Weight acceptance roughly corresponds to knee extension, propulsion to ankle extension, and leg lift to coactivated hip flexion and ankle flexion, respectively. The participant was lying relaxed in supine position on an examination table. EMG activity was recorded from left and right iliopsoas (II), rectus femoris (RF), vastus lateralis (VLat), semitendinosus (ST), tibialis anterior (TA), medial gastrocnemius (MG), and soleus (Sol) muscles using Delsys Trigno wireless EMG sensors (Delsys Incorporated). The Nuprep abrasive paste was applied on the skin (Weaver and Company, Aurora, CO) to reduce electrode-skin resistance and improve EMG signal quality. The muscle responses to single-pulse EES (300 $\mu$s pulse width) were mapped. These single-pulse EES were delivered using monopolar configurations (one lead electrode as cathode and case as anode) at currents ranging from 0.1mA to 3.5mA. All possible monopolar configurations were systematically tested. For each configuration, the EES current was gradually increased until all muscle responses reached saturation or until P1 reported discomfort. 3 repetitions for each amplitude were recorded. The recorded EMG responses were then used to compute spinal map activations for each monopolar configuration across a range of amplitudes. Eight monopolar configurations were then identified, each configuration generating a spinal map activation with the highest correlation with one of the identified hotspot spinal activations. If the muscle activation selectivity of one of these configurations was not satisfactory, that EES protocol was refined using multipolar electrode configurations, which use additional anodes to steer the electrical field towards the targeted posterior roots. The frequency and amplitude of each of the EES protocols was then tuned. Yet, the Activa RC IPG allowed the use of only a single stimulation frequency. Extensor muscles are more responsive to low-frequency stimulation (e.g. < 40 Hz), while the flexor muscles respond better to higher frequencies (e.g. > 80 Hz). Since the EES protocols targeted both the flexor and extensor muscles, the tuning identified 60Hz as the most effective stimulation frequency overall. The current amplitude for each EES protocol was tuned by asking the participant to walk while triggering that protocol and observing the muscle responses. The amplitudes that generated a clear enhancement of gait and that were below the P1's discomfort threshold was selected. Already the tonic stimulation using the of combination the left and the right knee extension EES protocols clearly enhanced P1's gait. Two of left and right knee extension EES protocols were then defined, a knee extension "boost" EES protocol with a higher amplitude to coincide with the natural activation of the knee extension hotspot, and another with a lower amplitude active during the remainder of the gait cycle **(Fig. 6)**.

Calibration of EES sequences in P1

In an EES calibration session, the EES protocols were assembled into two EES sequences: "Left Foot Off" and "Right Foot Off". These were designed to be initiated in synchrony with the beginning of the left and right leg lift hotspot, respectively. The duration of each protocol in the sequence was tuned by asking P1 to walk overground while he the EES sequences were delivered. G-Drive Plus was used to look at the overlap of EES protocols and activation of muscles targeted by those protocols. The sequences were modified until all the misalignments were removed **(Fig. 7)**. With non-human primates, whose gait cycle typically lasted more than one second, four EES sequences were used. With the help of EES, the gait cycle of P1 was often below 0.8 seconds. Due to the communication chain between G-Drive Plus and the IPG, a new command could only be sent to the IPG 200ms after the previous one. Therefore, using more than one EES sequence per side generated delays in the execution of EES sequences, which made the neuroprosthesis ineffective.

P1 corridor and freezing circuit sessions

These sessions tested the synergistic effects of DBS and EES therapies through four conditions: DBS off EES off, DBS on EES off, DBS off EES on, and DBS on EES on. Since the effect of DBS takes time to washout, a single session could only test either the DBS off or the DBS on conditions. For the DBS off sessions, P1 was instructed to turn his DBS therapy off in the morning before the session. P1 maintained the same daily regime of levodopa intake during the trial protocol. To ensure balanced comparison between the conditions tested on different days, sessions always stated at the same time, thus ensuring roughly the same levedopa levels at the beginning of each session. During these sessions, P1 was first equipped with up to 16 wireless Trigno® Avanti sensors with bipolar surface electrodes over the muscles of left and right legs: iliopsoas, rectus femoris, adductors, vastus lateralis, semitendinosus, tibialis anterior, medial gastrocnemius, and soleus. In addition, two NGIMU sensors were attached on the surface of the left and right shank. Additionally, 14 infra-red reflective markers were attached on the following anatomical landmarks: left and right toe, ankle, knee, hip, shoulder and two on each wrist. For the remainder of these sessions, P1 walked within the instrumented space of our gait laboratory as his EMG activity and kinematics were recorded and his spinal EES was controlled. The EMG and IMU signals were acquired using the Trigno® Avanti sensors with a 1259 Hz and 148 Hz sample rate, respectively, and additional IMU signals from NGIMU sensors at 30 Hz. The kinematic recordings were generated using a Vicon 3D motion capture system (Vicon Motion Systems, Oxford, UK), consisting of 14 infrared cameras, each set to record the reflection of infrared-reflective markers at 100 frames per second. The Vicon system covered a 12 m x 4 m x 2.5 m workspace. The chronophotographic images of participants were captured using a high-definition camera (FUJIFILM X-T2, 5 images/s, ISO 6400, shutter speed 1/250 sec). As he walked, P1 was always followed by a physiotherapist to prevent falls in the case of freezing. During the corridor sessions, on the mark of the experimenter, P1 was asked to stand up from his chair, walk from before one floor marker to beyond another floor marker in a straight line, turn around and come back again in the straight line, and repeat this the second time. Once he finished the task, P1 was free to sit down and rest. An epoch stating with P1 passing the marker on one side of the Gait lab and ending with P1 passing the marker on the other side of the Gait lab was defined as a trial. The time to complete a trial was defined as task time. The epoch between the P1 standing to walk on experimenter's mark and him finishing the task was defined as a "trial set". During the freezing circuit sessions, a circuit consisting of narrow corridors with obstacles was built. On the mark of the experimenter, P1 was asked to stand up from his chair and walk across the floor marker and through the circuit across the obstacles until he reached the floor marker on the other side of the Gait lab. Once he finished the task, P1 was asked to walk around the circuit to the initial position, sit down and rest. Each crossing of an obstacle was defined as a trial, during which P1 either froze or not. One passing of the entire circuit was defined as a trial set. In both types of sessions, P1 and the physiotherapist were not informed on whether the EES was on or off during that trial set. In the corridor sessions, after each trial, P1 was asked to comment on and score the quality of his gait on a scale from 0 to 5 with 0.25 increments. After P1 finished with the scoring, the physiotherapist was asked to also comment on and score the quality of P1's gait on the same scale.

Kinematically-controlled spinal cord neuroprosthesis in P1

Process to calibrate the closed-loop control of EES for P1 followed the procedure employed for the non-human primates **(Extended data Figure 5)**. On a day preceding the first overground walking session, a dataset was recorded in which P1 walked straight while being equipped with the NGIMU sensors, Trigno® Research+ system and recorded by cameras. He walked first in the DBS on EES off condition. These trial sets were used to calibrate thresholds on the pitch angles acquired from the NGIMU sensors positioned on the left and right leg shanks. P1 was then asked to again walk straight, now in the DBS on EES on condition where the Left Foot Off and Right Foot Off EES sequences were triggered by left and right leg shank pitch angles respectively passing their thresholds. The gait events were then labelled in both the DBS on EES off and DBS on EES on datasets using a custom-developed Matlab® program through visual inspection of the video frames. These labelled datasets were used to calibrate rLDA decoders that detected the initiation of the left leg lift and right leg lift hotspot initiation events. Decoder used accelerations and angular velocities acquired by the Trigno® Avanti sensors lowpass filtered with a 2nd order 0.25s long Savitzky-Golay filter[63,64] as inputs. To enable closed-loop control of EES sequences in real-time, a DecodingGate C++ software application (Visual Studio 2010, 2015, 2017) was developed. The DecodingGate acquired the Trigno® Avanti sensors recordings from G-Drive Plus, processed those recording, applied the rLDA decoding algorithms, and, upon detection of hotspot initiation events, sent the commands to the G-Drive Plus to initiate the relevant EES sequence. G-Drive Plus then relayed these commands to the IPG. Decoding Gate ran on a separate computer and exchanged data with G-Drive Plus via UDP packets through a local network. While calibrated from data recorded during straight walking in the DBS on conditions, the decoders remained accurate in the DBS off conditions, and when used in the freezing circuit task **(Fig. 5-6).**

Assessment of gait impairments in P1

After each corridor trial, the physiotherapists attributed a general gait quality score according to predefined criteria **(Supplementary Table 4).** The motion tracking of P1's legs was processed into kinematic variables. These variables were used to quantify key deficits of Parkinsonian gait. Gait speed was quantified by task time and stride length, and gait asymmetry for temporal and spatial parameters by the ratio of gait phases and step lengths between left and right legs. Gait is more symmetric as these ratios approach zero.

$$Gait\ Phase\ asymmetry = 100 \times \left| \log \left( \frac{\frac{Swing\ duration_R}{Stance\ duration_R}}{\frac{Swing\ duration_L}{Stance\ duration_L}} \right) \right|$$

$$Step\ length\ asymmetry = 100 \times \left| \log \left( \frac{Step\ length_R}{Step\ length_L} \right) \right|$$

The variability of gait patterns was also quantified using stride time coefficient of variation, which has been associated to gait instability and risk of fall in PD. The coefficient of variation is defined as the ratio between the standard deviation and the mean, and increases with variability. The gait upper body control was quantified by measuring the arm swing angle.

To assess whether P1's gait patterns across different experimental conditions was more similar to those of people with PD or to healthy people, a classification was implemented using a linear discriminant analysis decoder. This decoder was calibrated on gait parameters calculated from kinematic recordings from the group of 25 people with PD, and the group comprised 9 healthy age-matched controls. This decoder was used to classify whether each gait cycle performed by P1 was more similar to gait patterns from patients with PD or healthy gait. This procedure was implemented for each experimental condition separately, and calculated the portion of gait cycles classified as healthy.

Assessment of balance problems in P1

The impact of the spinal cord neuroprosthesis and DBS therapies on balance problems was evaluated using the standard Mini-BESTest clinical evaluation, and using the Activities-specific Balance Confidence (ABC) scale questionnaire.

Assessment of freezing-of-gait in P1

The impact of the spinal cord neuroprosthesis and DBS therapies on freezing-of-gait was evaluated by having a trained neurologist observe the videos of each of the freezing circuit trails and label freezing-of-gait epochs with 100 ms resolution. The impact was then measured by the fraction of the time P1 spent traversing the circuit that he spent under a freezing-of-gait epoch.

Blinding

No statistical methods were used to predetermine sample size. The experiments were not randomized. The investigators were not blinded to allocation during experiments and outcome assessment. Experienced technicians working at the facility and not part of the study were blinded during their assessments of Parkinson's disease scores. They were unaware of the intoxication protocol or any of the details related to the experiments. All data analyses, except for the semi-automatic kinematic reconstruction in the Simi Motion software and marking of gait events from video recordings, were performed using automatic computer routines.

Anatomical quantification of the impact of MPTP administration

The depletion of dopaminergic tissue was analysed in the 6 monkeys (M5, M6, M7, M8, M9, M11) that were involved in MI recordings after MPTP administration and in the experiments with the BSI. The monkeys were deeply anesthetized and perfused transcardially with a 4% solution of paraformaldehyde. Their brain was removed and stored at 4° C in 0.1 M phosphate-buffered saline azide (0.03%). The nigrostriatal dopaminergic neurons were counted using an unbiased stereological method and the density of dopaminergic fibers in the caudate and ventral and dorsal putamen was quantified as described previously (83). Briefly, tyrosine hydroxylase (TH) immunochemistry was performed using mouse anti-TH primary antibody (catalog MAB318, Millipore/Chemicon International, Technology, Billerica, MA, USA). Unbiased stereological counting of nigral TH$^{ON}$ neurons as well as striatal optical density measurement were performed using Exploranova Mercator (Explora Nova, La Rochelle, France). Technical reasons prevented quantification of dopaminergic fiber density in M5 and of all brain tissues in M11.

Statistical procedures

All the computed parameters were quantified and compared within each animal. All data are reported as mean values $\pm$ standard error of the mean (s.e.m.). Significance was analysed using the non-parametric Wilcoxon rank sum and signed-rank tests, bootstrapping or a Monte Carlo approach, as indicated for each test.

**SUPPLEMENTARY INFORMATION**

**Design and fabrication of the spinal implant**

Spatial distribution of leg motor neuron pools

In order to identify which dorsal roots to target using the spinal implants, a previously generated library of the spatial distributions of motor neuron pools of lower limb muscles in macaque monkey (3,36) was used. Briefly, the library was assembled using retrograde tracing method in seven monkeys. The monkeys received injections of Fastblue (4 % in sterile saline, 1000 $\mu$l per muscle) or Fluoro-Ruby (10 % in sterile saline, 1000 $\mu$l per muscle) in up to two distinct muscles of the left and right legs, allowing tracing up to four muscles per monkey. The following muscles recorded during locomotion were traced: gluteus medius, iliopsoas, rectus femoris, semitendinosus, gastrocnemius medialis, tibialis anterior, extensor digitorum longus, and flexor hallucis longus. The locations of the retrogradely traced motor neurons were reconstructed in 3D from serial sections of the spinal cord using Neurolucida. The reconstruction from all the animals are merged into a unified digital library using the morphology of the spinal segments as a landmark.

Anatomy of the spine

To precisely determine the dimensions of the arrays that fit within the epidural spinal space, the previously performed reconstruction of the macaque spine was leveraged (3,36). Briefly, the three-dimensional reconstruction of vertebra was conducted using the micro-computer tomography scanner Skyscan 1076 (Bruker $\mu$CT). The 3D shape of vertebrae was derived from these scans using NRecon and GPURecon Server (Bruker $\mu$CT). The spinal cords of several monkeys were imaged post-mortem. Segmentation and 3D models were constructed with Amira® (FEI Vizualisation Sciences Group). The spinal cord and vertebra reconstructions were co-registered into a unified 3D model. Measurements of relationships between vertebra and spinal segment morphologies were performed on fresh tissue taken from cadavers. For each monkey, the spinal segments were identified based on the innervation of the dorsal roots. The centre of the segment was defined as the entry point of the rootlets. After measuring the length of vertebra, and the relationships between vertebra and spinal segments, the entire spinal cord was extracted, and the roots moved perpendicular to the spinal cord to clearly visualize the segments. The location and length of each segment was then calculated.

Reconstruction of spinal segments and dorsal root trajectories

Reconstruction of spinal segments and dorsal root trajectories relied on the previously performed reconstruction of dorsal root trajectories (3,36). Briefly, the spinal cords of three monkeys were dissected, fixated overnight, and transferred to 30% phosphate buffered sucrose for cryoprotection. The dura mater was opened along the rostrocaudal axis, and gently moved on the side. For each spinal segment, the dorsal root ganglions were identified. The corresponding root was retracted cranially and laterally. The entire length of the root was painted, from the cut extremity to the entrance into the spinal segment. All the painted roots from S1 to L1 were repositioned to their original location along spinal segments, and the dura mater was sutured. The spinal cord was then frozen and cut into 80 $\mu$m thick slices using a cryostat (Leica Instruments). Reconstructions were conducted using every 4 sections, corresponding to intervals of 320 $\mu$m. The slides were assembled into the Neurolucida image analysis software (MBF Bioscience) to reconstruct the color-coded dorsal roots trajectories and lumbosacral segments in 3D.

Using model of the spinal cord and vertebral morphology for implant design

All the 3D reconstructions derived from micro-computed tomography imaging and anatomical experiments were merged to generate a global model including the bony structure of the vertebrae, the shape of spinal segments, the trajectory of each dorsal root and the anatomical locations of motor neuron columns. This model was leveraged to design spinal implants that positioned the electrodes above the L2-L7 spinal dorsal roots. In order to fit the electrode contacts and the tracts in the dorsal space between the vertebra and the spinal cord, two implants were designed: an 8-electrode "rostral" electrode array targeting left and right L2, L3 and L4 roots; and another 8-electrode "caudal" electrode array targeting the L5, L6 and L7 roots. Each implant was designed to access the roots on their way from the entry between the vertebra to their entry into the spinal cord.

Fabrication of spinal implants using the e-dura technology

A manufacturing procedure was developed that uses the e-dura technology (39) to fabricate the spinal implants. The manufacturing process involved five stages (Fig. 18):

1. Substrate
4" silicon wafers were used as a substrate for the implants. Then, a polystyrene sulfonic acid layer was spin-coated on the carrier to provide a water-release layer for the substrate stack. Then, a polydimethylsiloxane (PDMS) layer was cast on the substrate to a thickness of approximately 500 $\mu$m. Then, a laser-machined, 50 $\mu$m thick polyimide was mask manually laminated on the PDMS surface, and the carriers were mounted in a thermal evaporation chamber. A stack of chromium and gold have been evaporated on the carriers through the polyimide masks at a thickness of 5 nm (Cr) and 55 nm (Au). The chromium acts as adhesion promoter for the gold interconnect on PDMS. Finally, the polyimide shadow mask was peeled off the surface to reveal the interconnect design patterned in the metal stack.

2. Passivation
A 3 mm thick PDMS handling layer was casted in a Petri dish. Once cured, the top surface was exposed to an oxygen plasma. Then, a vapour-phase 1H,1H,2H,2H-perfluorooctyltriethoxysilane (Sigma-Aldrich) layer was applied in a vacuum chamber. This inhibits the adhesion of the silicone to subsequent PDMS layers deposited on the surface. Two subsequent 20 $\mu$m thick PDMS layers were spin-coated and cured on the thick PDMS, separated by the same adhesion inhibiting layer. Then this triple PDMS stack (3 mm, 20 $\mu$m, 20 $\mu$m in cross section) was cut with a blade and mount it on a glass slide. Then, a mechanical catheter puncher was used to make holes through the two thin PDMS layers and into the thick handling layer, in order to machine the passivation stack with through-vias. Each via was created by punching a series of 4 round holes of 690 $\mu$m diameter with 400 $\mu$m centre-to-centre spacing.

3. Assembly
The top surfaces of the substrate and triple stack encapsulation were exposed to oxygen plasma, and then mounted on an alignment rack with the two treated surfaces facing one another. The vias machined in the encapsulation were aligned with the interconnect patterned on the substrate. Then, the two parts were contacted in order to form a covalent bond between the silicone layers. Once bonded, the thick PDMS handling layer was peeled off the substrate, leaving the interconnect encapsulated by two 20 $\mu$m thick PDMS layers with openings corresponding to the position of the electrodes.

4. Electroactive coating
The electroactive coating was prepared as a composite material obtained by dispersing microscale platinum particles (Goodfellow PT006021, 3.5 $\mu$m maximum particle size) within a PDMS matrix. This creates a conductive paste that offers a balance between the charge injection properties of platinum and the mechanical properties of PDMS. This composite paste was applied on the encapsulation through the screen print PDMS layer to fill the openings, which creates an electrical contact with the interconnect. We then peel the screen print layer off to remove the excess coating and define the active stimulation sites.

## 5. Packaging

A blade was used to manually cut the assembled implant to the desired shape while still on wafer. The electrical wires were threaded in a PDMS guiding piece through holes that were machined at the same pitch as the gold tracks on the substrate. Then, this soft connector was aligned onto the interconnect with wires close to the ends of the gold tracks. Conductive Ag paste was pressure-dispensed to make the individual electrical connections between the wires and the gold tracks. (Epotek H27D). Once all electrical connections were made, a bolus of room temperature vulcanization sealant (one component silicone sealant 734, Dow Corning) was applied over the connector to secure the assembly mechanically After the sealant had cured, the implant was released from the silicon carrier by dissolving the PSS layer under the PDMS substrate with DI water.

Throughout the manufacturing process, all silicone layers were prepared by mixing PDMS (Sylgard 184, Dow Corning) using a weight ratio of 10:1 between pre-polymer and cross-linker. The deposited layers were cured for a minimum of 3 hours in temperature controlled oven set to 80°C.

## **Wireless control of spinal cord stimulation**

### Implantable pulse generator

The spinal implants were interfaced with an Activa RC implantable pulse generator (Medtronic, USA) commonly used for deep brain stimulation therapies. Activa RC supports the delivery of monopolar, constant-current or constant-voltage stimulation protocols using the case of the generator as the anode. The Activa RC firmware supports the definition of pre-defined stimulation sequences. Each sequence is composed of one or more stimulation protocols, each characterized by (i) stimulation channel (mapped to one of 16 electrodes of the spinal implants), (ii) stimulation frequency, (iii) stimulation pulse width, (iv) stimulation duration, and (v) stimulation amplitude. The stimulation protocols can overlap each other. Activa RC enables wireless upload of stimulation sequences and the ability to initiate and stop each of those sequences. Reception of a new command immediately interrupts the execution of a previous sequence.

### Wireless communication with the implanted pulse generator

A previously developed communication chain was used to wirelessly communicate with the implanted Activa RC pulse generator **(3)**. Briefly, the real-time analysis software running on the control computer elicited a stimulation sequence trigger command and sent it to a Neural Research Programmer software application (Medtronic, USA) running on the same computer. The Neural Research Programmer wirelessly transmitted the command by a Bluetooth link to a Bluetooth-to-infrared module placed in the jackets worn by the monkeys (ACTiSYS Inc.). The Bluetooth-to-infrared module relayed the command to the Patient's Programmer device (Medtronic, USA) in the jackets, which then transferred the command to the implanted Activa RC using a telemetry antenna placed on the monkeys' skin over the Activa RC. Once sent, the command reached the Activa RC with a latency of 105ms. This communication system is an investigational prototype restricted to research that is not approved for commercial use.

## **DECODING OF HOTSPOT INITIATION EVENTS FROM NEURAL SIGNALS**

### Concept

It has been sought to re-establish the natural dynamics of the spinal motor neuron activity by reinforcing the hotspots of motor neuron activity by stimulating the proprioceptive fibres of the spinal dorsal roots that feed into those motor neurons. Therefore, an aim was to deliver the stimulation protocols targeting appropriate roots beginning at the time that these hotspots become active. It was hypothesized that leg motor cortical neuronal activity can enable accurate detection of these hotspot initiation events using the neural decoding approach. Therefore, neural signal processing and decoding algorithms were developed and implemented that served to detect initiation of hotspots from the neural activity. In particular, the algorithms were implemented within the real-time software application.

Acquisition of calibration data

To calibrate the decoders, first a calibration dataset composed of synchronized neural signals and hotspot initiation events was collected **(Fig. 17a).** A dataset containing neural and video recordings of monkeys walking across a corridor without using stimulation was acquired. During the acquisition, the Central computer application (Blackrock Microsystems Inc., USA) band-pass filtered (0.5-7.5kHz) the wideband 96-channel neural signals recorded at 30 kilo-samples per second. Each crossing of this signal from above to below the electro-specific threshold was marked as a multiunit spike. The threshold was set to be 3.5 times the standard deviation of the filtered signal on this channel calculated over a period of five seconds. These multiunit spike events and the video recordings were saved on the computer. Once the dataset was recorded, the saved multiunit spikes were loaded into a custom-made Matlab program (The MathWorks, Inc., USA). The program was used to estimate multiunit spike rates using overlapping 150ms bins updated every 0.5 ms. Left and right foot off and foot strike gait events were identified through visual inspection of the video frames. The gait events were synchronized with the multiunit spike rates using a trigger saved with the neural data that marked the onset of video recordings. Using the spatiotemporal map of motor neuron activity, the temporal shifts between foot off and foot strike gait events and the initiation of weight acceptance and leg lift hotspots were estimated. These shifts were used to co-register weight acceptance and leg lift hotspot initiation events with the multiunit spike rates. The hotspot events were additionally corrected for the empirically estimated stimulation latency of 105ms. Then, the corrected hotspot initiation events and the derived 96 channels multiunit spike rates were used to calibrate a regularized linear discriminant analysis decoder.

Decoder calibration

Through the process of decoder calibration, corrected hotspot initiation events served to identify sets of motor cortical multiunit spike features that correspond to those events. Specifically, sets of left and right weight acceptance and leg lift hotspot events, *lwa, rwa, lll* and *rll,* were used to derive the left and right weight acceptance and leg lift hotspot classes of neural features, $C_{lwa}$, $C_{rwa}$, $C_{lll}$ and $C_{rll}$, respectively **(Fig. 17a):**

$$C_{lwa} = \left\{ \overline{a}_{lwa} \left| \overline{a}_{lwa}(i) = \begin{bmatrix} x_1\left(lwa(i) - \Delta t_{lwa}\right) \\ x_1\left(lwa(i) - \Delta t_{lwa} - \Delta t\right) \\ \vdots \\ x_1\left(lwa(i) - \Delta t_{lwa} - (N_{TP}-1)\cdot\Delta t\right) \\ x_2\left(lwa(i) - \Delta t_{lwa}\right) \\ \vdots \\ x_{N_{CH}}\left(lwa(i) - \Delta t_{lwa} - (N_{TP}-1)\cdot\Delta\right) \end{bmatrix} \right. C_{rwa} = \left\{ \overline{a}_{rwa} \left| \overline{a}_{rwa}(i) = \begin{bmatrix} x_1\left(rwa(i) - \Delta t_{rwa}\right) \\ x_1\left(rwa(i) - \Delta t_{rwa} - \Delta t\right) \\ \vdots \\ x_1\left(rwa(i) - \Delta t_{rwa} - (N_{TP}-1)\cdot\Delta t\right) \\ x_2\left(rwa(i) - \Delta t_{rwa}\right) \\ \vdots \\ x_{N_{CH}}\left(rwa(i) - \Delta t_{rwa} - (N_{TP}-1)\cdot\Delta\right) \end{bmatrix} \right.$$

$$C_{lll} = \left\{ \overline{a}_{lll} \left| \overline{a}_{lll}(i) = \begin{bmatrix} x_1\left(lll(i) - \Delta t_{lll}\right) \\ x_1\left(lll(i) - \Delta t_{lll} - \Delta t\right) \\ \vdots \\ x_1\left(lll(i) - \Delta t_{lll} - (N_{TP}-1)\cdot\Delta t\right) \\ x_2\left(lll(i) - \Delta t_{lll}\right) \\ \vdots \\ x_{N_{CH}}\left(lll(i) - \Delta t_{lll} - (N_{TP}-1)\cdot\Delta t\right) \end{bmatrix} \right] C_{rll} = \left\{ \overline{a}_{rll} \left| \overline{a}_{rll}(i) = \begin{bmatrix} x_1\left(rll(i) - \Delta t_{rll}\right) \\ x_1\left(rll(i) - \Delta t_{rll} - \Delta t\right) \\ \vdots \\ x_1\left(rll(i) - \Delta t_{rll} - (N_{TP}-1)\cdot\Delta t\right) \\ x_2\left(rll(i) - \Delta t_{rll}\right) \\ \vdots \\ x_{N_{CH}}\left(rll(i) - \Delta t_{rll} - (N_{TP}-1)\cdot\Delta t\right) \end{bmatrix} \right.$$

where $\overline{a}_{lwa}$, $\overline{a}_{rwa}$, $\overline{a}_{lll}$ and $\overline{a}_{rll}$ are feature vector members of classes $C_{lwa}$, $C_{rwa}$, $C_{lll}$ and $C_{rll}$, respectively; $N_{TP}=7$ (monkeys M8 and M9) or 5 (monkeys M10 and M11) is the number of multiunit spike rate measurements taken from the same neural channel; $\Delta t$=50ms (M9), 67ms (M8), 75ms (M10) or 125ms (M11) is the temporal difference between the two consecutive spike rate measurements taken from the same neural channel; $N_{CH}$=49 (M8), 64 (M9) or 96 (M10 and M11) is the number of neural channels; and $\Delta t_{lwa}$, $\Delta t_{rwa}$, $\Delta t_{lll}$ and $\Delta t_{rll}$ are the temporal offsets for each of the hotspots (for derivation of offsets (see "Calculation of temporal offsets" section). Another class $C_{OTHER}$ representing states other than hotspot initiation events was formed:

$$C_{OTHER} = \left\{ \bar{a}_O \middle| \bar{a}_O(i) = \begin{bmatrix} x_1(t_i) \\ x_1(t_i - \Delta t) \\ \vdots \\ x_1(t_i - (N_{TP}-1)\cdot\Delta t) \\ x_2(t_i) \\ \vdots \\ x_{N_{CH}}(t_i - (N_{TP}-1)\cdot\Delta t) \end{bmatrix} \begin{array}{l} t_i < lwa - \Delta t_{lwa} - 10ms \\ t_i > lwa - \Delta t_{lwa} + 10ms \\ t_i < rwa - \Delta t_{rwa} - 10ms \quad \forall lwa \\ t_i > rwa - \Delta t_{rwa} + 10ms \quad \forall rwa \\ t_i < lll - \Delta t_{lll} - 10ms \quad \forall lll \\ t_i > lll - \Delta t_{lll} + 10ms \quad \forall rll \\ t_i < rll - \Delta t_{rll} - 10ms \\ t_i > rll - \Delta t_{rll} + 10ms \end{array} \right\}$$

where $t_i$ are all times at least 10ms away from all *lwa, rwa, lll* and *rll* events. To limit the computational complexity of the decoder calibration and, therefore, limit the time needed to calibrate the decoder, up to 100 000 ti-s were selected by making random draws from a uniform distribution without repetitions. If less than 100 000 $t_i$-s were available, all $t_i$-s were used for calibration. $C_{lwa}$, $C_{rwa}$, $C_{lll}$, $C_{rll}$ and $C_{OTHER}$ were used to calibrate a multiclass regularized linear discriminant analysis (rLDA) **(3,84)** decoding model. We used previously described procedures to implement the regularization **(3,79,80),** using a regularization parameter value of 0.4.

Calculation of temporal offsets

It was sought to deliver the stimulation protocols that reinforced the hotspots of spinal motor neuron activity throughout the times at which the monkeys attempted to initiate movements that correspond to those hotspots. It was hypothesized that these times can be decoded by thresholding the probability of observing motor cortical neuronal activity that corresponds to hotspot initiation events at 0.8. However, the motor state probabilities often crossed the probability threshold before the motor state actually occurred, which was closer to the probability maximum.

To account for all of these latencies, offsets specific were derived to each hotspot initiation event, $\Delta t_{lwa}$, $\Delta t_{rwa}$, $\Delta t_{lll}$ and $\Delta t_{rll}$ as follows. First, the decoder was calibrated with all offsets set to 0 ms. Then, the probability of every feature vector $F(t)$ in the calibration dataset to belong to one of $C_{lwa}$, $C_{rwa}$, $C_{lll}$ and $C_{rll}$ classes was calculated using the decoder. When one of these hotspot event probabilities crossed a threshold of 0.8, that hotspot event was detected - marked as $e_{lwa}$, $e_{rwa}$, $e_{lll}$ and $e_{rll}$, respectively. On detection, the same hotspot event could not be detected as the next event for the following one second. Then, the median difference was calculated between the decoded and corrected hotspot initiation events, $\Delta\hat{t}_{lwa}$, $\Delta\hat{t}_{rwa}$, $\Delta\hat{t}_{lll}$ and $\Delta\hat{t}_{rll}$.

$$\Delta\hat{t}_{lwa} = M(lwa - e_{lwa}) \qquad \Delta\hat{t}_{rwa} = M(rwa - e_{rwa}) \qquad \Delta\hat{t}_{lll} = M(lll - e_{lll}) \qquad \Delta\hat{t}_{rll} = M(rll - e_{rll})$$

where *M* is the median operator. Only the closest estimated event to the gait event of the same type was taken into account. The median value was used to exclude type I and type II errors

from affecting the estimate of the offsets. Then, $\Delta\hat{t}_{lwa}$, $\Delta\hat{t}_{rwa}$, $\Delta\hat{t}_{lll}$ and $\Delta\hat{t}_{rll}$ were added to $\Delta t_{lwa}$, $\Delta t_{rwa}$, $\Delta t_{lll}$ and $\Delta t_{rll}$, respectively, and the decoder was calibrated with the new temporal offsets.

We This procedure was repeated until each of $\Delta\hat{t}_{lwa}$, $\Delta\hat{t}_{rwa}$, $\Delta\hat{t}_{lll}$ and $\Delta\hat{t}_{lll}$ was between -5ms and 5ms.

Calibration of decoders robust to the influence of stimulation on the neuronal activity

Once calibrated, the decoder was loaded into the real-time data processing and decoding software application running on the control computer. Then, it was possible to apply the brain-spine interface - neuroprosthetic system that triggered spinal stimulation on detection of hotspot initiation events from neural activity - to monkeys M8-M11. However, the spinal cord stimulation affected the motor cortical neuronal activity, thereby deteriorating the accuracy of brain-controlled spinal stimulation. To improve the robustness of the decoder to the effects of stimulation, the previously developed two-step decoder calibration technique (3) was adapted. This technique uses the "first-step" decoder calibrated using the data recorded without the use of stimulation to generate the dataset where neural data is affected with the accurately delivered stimulation. The goal was to synchronize the delivery of six stimulation protocols, each designed to reinforce one of left and right weight acceptance, propulsion and leg lift motor neuron activity hotspots. When delivered accurately, at least one of the stimulation protocols would be in effect throughout the majority of the gait cycle **(Fig. 19a).** Therefore, as the monkeys walk using the brain-spine interface to trigger all stimulation protocols, neuronal activity throughout the whole gait cycle would be affected by the stimulation. This would deteriorate the decoding accuracy and, in turn, desynchronize the delivery of the stimulation from the hotspots of motor neuron activity leading to suboptimal efficacy of the brain-spine interface **(Fig. 21).** The strategy to overcome this problem was to trigger composite sequences of stimulation protocols that occupy only half of the gait cycle using hotspot initiation events that occur only once in a gait cycle. Since gait cycles exceeded 1s, and less than 0.5s of neuronal data history was used to detect the hotspot events, it was concluded that the neuronal signals corresponding to that hotspot event would not be substantially affected by the stimulation response. First, a corridor dataset was acquired without spinal stimulation and used to calibrate the "first step" decoder as described above. Then, two additional corridor datasets were acquired, in which the first step decoder sparsely triggered composite stimulation sequences. In the first dataset, the decoder used only the detection of left weight acceptance hotpot events to trigger left composite stimulation sequence composed of left weight acceptance, left propulsion and right leg lift stimulation protocols. The second dataset used only the detection of right weight hotspot events to trigger the right composite stimulation sequence composed of right weight acceptance, right propulsion and left leg lift stimulation protocols. The protocols in each composite sequence were arranged to overlap according to the spatiotemporal map of motor neuron activity. As the composite sequences lasted for less than half of the gait cycle, the sparse once-per-gait-cycle triggering prevented the stimulation from influencing the neural activity used to detect the hotspot events and, thereby, preserved accurate decoding **(Fig. 19b).** These two datasets were combined with the original dataset recorded without stimulation and used altogether to calibrate the "second step" decoder. This decoder was then used to enable the brain-spine interface. Since calibrated on neural activity both affected and unaffected by stimulation, the second step decoder maintained its accuracy in both presence and absence of stimulation **(Fig. 19b).**

Temporal accuracy of the decoders

An ideal asynchronous neural decoder will use the neural activity to decode behavioural events that perfectly coincide with the actual behavioural events. However, due to variability of neural signals, finite sampling of calibration data and recording noise, behavioural events decoded even by a very accurate decoder will deviate in time from the actual events. Nonetheless, events decoded within an accepted tolerance window around the actual event are still useful for BCI applications. This acceptable tolerance will depend on the specific BCI application. Therefore, the effectiveness of an asynchronous decoder was measured by counting the proportion of actual gait events (left and right foot off and foot strike events) that had one and only one decoded event of the same type within the tolerance window of $\pm$ 200 ms.

**Delivery of EES protocols in control conditions**

Random delivery of EES protocols

Following the tuning and the testing of the BSI, a control that tested potential benefits of randomly delivered EES protocols was performed as follows. First, a decoder that inaccurately detected hotspot initiation events was calibrated, but maintained a similar detection frequency. Monkeys then walked across the corridor as this inaccurate decoder was used to trigger delivery of the same EES protocols used for BSI testing. Then, trials were identified that had the normalized mutual information between the actual and detected hotspot initiation events below 0.2 using a $\pm$200 ms tolerance window **(80).** These trials were classified as trials with random EES delivery.

Tuning of parameters of the continuous dorsal column stimulation

The amplitude of the continuous dorsal column stimulation in monkey M8 was tuned as follows. The monkey walked across the corridor as the continuous stimulation at 250Hz was initiated using the most rostral spinal array electrode positioned over the dorsal column at an amplitude of 1V. Then, the stimulation amplitude was modified to improve the locomotor behaviour of the monkey on the following crossing: the amplitude was increased if little or no effect was observed on the monkeys' EMG activity or gait, and decreased if the stimulation caused spastic movements or impeded the gait of the monkey in any other way. This procedure was repeated until it was possible to find an amplitude that caused modest spasticity and an amplitude that caused modest improvements. Then, a range of amplitudes was repeatedly tested between these two values during single crossings to determine the range of amplitudes that optimally improved the gait and balance of a monkey. In M8, this range spanned between 6.2V and 6.8V. Then, three amplitude values, 6.2V, 6.5V and 6.8V were tested in repeated crossings. The same procedure was used to tune the 500Hz stimulation and the same range of amplitudes, 6.2V to 6.8V, were obtained. Therefore, the same amplitude values were used to test the efficacy of the stimulation (6.2V, 6.5V and 6.8V). Post-hoc analysis revealed no differences of stimulation effects on muscle activity and kinematics between these three amplitudes. In M9, the same tuning procedure was used, but it was not possible to observe clear improvements in gait or balance of the monkey for any of the tested amplitudes for both 250Hz and 500Hz. We then chose to use the stimulation amplitude of 0.4V to demonstrate the effects of the continuous dorsal column stimulation. This amplitude at both 250Hz and 500Hz showed clear muscle responses, but did not cause any visible spastic movements or seem to impede the gait of the monkey in any way. Stimulation at a next possible higher amplitude of 0.5V caused clear spastic leg movements that impeded the gait of the monkey, while the stimulation at a next possible lower amplitude of 0.3V showed little or no stimulation-induced muscle activity. Therefore, it was chosen not to test the effects of the continuous dorsal column stimulation during repeated crossings at amplitudes other than 0.4V.

## Medical imaging of P1 STIMO-PARK participant

### Structural MRI scan

P1 underwent a structural MRI scan of the thoracolumbar (T10-L5) spine on a 1.5 T system (Magnetom Sola, Siemens Healthineers) with 16-channel body and 32-channel spine array coils. Shimming was used to correct for magnetic field inhomogeneities. Two 3D T2-weighted steady-state gradient-echo sequences were acquired with the following parameters: a) coronal True Fast Imaging with Steady state Precession (TrueFISP; Siemens Healthineers): repetition time (TR), 9.59 msec; echo time (TE), 4.80 msec; number of signal averages, 1; flip angle, 45 degrees; bandwidth, 566 Hz/pixel; field of view, 240 × 270 mm2; acquisition matrix, 384 × 216 pixels; section thickness, 0.6 mm; reconstructed voxel size, 0.6 × 0.6 × 0.6 mm3 b) sagittal Constructive Interference in Steady State (CISS, Siemens Healthineers): TR, 10.46 msec; TE, 5.23 msec; number of signal averages, 1; flip angle, 70 degrees; bandwidth, 211 Hz/pixel; field of view, 300 × 300 mm2; acquisition matrix, 320 × 320 pixels; section thickness, 0.9 mm; reconstructed voxel size, 0.9 × 0.9 × 0.9 mm3. The acquisition time was 17 min 10 sec and 7 min 09 sec for TrueFISP and CISS sequences, respectively.

### CT scan of the torso

Computed tomography (CT) of the torso with the pelvis was performed on a 256-detector row CT system (Revolution Apex, GE Healthcare) using the following data acquisition parameters: tube potential, 120 kVp; tube current, 133-201 mA, by enabling automatic tube current modulation; gantry revolution time, 0.35 sec; beam collimation, 128 × 0.625 mm; pitch, 0.508. Images were reconstructed with a field of view of ~175 × 175 and 350 × 350 mm2 for the spine and pelvis, respectively; at a section thickness/interval of 1.25/1.25 mm, with both smooth (Standard) and sharp (Bone plus) convolution kernels and a deep learning image reconstruction algorithm (TrueFidelity, GE Healthcare) for image noise reduction. The nominal voxel size was 0.34 × 0.34 × 1.25 and 0.68 × 0.68 × 1.25 mm3 for the spine and pelvis, respectively.

## Data processing and analysis

### Deriving spatiotemporal maps of motor neuron activity in NHPs

Spatiotemporal maps of motor neuron activity were constructed by projecting muscle activity into the spinal segments according to spatial distribution of motor pools. Contributions of each muscle were combined to compute the total activity at each spinal segment. The motor output pattern of each spinal segment $S_i$ was estimated by the following equation:

$$S_i = \frac{\sum_{j=1}^{n_i} MN_{ij} EMG_j}{\sum_{j=1}^{n_i} MN_{ij}}$$

where $EMG_j$ represents the normalized electromyography envelope of $j$-th muscle, $n_i$ is the number of $EMG_j$-s corresponding to the $i$-th segment, and $MN_{ij}$ is the number of motor neurons of $j$-th muscle in the $i$-th segment. The previously constructed library of distributions of the motor neurons across the lumbar spinal cord segments of different muscles **(3,36)** was used to derive $MN_{ij}$-s. Maps of motor neuron activity during the experimental sessions were computed using electromyography envelopes normalized to their 95th percentile over the whole sessions. Maps of motor neuron activity during single-pulse stimulation were derived from the peak-to-peak electromyography response and normalized by the maximal response for each muscle.

Deriving spatiotemporal spinal maps of motor neuron activity in human subjects

The same approach as in NHPs was used while considering left and right hip flexors (Il, RF), knee extensors (VLat, RF), ankle flexors (TA) and ankle extensors (MG). Separate left and right spatiotemporal spinal maps were generated based on activity of muscles on that side. $MN_{i,j}$ was approximated by the ratio of motoneurons innervating the muscle $j$ and present in the spinal segment $i,$ with respect to all motoneurons innervating the muscle $j,$ as derived from electrophysiological measures in a large population of patients undergoing surgery **(Supplementary Table 5).**

Calculation of gait parameters in NHPs

Before the recording sessions, the anatomical landmarks of monkeys' legs were painted using reflective paint. Videos of monkeys were recorded as they walked across corridor or ladder during the sessions using a 4-6 camera SIMI system (SIMI Motion Gmbh, Germany). After the session, the SIMI Motion software was used to reconstruct the 3D positions of each of the painted joints. The derived kinematics were rotated to align the x-axis with the forward movement of the monkey along the course; and to align x-y plane with the corridor or ladder surface. A custom-developed Matlab program was used to identify the foot off and foot strike events of both legs through visual inspection of the frames. Then, a custom Matlab program was used to calculate 83 gait parameters from each step by combining the derived 3D kinematics and the identified gait events (the list of gait parameters is provided in detail in Supplementary Table 2 below). Steps were defined as movement starting and ending with a foot strike event. Gait parameters of left and right steps were merged in a single dataset.

Analysis of gait kinematics using principal component analysis (PCA) in NHPs

Execution of locomotor tasks involves the repetition of stereotypical patterns of leg movements. However, legs move with numerous degrees of freedom, which makes the characterization of gait difficult. In contrast, efficacy of a given therapy aiming to improve leg movements can be faithfully illustrated only by using a small number of meaningful parameters that consolidate the full complexity of locomotor patterns. Here, 83 variables were that quantified kinematic features of locomotor patterns (see Supplementary Table 2 below). These variables were assigned to groups according to their relationship to the locomotor patterns. The groups include Gait cycle timing, Step dimensions, Endpoint velocity, Balance, Forward-Backward oscillations, Joint angles, Oscillatory amplitude, Oscillatory speed, Coordination and Reproducibility. To consolidate the dataset into a small number of parameters relevant to PD, principal component analysis (PCA) was used - a linear transformation of the dataset into a coordinate system where each new added axis maximizes the variance. The dataset was arranged in a matrix with 83 variables as the matrix columns and each row representing one gait cycle (one observation). Data collected from different conditions and different monkeys (where applicable) were pooled together in a single matrix and z-scored across columns. Two or three principal components were typically sufficient to account for more than 50% of the total variance of observations, demonstrating the high correlation between therapy-induced changes of sets of variables. To visualize the differences between conditions and monkeys (where applicable), the gait cycles of all the conditions and monkeys were illustrated in the new space created by the first two or three principal components by plotting "balloons" - ellipsoids with the center and principal semi-axis as the mean and standard deviation calculated across all the gait cycles for that condition and monkey. In this representation, the distance increases with the difference between the underlying locomotor patterns, allowing to readily visualize differences of locomotor patterns between conditions. PCA was also used to identify the sets of variables that vary together to account for the differences between conditions. This is done using the factor loadings - the correlation between each variable and each principal component axis. The result of this analysis is displayed using a color-coded representation of correlation values of all the variables with up to three leading principal components **(Figs. 10c, 14** and **24).** In this representation, variables that together strongly correlate (either positively or negatively) with a given principal component form variable sets that account for a specific difference between conditions.

Calculation of crossing time and steady speed in NHPs

The aggregate capacity of a monkey to walk as the time monkeys took to cross the central portion of the corridor or ladder was calculated. To this end, the coordinate system across recordings performed on different days was coregistered to a single coordinate system. The origin of this system was in the same place on the corridor or a ladder with the y axis in the middle direction is along the middle line of the corridor/ladder. This was achieved by marking 4 screws in the left top edge and right top edge of the corridor or ladder cage, respectively. Then, left edge screws $S_{li}(x_{li}, y_{li}, z_{li})$ were linearly regressed in the XY plane:

$$a_l x_{li} + b_l = y_{li} \,, i = 1,2,3,4$$

The least squares method was used to obtain:

$$\begin{bmatrix} a_l \\ b_l \end{bmatrix} = (A^T A)^{-1} A^T B$$

Where

$$A = \begin{bmatrix} x_{l1} & 1 \\ x_{l2} & 1 \\ x_{l3} & 1 \\ x_{l4} & 1 \end{bmatrix} , B = \begin{bmatrix} y_{l1} \\ y_{l2} \\ y_{l3} \\ y_{l4} \end{bmatrix}$$

The direction vector of the fitted line is:

$$\vec{d_l} = (1, a_l)$$

The rough direction vector of left edge in XY plane is:

$$\vec{r_l} = (x_{l4} - x_{l1}, y_{l4} - y_{l1})$$

Then, the unit direction vector of left edge was obtained:

$$\vec{n_l} = sgn(\vec{d_l} \cdot \vec{r_l}) \cdot \frac{\vec{d_l}}{|\vec{d_l}|}$$

Using the same procedure, the unit direction vector of right edge $\vec{n_r}$ was obtained. The y-axis unit vector was defined as the bisector of two edges:

$$\vec{n_y} = \frac{\vec{n_l} + \vec{n_r}}{|\vec{n_l} + \vec{n_r}|} = (x_n, y_n)$$

Therefore, the unit vectors of the standard coordinate system are:

$$\hat{i'} = \begin{bmatrix} y_n \\ -x_n \\ 0 \end{bmatrix}, \hat{j'} = \begin{bmatrix} x_n \\ y_n \\ 0 \end{bmatrix}, \hat{k'} = \begin{bmatrix} 0 \\ 0 \\ 1 \end{bmatrix}$$

Then, the rotation matrix was calculated from the coordinate system used to record the kinematics to the standard coordinate system as:

$$RM = inv \begin{bmatrix} y_n & -x_n & 0 \\ x_n & y_n & 0 \\ 0 & 0 & 1 \end{bmatrix}$$

To synchronize the origin of coordinate system, the center of these four screws in the X-Y plane was calculated:

$$c_x = \frac{1}{8} \sum_{i=1}^{4} (x_{li} + x_{ri})$$

$$c_y = \frac{1}{8}\sum_{i=1}^{4}(y_{li} + y_{ri})$$

The translation matrix is then:

$$TM = \begin{bmatrix} -c_x & -c_y & 0 \end{bmatrix}$$

Thus, the full transformation from the coordinate system used to record the kinematics to the standard coordinate system is:

$$(x' \quad y' \quad z') = (x \quad y \quad z) \cdot RM + TM$$

Then, the position of the left crest joint was marked at the start and end points of steady walking in each corridor or ladder crossing, and transformed them in the standard coordinate system. These points were used to identify the furthest point in the corridor/ladder where the monkey starts the steady walk, and the closest point where the monkey stops to walk steadily. Clear outliers were removed from this analysis. The space between these two points along the y-axis, common closest and furthest points of steady walk, defined the central portion of the corridor/ladder.

The full left crest joint kinematics transformed into the standard coordinate system were used to find times when the monkey crossed into this central portion. The time monkeys spent in this central portion during one crossing was identified as the crossing time. The steady speed was calculated as the length of the central portion along the y axis divided by the crossing time.

Reconstruction of body posture in NHPs

A whole-body CT scan was employed to generate a 3D model of the rhesus macaque skeleton and biomechanics. A control rig (Maya, Autodesk Inc., USA) comprised of 34 bones that accurately modelled whole-body posture was created. This skeleton allowed to animate and reproduce the behavior of the monkeys in 3D. The skeleton was mapped onto the whole-body kinematics recorded during behavioral experiments, and the corresponding mesh was rendered to obtain a high-fidelity replica of body posture at specific times of gait. The spinal curvature was computed from this model using the coordinates of twelve points in the sagittal plane distributed along the spine of the modelled skeleton. For each triplet of points <a,b,c>, the radius of the circumscribed circle that passes through these points was computed according to:

$$R = \left\| \frac{a^2 \cdot \big((b - a) \times (c - a)\big) \times (b - a)}{\|((b - a) \times (c - a))\|} \right\|$$

For the 12 points along the spine, this gave 10 values representing local curvature. The total spine curvature was summarized by taking the root-mean-square value of these calculations.

Analysis of neuronal population dynamics using canonical correlation analysis

To visualize and quantify putative changes in population dynamics before and after MPTP administration, canonical correlation analysis (CCA) was applied on all the identified neurons (85). Briefly, CCA aims to find linear transformations that maximize the correlation between neural dynamics in two low-dimensional neural manifolds (63). Here, the CCA was applied on manifolds describing neural activity pre-MPTP administration and post-MPTP administration: $\mathbf{L}_{pre}$ and $\mathbf{L}_{post}$. CCA is based on QR decomposition of the latent dynamics to obtain:

$$\mathbf{L}_{pre}^{\mathsf{T}} = \mathbf{Q}_{pre}\mathbf{R}_{pre}, \ \mathbf{L}_{post}^{\mathsf{T}} = \mathbf{Q}_{post}\mathbf{R}_{post}$$

Then, a singular value decomposition was on the product of the orthonormal bases $\mathbf{Q}_{pre}$ and $\mathbf{Q}_{post}$ given by the above QR decomposition to find the matrices:

$$\mathbf{Q}_{pre}^{\mathsf{T}}\mathbf{Q}_{post} = \mathbf{U}\,\mathbf{S}\,\mathbf{V}^{\mathsf{T}}$$

The matrices U and V are used to construct the mappings onto a new manifold that maximizes the pairwise correlations between each dimension of Pre-MPTP and Post-MPTP data.

$$\mathbf{M}_{pre} = \mathbf{R}_{pre}^{-1}\mathbf{U}, \qquad \mathbf{M}_{post} = \mathbf{R}_{post}^{-1}\mathbf{V}$$

To compute the canonical correlations, the neural dynamics $\mathbf{L}_{pre}$ and $\mathbf{L}_{post}$ were projected onto these "aligned" manifold axes and the pairwise correlation values were computed for each dimension. The resulting values are necessarily ordered from largest to smallest as a result of the singular value decomposition. To ensure that correlation values could not be explained trivially by the cyclical motor output, this procedure was repeated using the envelopes of the electromyography signals from the eight recorded leg muscles. To estimate the lower bound of the correlation values, neural trajectories recorded pre- and post-MPTP administration were shuffled and the same analysis was applied.

Additionally and or alternatively, the above disclosure may be combined with the following concept of neuromodulation of the lateral hypothalamus:

COMBINING SPINAL EPIDURAL ELECTRICAL STIMULATION WITH ELECTRICAL STIMULATION OF THE LATERAL HYPOTHALAMUS TO ALLEVIATE LOCOMOTOR DEFICITS OF SPINAL CORD INJURY

Problems or disadvantages overcome by the concept

Despite decades of research, there is currently no cure for motor deficits after spinal cord injury (SCI). Aside from the physical and psychological toll on individuals with SCI, the costs to society as a result of SCI are astronomical with one study estimating the total annual cost as $14.5 billion in the United States (V. Y. Ma, L. Chan, K. J. Carruthers, Incidence, prevalence, costs, and impact on disability of common conditions requiring rehabilitation in the united states: Stroke, spinal cord injury, traumatic brain injury, multiple sclerosis, osteoarthritis, rheumatoid arthritis, limb loss, and back pa. Arch. Phys. Med. Rehabil. 95, 986-995.e1 (2014)).

The demand for therapies that robustly improve neurologic function has therefore been high, but the various medical therapies that have attempted to improve motor function after SCI over the past several decades in both preclinical and clinical studies have largely failed. Rehabilitative strategies have exploited the remaining rim of tissue around the lesion site and promote improvements in motor function and long-term neuroplasticity through intensive training programs. This has largely been accomplished in combination with epidural electrical stimulation (EES) of the lumbar spinal cord. The administration of EES with robot-assisted will-powered overground training resulted in significant improvements in motor function and neuroplasticity of residual pathways in a rat model of contusion SCI (L. Asboth, L. Friedli, J. Beauparlant, C. Martinez-Gonzalez, S. Anil, E. Rey, L. Baud, G. Pidpruzhnykova, M. A. Anderson, P. Shkorbatova, L. Batti, S. Pagès, J. Kreider, B. L. Schneider, Q. Barraud, G. Courtine, Cortico-reticulo-spinal circuit reorganization enables functional recovery after severe spinal cord contusion. Nat Neurosci. 21, 576-588 (2018)).

In humans, the use of spatiotemporal EES in combination with active rehabilitation restored locomotor movements in three individuals unable to ambulate prior to the trial (F. B. Wagner, J.-B. Mignardot, C. G. L. Goff-Mignardot, R. Demesmaeker, S. Komi, M. Capogrosso, A. Rowald, I. Seáñez, M. Caban, E. Pirondini, M. Vat, L. A. McCracken, R. Heimgartner, I. Fodor, A. Watrin, P. Seguin, E. Paoles, K. V. D. Keybus, G. Eberle, B. Schurch, E. Pralong, F. Becce, J. Prior, N. Buse, R. Buschman, E. Neufeld, N. Kuster, S. Carda, J. von Zitzewitz, V. Delattre, T. Denison, H. Lambert, K. Minassian, J. Bloch, G. Courtine, Targeted neurotechnology restores walking in humans with spinal cord injury. Nature. 563, 65-71 (2018)).

The improvements in motor function mediated by rehabilitative and EES paradigms are encouraging, but there still remains a significant gap between the restored locomotor movements and normal locomotion.

Therefore, there is a need for additional inventions that are capable of further augmenting motor recovery after incomplete SCI. Preclinical and clinical studies showed that EES augments the functional impact of neural signals from residual pathways originating from the brain. This understanding suggested that tapping into the brain circuits associated with these residual pathways may improve locomotor performance after SCI.

There is a paucity of therapies that effectively target the brain after SCI. This lack of treatment options contrasts with evidence that demonstrated the critical role of the brain in locomotor recovery. Previous research has demonstrated that automated training of the lower limbs with EES results in poor functional recovery if the brain is not participating in the training: the active and motivated participation in the rehabilitative training process is essential to mediate neuroplasticity of residual descending pathways (R. van den Brand, J. Heutschi, Q. Barraud, J. DiGiovanna, K. Bartholdi, M. Huerlimann, L. Friedli, I. Vollenweider, E. M. Moraud, S. Duis, N. Dominici, S. Micera, P. Musienko, G. Courtine, Restoring Voluntary Control of Locomotion after Paralyzing Spinal Cord Injury. Science. 336, 1182-1185 (2012)). One area of the brain that has never previously been considered in the context of SCI but has a critical role in locomotion is the lateral hypothalamus (LH). There is a large body of evidence demonstrating that the lateral hypothalamus plays an important role in the production of locomotion and also serves as an interface between motivated behaviour and subsequent locomotor responses (L. H. Kim, S. Sharma, S. A. Sharples, K. A. Mayr, C. H. T. Kwok, P. J. Whelan, Integration of Descending Command Systems for the Generation of Context-Specific Locomotor Behaviors. Front Neurosci-switz. 11, 581 (2017)).

It is therefore a further object of this disclosure to provide an enhanced system and method for neuromodulation and/or neurostimulation system for mitigating locomotor deficits of a human patient, especially in that the system and method allow an enhanced and more accurate treatment of patients suffering neural disorders, especially due to Parkinson's disease, but also other diseases such as stroke, Alzheimer's disease and the like, and who further suffer especially from motor and locomotion disorders.

This object is inter alia solved by the subject-matter of aspect 1 as described below. Accordingly, a system for neuromodulation and/or neurostimulation system for mitigating locomotor deficits a human patient, such locomotor deficits being locomotor deficits of/or neuronal disorders, especially at least one of Parkinson's disease epilepsy, chronic pain (like (cluster) headache or migraine), depression, Alzheimer's disease, obsessive compulsive disorders, and obesity, at least comprising a deep brain neuromodulation element, which is capable to be arranged at the region of the hypothalamus.

The system is inter alia based on the general idea and scientific finding that by stimulating the region of the hypothalamus of the brain of a human patient significant improvement of locomotor deficits, but also other function of the human body can be reached.

Such further functions can be inter alia autonomic function or the like.

It is possible to combine the system with a closed-loop integration of deep brain stimulation and epidural electrical spinal cord stimulation to activate the sympathetic and motor systems in neurodegenerative disorders.

In particular, Individuals with neurodegenerative disorders, including but not limited to: Parkinson's disease, multiple system atrophy, pure autonomic failure, amyotrophic lateral sclerosis, and multiple sclerosis suffer from motor impairments and autonomic dysfunctions. Motor impairments may include parkinsonian features such as rigidity and bradykinesia, while autonomic impairments include orthostatic hypotension and urinary dysfunction. Treating these motor and autonomic complications in tandem is extremely difficult, if not impossible, using current pharmacological approaches. Consequently, these individuals are left in limbo, in a balance between attempting to control motor complications, while attempting to not worsen autonomic control.

In this regard, the inventors propose an system that is capable to and enables joint treatment of motor symptoms and autonomic symptoms. This system uses a combination of deep brain stimulation targeting motor areas involved in parkinsonian features and epidural electrical stimulation of the thoracic, lumbar, or sacral spinal cord. These stimuli are integrated through a smart, closed-loop approach that enables constant titration of deep brain stimulation or epidural electrical stimulation patterns, depending on the requirements of the person. For example, one can imagine at least these two different scenarios:

The individual (patient) wants to stand up and walk. This will require constant titration of stimulation amplitudes for both deep brain stimulation and epidural stimulation, in order to maintain blood pressure and prevent orthostatic hypotension, while also enabling the required reduction in rigidity to walk.

The individual (patient) wants to sit-up and watch television. In this situation the individual may not require constant adjustment to their deep brain stimulation, but will require constant epidural electrical stimulation to avoid episodes of orthostatic hypotension.

The integration of these two systems, along with sensors for motor and autonomic features, is critical to maintain tandem treatment of both conditions.

Supporting evidence: The inventors recently implanted one individual with multiple system atrophy. She received both deep brain stimulation in the subthalamic nucleus as well as epidural electrical stimulation over the lower thoracic spinal cord segments. The inventors have programmed stimulation patterns that adjust with the position of her body, to avoid orthostasis events. Deep brain stimulation reduces the need for pharmacological management, which interferes with autonomic control. This patient was bedridden only a few months ago. She can now stand-up, walk around, and even dance with her husband. So, a system is provided that automates the process of parameter optimization and electrode configuration for epidural electrical stimulation of the spinal cord targeting the autonomic circuitry, and integrates this with stimulation patterns required for deep brain stimulation to alleviate motor symptoms in individuals with neurodegenerative diseases. A joint use of these technologies as well as the algorithms required to enable their multi-modal treatment of neurodegeneration is described.

Especially, the region is the lateral hypothalamus. This region has been found to be very effective for a neuromodulation with the aim of mitigating locomotor deficits of a human patient, see explanations and clinical data below.

The system may further comprise a current and/or pulse generator, especially wherein the current and/or pulse generator is an implanted current and/or pulse generator.

Furthermore, it is possible that the system further comprises a neuromodulation module, which is capable to provide EES to the spinal cord, especially at lumbar level of the spinal cord and/or at lumbosacral level of the spinal cord. This region has been shown and proven in clinical results that neuromodulation has advantageous effects, see the below clinical data.

Additionally it is possible that the system further comprises a cortical neurosensor. This sensor allows also a closed-loop procedure. Furthermore, it helps to get more input signals such that the neuromodulation or neurostimulation can be adjusted.

It is possible that the system further comprises at least one muscle activity sensor. Such sensor can sense the muscle activity, which can then provide signal input to assess the effect of the neuromodulation. This way, a further adjustment possibility can be provided for the neuromodulation delivered to the patient.

Moreover it is possible that the system further comprises at least one behavioral sensor. For example, these sensors send the signals to one or more controllers by wired or wireless links. The controllers may process the acquired signals, input them in the decoding algorithms that infer person's movement intentions from the input signals, and, based on the inferred movement intentions, send the commands to one or more communicators. The communicators may relay the commands to the implanted current generators that deliver the spinal epidural electrical stimulation as neuromodulation for the spinal cord.

The system may further comprise at least one controller and/or communicator.

It is possible that the system is capable to provide EES neuromodulation at a stimulation frequency of approx. 40 Hz.

It is possible that the system is capable to provide EES neuromodulation at a pulse width of approx. 0.2 ms.

It is possible that the system is capable to provide EES neuromodulation at an amplitude range of 50-300 uA.

It is possible that the system is capable to provide LH-DBS neuromodulation at a frequency of approx. 50 Hz.

It is possible that the system is capable to provide LH-DBS neuromodulation at a pulse width of approx. 0.2 ms.

It is possible that the system is capable to provide LH-DBS neuromodulation at an amplitude range of approx. 85-210 uA.

Further parameters can be for example:

- Frequency range - 10Hz - 260 Hz, preferred 20 Hz and 180 Hz;

- Pulse width range - 10 - 300 us;

- Amplitude range - 0.1 mA to 25mA - set based on patient feeling;

- Electrode configurations (monopolar, multipolar and directional);

- Preferred parameters for patient 1: 20 Hz or 180 Hz.

The present disclosure also relates to a method for neuromodulation. Accordingly, a method for neuromodulation and/or neurostimulation system for mitigating locomotor deficits of a human patient is provided, at least comprising the step of providing deep brain neuromodulation at the region of the hypothalamus.

Especially it is possible that the region is the lateral hypothalamus.

The method can be performed with the system as described and disclosed in this whole disclosure (as described above and below).

Further details and advantages shall now be described in connection with additional drawings.

It is shown in:

**Fig. 33**     the effect of DBS of the LH alone and in combination with EES restores walking after contusion SCI;

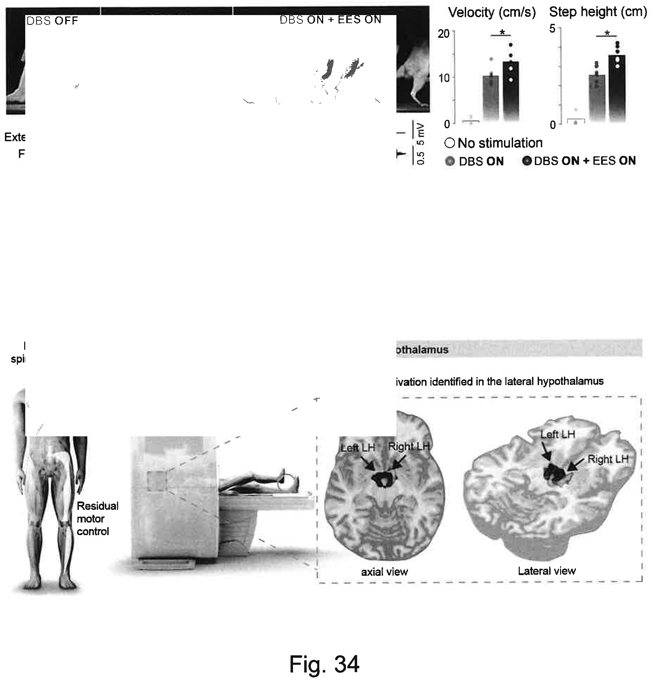

**Fig. 34**     functional MRI to determine peak activation areas in the deep brain region Left panel;

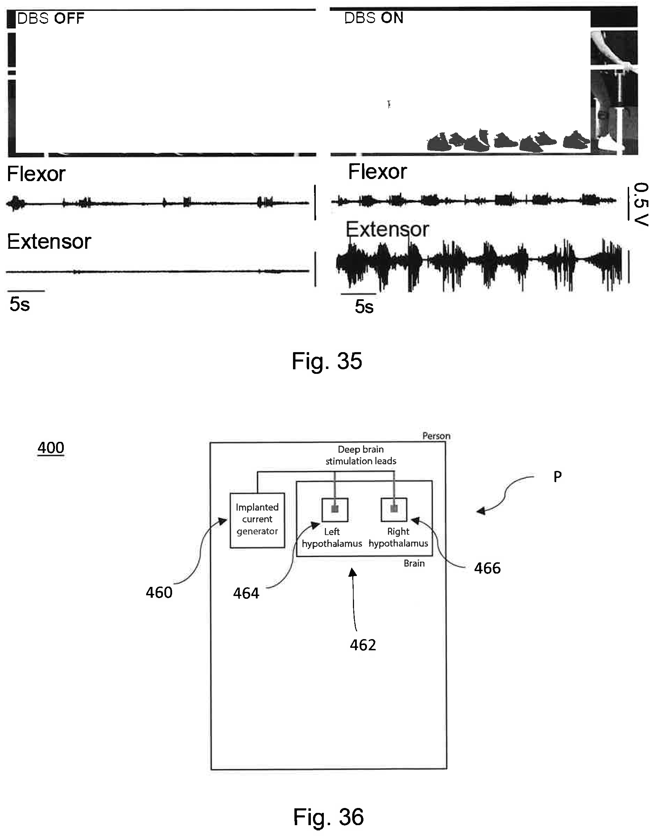

**Fig. 35**     lateral hypothalamus stimulation (DBS) promotes walking in clinical trial participant suffering from gait deficits secondary to spinal cord injury;

**Fig. 36**     an embodiment for a system to provide neuromodulation inter alia for the LH;

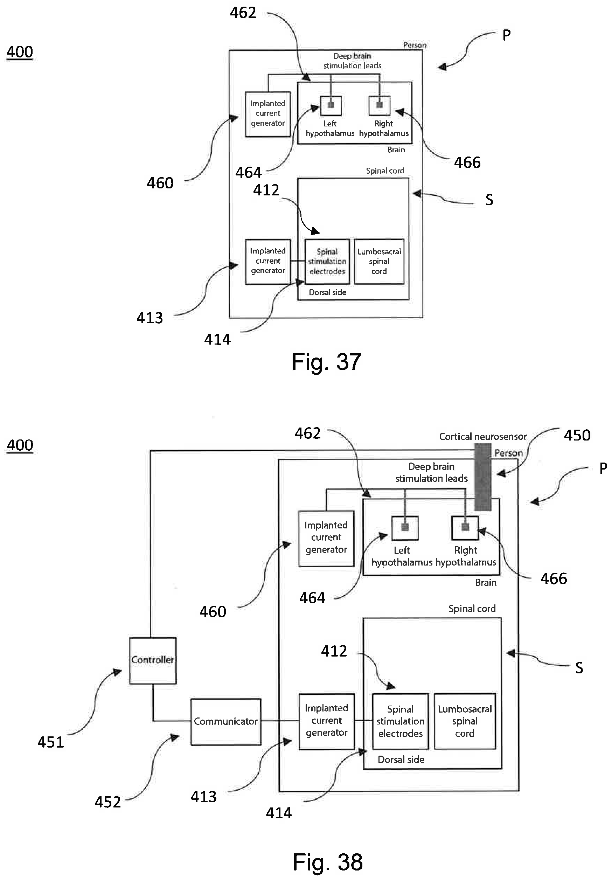

**Fig. 37**     a further embodiment for a system to provide neuromodulation inter alia for the LH;

**Fig. 38**     a further embodiment for a system to provide neuromodulation inter alia for the LH;

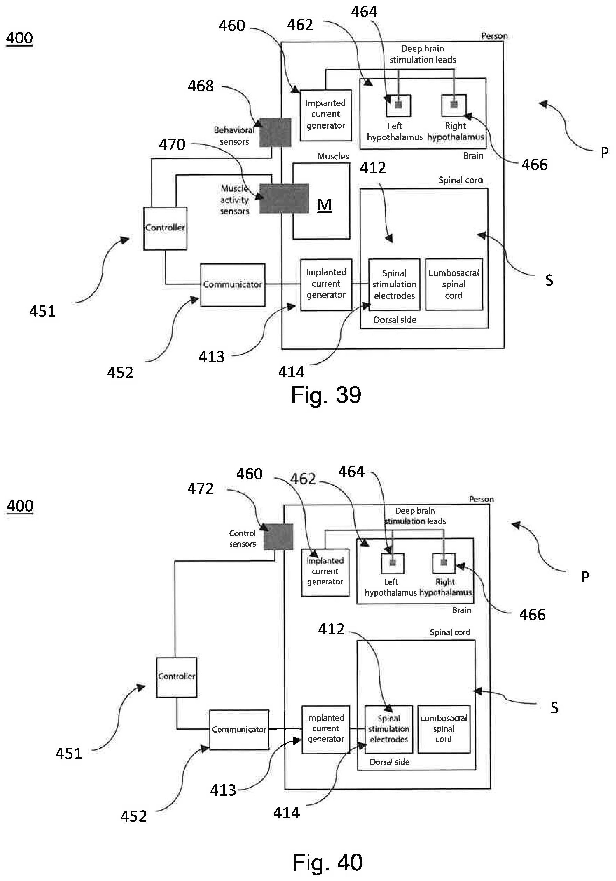

**Fig. 39** a further embodiment for a system to provide neuromodulation inter alia for the LH; and

**Fig. 40** a further embodiment for a system to provide neuromodulation inter alia for the LH.

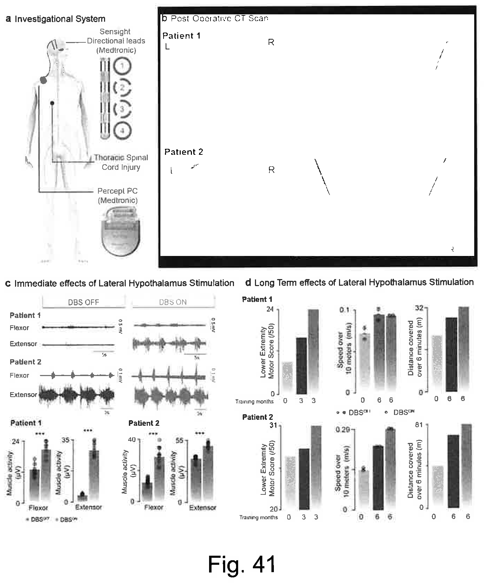

**Fig. 41** overview of a clinical trial on two human subjects with incomplete SCI, showing that DBS of the lateral hypothalamus enhances recovery of motor functions. In detail: **41a.** Hardware components used for DBS in patients with incomplete SCI. **41b.** Post-operative imaging confirmed correct positioning of electrodes in the lateral hypothalamus. **41c.** Both patients recovered walking capacities immediately after implantation while DBS of the lateral hypothalamus is turned on. **41d.** Moreover, patients recovered lower extremity motor score (LEMS), and progressed on the 10-meter walk test and 6-minute walk test after a few months of DBS-supported rehabilitation.

The lateral hypothalamus (LH) has largely been ignored in clinical discussions surrounding locomotion, even though this nucleus has been demonstrated to play a critical role in the production of natural locomotion in preclinical studies for decades. Indeed, based on electrical stimulation studies, the descending control of locomotion has historically been attributed to three key regions conserved across vertebrate species: the subthalamic locomotor region (SLR) which includes the LH, the mesencephalic locomotor region (MLR), and the medullary reticular formation. While the MLR and medullary reticular formation have been investigated in SCI, the LH has surprisingly largely been ignored. In one study, MLR stimulation resulted in an improvement in quadrupedal locomotor function after SCI in rats. These preclinical data motivated a clinical trial that aims to stimulate the MLR of humans with SCI to improve locomotor recovery (University of Zurich, ClinicalTrials.gov ID: NCT03053791) (L. C. Bachmann, A. Matis, N. T. Lindau, P. Felder, M. Gullo, M. E. Schwab, Deep brain stimulation of the midbrain locomotor region improves paretic hindlimb function after spinal cord injury in rats. Sci Transl Med. 5, 208ra146 (2013)). However, data from the laboratory of the inventors demonstrates that MLR stimulation in rats undergoing rehabilitation after SCI is stressful for the animal, casting doubts that this target is viable for long-term rehabilitation studies in humans (M. Bonizzato, N. D. James, G. Pidpruzhnykova, N. Pavlova, P. Shkorbatova, L. Baud, C. Martinez-Gonzalez, J. W. Squair, J. DiGiovanna, Q. Barraud, S. Micera, G. Courtine, Multi-pronged neuromodulation intervention engages the residual motor circuitry to facilitate walking in a rat model of spinal cord injury. Nat Commun. 12, 1925 (2021)). The inventors were able to reduce this stress by using a sophisticated brain-MLR interface in which MLR stimulation was guided by motor intention of the rat that we decoded from primary motor cortex activity.

However, the inventors argue that the functional benefits do not outweigh the complexity and invasiveness of the underlying technology and approach. Moreover, the anatomical location of MLR in the midbrain increases the risk for significant neurological morbidity and even death following the implantation of DBS electrodes (M.-L. Welter, A. Demain, C. Ewenczyk, V. Czernecki, B. Lau, A. E. Helou, H. Belaid, J. Yelnik, C. François, E. Bardinet, C. Karachi, D. Grabli, PPNa-DBS for gait and balance disorders in Parkinson's disease: a double-blind, randomised study. J Neurol. 262, 1515-1525 (2015)). In one double-blind randomized study evaluating the functional benefits of DBS delivered in the PPN (pedunculopontine nucleus; a part of the MLR) for patients with Parkinson's disease, one patient was excluded because of a midbrain haemorrhage that required life support in the intensive care unit and ultimately left the patient wheel-chair bound, anarthric, and requiring a gastrostomy feeding tube (M.-L. Welter, A. Demain, C. Ewenczyk, V. Czernecki, B. Lau, A. E. Helou, H. Belaid, J. Yelnik, C. François, E. Bardinet, C. Karachi, D. Grabli, PPNa-DBS for gait and balance disorders in Parkinson's disease: a double-blind, randomised study. J Neurol. 262, 1515-1525 (2015)). Clinically, the risk of catastrophic outcomes after haemorrhage in the midbrain and other areas of the brainstem including the pons and medulla is especially high given the critical involvement of these regions in respiratory and cardiac function and motor function as the corticospinal tract from the motor cortex to the spinal cord traverses these regions (K. Ikeda, K. Kawakami, H. Onimaru, Y. Okada, S. Yokota, N. Koshiya, Y. Oku, M. Iizuka, H. Koizumi, The respiratory control mechanisms in the brainstem and spinal cord: integrative views of the neuroanatomy and neurophysiology. J Physiological Sci. 67, 45-62 (2017); R. A. L. Dampney, Central neural control of the cardiovascular system: current perspectives. Adv Physiol Educ. 40, 283-296 (2016)). The benefit of implanting the MLR would have to be very high to justify this risk in patients with SCI, given that chronic SCI is not an immediately life-threatening condition and that improvement of gait is more related to an improvement in quality of life as opposed to survival. Finally, the MLR is poorly defined anatomically, which increases the chance that the therapy may not be consistently effective for SCI patients as has been seen with the mixed results of PPN deep brain stimulation for Parkinson's disease (M.-L. Welter, A. Demain, C. Ewenczyk, V. Czernecki, B. Lau, A. E. Helou, H. Belaid, J. Yelnik, C. François, E. Bardinet, C. Karachi, D. Grabli, PPNa-DBS for gait and balance disorders in Parkinson's disease: a double-blind, randomised study. J Neurol. 262, 1515-1525 (2015).; A. Nowacki, S. Galati, J. Ai-Schlaeppi, C. Bassetti, A. Kaelin, C. Pollo, Pedunculopontine nucleus: An integrative view with implications on Deep Brain Stimulation. Neurobiol Dis. 128, 75-85 (2018)). We conclude that there is a clear need to identify safer and potentially more effective targets in the brain to augment locomotor function after SCI. Compared to the MLR, the SLR, which includes the LH, has received little attention (L. C. Bachmann, A. Matis, N. T. Lindau, P. Felder, M. Gullo, M. E. Schwab, Deep brain stimulation of the midbrain locomotor region improves paretic hindlimb function after spinal cord injury in rats. Sci Transl Med. 5, 208ra146 (2013)).

Studies in uninjured anesthetized rats have consistently demonstrated that stimulation of the LH elicits natural locomotor movements (H. M. Sinnamon, Locomotor stepping elicited by electrical stimulation of the hypothalamus persists after lesion of descending fibers of passage. Physiol Behav. 48, 261-266 (1990).; H. M. Sinnamon, C. K. Stopford, Locomotion elicited by lateral hypothalamic stimulation in the anesthetized rat does not require the dorsal midbrain. Brain Res. 402, 78-86 (1987).; H. M. Sinnamon, Preoptic and hypothalamic neurons and the initiation of locomotion in the anesthetized rat. Prog Neurobiol. 41, 323-344 (1993)).

Previous data also suggests that a subset of reticulospinal neurons respond monosynaptically to stimulation of the hypothalamus, which has been supported by anatomic tracing studies (K. V. Baev, V. K. Berezovskii, T. T. Kebkalo, L. A. Savos'kina, Projections of neurons of the hypothalamic locomotor region to some brainstem and spinal cord structures in the cat. Neurophysiology+. 17, 595-600 (1985); G. N. Orlovskii, Connections between reticulospinal neurons and locomotor regions of the brainstem. Neurosci Transl. 4, 58-64 (1970)). This is especially important as it has been shown by the laboratory of the inventors in rats that reticulospinal neurons form an essential bypass circuit from the motor cortex that mediates locomotor recovery after SCI after a clinically relevant spinal cord contusion, which destroys all direct corticospinal projections to the lumbar spinal cord (L. Asboth, L. Friedli, J. Beauparlant, C. Martinez-Gonzalez, S. Anil, E. Rey, L. Baud, G. Pidpruzhnykova, M. A. Anderson, P. Shkorbatova, L. Batti, S. Pagès, J. Kreider, B. L. Schneider, Q. Barraud, G. Courtine, Cortico-reticulo-spinal circuit reorganization enables functional recovery after severe spinal cord contusion. Nat Neurosci. 21, 576-588 (2018)). Another study examined the effect of LH stimulation on lumbar spinal reflex activity and found that LH stimulation increased the amplitude of the monosynaptic discharge, demonstrating that the LH can affect spinal motoneuron excitability (P. R. Miles, W. E. Gladfelter, Lateral hypothalamus and reflex discharges over ventral roots of rat spinal nerves. Physiol Behav. 4, 671-675 (1969)). More recently, studies in mice have demonstrated that the inhibition of GAD65 cells in the LH decreases voluntary locomotion while hyperactivity of these cells results in hyperlocomotion (C. Kosse, C. Schöne, E. Bracey, D. Burdakov, Orexin-driven GAD65 network of the lateral hypothalamus sets physical activity in mice. Proc National Acad Sci. 114, 4525-4530 (2017)).

Previous experiments in cats and monkeys have also demonstrated that stimulation of the hypothalamus can significantly augment movements resulting from stimulation of the motor cortex (J. P. Murphy, E. Gellhorn, THE INFLUENCE OF HYPOTHALAMIC STIMULATION ON CORTICALLY INDUCED MOVEMENTS AND ON ACTION POTENTIALS OF THE CORTEX. J Neurophysiol. 8, 341-364 (1945); R. Rhines, H. W. Magoun, BRAIN STEM FACILITATION OF CORTICAL MOTOR RESPONSE. J Neurophysiol. 9, 219-229 (1946)). For example, Murphy and Gellhorn reported that a monkey otherwise paralyzed from experimental poliomyelitis could move its hindlimbs during states of excitement and rage, which prompted them to study the effect of hypothalamic stimulation on motor function (J. P. Murphy, E. Gellhorn, THE INFLUENCE OF HYPOTHALAMIC STIMULATION ON CORTICALLY INDUCED MOVEMENTS AND ON ACTION POTENTIALS OF THE CORTEX. J Neurophysiol. 8, 341-364 (1945)).

While these studies have been performed in animals with intact spinal cords, these results encouraged the inventors to investigate the effects of LH modulation on locomotor performance after SCI. The inventors delivered electrical stimulation in the lateral hypothalamus of rats that underwent a clinically relevant contusion of the spinal cord. The inventors found that deep brain stimulation (DBS) of the lateral hypothalamus instantly augmented the tonus of the legs, which enabled rats to produce a natural locomotor activity with partial weight bearing. Moreover, this intervention interacted synergistically with EES. The combination of DBS in the lateral hypothalamus and EES over the lumbar spinal cord enables rats with leg paralysis to produce full weight bearing locomotion. The inventors produced these outcomes in all the tested rats to date, and in multiple independent groups of rats, thus showing the robustness of the delivery and efficacy of this treatment paradigm **(Fig. 33)**.

**Fig. 33** shows the effect of DBS of the LH alone and in combination with EES restores walking after contusion SCI. Chronophotography and the left limb EMGs during locomotion showing the recovery walking when DBS is delivered in the LH. This recovery is further potentiated when EES is associated. Bar plots for several gait parameters demonstrating augmented hindlimb locomotor function with the addition of LH DBS. Repeated measures one-way ANOVA with post-hoc Tukey test (*$p < 0.05$; error bars SEM).

The inventors have evidence from rodent models of spinal cord injury (SCI) that key neurons restoring walking after SCI are located in the Lateral Hypothalamus (LH), a highly-specialized region of the brain that regulates arousal, feeding and motivated behavior.

The results of the inventors stimulating the LH excitatory neurons using optogenetics in mice demonstrate that LH neurons are not only necessary to walk after SCI, but are also sufficient to augment walking capacities after paralysis.

In our models, the inventors found that the LH possesses the relevant projectome to improve walking after the type of SCI commonly observed in humans, and that its receptome is favorably positioned to increase the influence of motivation.

Stimulation of the LH using DBS electrodes augments recovery of walking in a rat model of SCI. When combined with Electrical Epidural Stimulation (EES) in the presence of serotonergic agonists, the recovery of walking is even further improved **(Fig. 34)**.

**Fig. 34** shows functional MRI to determine peak activation areas in the deep brain region Left panel. The first implanted patient has an incomplete spinal cord injury with residual motor control on the left leg. Right panel. The patient's leg is moved alternating right and leg movements during functional MRI measurements. Focal activation points are identified (dark black area) in the lateral hypothalami of both sides.

The inventors tested the following stimulation pattern revealing efficient effect of LH stimulation for recovery of walking. So far, the following stimulation parameters have been found to be effective:

EES:

- Frequency - approx. 40 Hz;
- Pulse width - 0.2 ms;
- Amplitude range - 50-300 μA;
- (suitable) Electrode configurations capable to stimulate a lumbar or lumbosacral level.

LH-DBS:

- Frequency - 50 Hz;
- Pulse width - 0.2 ms;
- Amplitude range - 85-210 μA;
- (suitable) Electrode configurations

Clinical evidence

To date, no clinical trial has studied the impact of DBS delivered in the LH to augment locomotion after SCI. However, the inventors collected fMRI data (CER-VD protocol 207/10, version of 23.06.2014) in a cohort of 70 human subjects that demonstrates the robust activation of the LH during a locomotor task. The functional activation of the LH during locomotion is consistent with the large amount of preclinical data that showed the critical role of this region in the production of locomotion. Moreover, DBS of the hypothalamus is not a new therapeutic target, since several clinical trials for other diseases have used this approach safely for at least a decade.

DBS of the hypothalamus has spanned a range of indications in humans. Previous research in animals has demonstrated that the hypothalamus plays a role in regulating feeding behaviour and body weight (L. E. Harrell, J. M. Decastro, S. Balagura, A critical evaluation of body weight loss following lateral hypothalamic lesions. Physiol Behav. 15, 133-136 (1975); R. Keesey, T. Powley, Self-stimulation and body weight in rats with lateral hypothalamic lesions. Am J Physiology-legacy Content. 224, 970-978 (1973)).

These findings have served as an impetus for trials in humans that have looked at the effect of DBS on refractory obesity. Whiting et al. performed bilateral DBS electrode implantation into the LH in three patients who failed bariatric surgery (D. M. Whiting, N. D. Tomycz, J. Bailes, L. de Jonge, V. Lecoultre, B. Wilent, D. Alcindor, E. R. Prostko, B. C. Cheng, C. Angle, D. Cantella, B. B. Whiting, J. S. Mizes, K. W. Finnis, E. Ravussin, M. Y. Oh, Lateral hypothalamic area deep brain stimulation for refractory obesity: a pilot study with preliminary data on safety, body weight, and energy metabolism. J Neurosurg. 119, 56-63 (2013)).

With continuous DBS and a mean follow-up of 35 months, no serious adverse events were observed with no hardware complications (including infection and wound erosion) except an electrode failure in one patient. There were no observed negative effects on psychological function, and serial blood testing did not reveal any changes in pituitary hormones and neuroendocrine studies (D. M. Whiting, N. D. Tomycz, J. Bailes, L. de Jonge, V. Lecoultre, B. Wilent, D. Alcindor, E. R. Prostko, B. C. Cheng, C. Angle, D. Cantella, B. B. Whiting, J. S. Mizes, K. W. Finnis, E. Ravussin, M. Y. Oh, Lateral hypothalamic area deep brain stimulation for refractory obesity: a pilot study with preliminary data on safety, body weight, and energy metabolism. J Neurosurg. 119, 56-63 (2013)).

Another case report of an individual bilaterally implanted with DBS electrodes in the hypothalamus for morbid obesity unexpectedly induced autobiographical memory effects and was thought to possibly improve hippocampus-dependent memory function (C. Hamani, M. P. McAndrews, M. Cohn, M. Oh, D. Zumsteg, C. M. Shapiro, R. A. Wennberg, A. M. Lozano, Memory enhancement induced by hypothalamic/fornix deep brain stimulation. Ann Neurol. 63, 119-123 (2008)). This was followed by a phase I clinical trial in 6 patients with mild Alzheimer disease to assess improvements in cognitive function (A. W. Laxton, D. F. Tang-Wai, M. P. McAndrews, D. Zumsteg, R. Wennberg, R. Keren, J. Wherrett, G. Naglie, C. Hamani, G. S. Smith, A. M. Lozano, A phase I trial of deep brain stimulation of memory circuits in Alzheimer's disease. Ann Neurol. 68, 521-534 (2010)). These patients underwent chronic stimulation at 3.0 to 3.5 V with a frequency of 130 Hz for 12 months. In all patients, surgery was well tolerated with patients being discharged 1 to 3 days after surgery. At stimulation intensities of 7 to 10 V, patients experienced a warmth sensation, flushing, sweating, and increase in heart rate and blood pressure. At chronic stimulation levels of 50% of the voltage that resulted in these effects, there was no evidence of hypothalamic dysfunction or metabolic or endocrine abnormalities after 12 months of DBS (A. W. Laxton, D. F. Tang-Wai, M. P. McAndrews, D. Zumsteg, R. Wennberg, R. Keren, J. Wherrett, G. Naglie, C. Hamani, G. S. Smith, A. M. Lozano, A phase I trial of deep brain stimulation of memory circuits in Alzheimer's disease. Ann Neurol. 68, 521-534 (2010)).

Cluster headache, a debilitating primary headache condition, has been associated with activation of the posterior hypothalamus based on functional imaging during attacks (D. Fontaine, Y. Lazorthes, P. Mertens, S. Blond, G. Géraud, N. Fabre, M. Navez, C. Lucas, F. Dubois, S. Gonfrier, P. Paquis, M. Lantéri-Minet, Safety and efficacy of deep brain stimulation in refractory cluster headache: a randomized placebo-controlled double-blind trial followed by a 1-year open extension. J Headache Pain. 11, 23-31 (2010)). Fontaine et al. performed a randomized placebo-controlled double-blind trial to assess the safety and efficacy of DBS of the hypothalamus for cluster headache. This trial compared DBS of the posterior hypothalamic region with a sham control. 11 patients were enrolled in the study, and there were three serious adverse events including subcutaneous infection, transient loss of consciousness and micturition syncope. There were no significant changes in hormonal function or electrolyte balance.

Taken together, these clinical trials demonstrate that DBS of the hypothalamus can be performed safely with relatively little morbidity even with long-term continuous stimulation, and it has already been tested for a variety of different indications. To this date, the inventors implanted one individual with incomplete SCI and obtained the following results:

Patient data

The inventors have data from one patient with incomplete SCI (AIS-C, lower extremity motor score of 0 on the right and 8 on the left) that was implanted with bilateral DBS electrodes in the right and left lateral hypothalamus.

Functional MRI data during a randomly repeated and alternating leg motor task was performed prior to surgery and diffusion tensor imaging guided exact location and trajectory for DBS electrodes in the lateral hypothalamus **(Fig. 34).**

Each implanted DBS lead (left and right) is composed of a 1-3-3-1 electrode configuration to more precisely direct the stimulation. Each electrode on both leads was tested after surgery and a mapping of sensations was performed.

Stimulation of the lateral hypothalamus on both sides during walking demonstrated increased EMG activity in representative flexor and extensor muscles **(Fig. 35)** while testing in the same walking conditions (body weight support and task).

Stimulation parameters:

- Frequency range - 10Hz - 260 Hz, preferred 20 Hz and 180 Hz;

- Pulse width range - 10 - 300 $\mu$s;

- Amplitude range - 0.1 mA to 25mA - set based on patient feeling;

- Electrode configurations (monopolar, multipolar and directional);

- Preferred parameters for patient 1: 20 Hz or 180 Hz.

The inventors also have data from one patient with SCI that improved motor score after 2 months of LH-DBS supported rehabilitation (3 days / week).

**Fig. 35** shows that Lateral hypothalamus stimulation (DBS) promotes walking in clinical trial participant suffering from gait deficits secondary to spinal cord injury. Left panel. In the absence of DBS, regular interchange of activity between flexor and extensor muscles is absent. Chronophotography shows the participant struggling to move along the corridor by supporting himself mostly by hands. The plots show the activity of a leg flexor and a leg extensor muscles. Right panel. DBS restores the activation of the leg muscles to support the overground locomotion. Chronophotography shows the participant walking along the corridor. The plots show the activity of a leg flexor and a leg extensor muscles.

**Fig. 36** shows an embodiment for a system 400 to provide neuromodulation inter alia for the LH.

Similar structural or functional features and elements of the systems previously described have similar reference numbers, but are increased by the value 400, 300, 200 or 100.

Further, the systems as previously shown in **Fig. 1** to **Fig. 24** can be combined in parts or as a whole, complimentary and in full with the system 400.

This disclosure describes therapeutic systems, each composed of one or more devices, that deliver electrical deep brain stimulation in the region of the lateral hypothalamus, alone or in combination with the electrical epidural stimulation of the spinal cord, in order to alleviate locomotor deficits in neuromotor disorders.

The system 400 comprises an implanted current generator 460. This can be an implantable pulse generator.

Further, there are DBS leads 462 for the modulation/stimulation of the LH.

There is a DBS neuromodulation element 464 for the modulation/stimulation of the left LH.

Additionally, there is a DBS neuromodulation element 466 for the modulation/stimulation of the left LH.

The person/patient P is implanted with the complete system 400 composed of one or more implanted current generators 460 connected by cables to one or more deep brain stimulation leads 462, 464, 466 with electrodes implanted into one or two lateral hippocampi.

The system 400 has the following functionality:

The system 400 is a system 400 where electrical current is delivered at one or both lateral hypothalami using one or more electrode leads 464, 466, each containing one or more electrodes. These leads are connected by cables to one or more "implanted current generator" devices 460 that deliver electrical current through their contacts **(Fig. 36).** Therefore, the implanted current generators deliver the current through their contact or multiple contacts, over the cable and then through one or more implanted electrodes. The implanted current generator 460 can work independently until its batteries have been discharged. The implanted current generator 460 may be rechargeable. The implanted current generator 460 may be controlled by another device that can modify the current delivery protocols, including stopping the delivery of the current.

Users P are implanted with all the devices of the system 400: one or more implanted current generators and one or more electrode leads 464, 466, each containing one or more electrodes. The electrodes are positioned within one or both lateral hypothalami. The electrodes are then connected to the implanted current generators by cables that carry electrical current from the implanted pulse generators through the cables and electrodes into the lateral hypothalami.

The deep brain electrical stimulation of the lateral hypothalami uses for example tonic programming. Also other suitable stimulation programs are possible.

The process of building and using this system 400 is as follows:

Subjects P are equipped with the following system elements:

- Two implanted current generator 460, for example the Perept PC (Medtronic).

- Two deep brain electrode leads - two Sensight Directional leads (Medtronic), one implanted in each lateral hypothalamus.

Each implanted current stimulator is programmed with its stimulation program. Each program is defined by the set of deep brain leads that deliver the stimulation current, current amplitude, pulse waveform, and stimulation frequency. These programs are calibrated as follows:

The DBS program is defined by the expert with feedback from the patient. The DBS program remains active until and throughout the session. At the session, the patient is asked to walk overground or on the treadmill. During this process, the experts examine the quality of the gait.

The evaluation of gait is compared to the gait of other sessions recorded using other DBS programs.

The DBS program is loaded into the firmware of the implanted current generator. The DBS is then activated and runs continuously. The DBS program may be modified during subsequent re-calibration.

**Fig. 37** shows a further embodiment for a system 400 to provide neuromodulation inter alia for the LH.

It has all the structural and functional features as the system 400 of **Fig. 36.**

Similar structural or functional features and elements of the systems previously described have similar reference numbers, but are increased by the value 400, 300, 200 or 100.

Further, the systems as previously shown in **Fig. 1** to **Fig. 24** can be combined in parts or as a whole, complimentary and in full with the system 400.

This disclosure describes therapeutic systems, each composed of one or more devices, that deliver electrical deep brain stimulation in the region of the lateral hypothalamus, alone or in combination with the electrical epidural stimulation of the spinal cord, in order to alleviate locomotor deficits in neuromotor disorders.

**Fig. 37** shows a system 400 as shown in **Fig. 36** that also includes system 10 (see also **Fig. 1),** where electrical current is delivered over the dorsal side of the lumbosacral spinal cord using one or more electrodes 412, 414 positioned epidurally. These electrodes are connected by cables to one or more "implanted current generator" devices 413, 460 (or implanted pulse generators (IPGs)) that deliver electrical current through their contacts **(Fig. 37).** The implanted current generators can generate a temporal sequence of electrical currents on each of their contacts. Therefore, the implanted current generators deliver the current through their contact or multiple contacts, over the cable and then through one or more implanted electrodes. The implanted current generator can work independently until its batteries have been discharged. The implanted current generator may be rechargeable. The implanted current generator 413, 460 may be controlled by another device that can modify the sequence of current delivery, including stopping the delivery of the current. The user P may or may not receive feedback about the delivered spinal stimulation by different modalities, including but not limited to sound, touch, vibration and visual stimuli.

The person P is implanted with system 400. In addition, the person P is implanted with the complete system composed of one or more implanted current generators connected by cables to one or more electrodes placed epidurally over the dorsal side of the spinal cord SC.

In other words: Users P are implanted with one or more implanted current generators 413 and one or more electrodes 414 that can be arranged into one or more electrode arrays. The electrodes 414 are positioned within the spinal column over the dorsal side of the lumbosacral spinal cord. The electrodes 414 are then connected to the implanted current generators 413 by cables that carry electrical current from the implanted pulse generators through the cables across the electrodes into the vicinity of the spinal cord.

**Fig. 38** shows a further embodiment for a system to provide neuromodulation inter alia for the LH.

It has all the structural and functional features as the system 400 of **Fig. 36** and also **Fig. 37.**

Similar structural or functional features and elements of the systems previously described have similar reference numbers, but are increased by the value 400, 300, 200 or 100.

Further, the systems as previously shown in **Fig. 1** to **Fig. 24** can be combined in parts or as a whole, complimentary and in full with the system 400.

This disclosure describes therapeutic systems, each composed of one or more devices, that deliver electrical deep brain stimulation in the region of the lateral hypothalamus, alone or in combination with the electrical epidural stimulation of the spinal cord, in order to alleviate locomotor deficits in neuromotor disorders.

**Fig. 38** shows a system as in **Fig. 37** that also includes one or more devices that infer users' movement intentions from their cortical activity **(Fig. 38).** The additional devices include one or more "cortical neurosensor" devices that record cortical activity, one or more "controller" devices 451 that acquire the signals from the cortical neurosensor(s), use these signals to infer parameters of users' movement intentions, and generate sequences of current delivery based of limitations of the spinal epidural electrical stimulation system as described in connection with **Fig. 37,** and one or more "communicator" devices 452 that communicate the sequences of current delivery to the implanted current generators used for spinal epidural electrical stimulation. One or more of these devices can be integrated in a single device.

The person P is implanted with system 400. In addition, the person is equipped by one or more invasive or non-invasive cortical neurosensors. The cortical neurosensor(s) send the signals to one or more Controllers by wired and / or wireless links. The Controller(s) 451 processes the acquired neural signals, input them in the decoding algorithms that infer person's movement intentions from the input signals, and, based on the inferred movement intentions, send the commands to one or more Communicators. The Communicator(s) 452 relay(s) the commands to the implanted current generators that deliver the spinal epidural electrical stimulation as described in **Fig. 37.**

System 400 as shown in **Fig. 38** works just as system 400 of **Fig. 37.** In addition, the following functional and structural features are realized:

Users P are equipped with one or more cortical neurosensors 450.

The cortical neurosensors 450 can be non-invasive, including but not limited to electroencephalography (EEG) electrode sets, sets of functional near-infrared (fNIR) sensors, sets of magnetoencephalography (MEG) sensors and functional magnetic resonance imagining (fMRI) sensors.

The cortical neurosensors 450 can also be invasive and, therefore, equipped through a surgery.

The invasive cortical neurosensors include but not limited to stereotactic EEG, epidural and subdural electrocorticography (ECoG), penetrating cortical glass electrodes, and penetrating cortical microelectrodes.

The cortical neurosensor electrodes are connected to one or more cortical neurosensor modules that allow readout of recorded cortical activity.

Users P are equipped with one or more Controller devices 451. Each Controller 451 acquires the signals from one or more cortical neurosensors, uses these signals to infer parameters of users' movement intentions, and generates one or more sequences of current delivery based of limitations of the system. Controllers 451 then send commands that encode the sequences of current delivery to one or more Communicator devices 452. The Controller devices 451 can be implanted or external to the user.

Users P are equipped with one or more Communicator devices 452 that acquire the commands from the Controllers 451, and send these commands to the implanted current generators used for spinal epidural electrical stimulation. The Communicators 452 can be implanted or external to the user.

**Fig. 39** shows a further embodiment for a system to provide neuromodulation inter alia for the LH.

It has all the structural and functional features as the system 400 of **Fig. 36** and also **Fig. 37** and also **Fig. 38.** When compared with the system 400 of **Fig. 38,** there is no cortical neurosensor provided here.

Similar structural or functional features and elements of the systems previously described have similar reference numbers, but are increased by the value 400, 300, 200 or 100.

Further, the systems as previously shown in **Fig. 1** to **Fig. 24** can be combined in parts or as a whole, complimentary and in full with the system 400.

This disclosure describes therapeutic systems, each composed of one or more devices, that deliver electrical deep brain stimulation in the region of the lateral hypothalamus, alone or in combination with the electrical epidural stimulation of the spinal cord, in order to alleviate locomotor deficits in neuromotor disorders.

**Fig. 39** shows a system as in **Fig. 37** that also includes one or more devices that infer users' movement intentions from the activity of their muscles M or their behavior, including but not limited to movements of their limbs, their torso or head. The additional devices include one or more "muscle activity sensor" devices 470 that record users' muscle activity and / or one or more "behavioral sensor" devices 468 that record users' behavior; one or more controller devices 451 that acquires the signals from the muscle activity sensors 470 and / or behavioral sensors 468, use these signals to infer parameters of users' movement intentions, and generate sequences of current delivery based of limitations of the spinal epidural electrical stimulation system 400 as described in **Fig. 37;** and one or more communicator devices 452 that communicate the sequences of current delivery to the implanted current generators 413 used for spinal epidural electrical stimulation. One or more of these devices can be integrated in a single device.

The person is implanted with system 400. In addition, the person is equipped by one or more invasive or non-invasive muscle activity sensors 470 and/or one or more behavioral sensors 468. These sensors send the signals to one or more Controllers by wired or wireless links. The Controllers process the acquired signals, input them in the decoding algorithms that infer person's movement intentions from the input signals, and, based on the inferred movement intentions, send the commands to one or more Communicators. The Communicators relay the commands to the implanted current generators that deliver the spinal epidural electrical stimulation as described in **Fig. 37.**

The system 400 works as follows:
Users P are equipped with one or more muscle activity sensors 470 that record activity of the muscles and / or one or more behavioral sensors 468.

The muscle activity sensors 470 can be invasive and, if invasive, are equipped though a surgery.

The behavioral sensors 468 can be invasive and, if invasive, are equipped through a surgery. The muscle activity electrodes 470 are connected to one or more muscle activity sensor modules that allow readout of recorded muscle activity.

Users P are equipped with one or more Controller devices 451. Each Controller 451 acquires the signals from one or more muscle activity sensors 470 and / or behavioral sensors 468, uses these signals to infer parameters of users' movement intentions, and generates one or more sequences of current delivery based of limitations of the system 400. Controllers 451 then send commands that encode the sequences of current delivery to one or more Communicator devices 452. The Controller devices 451 can be implanted or external to the user P.

Users P are equipped with one or more Communicator devices 452 that acquire the commands from the Controllers 451, and send these commands to the implanted current generators 413 used for spinal epidural electrical stimulation. The Communicators 452 can be implanted or external to the user P.

**Fig. 40** shows a further embodiment for a system to provide neuromodulation inter alia for the LH.

It has all the structural and functional features as the system 400 of **Fig. 36** and also **Fig. 37** and also **Fig. 38.** When compared with the system 400 of **Fig. 38,** there is no cortical neurosensor provided here. When compared with the system 400 of **Fig. 39,** there are no muscle sensors and behavorial sensors provided here.

Similar structural or functional features and elements of the systems previously described have similar reference numbers, but are increased by the value 400, 300, 200 or 100.

Further, the systems as previously shown in **Fig. 1** to **Fig. 24** can be combined in parts or as a whole, complimentary and in full with the system 400.

This disclosure describes therapeutic systems, each composed of one or more devices, that deliver electrical deep brain stimulation in the region of the lateral hypothalamus, alone or in combination with the electrical epidural stimulation of the spinal cord, in order to alleviate locomotor deficits in neuromotor disorders.

As the system 400 of **Fig. 37,** also the system 400 of **Fig. 40** also includes one or more devices that collect users' commands, including but not limited to verbal commands and button presses **(Fig. 40).** The additional devices include one or more "control sensor" devices 472 that collect users' commands; one or more controller devices 451 that generate sequences of current delivery from users' commands based of limitations of the spinal epidural electrical stimulation system as described in connection with the system of **Fig. 37;** and one or more communicator devices 452 that communicate the sequences of current delivery to the implanted current generators used for spinal epidural electrical stimulation. One or more of these devices can be integrated in a single device.

The person P is implanted with system 400. In addition, the person P is equipped by one or more control sensors 472. These control sensors 472 send the signals to one or more Controllers 451 by wired and / or wireless links. The Controllers 451 use the acquired signals to derive the stimulation commands, and send these commands to the Communicators 452. The Communicators 452 relay the commands to the implanted current generators that deliver the spinal epidural electrical stimulation as described in connection with the system 400 of **Fig. 37.**

**Fig. 40** shows a system 400 as for example in **Fig. 37** where the spinal epidural electrical stimulation is delivered in a previously designed sequence out of users' control.

The sequence can, but does not have to, modulate over time.

The sequence can further, but does not have to, modulate over different spinal stimulation locations.

Also, all other stimulation parameters can modulate over time, or remain fixed.

These include, but are not limited to, stimulation frequency, stimulation amplitude, stimulation pulse width and any other parameters that regulate the spinal stimulation.

The system 400 operates as follows:

Users P are equipped with one or more control sensors 472 that collect users commends, including but not limited to verbal commands and button presses. The control sensors 472 can be invasive and, if invasive, are equipped though a surgery. The control sensor electrodes are connected to one or more control sensor modules that allow readout of users' controls.

Users are equipped with one or more Controller devices 451. Each Controller 451 acquires the signals from one or more control sensors 472, and uses these signals to generate one or more sequences of current delivery based of limitations of the system. Controllers 451 then send commands that encode the sequences of current delivery to one or more Communicator devices 452. The Controller devices 452 can be implanted or external to the user.

Users P are equipped with one or more Communicator devices 452 that acquire the commands from the Controllers 451, and send these commands to the implanted current generators used for spinal epidural electrical stimulation. The Communicators 452 can be implanted or external to the user P.

The systems 400 as show in **Fig. 37** to **Fig. 40** have the following functional features:

The deep brain electrical stimulation of the lateral hypothalami using tonic programming combined with the epidural electrical stimulation of the lumbosacral spinal cord using tonic programming. The process of building and using this system 400, which corresponds to the systems 400 as shown in **Fig. 37** to **Fig. 40** described above, is as follows:

Subjects P are equipped with:

Two deep brain electrode leads - one implanted in each lateral hypothalamus with transcutaneous connectors.

Spinal electrodes - one custom-made electrode array implanted over the dorsal side of the lumbosacral spinal cord.

The externalized current stimulator used for DBS is programmed with its stimulation program. Each program is defined by the set of deep brain lead electrodes that deliver the stimulation current, current amplitude, pulse waveform, and stimulation frequency. These programs are calibrated as follows:

The DBS program is defined by the expert. The DBS program remains active until and throughout the session. During the session, the subject walks overground or on the treadmill while the experts examine the quality of the gait. The evaluation of gait is compared to the gait of other sessions recorded using other DBS programs.

The externalized current stimulator used for EES is programmed with its stimulation program. Each program is defined by the set of spinal array electrodes that deliver the stimulation current, current amplitude, pulse waveform, and stimulation frequency.

These programs are calibrated as follows:

During the session, the user P is asked to walk overground or on the treadmill while the experts turn an EES tonic program on and examine the quality of the gait. The parameters of the EES program are modified and the process is repeated. Using this iterative process, the experts select the most effective EES program.

## Clinical trial on two human subjects with incomplete SCI

**Fig. 41** provides an overview of a clinical trial conducted by the inventors on two human patients with incomplete SCI (clinical trial NCT04965727, https://clinicaltrials.gov/ct2/show/NCT04965727).

Here, DBS electrodes (Sensight directional leads, Medtronic) were implanted in the left and right lateral hypothalamus of the two patients with incomplete SCI.

Both leads were connected to a Percept PC (Medtronic) implanted under the collarbone.

Hardware components used for DBS in patients with incomplete SCI are depicted in Fig. **41a.**

The day after surgery, a merge of the post-operative computer tomography scan and the preoperative MRI confirmed correct positioning of electrodes of both patients in the right and left lateral hypothalamus region **(Fig 41b).**

As early as the first session after implantation, the stimulation facilitated walking in parallel bars and significantly increased muscle activity of flexor (tibialis anterior) and extensor (vastus lateralis) muscles **(Fig. 41c).**

Following a calibration phase of 4 weeks, both patients underwent a training of 3 months (3x3 hours per week) with DBS$^{ON}$ during the training session.

Clinical measurements were performed before training (month 0), after 3 months (month 3) of training, and after additional 3 months of home-use (month 6).

Both patients recovered lower extremity motor score (LEMS) after 3 months of DBS-supported rehabilitation, and progressed on the 10-meter walk test and 6-minute walk test after 6 months of DBS-supported rehabilitation **(Fig. 41d).**

These increases of motor functionalities enabled both patients to improve their walking performance in daily life activities.

The first patient achieved to walk overground with a walker without using any leg orthosis, which was not feasible before therapy.

The second patient was able to climb over 30 stairs following rehabilitation (in comparison to 5 before implantation).

Both patients reported a facilitation of control of the lower limbs and an increase in their training endurance.

This unexpected result suggested that neurorehabilitation supported by DBS of the lateral hypothalamus promotes a reorganization of residual descending pathways that improves neurological recovery, even when the stimulation is turned off, and is further enhanced when stimulation is turned on.

The following aspects are explicitly disclosed in connection with the present disclosure:

**Aspect 1:** A system for neuromodulation and/or neurostimulation system for mitigating locomotor deficits a human patient, such locomotor deficits being locomotor deficits of/or neuronal disorders, especially at least one of Parkinson's disease epilepsy, chronic pain (like (cluster) headache or migraine), depression, Alzheimer's disease, obsessive compulsive disorders, and obesity, at least comprising a deep brain neuromodulation element, which is capable to be arranged at the region of the hypothalamus.

**Aspect 2:** The system of aspect 1, characterized in that the region is the lateral hypothalamus.

**Aspect 3:** The system of aspect 1 or aspect 2, characterized in that the system further comprises a current and/or pulse generator, especially wherein the current and/or pulse generator is an implanted current and/or pulse generator.

**Aspect 4:** The system of one of the previous aspects, characterized in that the system further comprises a neuromodulation module, which is capable to provide EES to the spinal cord, especially at lumbar level of the spinal cord and/or at lumbosacral level of the spinal cord.

**Aspect 5:** The system of one of the previous aspects, characterized in that the system further comprises a cortical neurosensor.

**Aspect 6:** The system of one of the previous aspects, characterized in that the system further comprises at least one muscle activity sensor.

**Aspect 7:** The system of one of the previous aspects, characterized in that the system further comprises at least one behavioral sensor.

**Aspect 8:** The system of one of the previous aspects, characterized in that the system further comprises at least one controller and/or communicator.

**Aspect 9:** The system of one of the previous aspects, characterized in that the system is capable to provide EES neuromodulation at a stimulation frequency of approx. 40 Hz.

**Aspect 10:** The system of one of the previous aspects, characterized in that the system is capable to provide EES neuromodulation at a pulse width of approx. 0.2 ms.

**Aspect 11:** The system of one of the previous aspects, characterized in that the system is capable to provide EES neuromodulation at an amplitude range of 50-300 $\mu$A.

**Aspect 12:** The system of one of the previous aspects, characterized in that the system is capable to provide LH-DBS neuromodulation at a frequency of approx. 50 Hz.

**Aspect 13:** The system of one of the previous aspects, characterized in that the system is capable to provide LH-DBS neuromodulation at a pulse width of approx. 0.2 ms.

**Aspect 14:** The system of one of the previous aspects, characterized in that the system is capable to provide LH-DBS neuromodulation at an amplitude range of approx. 85-210 $\mu$A.

**Aspect 15:** A method for neuromodulation and/or neurostimulation system for mitigating locomotor deficits of a human patient, at least comprising the step of providing deep brain neuromodulation at the region of the hypothalamus.

**Aspect 16:** The method of aspect 15, characterized in that the region is the lateral hypothalamus.

**Aspect 17:** The system according to one of aspects 1 to 14, characterized in that the system is at least partially or completely combined and/or integrated with or into the system according to one of claims 1 to 15 or any part of the present disclosure.

**Aspect 18:** The method according to one of aspects 15 or 16, characterized in that the method is at least partially or completely combined and/or integrated and/or performed with or into the system according to one of claims 1 to 15 or one of aspects 1 to 14 or any part of the present disclosure.

**Aspect 19:** A combined neuromodulation and/or neurostimulation system for mitigating locomotor deficits of/or neuronal disorders, especially at least one of Parkinson's disease, epilepsy, chronic pain (like (cluster) headache or migraine), depression, Alzheimer's disease, obsessive compulsive disorders, obesity, and/or other locomotor deficits and/or neuronal disorders after spinal cord injury (SCI), stroke, and/or other neurological disorders, said system comprising:

- at least one Deep Brain Stimulation (DBS) System for providing Deep Brain Stimulation to brain tissue of a subject,

- at least one control unit, configured and adapted to provide stimulation data,

- at least one stimulation unit, operatively connected to the at least one control unit, the at least one stimulation unit being configured and adapted to deliver epidural electrical stimulation (EES) to the dorsal side of the spinal cord of said subject, and

- at least one implantable pulse generator (IPG),

wherein the at least one stimulation unit includes one or more electrodes configured to be implanted epidurally, the one or more electrodes being operatively connected to the at least one IPG.

**Aspect 20:** The combined neuromodulation and/or neurostimulation system according to aspect 19, wherein the at least one stimulation unit is configured and adapted to deliver EES to the lumbosacral region of the spinal cord, preferably at spinal cord segments from L2 to S1.

**Aspect 21:** The combined neuromodulation/neurostimulation system according to aspect 19 or aspect 20, wherein the Deep Brain Stimulation (DBS) System is configured to provide low frequency stimulation to the brain tissue, for example in the range between 0-150Hz, especially in the range of 20-100Hz, more especially in the range of 20-70 Hz, preferably around 20-45 Hz or 20-60 Hz.

**Aspect 22:** The combined neuromodulation/neurostimulation system according to aspect 19 or aspect 20, wherein the Deep Brain Stimulation (DBS) System is configured to provide low frequency stimulation to the brain tissue by means of charge balanced biphasic stimulation pulses.

**Aspect 23:** The combined neuromodulation/neurostimulation system according to aspect 19 or aspect 20, wherein the Deep Brain Stimulation (DBS) System is configured to provide stimulation pulses with a pulse width of approx. 90 $\mu$s.

**Aspect 24:** The combined neuromodulation/neurostimulation system according to aspect 19 or aspect 20, wherein the Deep Brain Stimulation (DBS) System is configured to provide stimulation to the subthalamic nucleus (STN).

**Aspect 25:** The combined neuromodulation/neurostimulation system according to any one of the preceding aspects, wherein the stimulation unit is configured provide stimulation to spinal cord tissue and/or the tissue of the spinal cord dorsal roots, for example in the range between 0-150Hz, especially in the range of 20-100Hz, more especially in the range of 20-70 Hz, preferably around 20-60 Hz, more preferably at a frequency of 60Hz plus minus 10 Hz.

**Aspect 26:** The combined neuromodulation/neurostimulation system according to any one of the preceding aspects, wherein the stimulation unit is configured provide stimulation to spinal cord tissue and/or the tissue of the spinal cord dorsal roots at currents in the range of approx. 0.4-1.7mA.

**Aspect 27:** The combined neuromodulation/neurostimulation system according to any one of the preceding aspects, wherein the stimulation unit is configured provide stimulation to spinal cord tissue and/or the tissue of the spinal cord dorsal roots with a pulse width of approx. 200-300 μs.

**Aspect 28:** The combined neuromodulation/neurostimulation system according to any one of the preceding aspects, wherein the stimulation unit comprises at least a lead paddle with an electrode array and is configured provide stimulation to spinal cord tissue and/or the tissue of the spinal cord dorsal roots with the electrodes of the lead paddle, where the cathode(s) is are provided by the electrodes of the lead paddle and/or the anode(s) is/are provided at least partially by either the electrodes of the lead paddle or the IPG case.

**Aspect 29:** The combined neuromodulation/neurostimulation system according to any one of the preceding aspects, wherein the system further comprises at least one module for administering a pharmacological agent to the subject, preferably wherein said pharmacological includes Levodopa.

**Aspect 30:** The combined neuromodulation/neurostimulation system according to any one of the preceding aspects, wherein the system further comprises a Brain Spinal Interface (BSI) with microelectrode arrays, which are configured to be placed at the left and right leg primary motor cortex.

**Aspect 31:** The combined neuromodulation/neurostimulation system according to aspect 30, wherein the Brain Spinal Interface (BSI) further includes

- one or more sensors, preferably wherein said one or more sensors include one or more among a cortical neurosensor for recording a cortical activity of a subject, a motion sensor, a haptic sensor, and/or a physiological sensor;

- one or more controller devices, configured and adapted to receive signals from the one or more cortical neurosensors, process the received signals to determine one or more parameters regarding movement intentions of the subject, and generate one or more stimulation parameters based on the determined one or more movement intentions parameters, and

- one or more communicator devices, configured and adapted to communicate the generated one or more stimulation parameters to the at least one IPG.

**Aspect 32:** The combined neuromodulation/neurostimulation system according to any one of the preceding aspects, wherein the system further includes

- one or more muscle activity sensors and/or one or more behavioral sensors for recording muscle activity and/or a behavior of a subject;

- one or more controller devices, configured and adapted to receive signals from the one or more muscle activity sensors and/or one or more behavioral sensors, process the received signals to determine one or more parameters regarding movement intentions of the subject, and generate one or more stimulation parameters based on the one or more determined movement intentions parameters, and

- one or more communicator devices, configured and adapted to communicate the generated stimulation parameters to the at least one IPG.

**Aspect** 33: The combined neuromodulation/neurostimulation system according to aspect 32, wherein the one or more muscle activity sensors and/or one or more behavioral sensors, the one or more controller devices and/or the one or more communicator devices are integrated in a single unit.

Note that the example control and estimation routines included herein can be used with various neuromodulation and/or neurostimulation system configurations. The control methods and routines disclosed herein may be stored as executable instructions in non-transitory memory and may be carried out by the control unit in combination with the various sensors, actuators, and other system hardware in connection with a medical neurostimulation system. The specific routines described herein may represent one or more of any number of processing strategies such as event-driven, interrupt-driven, multi-tasking, multi-threading, and the like. As such, various actions, operations, and/or functions illustrated may be performed in the sequence illustrated, in parallel, or in some cases omitted. Likewise, the order of processing is not necessarily required to achieve the features and advantages of the example embodiments described herein, but is provided for ease of illustration and description. One or more of the illustrated actions, operations and/or functions may be repeatedly performed depending on the particular strategy being used. Further, the described actions, operations and/or functions may graphically represent code to be programmed into non-transitory memory of the computer readable storage medium in the control unit, where the described actions are carried out by executing the instructions in a system including the various hardware components in combination with an electronic control unit.

**SUPPLEMENTARY TABLES**

Supplementary table 1. Behavioral experiments conducted with each NHP.

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| M1 | Pre-MPTP 1: corridor 19.7.2012 | N/A | 5 | | | | | | | | | |
| | Post-MPTP 1: corridor 22.11.2012 | 7 (assessed on 22.11.2012) | 5 | | | | | | | | | |
| M2 | Pre-MPTP 1: corridor 13.7.2012 | N/A | 6 | | | | | | | | | |
| | Post-MPTP 1: corridor 22.11.2012 | 9 (assessed on 22.11.2012) | 4 | | | | | | | | | |
| M3 | Pre-MPTP 1: corridor 26.2.2013 | N/A | 5 | | | | | | | | | |
| | Pre-MPTP 2: corridor 27.2.2013 | N/A | 5 | | | | | | | | | |
| | Pre-MPTP 3: ladder 9.5.2013 | N/A | 5 | | | | | | | | | |
| | Post-MPTP 1: corridor 17.10.2013 | 6 (assessed on 17.10.2013) | 4 | | | | | | | | | |

EP 4 778 565 A2

68

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 1: ladder 17.10.2013 | 6 (assessed on 17.10.2013) | 8 | | | | | | | | | |
| | Post-MPTP 2: corridor 25.11.2013 | 7 (assessed on 25.11.2013) | 5 | | | | | | | | | |
| M4 | Pre-MPTP 1: corridor 9.4.2013 | N/A | 9 | | | | | | | | | |
| | Pre-MPTP 1: ladder 9.4.2013 | N/A | 12 | | | | | | | | | |
| | Post-MPTP 1: ladder 2.6.2013 | 5 (assessed on 2.6.2013) | 6 | | | | | | | | | |
| | Post-MPTP 2: corridor 3.12.2013 | 6 (assessed on 3.12.2013) | 5 | | | | | | | | | |
| M5 | Pre-MPTP 1: corridor 23.11.2013 | N/A | 12 | | | | | | | | | |
| | Pre-MPTP 1: ladder 23.11.2013 | N/A | 8 | | | | | | | | | |

EP 4 778 565 A2

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Pre-MPTP 1: treadmill 23.11.2013 | N/A | 7 | | | | | | | | | |
| | Pre-MPTP 2: corridor 24.11.2013 | N/A | 10 | | | | | | | | | |
| | Pre-MPTP 2: ladder 24.11.2013 | N/A | 9 | | | | | | | | | |
| | Pre-MPTP 2: treadmill 24.11.2013 | N/A | 7 | | | | | | | | | |
| | Post-MPTP 1: treadmill 19.12.2013 | 0 (assessed on 19.12.2013) | 1 | | | | | | | | | |
| | Post-MPTP 2: treadmill 26.12.2013 | 0 (assessed on 26.12.2013) | 1 | | | | | | | | | |
| | Post-MPTP 3: treadmill 9.1.2014 | 2 (assessed on 9.1.2014) | 6 | | | | | | | | | |
| | Post-MPTP 4: treadmill 16.1.2014 | 2 (assessed on 16.1.2014) | 5 | | | | | | | | | |
| | Post-MPTP 5: treadmill 23.1.2014 | 6 (assessed on 16.1.2014) | 1 | | | | | | | | | |

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 6: treadmill 13.2.2014 | 5 (assessed on 13.2.2014) | 1 | | | | | | | | | |
| | Post-MPTP 7: treadmill 21.4.2014 | 6 (assessed on 21.4.2014) | 5 | | | | | | | | | |
| | Post-MPTP 8: corridor 23.4.2014 | 6 (assessed on 23.4.2014) | 10 | | | | | | | | | |
| | Post-MPTP 8: ladder 23.4.2014 | 6 (assessed on 23.4.2014) | 11 | | | | | | | | | |
| | Post-MPTP 8: treadmill 23.4.2014 | 6 (assessed on 23.4.2014) | 4 | | | | | | | | | |
| | Post-MPTP 9: corridor 28.4.2014 | 5 (assessed on 28.4.2014) | 6 | | | | | | | | | |
| | Post-MPTP 10: treadmill 5.6.2014 | 4 (assessed on 5.6.2014) | 3 | | | | | | | | | |
| | Post-MPTP 11: treadmill 6.6.2014 | 4 (assessed on 6.6.2014) | 3 | | | | | | | | | |
| | Post-MPTP 12: treadmill 7.6.2014 | 4 (assessed on 7.6.2014) | 7 | | | | | | | | | |

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 13: treadmill 24.7.2014 | 4 (assessed on 26.7.2014) | 2 | | | | | | | | | |
| | Post-MPTP 14: treadmill 25.7.2014 | 4 (assessed on 26.7.2014) | 5 | | | | | | | | | |
| | Post-MPTP 15: treadmill 26.7.2014 | 4 (assessed on 26.7.2014) | 4 | | | | | | | | | |
| | Post-MPTP 16: treadmill 28.7.2014 | 4 (assessed on 28.7.2014) | 5 | | | | | | | | | |
| | Post-MPTP 17: treadmill 29.7.2014 | 4 (assessed on 29.7.2014) | 3 | | | | | | | | | |
| | Post-MPTP 18: treadmill 30.7.2014 | 4 (assessed on 30.7.2014) | 3 | | | | | | | | | |
| | | | | | | | | | | | | |
| M6 | Pre-MPTP 1: treadmill 22.11.2013 | N/A | 1 | | | | | | | | | |
| | Pre-MPTP 2: corridor 24.11.2013 | N/A | 10 | | | | | | | | | |
| | Pre-MPTP 2: ladder 24.11.2013 | N/A | 6 | | | | | | | | | |

EP 4 778 565 A2

72

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Pre-MPTP 2: treadmill 24.11.2013 | N/A | 1 | | | | | | | | | |
| | Pre-MPTP 3: corridor 26.11.2013 | N/A | 11 | | | | | | | | | |
| | Pre-MPTP 3: ladder 26.11.2013 | N/A | 10 | | | | | | | | | |
| | Pre-MPTP 3: treadmill 26.11.2013 | N/A | 1 | | | | | | | | | |
| | Post-MPTP 1: treadmill 19.12.2013 | 0 (assessed on 19.12.2013) | 1 | | | | | | | | | |
| | Post-MPTP 2: treadmill 26.12.2013 | 0 (assessed on 26.12.2013) | 1 | | | | | | | | | |
| | Post-MPTP 3: treadmill 2.1.2014 | 2 (assessed on 02.01.2014) | 3 | | | | | | | | | |
| | Post-MPTP 4: treadmill 9.1.2014 | 7 (assessed on 09.01.2014) | 2 | | | | | | | | | |
| | Post-MPTP 5: treadmill 16.1.2014 | 7 (assessed on 16.01.2014) | 2 | | | | | | | | | |

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 6: treadmill 23.1.2014 | 6 (assessed on 23.01.2014) | 3 | | | | | | | | | |
| | Post-MPTP 7: treadmill 30.1.2014 | 5 (assessed on 30.01.2014) | 1 | | | | | | | | | |
| | Post-MPTP 8: treadmill 6.2.2014 | 5 (assessed on 06.02.2014) | 3 | | | | | | | | | |
| | Post-MPTP 9: treadmill 13.2.2014 | 7 (assessed on 13.02.2014) | 3 | | | | | | | | | |
| | Post-MPTP 10: treadmill 18.2.2014 | 7 (assessed on 18.02.2014) | 4 | | | | | | | | | |
| | Post-MPTP 11: corridor 22.2.2014 | 7 (assessed on 22.02.2014) | 4 | | | | | | | | | |
| | Post-MPTP 11: treadmill 22.2.2014 | 7 (assessed on 22.02.2014) | 8 | | | | | | | | | |
| | Post-MPTP 12: corridor 25.2.2014 | 7 (assessed on 25.02.2014) | 6 | | | | | | | | | |
| | Post-MPTP 12: treadmill 25.2.2014 | 7 (assessed on 25.02.2014) | 4 | | | | | | | | | |

(continued)

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 13: treadmill 27.2.2014 | 7 (assessed on 27.02.2014) | 3 | | | | | | | | | |
| | Post-MPTP 14: corridor 7.4.2014 | 6 (assessed on 07.04.2014) | 15 | | | | | | | | | |
| | Post-MPTP 14: treadmill 7.4.2014 | 6 (assessed on 07.04.2014) | 1 | | | | | | | | | |
| | Post-MPTP 15: corridor 10.4.2014 | 6 (assessed on 10.04.2014) | 9 | | | | | | | | | |
| | Post-MPTP 15: treadmill 10.4.2014 | 6 (assessed on 10.04.2014) | 2 | | | | | | | | | |
| | Post-MPTP 16: corridor 16.4.2014 | 6 (assessed on 16.04.2014) | 15 | | | | | | | | | |
| | Post-MPTP 16: treadmill 16.4.2014 | 6 (assessed on 16.04.2014) | 1 | | | | | | | | | |
| | Post-MPTP 17: treadmill 18.4.2014 | 6 (assessed on 16.04.2014) | 2 | | | | | | | | | |
| | Post-MPTP 18: corridor 5.5.2014 | 5 (assessed on 05.05.2014) | 13 | | | | | | | | | |

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 18: ladder 5.5.2014 | 5 (assessed on 05.05.2014) | 12 | | | | | | | | | |
| | Post-MPTP 18: treadmill 5.5.2014 | 5 (assessed on 05.05.2014) | 1 | | | | | | | | | |
| | Post-MPTP 19: treadmill 6.5.2014 | 6 (assessed on 06.05.2014) | 2 | | | | | | | | | |
| | Post-MPTP 19: treadmill 8.5.2014 | 5 (assessed on 08.05.2014) | 1 | | | | | | | | | |
| | Post-MPTP 20: corridor 9.5.2014 | 5 (assessed on 09.05.2014) | 15 | | | | | | | | | |
| | Post-MPTP 20: ladder 9.5.2014 | 5 (assessed on 09.05.2014) | 15 | | | | | | | | | |
| | Post-MPTP 21: treadmill 9.5.2014 | 5 (assessed on 09.05.2014) | 1 | | | | | | | | | |
| | Post-MPTP 22: corridor 4.6.2014 | 5 (assessed on 04.06.2014) | 4 | | | | | | | | | |
| | Post-MPTP 23: treadmill 5.6.2014 | 5 (assessed on 05.06.2014) | 2 | | | | | | | | | |

EP 4 778 565 A2

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 24: treadmill 6.6.2014 | 5 (assessed on 06.06.2014) | 8 | | | | | | | | | |
| | Post-MPTP 25: treadmill 7.6.2014 | 5 (assessed on 07.06.2014) | 4 | | | | | | | | | |
| | Post-MPTP 26: treadmill 24.7.2014 | 5 (assessed on 26.07.2014) | 11 | | | | | | | | | |
| | Post-MPTP 27: treadmill 25.7.2014 | 5 (assessed on 26.07.2014) | 8 | | | | | | | | | |
| | Post-MPTP 28: treadmill 26.7.2014 | 5 (assessed on 26.07.2014) | 5 | | | | | | | | | |
| | Post-MPTP 29: treadmill 28.7.2014 | 5 (assessed on 28.07.2014) | 8 | | | | | | | | | |
| | Post-MPTP 30: treadmill 29.7.2014 | 5 (assessed on 29.07.2014) | 6 | | | | | | | | | |
| | Post-MPTP 31: treadmill 30.7.2014 | 5 (assessed on 30.07.2014) | 5 | | | | | | | | | |
| M7 | Pre-MPTP 1: corridor 2.12.2013 | N/A | 11 | | | | | | | | | |

EP 4 778 565 A2

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Pre-MPTP 1: ladder 2.12.2013 | N/A | 11 | | | | | | | | | |
| | Pre-MPTP 1: treadmill 2.12.2013 | N/A | 8 | | | | | | | | | |
| | Pre-MPTP 2: corridor 4.12.2013 | N/A | 15 | | | | | | | | | |
| | Pre-MPTP 2: ladder 4.12.2013 | N/A | 5 | | | | | | | | | |
| | Pre-MPTP 2: treadmill 4.12.2013 | N/A | 1 | | | | | | | | | |
| | Pre-MPTP 3: corridor 5.12.2013 | N/A | 12 | | | | | | | | | |
| | Pre-MPTP 3: treadmill 5.12.2013 | N/A | 4 | | | | | | | | | |
| | Pre-MPTP 4: corridor 12.12.2013 | N/A | 11 | | | | | | | | | |
| | Pre-MPTP 4: treadmill 12.12.2013 | N/A | 8 | | | | | | | | | |

(continued)

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Pre-MPTP 5: corridor 13.12.2013 | N/A | 10 | | | | | | | | | |
| | Pre-MPTP 5: treadmill 13.12.2013 | N/A | 1 | | | | | | | | | |
| | Post-MPTP 1: treadmill 19.12.2013 | 0 (assessed on 19.12.2013) | 7 | | | | | | | | | |
| | Post-MPTP 2: treadmill 26.12.2013 | 0 (assessed on 26.12.2013) | 1 | | | | | | | | | |
| | Post-MPTP 3: treadmill 2.1.2014 | 3 (assessed on 02.01.2014) | 2 | | | | | | | | | |
| | Post-MPTP 4: treadmill 27.1.2014 | 6 (assessed on 27.01.2014) | 1 | | | | | | | | | |
| | Post-MPTP 5: treadmill 6.2.2014 | 6 (assessed on 06.02.2014) | 2 | | | | | | | | | |
| | Post-MPTP 6: treadmill 13.2.2014 | 6 (assessed on 13.02.2014) | 2 | | | | | | | | | |
| | Post-MPTP 7: treadmill 19.2.2014 | 6 (assessed on 19.02.2014) | 6 | | | | | | | | | |

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
|  | Post-MPTP 8: corridor 20.2.2014 | 6 (assessed on 20.02.2014) | 10 |  |  |  |  |  |  |  |  |  |
|  | Post-MPTP 8: ladder 20.2.2014 | 6 (assessed on 20.02.2014) | 13 |  |  |  |  |  |  |  |  |  |
|  | Post-MPTP 9: treadmill 21.2.2014 | 6 (assessed on 21.02.2014) | 1 |  |  |  |  |  |  |  |  |  |
|  | Post-MPTP 10: corridor 24.2.2014 | 6 (assessed on 24.02.2014) | 12 |  |  |  |  |  |  |  |  |  |
|  | Post-MPTP 10: treadmill 24.2.2014 | 6 (assessed on 24.02.2014) | 2 |  |  |  |  |  |  |  |  |  |
|  | Post-MPTP 11: ladder 26.2.2014 | 6 (assessed on 26.02.2014) | 12 |  |  |  |  |  |  |  |  |  |
|  | Post-MPTP 12: corridor 28.2.2014 | 6 (assessed on 26.02.2014) | 12 |  |  |  |  |  |  |  |  |  |
|  | Post-MPTP 12: treadmill 28.2.2014 | 6 (assessed on 26.02.2014) | 1 |  |  |  |  |  |  |  |  |  |
| **M8** | Pre-MPTP 1: ladder 24.10.2017 | N/A | 9 |  |  |  |  |  |  |  |  |  |

EP 4 778 565 A2

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Pre-MPTP 2: treadmill 25.10.2017 | N/A | 20 | | | | | | | | | |
| | Pre-MPTP 3: corridor 30.11.2017 | N/A | 12 | | | | | | | | | |
| | Pre-MPTP 4: treadmill 30.11.2017 | N/A | 4 | | | | | | | | | |
| | Pre-MPTP 5: corridor 02.12.2017 | N/A | 24 | | | | | | | | | |
| | Pre-MPTP 6: corridor 05.12.2017 | N/A | 8 | 3 at 40Hz 1 at 50 Hz | 3 at 40Hz 2 at 50 Hz 2 at 60 Hz | | | | | | | |
| | Pre-MPTP 7: corridor 07.12.2017 | N/A | 10 | 8 at 40Hz | 9 at 40Hz | 5 at 30Hz 6 at 40Hz 1 at 50Hz | | | | | | |
| | Pre-MPTP 8: corridor 13.12.2017 | N/A | 12 | | | | | | | | | |
| | Post-MPTP 1: treadmill 15.1.2018 | 10 (assessed on 13.01.2018) | 4 | | | | | | | | | |
| | Post-MPTP 1: corridor 15.1.2018 | 10 (assessed on 13.01.2018) | 10 | | | | | | | | | |

EP 4 778 565 A2

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 2: treadmill 27.1.2018 | 10 (assessed on 27.01.2018) | 6 | | | | | | | | | |
| | Post-MPTP 2: corridor 27.1.2018 | 10 (assessed on 27.01.2018) | 4 | | | | | | | | | |
| | Post-MPTP 3: treadmill 29.1.2018 | 10 (assessed on 27.01.2018) | 13 | | | | | | | | | |
| | Post-MPTP 4: treadmill 30.1.2018 | 10 (assessed on 27.01.2018) | 10 | | | | | | | | | |
| | Post-MPTP 4: corridor 30.1.2018 | 10 (assessed on 27.01.2018) | 14 | | | | | | | | | |
| | Post-MPTP 5: corridor 31.1.2018 | 10 (assessed on 03.02.2018) | 20 | | | | | | | | | |
| | Post-MPTP 6: corridor 2.2.2018 | 10 (assessed on 03.02.2018) | 16 | | 8 at 40Hz 9 at 50 Hz 6 at 60 Hz | | | | 7 at 250Hz 6 at 500 Hz | | | |
| | Post-MPTP 6: ladder 2.2.2018 | 10 (assessed on 03.02.2018) | 4 | | 8 at 50 Hz 10 at 60 Hz | | | | | | | |
| | Post-MPTP 7: corridor 3.2.2018 | 10 (assessed on 03.02.2018) | 7 | | 6 at 40Hz 10 at 50 Hz 7 at 60 Hz | 5 at 50Hz | 5 at 50Hz | | | | | |

EP 4 778 565 A2

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| | Post-MPTP 7: ladder 3.2.2018 | 10 (assessed on 03.02.2018) | 7 | | 8 at 50 Hz 4 at 60 Hz | | | | | | | |
| M9 | Pre-MPTP 1: corridor 28.11.2018 | N/A | 14 | | | | | | | | | |
| | Pre-MPTP 2: corridor 02.01.2019 | N/A | 11 | | | | | | | | | |
| | Post-MPTP 1: corridor 21.2.2019 | 10 (assessed on 14.02.2019) | 19 | | | | | | 12 at 20Hz 17 at 125Hz | | | |
| | Post-MPTP 2: corridor 23.2.2019 | 10 (assessed on 14.02.2019) | 10 | | 11 at 80 Hz | | | | | | | |
| | Post-MPTP 3: corridor 25.2.2019 | 10 (assessed on 14.02.2019) | | | | | | | | | | 22 at DBS 125Hz and Brain control at 80Hz |
| | Post-MPTP 4: corridor 28.2.2019 | 10 (assessed on 12.03.2019) | 6 | | | 6 at 40 Hz 5 at 60 Hz 5 at 80 Hz | 6 at 80Hz | | 5 at 80Hz | 6 at 250Hz 5 at 500Hz | | |

EP 4 778 565 A2

| Monkey | Session | PD score | # of blocks Stimulation Off | # of blocks Brain Control: LFS | # of blocks Brain Control: RFS | # of blocks Brain Control: LFS+RFS | # of blocks Brain Control: Both | # of blocks Brain control: Both - anticipated | # of blocks Brain control: Both - delayed | # of blocks Continuous stimulation | # of blocks DBS | # of blocks Brain control with DBS |
|---|---|---|---|---|---|---|---|---|---|---|---|---|
| **M10** | Pre-MPTP 1: corridor 12.12.2017 | N/A | 5 | 1 at 30Hz<br>3 at 40Hz<br>1 at 50 Hz<br>1 at 60 Hz | 1 at 30Hz<br>1 at 40Hz<br>3 at 50 Hz<br>1 at 60 Hz | 4 at 30Hz<br>2 at 40Hz | | | | | | |
| **M11** | Post-MPTP 1: corridor 27.2.2017 | 5 (assessed on 27.02.2017) | 9 | | | 3 at 30Hz<br>8 at 40Hz<br>4 at 50 Hz<br>7 at 60 Hz<br>6 at 80 Hz | | | | | | |
| | Post-MPTP 1: ladder 27.2.2017 | 5 (assessed on 27.02.2017) | 6 | | | 6 at 60 Hz<br>10 at 80 Hz | | | | | | |
| | Post-MPTP 2: corridor 28.2.2017 | 5 (assessed on 27.02.2017) | 11 | | | 10 at 80 Hz | | | | | | |

EP 4 778 565 A2

**Supplementary table 2 | Computed gait parameters from kinematic recordings in NHPs.**

| ..PARAMETER | VARIABLE |
|---|---|
| Gait cycle timing | |
| 1 | Limb side (right or left) |
| 2 | Duration of gait cycle in seconds |
| 3 | Speed of animal |
| 4 | Stance duration in seconds (time between gait cycle onset and stance end) |
| 5 | Swing duration in seconds (time between stance end and gait cycle end) |
| 6 | Stance phase portion of the gait cycle (in percentage of gait cycle) |
| 7 | Time at stance / time of swing onset |
| Step dimensions | |
| 8 | Stride length |
| 9 | Step length |
| 10 | Path length of ankle marker (distance travelled by ankle marker from swing start to the end of the gait cycle) |
| 11 | Maximum backward position (crest - ankle positions) |
| 12 | Maximum forward position of the hip |
| 13 | Max limb length |
| 14 | Step height |
| 15 | Normalized step height |
| Endpoint velocity | |
| 16 | Maximal speed of Foot across the entire gait cycle |
| 17 | Gait cycle phase of the maximal speed of Foot |
| 18 | Acceleration of Foot at swing onset |
| 19 | Speed of Foot at swing onset |
| 20 | Angular velocity of Foot at swing onset |
| Balance | |
| 21 | Lateral displacement across the swing phase |
| 22 | Normalized maximal position of hip marker on Y axis |
| 23 | Normalized minimal position of hip marker on Y axis |
| 24 | Difference between normalized max and min positions of hip marker on Y axis |
| Forward-Backward oscillations | |
| 25 | Min of Crest-Hip elevation angle |
| 26 | Min of Hip-Knee elevation angle |
| 27 | Min of Knee-Ankle elevation angle |
| 28 | Min of Ankle-Foot elevation angle |
| 29 | Min of limb axis (Crest-Foot vector) angle in XY plane |
| 30 | Max of Crest-Hip elevation angle |
| 31 | Max of Hip-Knee elevation angle |
| 32 | Max of Knee-Ankle elevation angle |
| 33 | Max of Ankle-Foot elevation angle |
| 34 | Max of limb axis (Crest-Foot vector) angle in XY plane |
| Joint angles | |
| 35 | Max of Crest-Hip-Knee angle |
| 36 | Max of Hip-Knee-Ankle angle |
| 37 | Max of Knee-Ankle-Foot angle |
| 38 | Max of lateral limb axis (Crest-Foot vector) angle in YZ plane |
| 39 | Min of Crest-Hip-Knee angle |
| 40 | Min of Hip-Knee-Ankle angle |
| 41 | Min of Knee-Ankle-Foot angle |
| 42 | Min of lateral limb axis (Crest-Foot vector) angle in YZ plane |

(continued)

Oscillatory amplitude

| 43 | Difference between max and min of Crest-Hip elevation angle |
| 44 | Difference between max and min of Hip-Knee elevation angle |
| 45 | Difference between max and min of Knee-Ankle elevation angle |
| 46 | Difference between max and min of Ankle-Foot elevation angle |
| 47 | Difference between max and min of Crest-Foot elevation angle |
| 48 | Difference between max and min of Crest-Hip-Knee angle |
| 49 | Difference between max and min of Hip-Knee-Ankle angle |
| 50 | Difference between max and min of Knee-Ankle-Foot angle |
| 51 | Difference between max and min of the lateral limb axis (Crest-Foot vector) angle in YZ plane |

Oscillatory speed

| 52 | Min of limb axis (Crest-Foot vector) angle velocity in XY plane |
| 53 | Min of Hip angle velocity |
| 54 | Min of Knee angle velocity |
| 55 | Min of Ankle angle velocity |
| 56 | Max of limb axis (Crest-Foot vector) angle velocity in XY plane |
| 57 | Max of Hip angle velocity |
| 58 | Max of Knee angle velocity |
| 59 | Max of Ankle angle velocity |
| 60 | Difference between max and min values of limb axis (Crest-Foot vector) angle velocity in XY plane |
| 61 | Difference between max and min values of Hip angle velocity |
| 62 | Difference between max and min values of Knee angle velocity |
| 63 | Difference between max and min values of Ankle angle velocity |

Coordination

| 64 | Phase difference between two elevation angles: Hip-Knee minus Crest-Hip |
| 65 | Phase difference between two elevation angles: Knee-Ankle minus Hip-Knee |
| 66 | Phase difference between two elevation angles: Ankle-Foot minus Knee-Ankle |
| 67 | Gait cycle phase difference between min Crest-Hip elevation angle and min Hip-Knee elevation angle |
| 68 | Gait cycle phase difference between max Crest-Hip elevation angle and max Hip-Knee elevation angle |
| 69 | Gait cycle phase difference between min Hip-Knee elevation angle and min Knee-Ankle elevation angle |
| 70 | Gait cycle phase difference between max Hip-Knee elevation angle and max Knee-Ankle elevation angle |
| 71 | Gait cycle phase difference between min Knee-Ankle elevation angle and min Ankle-Foot elevation angle |
| 72 | Gait cycle phase difference between max Knee-Ankle elevation angle and max Ankle-Foot elevation angle |

Reproducibility

| 73 | PC1 variance resulting from PCA applied on leg elevation angles |
| 74 | PC2 variance resulting from PCA applied on leg elevation angles |
| 75 | PC3 variance resulting from PCA applied on leg elevation angles |
| 76 | Phase of Crest-Hip elevation angle at maximal amplitude |
| 77 | Maximal amplitude of Crest-Hip elevation angle |
| 78 | Phase of Hip-Knee elevation angle at maximal amplitude |
| 79 | Maximal amplitude of Hip-Knee elevation angle |
| 80 | Phase of Knee-Ankle elevation angle at maximal amplitude |
| 81 | Maximal amplitude of Knee-Ankle elevation angle |
| 82 | Phase of Ankle-Foot elevation angle at maximal amplitude |

(continued)

| Reproducibility | |
|---|---|
| 83 | Maximal amplitude of Ankle-Foot elevation angle |

**Supplementary table 3 | Computed gait parameters from kinematic recordings in humans**

| PARAMETER | VARIABLE |
|---|---|
| Gait cycle timing | |
| 1 | Limb side (right or left) |
| 2 | Duration of gait cycle in seconds |
| 3 | Step duration |
| 4 | Stance duration in seconds (time between gait cycle onset and stance end) |
| 5 | Swing duration in seconds (time between stance end and gait cycle end) |
| 6 | Single stance phase portion of the gait cycle (in percentage of gait cycle) |
| 7 | Double stance phase portion of the gait cycle (in percentage of gait cycle) |
| Step dimensions | |
| 8 | Stride length |
| 9 | Step length |
| 10 | Step width |
| 11 | Stride speed |
| 12 | the gait cycle) Path length of ankle marker (distance travelled by ankle marker from swing start to the end of |
| 13 | Path length of toe marker |
| 14 | Step height |
| 15 | Max limb length |
| 16 | Amplitude of lateral movement of the hip |
| 17 | Amplitude of vertical movement of the hip |
| Oscillatory oscillations | |
| 18 | Min of hip-knee joint angle |
| 19 | Max of hip-knee joint angle |
| 20 | Difference between max and min of hip-knee elevation angle |
| 21 | Max of knee-ankle joint angle |
| 22 | Max of knee-ankle joint angle |
| 23 | Difference between max and min of knee-ankle elevation angle |
| 24 | Min of ankle-foot angle |
| 25 | Max of ankle-foot angle |
| 26 | Difference between max and min of ankle-foot angle |
| 27 | Min of leg elevation angle |
| 28 | Max of leg elevation angle |
| 29 | Difference between max and min of leg elevation angle |
| Oscillatory speed | |
| 30 | Max of hip angle velocity |
| 31 | Max of knee angle velocity |
| 32 | Max of ankle angle velocity |
| 33 | Max of limb axis (crest-foot vector) angle velocity in XY plane |
| Reproducibility | |
| 34 | Path length in 3 main PD components |
| 35 | Path width in 3 main PD components |
| 36 | Planarity in 3 main PD components |
| 37 | Phase of hip elevation angle at maximal amplitude |
| 38 | Phase of knee elevation angle at maximal amplitude |

(continued)

Reproducibility

39                Phase of foot elevation angle at maximal amplitude

**Supplementary Table 4 | Criteria used to determine the physiotherapist scores.**

| Item / Score | 5 | 4 | 3 | 2 | 1 | 0 |
|---|---|---|---|---|---|---|
| Safety | Safe | | | Supervision, risk of fall | Close supervision, high risk of fall | Assistance required to avoid fall |
| Fluidity | Good | Reduced | Absent | | | |
| Consistency across gait cycles | Good | Reduced | | Absent | | |
| Symmetry | Symmetric | | Asymmetric | | | |
| Stepping thread | Normal heel to toe sequence | | Abnormal | | | |
| Upper limb mobility | Good | | | Reduced | | |
| Freezing, festination | Absent | | | | Present | |
| Leg crossing | Absent | | | | Present | |

**Supplementary Table 5 | Coefficients to transfer EMG activity of muscles into activity of spinal segments.**

| Spinal segment | Iliopsoas | Rectus Femoris and Vastus Lateralis | Tibialis Anterior | Medial Gastrocnemius |
|---|---|---|---|---|
| L1 | 0.45 | 0.12 | 0 | 0 |
| L2 | 0.3 | 0.27 | 0 | 0 |
| L3 | 0.17 | 0.37 | 0.04 | 0.04 |
| L4 | 0.06 | 0.24 | 0.34 | 0.34 |
| L5 | 0.01 | 0 | 0.43 | 0.43 |
| S1 | 0.01 | 0 | 0.19 | 0.19 |
| S2 | 0 | 0 | 0 | 0 |

## Bibliography

1 Stolze, H. et al. Prevalence of gait disorders in hospitalized neurological patients. Mov Disord 20, 89-94, doi:10.1002/mds.20266 (2005).

2 Snijders, A. H. et al. Physiology of freezing of gait. Ann Neurol 80, 644-659, doi:10.1002/ana.24778 (2016).

3 Capogrosso, M. et al. A brain-spine interface alleviating gait deficits after spinal cord injury in primates. Nature 539, 284-288, doi:10.1038/nature20118 (2016).

4 Bezard, E., Imbert, C., Deloire, X., Bioulac, B. & Gross, C. E. A chronic MPTP model reproducing the slow evolution of Parkinson's disease: evolution of motor symptoms in the monkey. Brain Res 766, 107-112 (1997).

5 Wagner, F. B. et al. Targeted neurotechnology restores walking in humans with spinal cord injury. Nature 563, 65-71, doi:10.1038/s41586-018-0649-2 (2018).

6 Ajiboye, A. B. et al. Restoration of reaching and grasping movements through brain-controlled muscle stimulation in a person with tetraplegia: a proof-of-concept demonstration. Lancet, doi:10.1016/S0140-6736(17)30601-3 (2017).

7 Bezard, A. L. et al. An exoskeleton controlled by an epidural wireless brain-machine interface in a tetraplegic patient: a proof-of-concept demonstration. Lancet Neurol, doi:10.1016/S1474-4422(19)30321-7 (2019).

8 Bezard, E. et al. Relationship between the appearance of symptoms and the level of nigrostriatal degeneration in a progressive 1-methyl-4-phenyl-1,2,3,6-tetrahydropyridine-lesioned macaque model of Parkinson's disease. The Journal of neuroscience : the official journal of the Society for Neuroscience 21, 6853-6861 (2001).

9 Knutsson, E. An analysis of Parkinsonian gait. Brain : a journal of neurology 95, 475-486 (1972).

10 Creaby, M. W. & Cole, M. H. Gait characteristics and falls in Parkinson's disease: A systematic review and meta-analysis. Parkinsonism & related disorders, doi:10.1016/j.parkreldis.2018.07.008 (2018).

11 Imbert, C., Bezard, E., Guitraud, S., Boraud, T. & Gross, C. E. Comparison of eight clinical rating scales used for the assessment of MPTP-induced parkinsonism in the Macaque monkey. J Neurosci Methods 96, 71-76, doi:10.1016/s0165-0270(99)00184-3 (2000).

12 Friedli, L. et al. Pronounced species divergence in corticospinal tract reorganization and functional recovery after lateralized spinal cord injury favors primates. Science translational medicine 7, 302ra134, doi:10.1126/scitranslmed.aac5811 (2015).

13 Courtine, G. et al. Transformation of nonfunctional spinal circuits into functional states after the loss of brain input. Nature Neuroscience 12, 1333-1342, doi:10.1038/nn.2401 (2009).

14 Lozano, A. M. & Lipsman, N. Probing and regulating dysfunctional circuits using deep brain stimulation. Neuron 77, 406-424, doi:10.1016/j.neuron.2013.01.020 (2013).

15 Santana, M. B. et al. Spinal cord stimulation alleviates motor deficits in a primate model of Parkinson disease. Neuron 84, 716-722, doi:10.1016/j.neuron.2014.08.061 (2014).

16 MacDonald, V. & Halliday, G. M. Selective loss of pyramidal neurons in the presupplementary motor cortex in Parkinson's disease. Mov Disord 17, 1166-1173, doi:10.1002/mds.10258 (2002).

17 Goldberg, J. A. et al. Enhanced synchrony among primary motor cortex neurons in the 1-methyl-4-phenyl-1,2,3,6-tetrahydropyridine primate model of Parkinson's disease. The Journal of neuroscience : the official journal of the Society for Neuroscience 22, 4639-4653, doi:20026424 (2002).

18 Pozzi, N. G. et al. Freezing of gait in Parkinson's disease reflects a sudden derangement of locomotor network dynamics. Brain : a journal of neurology 142, 2037-2050, doi:10.1093/brain/awz141 (2019).

19 Cagnan, H. et al. Temporal evolution of beta bursts in the parkinsonian cortical and basal ganglia network. Proc Natl Acad Sci U S A 116, 16095-16104, doi:10.1073/pnas.1819975116 (2019).

20 de Hemptinne, C. et al. Therapeutic deep brain stimulation reduces cortical phase-amplitude coupling in Parkinson's disease. Nat Neurosci 18, 779-786, doi:10.1038/nn.3997 (2015).

21 Burciu, R. G. & Vaillancourt, D. E. Imaging of Motor Cortex Physiology in Parkinson's Disease. Mov Disord 33, 1688-1699, doi:10.1002/mds.102 (2018).

22 Pasquereau, B. & Turner, R. S. Primary motor cortex of the parkinsonian monkey: differential effects on the spontaneous activity of pyramidal tract-type neurons. Cereb Cortex 21, 1362-1378, doi:10.1093/cercor/bhq217 (2011).

23 Pasquereau, B., DeLong, M. R. & Turner, R. S. Primary motor cortex of the parkinsonian monkey: altered encoding of active movement. Brain : a journal of neurology 139, 127-143, doi:10.1093/brain/awv312 (2016).

24 de Hemptinne, C. et al. Exaggerated phase-amplitude coupling in the primary motor cortex in Parkinson disease. Proc Natl Acad Sci U S A 110, 4780-4785, doi:10.1073/pnas.1214546110 (2013).

25 Devergnas, A., Pittard, D., Bliwise, D. & Wichmann, T. Relationship between oscillatory activity in the cortico-basal ganglia network and parkinsonism in MPTP-treated monkeys. Neurobiology of disease 68, 156-166, doi:10.1016/j.nbd.2014.04.004 (2014).

26 Yin, M. et al. Wireless Neurosensor for Full-Spectrum Electrophysiology Recordings during Free Behavior. Neuron 84, 1170-1182, doi:10.1016/j.neuron.2014.11.010 (2014).

27 Yu, H., Sternad, D., Corcos, D. M. & Vaillancourt, D. E. Role of hyperactive cerebellum and motor cortex in Parkinson's disease. Neuroimage 35, 222-233, doi:10.1016/j.neuroimage.2006.11.047 (2007).

28 Sabatini, U. et al. Cortical motor reorganization in akinetic patients with Parkinson's disease: a functional MRI study. Brain : a journal of neurology 123 ( Pt 2), 394-403, doi:10.1093/brain/123.2.394 (2000).

29 Eckert, T., Peschel, T., Heinze, H. J. & Rotte, M. Increased pre-SMA activation in early PD patients during simple self-initiated hand movements. J Neurol 253, 199-207, doi:10.1007/s00415-005-0956-z (2006).

30 Catalan, M. J., Ishii, K., Honda, M., Samii, A. & Hallett, M. A PET study of sequential finger movements of varying length in patients with Parkinson's disease. Brain : a journal of neurology 122 ( Pt 3), 483-495, doi:10.1093/brain/122.3.483 (1999).

31 Turner, R. S., Grafton, S. T., McIntosh, A. R., DeLong, M. R. & Hoffman, J. M. The functional anatomy of parkinsonian bradykinesia. Neuroimage 19, 163-179 (2003).

32 Fuentes, R., Petersson, P., Siesser, W. B., Caron, M. G. & Nicolelis, M. A. Spinal cord stimulation restores locomotion in animal models of Parkinson's disease. Science 323, 1578-1582, doi:10.1126/science.1164901 (2009).

33 Gallego, J. A. et al. Cortical population activity within a preserved neural manifold underlies multiple motor behaviors. Nature communications 9, 4233, doi:10.1038/s41467-018-06560-z (2018).

34 Yakovenko, S., Mushahwar, V., VanderHorst, V., Holstege, G. & Prochazka, A. Spatiotemporal activation of lumbosacral motoneurons in the locomotor step cycle. J Neurophysiol 87, 1542-1553 (2002).

35 Cappellini, G., Ivanenko, Y. P., Dominici, N., Poppele, R. E. & Lacquaniti, F. Migration of motor pool activity in the spinal cord reflects body mechanics in human locomotion. J Neurophysiol 104, 3064-3073, doi:10.1152/jn.00318.2010 (2010).

36 Capogrosso, M. et al. Configuration of electrical spinal cord stimulation through real-time processing of gait kinematics. Nat Protoc 13, 2031-2061, doi:10.1038/s41596-018-0030-9 (2018).

37 Formento, E. et al. Electrical spinal cord stimulation must preserve proprioception to enable locomotion in humans with spinal cord injury. Nat Neurosci 21, 1728-1741, doi:10.1038/s41593-018-0262-6 (2018).

38 Schuettler, M., Stiess, S., King, B. V. & Suaning, G. J. Fabrication of implantable microelectrode arrays by laser cutting of silicone rubber and platinum foil. J Neural Eng 2, S121-128, doi:10.1088/1741-2560/2/1/013 (2005).

39 Minev, I. R. et al. Biomaterials. Electronic dura mater for long-term multimodal neural interfaces. Science 347, 159-163, doi:10.1126/science.1260318 (2015).

40 Wenger, N. et al. Spatiotemporal neuromodulation therapies engaging muscle synergies improve motor control after spinal cord injury. Nature medicine 22, 138-145, doi:10.1038/nm.4025 (2016).

41 Revuelta, G. J., Uthayathas, S., Wahlquist, A. E., Factor, S. A. & Papa, S. M. Non-human primate FOG develops with advanced parkinsonism induced by MPTP Treatment. Experimental neurology 237, 464-469, doi:10.1016/j.expneurol.2012.07.021 (2012).

42 Pinto de Souza, C. et al. Spinal cord stimulation improves gait in patients with Parkinson's disease previously treated with deep brain stimulation. Mov Disord 32, 278-282, doi:10.1002/mds.26850 (2017).

43 de Lima-Pardini, A. C. et al. Effects of spinal cord stimulation on postural control in Parkinson's disease patients with freezing of gait. Elife 7, doi:10.7554/eLife.37727 (2018).

44 Nishioka, K. & Nakajima, M. Beneficial Therapeutic Effects of Spinal Cord Stimulation in Advanced Cases of Parkinson's Disease With Intractable Chronic Pain: A Case Series. Neuromodulation 18, 751-753, doi:10.1111/ner.12315 (2015).

45 St George, R. J., Nutt, J. G., Burchiel, K. J. & Horak, F. B. A meta-regression of the long-term effects of deep brain stimulation on balance and gait in PD. Neurology 75, 1292-1299, doi:10.1212/WNL.0b013e3181f61329 (2010).

46 Stuart, S. et al. Cortical activity during walking and balance tasks in older adults and in people with Parkinson's disease: A structured review. Maturitas 113, 53-72, doi:10.1016/j.maturitas.2018.04.011 (2018).

47 McCabe, G., Moore, D., Introduction to the Practice of Statistics. Nurse Res 13, 89-90, doi:10.7748/nr.13.2.89.s12 (2005).

48 Ahmed, M. et al. Lentiviral overexpression of GRK6 alleviates L-dopa-induced dyskinesia in experimental Parkinson's disease. Sci Transl Med 2, 28ra28 (2010).

49 Bezard, E. et al. Attenuation of levodopa-induced dyskinesia by normalizing dopamine D3 receptor function. Nature medicine 9, 762-767, doi:10.1038/nm875 (2003).

50 Bezard, E. et al. The mGluR5 negative allosteric modulator dipraglurant reduces dyskinesia in the MPTP macaque model. Movement disorders : official journal of the Movement Disorder Society 29, 1074-1079, doi:10.1002/mds.25920 (2014).

51 Fasano, S. et al. Inhibition of Ras-guanine nucleotide-releasing factor 1 (Ras-GRF1) signaling in the striatum reverts motor symptoms associated with L-dopa-induced dyskinesia. Proc Natl Acad Sci U S A 107, 21824-21829, doi:10.1073/pnas.1012071107 (2010).

52 Porras, G. et al. PSD-95 expression controls I-DOPA dyskinesia through dopamine D1 receptor trafficking. J Clin Invest 122, 3977-3989, doi:59426 [pii] 10.1172/JCI59426 (2012).

53 Shen, W. et al. M4 Muscarinic Receptor Signaling Ameliorates Striatal Plasticity Deficits in Models of L-DOPA-Induced Dyskinesia. Neuron 88, 762-773, doi:10.1016/j.neuron.2015.10.039 (2015).

54 Urs, N. M. et al. Targeting beta-arrestin2 in the treatment of L-DOPA-induced dyskinesia in Parkinson's disease. Proc Natl Acad Sci U S A 112, E2517-2526, doi:10.1073/pnas.1502740112 (2015).

55 Meissner, W. et al. Time-course of nigrostriatal degeneration in a progressive MPTP-lesioned macaque model of Parkinson's disease. Mol Neurobiol 28, 209-218, doi:10.1385/MN:28:3:209 (2003).

56 Courtine, G. et al. Kinematic and EMG determinants in quadrupedal locomotion of a non-human primate (Rhesus). J Neurophysiol 93, 3127-3145, doi:10.1152/jn.01073.2004 (2005).

57 Tass, P. A. et al. Coordinated reset has sustained aftereffects in Parkinsonian monkeys. Ann Neurol 72, 816-820, doi:10.1002/ana.23663 (2012).

58 Moraud, E. M. et al. Mechanisms Underlying the Neuromodulation of Spinal Circuits for Correcting Gait and Balance Deficits after Spinal Cord Injury. Neuron 89, 814-828, doi:10.1016/j.neuron.2016.01.009 (2016).

59 Capogrosso, M. et al. A computational model for epidural electrical stimulation of spinal sensorimotor circuits. The Journal of neuroscience : the official journal of the Society for Neuroscience 33, 19326-19340, doi:10.1523/JNEUROSCI.1688-13.2013 (2013).

60 Baufreton, J. et al. Inhaling xenon ameliorates I-dopa-induced dyskinesia in experimental parkinsonism. Mov Disord, doi:10.1002/mds.27404 (2018).

61 Zampieri, C. et al. The instrumented timed up and go test: potential outcome measure for disease modifying therapies in Parkinson's disease. J Neurol Neurosurg Psychiatry 81, 171-176, doi:10.1136/jnnp.2009.173740 (2010).

62 Palmerini, L., Mellone, S., Avanzolini, G., Valzania, F. & Chiari, L. Quantification of motor impairment in Parkinson's disease using an instrumented timed up and go test. IEEE Trans Neural Syst Rehabil Eng 21, 664-673, doi:10.1109/tnsre.2012.2236577 (2013).

63 Gallego, J. A., Perich, M. G., Miller, L. E. & Solla, S. A. Neural Manifolds for the Control of Movement. Neuron 94, 978-984, doi:10.1016/j.neuron.2017.05.025 (2017).

64 Chestek, C. A. et al. Long-term stability of neural prosthetic control signals from silicon cortical arrays in rhesus macaque motor cortex. J Neural Eng 8, 045005, doi:10.1088/1741-2560/8/4/045005 (2011).

65 Santhanam, G. et al. HermesB: a continuous neural recording system for freely behaving primates. IEEE Trans Biomed Eng 54, 2037-2050, doi:10.1109/tbme.2007.895753 (2007).

66 Linderman, M. D. et al. Neural recording stability of chronic electrode arrays in freely behaving primates. Conf Proc IEEE Eng Med Biol Soc 1, 4387-4391, doi:10.1109/iembs.2006.260814 (2006).

67 Dickey, A. S., Suminski, A., Amit, Y. & Hatsopoulos, N. G. Single-unit stability using chronically implanted multielectrode arrays. J Neurophysiol 102, 1331-1339, doi:90920.2008 [pii] 10.1152/jn.90920.2008 (2009).

68 Rokni, U., Richardson, A. G., Bizzi, E. & Seung, H. S. Motor learning with unstable neural representations. Neuron 54, 653-666, doi:10.1016/j.neuron.2007.04.030 (2007).

69 Taylor, D. M., Tillery, S. I. & Schwartz, A. B. Direct cortical control of 3D neuroprosthetic devices. Science 296, 1829-1832, doi:10.1126/science.1070291 (2002).

70 Downey, J. E., Schwed, N., Chase, S. M., Schwartz, A. B. & Collinger, J. L. Intracortical recording stability in human brain-computer interface users. J Neural Eng 15, 046016, doi:10.1088/1741-2552/aab7a0 (2018).

71 Perge, J. A. et al. Reliability of directional information in unsorted spikes and local field potentials recorded in human motor cortex. J Neural Eng 11, 046007, doi:10.1088/1741-2560/11/4/046007 (2014).

72 Perge, J. A. et al. Intra-day signal instabilities affect decoding performance in an intracortical neural interface system. J Neural Eng 10, 036004, doi:10.1088/1741-2560/10/3/036004 (2013).

73 Simeral, J. D., Kim, S. P., Black, M. J., Donoghue, J. P. & Hochberg, L. R. Neural control of cursor trajectory and click by a human with tetraplegia 1000 days after implant of an intracortical microelectrode array. J Neural Eng 8, 025027, doi:10.1088/1741-2560/8/2/025027 (2011).

74 Barrese, J. C. et al. Failure mode analysis of silicon-based intracortical microelectrode arrays in non-human primates. J Neural Eng 10, 066014, doi:10.1088/1741-2560/10/6/066014 (2013).

75 Bjornsson, C. S. et al. Effects of insertion conditions on tissue strain and vascular damage during neuroprosthetic device insertion. J Neural Eng 3, 196-207, doi:S1741-2560(06)17189-X [pii] 10.1088/1741-2560/3/3/002 (2006).

76 Shain, W. et al. Controlling cellular reactive responses around neural prosthetic devices using peripheral and local intervention strategies. IEEE Trans Neural Syst Rehabil Eng 11, 186-188, doi:10.1109/TNSRE.2003.814800 (2003).

77 Welch, P. The use of fast Fourier transform for the estimation of power spectra: A method based on time averaging over short, modified periodograms. IEEE Transactions on Audio and Electroacoustics 15, 70-73, doi:10.1109/TAU.1967.1161901 (1967).

78 McLachlan, G. J. & Peel, D. Finite mixture models. (Wiley, 2000).

79 Milekovic, T. et al. Stable long-term BCI-enabled communication in ALS and locked-in syndrome using LFP signals. J Neurophysiol 120, 343-360, doi:10.1152/jn.00493.2017 (2018).

80 Milekovic, T., Ball, T., Schulze-Bonhage, A., Aertsen, A. & Mehring, C. Detection of error related neuronal responses recorded by electrocorticography in humans during continuous movements. PLoS One 8, e55235, doi:10.1371/journal.pone.0055235 (2013).

81 Fasano, A., Aquino, C. C., Krauss, J. K., Honey, C. R. & Bloem, B. R. Axial disability and deep brain stimulation in patients with Parkinson disease. Nat Rev Neurol 11, 98-110, doi:10.1038/nrneurol.2014.252 (2015).

82 Moreau, C. et al. STN-DBS frequency effects on freezing of gait in advanced Parkinson disease. Neurology 71, 80-84, doi:10.1212/01.wnl.0000303972.16279.46 (2008).

83 Rosenblad, C. et al. Vector-mediated l-3,4-dihydroxyphenylalanine delivery reverses motor impairments in a primate model of Parkinson's disease. Brain : a journal of neurology 142, 2402-2416, doi:10.1093/brain/awz176 (2019).

84 Friedman, J. Regularized Discriminant-Analysis. J Am Stat Assoc, 165-175 (1989).

85 Gallego, J. A., Perich, M. G., Chowdhury, R., Solla, S. A. & Miller, L. E. Long-term stability of cortical population dynamics underlying consistent behavior. Nature Neurosci. (in press).

**References**

10, 110, 210, 310     Neuromodulation/neurostimulation system
12, 112, 212, 312     Stimulation unit

| | |
|---|---|
| 13, 113, 213, 313 | Implantable Pulse Generator (IPG) |
| 14, 114, 214, 314 | Epidurally implanted electrodes |
| 150 | Cortical neurosensor |
| 250 | Muscle activity sensor |
| 260 | Behavioral sensor |
| 350 | Control sensor |
| 151, 251, 351 | Controller device |
| 152, 252, 352 | Communicator device |
| | |
| 400 | System to provide neuromodulation inter alia for the LH |
| | |
| 412 | Stimulation unit |
| 413 | Implantable Pulse Generator (IPG) |
| 414 | Epidurally implanted electrodes |
| | |
| 450 | Cortical Neurosensor |
| 451 | Controller |
| 452 | Communicator |
| 460 | Implanted current generator |
| 462 | DBS leads for stimulation of LH |
| 464 | DBS neuromodulation element for stimulation of left LH |
| 466 | DBS neuromodulation element for stimulation of right LH |
| 468 | Behavioral Sensor |
| 470 | Muscle Sensor |
| 472 | Control Sensors |
| | |
| M | Muscles |
| S | Spinal cord |
| P | Subject / Patient |

**Claims**

1. A neuromodulation system (110) for mitigating locomotor deficits of a subject (P), the system (110) comprising:

   at least one cortical neurosensor (150) configured to record a cortical activity of the subject (P);
   at least one controller device (151) configured to receive signals from the at least one cortical neurosensor (150), process the received signals to determine one or more parameters regarding movement intentions of the subject (P), and generate one or more stimulation parameters based on the determined one or more movement intention parameters;
   at least one stimulation unit (112) comprising one or more electrodes (114) configured to be implanted epidurally over the dorsal side of the spinal cord (S) of the subject (S) and apply epidural electrical stimulation, EES;
   at least one implantable pulse generator, IPG, (113) operatively connected to the one or more electrodes (114); and
   at least one communicator device (152) configured to communicate the generated one or more stimulation parameters from the at least one controller device (151) to the at least one IPG (113),

   wherein the at least one IPG (113) is configured to operate the one or more electrodes (114) to deliver EES to the dorsal side of the spinal cord (S) in accordance with the generated one or more stimulation parameters.

2. The system (110) of claim 1, wherein the at least one cortical neurosensor (150) comprises one more among: penetrating intracortical microelectrode(s), subdural electrocorticography (ECoG) electrode(s), epidural electrocorticography electrode(s), and stereotactic EEG electrode(s).

3. The system (110) of claim 1 or 2, wherein the at least one cortical neurosensor (150) is configured to be placed at the primary motor cortex of the subject (P).

4. The system (110) of claim 3, wherein the at least one cortical neurosensor (150) is configured to be placed at the leg primary motor cortex, preferably at both left and right leg primary motor cortex.

5. The system (110) of any one of claims 1 to 4, wherein the at least one controller device (151) is configured to detect initiation events of spatially-restricted motor neuron activation hotspots in the spinal cord from the cortical activity, and wherein the at least one IPG (113) is configured to control the one or more electrodes (114) to deliver EES in synchrony with the detected hotspot initiation events.

6. The system (110) of claim 5, wherein the hotspot initiation events comprise at least one of: a weight acceptance hotspot initiation event, a propulsion hotspot initiation event, and a leg lift hotspot initiation event, for each of the left leg and the right leg of the subject (P).

7. The system (110) of any one of the preceding claims, wherein the at least one controller device (151) is configured to implement a regularized linear discriminant analysis (rLDA) decoder, and wherein a hotspot initiation event is detected when a probability output of the decoder exceeds a predefined threshold, optionally a threshold of 0.8.

8. The system (110) of any one of the preceding claims, wherein the at least one communicator device (152) is configured to communicate the stimulation parameters to the at least one IPG (113) with a latency of 200 ms or less from detection of a movement intention event, preferably a latency of approximately 105 ms.

9. The system (110) of any one of the preceding claims, wherein the one or more electrodes (114) are configured to target posterior roots of the lumbosacral spinal cord at segments from L2 to S1, and wherein the at least one IPG (113) is configured to control the one or more electrodes (114) to deliver EES at a frequency in the range of 20-100 Hz, preferably in the range of 50-70 Hz, more preferably of about 60 Hz.

10. The system (110) of any one of the preceding claims, further comprising a Deep Brain Stimulation, DBS, system configured to deliver DBS to brain tissue of the subject (S).

11. The system (110) of claim 10, wherein the DBS system is configured to deliver DBS to the subthalamic nucleus, STN.

12. The system (110) of claim 10, wherein the DBS system is configured to deliver DBS to the lateral hypothalamus, LH.

13. The system (110) of any preceding claim, wherein the locomotor deficits are associated with Parkinson's disease, PD.

14. The system (110) of any preceding claim, further comprising a module configured to administer a pharmacological agent to the subject (P), optionally wherein the pharmacological agent is levodopa.

15. The system (110) of any one of claims 1 to 12, wherein the locomotor deficits result from spinal cord injury, SCI.

10

Person

P

13 — Implanted current generator

Spinal column

14 — Spinal stimulation electrodes

Spinal cord

12

S

Dorsal side

## Fig. 1

110

150 — Cortical neurosensor

Person

P

151 — Controler

Brain

Cortex

113

152 — Communicator

Implanted current generator

Spinal column

114 — Spinal stimulation electrodes

Spinal cord

112

S

Dorsal side

## Fig. 2

Fig. 3

Fig. 4

IPG case

Fig. 5a

Specify 5-6-5

Fig. 5b

| Protocol | Cathode | Anode | Current | Pulsewidth | Frequency |
|---|---|---|---|---|---|
| Left knee extension | 5 | 3 | 0.6mA | 300µs | 60Hz |
| Left knee extension boost | 5 | 3 | 0.7mA | 300µs | 60Hz |
| Left ankle extension | 15 | 7 | 0.4mA | 300µs | 60Hz |
| Left hip flexion | 1 | 0 | 1.4mA | 300µs | 60Hz |
| Left ankle flexion | 7 | IPG case | 0.5mA | 200µs | 60Hz |
| Right knee extension | 10 | 9 | 0.9mA | 300µs | 60Hz |
| Right knee extension boost | 10 | 9 | 1mA | 300µs | 60Hz |
| Right ankle extension | 14 | 12 | 0.5mA | 300µs | 60Hz |
| Right hip flexion | 8 | IPG case | 1.7mA | 300µs | 60Hz |
| Right ankle flexion | 12 | 14 | 0.6mA | 200µs | 60Hz |

Fig. 6

| Sequence | Protocol | 0 – 0.3s | 0.3-0.7s | 0.7-1.4s |
|---|---|---|---|---|
| Left Foot Off | Left knee extension | | | |
| | Left knee extension boost | | | |
| | Right knee extension boost | | | |
| | Right ankle extension | | | |
| | Left hip flexion | | | |
| | Left knee flexion | | | |
| Right Foot Off | Right knee extension | | | |
| | Right knee extension boost | | | |
| | Left knee extension boost | | | |
| | Left ankle extension | | | |
| | Right hip flexion | | | |
| | Right knee flexion | | | |

## Fig. 7

## Fig. 8

| Animal ID | Macaque species | Walking experiments before MPTP | Walking experiments after MPTP | Wireless muscle activity system | Micro electrode array | Spike sorting | Spinal implant | Single pulse spinal cord stimulation | Brain-control stimulation before MPTP | Brain-control stimulation after MPTP |
|---|---|---|---|---|---|---|---|---|---|---|
| M1 | Mulata | ✓ | ✓ | - | - | - | - | - | - | - |
| M2 | Mulata | ✓ | ✓ | - | - | - | - | - | - | - |
| M3 | Mulata | ✓ | ✓ | - | - | - | - | - | - | - |
| M4 | Mulata | ✓ | ✓ | - | - | - | - | - | - | - |
| M5 | Mulata | ✓ | ✓ | ✓ | ✓ | - | - | - | - | - |
| M6 | Mulata | ✓ | ✓ | ✓ | ✓ | ✓ | - | - | - | - |
| M7 | Mulata | ✓ | ✓ | ✓ | ✓ | ✓ | - | - | - | - |
| M8 | Mulata | ✓ | ✓ | ✓ | ✓ | - | ✓,& | ✓ | ✓ | ✓ |
| M9 | Fascicularis | ✓ | ✓ | ✓ | ✓ | - | ✓,& | ✓ | - | ✓ |
| M10 | Mulata | - | - | ✓ | ✓ | - | ✓,& | ✓ | ✓ | * |
| M11 | Mulata | - | - | * | ✓ | - | ✓,º | * | - | ✓ |

✓ Performed

- Not performed

* Could not be performed due to technical issues or health conditions

& Commercial spinal implant

º *e-dura* spinal implant

Fig. 9

Fig. 10

Fig. 10 (Continued)

Fig. 11

Fig. 12

Fig. 13

Fig. 13(Continued)

Fig. 14

Fig. 15

Fig. 15(Continued)

a  Spatiotemporal map of motor neuron activity before MPTP administration

M6   M7   M8   M9   M10

stance  swing  stance  swing  stance  swing  stance  swing  stance  swing

■ Weigth acceptance ■ Propulsion ■ Leg lift

b  Spatiotemporal map of motor neuron activity after MPTP administration

M6   M7   M8   M9

stance  swing  stance  swing  stance  swing  stance  swing

2D correlation to a map of motor neuron activity before MPTP

M6   M7   M8   M9

(R)

Before MPTP  After MPTP

Fig. 16

EP 4 778 565 A2

C Design of hotspot stimulation protocols

**M8**

Right weight acceptance — 1.1mA, 2mA — 0% 70% 0% 100%

Right propulsion — 1mA, 2mA — 0% 10% 0% 100%

Right leg lift — 4.1mA, 7mA — 0% 19% 0% 100%

**M9**

Right weight acceptance — 0.2mA, 0.6mA — 0% 12% 0% 100%

Right propulsion — 1.5mA, 1.7mA — 0% 15% 0% 100%

Right leg lift — 0.3mA, 0.7mA — 0% 35% 0% 100%

**M10**

Right weight acceptance — 1.1mA, 2mA — 0% 9% 0% 100%

Right propulsion — 0.8mA, 2mA — 0% 16% 0% 100%

Right leg lift — 7.6mA, 10mA — 0% 50% 0% 100%

Fig. 16 (Continued)

**a** Fabrication of the implant

**b** Planed position of the implant

# Fig. 17

Fig. 18

Fig. 19

Fig. 20

Fig. 20(Continued)

Fig. 21

Fig. 21(Continued)

Fig. 22

Fig. 22 (Continued)

Fig. 22 (Continued)

**a** Gait modulation during continuous dorsal column stimulation

Fig. 23

**b**

Fig. 23 (Continued)

**C** Spatiotemporal map of motor neuron activation

**d** Overground crossing performance

Fig. 23 (Continued)

Fig. 24

**a** Non-human primates: Gait recording platform

Muscle activity (EMG)
Electromyography (EMG) implanted sensors

EMG recording

Corridor

3D motion tracking

Kinematic recording

Leg kinematics

Analysis

**b** Healthy

MPTP

**c**

PD score

Late stage

Early stage

M6
M7

**d** Non-human primates: Kinematic characterisation

PC3 (8%)

M7
M6

(n=9)

PC1 (30%)

Stride length (cm)

Amplitude of leg oscillation (deg)

Max endpoint velocity (m/s)

Lateral hip displacement (a.u.)

Before MPTP       After MPTP

**e** Non-human primates: Motor neuron activation dynamics

Il
RF
ST
MG
FHL
GLU
EDL
TA

stance   swing
Time (s)

L1
L2
L3
L4
L5
L6
L7

Il

TA

Motor neuron pool distribution

L1
L2
L3
L4
L5
L6
L7

stance   swing

Before MPTP Hotspots

L1
L2
L3
L4
L5
L6
L7

stance   swing

0          1

Flexion hip

Propulsion

Flexion ankle

Weight acceptance

stance   swing

0          1
Normalized Gaussian fit

MPTP

L1
L2
L3
L4
L5
L6
L7

stance   swing

0          1
Normalized motor neuron activation

# Fig. 25

**f** Humans: Gait recording platform

**g** Optimal biomechanical model (Target)

Individual with Parkinson's disease (P1)

**h** **i** Humans: Kinematic characterisation

**j** Humans: Motor neuron activation dynamics

Fig. 25 (Continued)

Fig. 26

**e** Brain decoding in NHPs

**f** Brain decoding in humans

Fig. 26 (Continued)

Fig. 27

d

DBS$^{ON}$-EES$^{OFF}$    DBS$^{ON}$-EES$^{ON}$    Target

L1
L2
L3
L4
L5
S1

stance    swing    stance    swing    stance    swing

0.7

Correlation to target spinal map

0.2

0    1
Normalized motor neuron activation

e

Physiotherapist's gait quality score

5

0

Parkinson's disease    Healthy

PC2 (9%)

P1

PC1 (47%)

35

Distance to target (au)

25

f

60%

Portion of steps classified as healthy

0%

Target    DBS$^{OFF}$-EES$^{OFF}$    DBS$^{ON}$-EES$^{OFF}$    DBS$^{OFF}$-EES$^{ON}$    DBS$^{ON}$-EES$^{ON}$

Fig. 27 (Continued)

EP 4 778 565 A2

129

Fig. 28

Fig. 28 (Continued)

Fig. 29

Fig. 30

b     Generation of a personalized neurobiomechanical model

**Step 1**: Personalize a Lower Limb musculoskeletal model     **Step 2**: Optimize a reflex-based gait controller

Fig. 30 (Continued)

Muscles abbreviations | **BFSH**: Biceps Femoris Short Head, **Gas**: Gastrocnemius, **GMax**: Gluteus Maximus, **HAMS**: Hamstring muscles, **IL**: Iliopsoas, **Sol**: Soleus, **TA**: Tibialis Anterior, **Vas**: Vasti

Gait cycles stages | **ES**: Early Stnace, **MS**: Mid-Stance, **PS**: Pre-Swing, **LP**: Landing Preparation

Low level control laws | **C**: Constant signal, **L**: muscle Length, **V**: muscle Velocity, **PD**: Proportional-Derivative control, **F**: muscle Force, ±: positive and negative feedback

Fig. 31

Fig. 32

Fig. 34

DBS OFF

DBS ON

Flexor

Flexor

Extensor

Extensor

0.5 V

5s

5s

Fig. 35

400

Person

Deep brain
stimulation leads

Implanted
current
generator

Left
hypothalamus

Right
hypothalamus

Brain

P

460

464

466

462

Fig. 36

Fig. 37

Fig. 38

400

468

470

451

452                    413

                            414

Behavioral
sensors

Muscle
activity
sensors

Controller

Communicator

460    462    464

Implanted
current
generator

Muscles

Implanted
current
generator

M

412

Deep brain
stimulation leads

Left
hypothalamus

Right
hypothalamus

Brain

Spinal cord

Spinal
stimulation
electrodes

Lumbosacral
spinal
cord

Dorsal side

Person

P

466

S

Fig. 39

400

472

451

452                    413

                            414

Control
sensors

Controller

Communicator

460    462    464

Implanted
current
generator

Implanted
current
generator

412

Deep brain
stimulation leads

Left
hypothalamus

Right
hypothalamus

Brain

Spinal cord

Spinal
stimulation
electrodes

Lumbosacral
spinal
cord

Dorsal side

Person

P

466

S

Fig. 40

Fig. 41

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **NOGA**. Combined neuromodulatory approaches in the central nervous system for treatment of spinal cord injury. *Current Opinion in Neurology*, December 2021, vol. 34 (6)
- **BAIZABAL-CARVALLO, JF**. Low-frequency deep brain stimulation for movement disorders. *Parkinsonism & Related Disorders*, October 2016, vol. 31, 14-22
- **CHO et al.** *Neurosurgery, unbiased interrogation of whole brain circuits identifies neurons that restore walking after spinal cord injury*, April 2022, vol. 68, 61
- **BONIZZATO et al.** Multi-pronged neuromodulation intervention engages the residual motor circuitry to facilitate walking in a rat model of spinal cord injury. *Nature Communications*, 2021, vol. 12
- **BAIZABAL-CARVALLO et al.** Low-Frequency Deep Brain Stimulation for Movement Disorders. *Parkinsonism and Related Disorders*, October 2016, vol. 31, 14-22
- **V. Y. MA** ; **L. CHAN** ; **K. J. CARRUTHERS**. Incidence, prevalence, costs, and impact on disability of common conditions requiring rehabilitation in the united states: Stroke, spinal cord injury, traumatic brain injury, multiple sclerosis, osteoarthritis, rheumatoid arthritis, limb loss, and back pa. *Arch. Phys. Med. Rehabil.*, 2014, vol. 95, 986-995, 1
- **L. ASBOTH** ; **L. FRIEDLI** ; **J. BEAUPARLANT** ; **C. MARTINEZ-GONZALEZ** ; **S. ANIL** ; **E. REY** ; **L. BAUD** ; **G. PIDPRUZHNYKOVA** ; **M. A. ANDERSON** ; **P. SHKORBATOVA**. Cortico-reticulo-spinal circuit reorganization enables functional recovery after severe spinal cord contusion.. *Nat Neurosci.*, 2018, vol. 21, 576-588
- **F. B. WAGNER** ; **J.-B. MIGNARDOT** ; **C. G. L. GOFF-MIGNARDOT** ; **R. DEMESMAEKER** ; **S. KOMI** ; **M. CAPOGROSSO** ; **A. ROWALD** ; **I. SEÁÑEZ** ; **M. CABAN** ; **E. PIRONDINI**. Targeted neurotechnology restores walking in humans with spinal cord injury.. *Nature*, 2018, vol. 563, 65-71
- **R. VAN DEN BRAND** ; **J. HEUTSCHI** ; **Q. BARRAUD** ; **J. DIGIOVANNA** ; **K. BARTHOLDI** ; **M. HUERLIMANN** ; **L. FRIEDLI** ; **I. VOLLENWEIDER** ; **E. M. MORAUD** ; **S. DUIS**. Restoring Voluntary Control of Locomotion after Paralyzing Spinal Cord Injury.. *Science*, 2012, vol. 336, 1182-1185

- **L. H. KIM** ; **S. SHARMA** ; **S. A. SHARPLES** ; **K. A. MAYR** ; **C. H. T. KWOK** ; **P. J. WHELAN**. Integration of Descending Command Systems for the Generation of Context-Specific Locomotor Behaviors. *Front Neurosci-switz*, 2017, vol. 11, 581
- **L. C. BACHMANN** ; **A. MATIS** ; **N. T. LINDAU** ; **P. FELDER** ; **M. GULLO** ; **M. E. SCHWAB**. Deep brain stimulation of the midbrain locomotor region improves paretic hindlimb function after spinal cord injury in rats. *Sci Transl Med.*, 2013, vol. 5, 208-146
- **M. BONIZZATO** ; **N. D. JAMES** ; **G. PIDPRUZHNYKOVA** ; **N. PAVLOVA** ; **P. SHKORBATOVA** ; **L. BAUD** ; **C. MARTINEZ-GONZALEZ** ; **J. W. SQUAIR** ; **J. DIGIOVANNA** ; **Q. BARRAUD**. Multi-pronged neuromodulation intervention engages the residual motor circuitry to facilitate walking in a rat model of spinal cord injury. *Nat Commun*, 2021, vol. 12, 1925
- **M.-L. WELTER** ; **A. DEMAIN** ; **C. EWENCZYK** ; **V. CZERNECKI** ; **B. LAU** ; **A. E. HELOU** ; **H. BELAID** ; **J. YELNIK** ; **C. FRANÇOIS** ; **E. BARDINET**. PPNa-DBS for gait and balance disorders in Parkinson's disease: a double-blind, randomised study. *J Neurol*, 2015, vol. 262, 1515-1525
- **M.-L. WELTER** ; **A. DEMAIN** ; **C. EWENCZYK** ; **V. CZERNECKI** ; **B. LAU** ; **A. E. HELOU** ; **H. BELAID** ; **J. YELNIK** ; **C. FRANÇOIS** ; **E. BARDINET**. PPNa-DBS for gait and balance disorders in Parkinson's disease: a double-blind, randomised study. *J Neurol.*, 2015, vol. 262, 1515-1525
- **K. IKEDA** ; **K. KAWAKAMI** ; **H. ONIMARU** ; **Y. OKADA** ; **S. YOKOTA** ; **N. KOSHIYA** ; **Y. OKU** ; **M. LIZUKA** ; **H. KOIZUMI**. The respiratory control mechanisms in the brainstem and spinal cord: integrative views of the neuroanatomy and neurophysiology. *J Physiological Sci.*, 2017, vol. 67, 45-62
- **R. A. L. DAMPNEY**. Central neural control of the cardiovascular system: current perspectives.. *Adv Physiol Educ.*, 2016, vol. 40, 283-296
- **M.-L. WELTER** ; **A. DEMAIN** ; **C. EWENCZYK** ; **V. CZERNECKI** ; **B. LAU** ; **A. E. HELOU** ; **H. BELAID** ; **J. YELNIK** ; **C. FRANÇOIS** ; **E. BARDINET**. Grabli, PPNa-DBS for gait and balance disorders in Parkinson's disease: a double-blind, randomised study. *J Neurol.*, 2015, vol. 262, 1515-1525

- **A. NOWACKI** ; **S. GALATI** ; **J. AI-SCHLAEPPI** ; **C. BASSETTI** ; **A. KAELIN** ; **C. POLLO**. Pedunculopontine nucleus: An integrative view with implications on Deep Brain Stimulation. *Neurobiol Dis.*, 2018, vol. 128, 75-85
- **H. M. SINNAMON**. Locomotor stepping elicited by electrical stimulation of the hypothalamus persists after lesion of descending fibers of passage. *Physiol Behav.*, 1990, vol. 48, 261-266
- **H. M. SINNAMON** ; **C. K. STOPFORD**. Locomotion elicited by lateral hypothalamic stimulation in the anesthetized rat does not require the dorsal midbrain. *Brain Res.*, 1987, vol. 402, 78-86
- **H. M. SINNAMON**. Preoptic and hypothalamic neurons and the initiation of locomotion in the anesthetized rat.. *Prog Neurobiol.*, 1993, vol. 41, 323-344
- **K. V. BAEV** ; **V. K. BEREZOVSKII** ; **T. T. KEBKALO** ; **L. A. SAVOS'KINA**. Projections of neurons of the hypothalamic locomotor region to some brainstem and spinal cord structures in the cat. *Neurophysiology*, 1985, vol. 17, 595-600
- **G. N. ORLOVSKII**. Connections between reticulospinal neurons and locomotor regions of the brainstem.. *Neurosci Transl.*, 1970, vol. 4, 58-64
- **L. ASBOTH** ; **L. FRIEDLI** ; **J. BEAUPARLANT** ; **C. MARTINEZ-GONZALEZ** ; **S. ANIL** ; **E. REY** ; **L. BAUD** ; **G. PIDPRUZHNYKOVA** ; **M. A. ANDERSON** ; **P. SHKORBATOVA**. Cortico-reticulo-spinal circuit reorganization enables functional recovery after severe spinal cord contusion. *Nat Neurosci.*, 2018, vol. 21, 576-588
- **P. R. MILES** ; **W. E. GLADFELTER**. Lateral hypothalamus and reflex discharges over ventral roots of rat spinal nerves. *Physiol Behav.*, 1969, vol. 4, 671-675
- **C. KOSSE** ; **C. SCHÖNE** ; **E. BRACEY** ; **D. BURDAKOV**. Orexin-driven GAD65 network of the lateral hypothalamus sets physical activity in mice.. *Proc National Acad Sci.*, 2017, vol. 114, 4525-4530
- **J. P. MURPHY** ; **E. GELLHORN**. THE INFLUENCE OF HYPOTHALAMIC STIMULATION ON CORTICALLY INDUCED MOVEMENTS AND ON ACTION POTENTIALS OF THE CORTEX. *J Neurophysiol*, 1945, vol. 8, 341-364
- **R. RHINES** ; **H. W. MAGOUN**. BRAIN STEM FACILITATION OF CORTICAL MOTOR RESPONSE. *J Neurophysiol.*, 1946, vol. 9, 219-229
- **J. P. MURPHY** ; **E. GELLHORN**. THE INFLUENCE OF HYPOTHALAMIC STIMULATION ON CORTICALLY INDUCED MOVEMENTS AND ON ACTION POTENTIALS OF THE CORTEX. *J Neurophysiol.*, 1945, vol. 8, 341-364
- **L. E. HARRELL** ; **J. M. DECASTRO** ; **S. BALAGURA**. A critical evaluation of body weight loss following lateral hypothalamic lesions. *Physiol Behav.*, 1975, vol. 15, 133-136
- **R. KEESEY** ; **T. POWLEY**. Self-stimulation and body weight in rats with lateral hypothalamic lesions.. *Am J Physiology-legacy Content*, 1973, vol. 224, 970-978
- **D. M. WHITING** ; **N. D. TOMYCZ** ; **J. BAILES** ; **L. DE JONGE** ; **V. LECOULTRE** ; **B. WILENT** ; **D. ALCINDOR** ; **E. R. PROSTKO** ; **B. C. CHENG** ; **C. ANGLE**. Lateral hypothalamic area deep brain stimulation for refractory obesity: a pilot study with preliminary data on safety, body weight, and energy metabolism. *J Neurosurg.*, 2013, vol. 119, 56-63
- **D. M. WHITING** ; **N. D. TOMYCZ** ; **J. BAILES** ; **L. DE JONGE** ; **V. LECOULTRE** ; **B. WILENT** ; **D. ALCINDOR** ; **E. R. PROSTKO** ; **B. C. CHENG** ; **C. ANGLE**. Lateral hypothalamic area deep brain stimulation for refractory obesity: a pilot study with preliminary data on safety, body weight, and energy metabolism.. *J Neurosurg.*, 2013, vol. 119, 56-63
- **C. HAMANI** ; **M. P. MCANDREWS** ; **M. COHN** ; **M. OH** ; **D. ZUMSTEG** ; **C. M. SHAPIRO** ; **R. A. WENNBERG** ; **A. M. LOZANO**. Memory enhancement induced by hypothalamic/fornix deep brain stimulation. *Ann Neurol.*, 2008, vol. 63, 119-123
- **A. W. LAXTON** ; **D. F. TANG-WAI** ; **M. P. MCANDREWS** ; **D. ZUMSTEG** ; **R. WENNBERG** ; **R. KEREN** ; **J. WHERRETT** ; **G. NAGLIE** ; **C. HAMANI** ; **G. S. SMITH**. A phase I trial of deep brain stimulation of memory circuits in Alzheimer's disease.. *Ann Neurol.*, 2010, vol. 68, 521-534
- **A. W. LAXTON** ; **D. F. TANG-WAI** ; **M. P. MCANDREWS** ; **D. ZUMSTEG** ; **R. WENNBERG** ; **R. KEREN** ; **J. WHERRETT** ; **G. NAGLIE** ; **C. HAMANI** ; **G. S. SMITH**. A phase I trial of deep brain stimulation of memory circuits in Alzheimer's disease. *Ann Neurol.*, 2010, vol. 68, 521-534
- **D. FONTAINE** ; **Y. LAZORTHES** ; **P. MERTENS** ; **S. BLOND** ; **G. GÉRAUD** ; **N. FABRE** ; **M. NAVEZ** ; **C. LUCAS** ; **F. DUBOIS** ; **S. GONFRIER**. Safety and efficacy of deep brain stimulation in refractory cluster headache: a randomized placebo-controlled double-blind trial followed by a 1-year open extension. *J Headache Pain.*, 2010, vol. 11, 23-31
- **STOLZE, H et al.** Prevalence of gait disorders in hospitalized neurological patients. *Mov Disord*, 2005, vol. 20, 89-94
- **SNIJDERS, A. H. et al.** Physiology of freezing of gait.. *Ann Neurol*, 2016, vol. 80, 644-659
- **CAPOGROSSO, M. et al.** A brain-spine interface alleviating gait deficits after spinal cord injury in primates.. *Nature*, 2016, vol. 539, 284-288

- **BEZARD, E** ; **IMBERT, C.** ; **DELOIRE, X** ; **BIOULAC, B.** ; **GROSS, C. E.** A chronic MPTP model reproducing the slow evolution of Parkinson's disease: evolution of motor symptoms in the monkey.. *Brain Res*, 1997, vol. 766, 107-112
- **WAGNER, F. B et al.** Targeted neurotechnology restores walking in humans with spinal cord injury. *Nature*, 2018, vol. 563, 65-71
- **AJIBOYE, A. B. et al.** Restoration of reaching and grasping movements through brain-controlled muscle stimulation in a person with tetraplegia: a proof-of-concept demonstration. *Lancet*, 2017
- **BEZARD, A. L. et al.** An exoskeleton controlled by an epidural wireless brain-machine interface in a tetraplegic patient: a proof-of-concept demonstration. *Lancet Neurol,*, 2019
- **BEZARD, E. et al.** Relationship between the appearance of symptoms and the level of nigrostriatal degeneration in a progressive 1-methyl-4-phenyl-1,2,3,6-tetrahydropyridine-lesioned macaque model of Parkinson's disease. *The Journal of neuroscience : the official journal of the Society for Neuroscience*, 2001, vol. 21, 6853-6861
- **KNUTSSON**. E. An analysis of Parkinsonian gait. *Brain : a journal of neurology*, 1972, vol. 95, 475-486
- **CREABY, M. W.** ; **COLE, M. H**. Gait characteristics and falls in Parkinson's disease: A systematic review and meta-analysis. *Parkinsonism & related disorders*, 2018
- **IMBERT, C** ; **BEZARD, E** ; **GUITRAUD, S** ; **BORAUD, T.** ; **GROSS, C. E**. Comparison of eight clinical rating scales used for the assessment of MPTP-induced parkinsonism in the Macaque monkey. *J Neurosci Methods*, 2000, vol. 96, 71-76
- **FRIEDLI, L. et al.** Pronounced species divergence in corticospinal tract reorganization and functional recovery after lateralized spinal cord injury favors primates.. *Science translational medicine*, 2015, vol. 7, 302-134
- **COURTINE, G. et al.** Transformation of nonfunctional spinal circuits into functional states after the loss of brain input.. *Nature Neuroscience*, 2009, vol. 12, 1333-1342
- **LOZANO, A. M.** ; **LIPSMAN, N.** Probing and regulating dysfunctional circuits using deep brain stimulation.. *Neuron*, 2013, vol. 77, 406-424
- **SANTANA, M. B et al.** Spinal cord stimulation alleviates motor deficits in a primate model of Parkinson disease. *Neuron*, 2014, vol. 84, 716-722
- **MACDONALD, V.** ; **HALLIDAY, G. M**. Selective loss of pyramidal neurons in the presupplementary motor cortex in Parkinson's disease. *Mov Disord*, 2002, vol. 17, 1166-1173
- **GOLDBERG, J. A. et al.** Enhanced synchrony among primary motor cortex neurons in the 1-methyl-4-phenyl-1,2,3,6-tetrahydropyridine primate model of Parkinson's disease. *The Journal of neuroscience : the official journal of the Society for Neuroscience*, 2002, vol. 22, 4639-4653
- **POZZI, N. G. et al.** Freezing of gait in Parkinson's disease reflects a sudden derangement of locomotor network dynamics. *Brain : a journal of neurology*, 2019, vol. 142, 2037-2050
- **CAGNAN, H. et al.** Temporal evolution of beta bursts in the parkinsonian cortical and basal ganglia network.. *Proc Natl Acad Sci U S A*, 2019, vol. 116, 16095-16104
- **DE HEMPTINNE, C. et al.** Therapeutic deep brain stimulation reduces cortical phase-amplitude coupling in Parkinson's disease.. *Nat Neurosci*, 2015, vol. 18, 779-786
- **BURCIU, R. G.** ; **VAILLANCOURT, D. E.** Imaging of Motor Cortex Physiology in Parkinson's Disease.. *Mov Disord*, 2018, vol. 33, 1688-1699
- **PASQUEREAU, B.** ; **TURNER, R. S**. Primary motor cortex of the parkinsonian monkey: differential effects on the spontaneous activity of pyramidal tract-type neurons. *Cereb Cortex*, 2011, vol. 21, 1362-1378
- **PASQUEREAU, B.** ; **DELONG, M. R.** ; **TURNER, R. S**. Primary motor cortex of the parkinsonian monkey: altered encoding of active movement.. *Brain : a journal of neurology*, 2016, vol. 139, 127-143
- **DE HEMPTINNE, C. et al.** Exaggerated phase-amplitude coupling in the primary motor cortex in Parkinson disease.. *Proc Natl Acad Sci U S A*, 2013, vol. 110, 4780-4785
- **DEVERGNAS, A** ; **PITTARD, D** ; **BLIWISE, D** ; **WICHMANN, T**. Relationship between oscillatory activity in the cortico-basal ganglia network and parkinsonism in MPTP-treated monkeys. *Neurobiology of disease*, 2014, vol. 68, 156-166
- **YIN, M. et al.** Wireless Neurosensor for Full-Spectrum Electrophysiology Recordings during Free Behavior.. *Neuron*, 2014, vol. 84, 1170-1182
- **YU, H.** ; **STERNAD, D** ; **CORCOS, D. M.** ; **VAILLANCOURT, D. E.** Role of hyperactive cerebellum and motor cortex in Parkinson's disease.. *Neuroimage*, 2007, vol. 35, 222-233
- **SABATINI, U. et al.** Cortical motor reorganization in akinetic patients with Parkinson's disease: a functional MRI study. *Brain : a journal of neurology*, 2000, vol. 123, 394-403
- **ECKERT, T.** ; **PESCHEL, T.** ; **HEINZE, H. J.** ; **ROTTE, M**. Increased pre-SMA activation in early PD patients during simple self-initiated hand movements. *J Neurol*, 2006, vol. 253, 199-207

- **CATALAN, M. J.** ; **ISHII, K** ; **HONDA, M.** ; **SAMII, A.** ; **HALLETT, M**. A PET study of sequential finger movements of varying length in patients with Parkinson's disease. *Brain : a journal of neurology*, 1999, vol. 122, 483-495

- **TURNER, R. S** ; **GRAFTON, S. T** ; **MCINTOSH, A. R** ; **DELONG, M. R.** ; **HOFFMAN, J. M**. The functional anatomy of parkinsonian bradykinesia.. *Neuroimage*, 2003, vol. 19, 163-179

- **FUENTES, R.** ; **PETERSSON, P** ; **SIESSER, W. B.** ; **CARON, M. G** ; **NICOLELIS, M. A**. Spinal cord stimulation restores locomotion in animal models of Parkinson's disease. *Science*, 2009, vol. 323, 1578-1582

- **GALLEGO, J. A. et al.** Cortical population activity within a preserved neural manifold underlies multiple motor behaviors. *Nature communications*, 2018, vol. 9, 4233

- **YAKOVENKO, S** ; **MUSHAHWAR, V** ; **VANDERHORST, V** ; **HOLSTEGE, G** ; **PROCHAZKA, A**. Spatiotemporal activation of lumbosacral motoneurons in the locomotor step cycle. *J Neurophysiol*, 2002, vol. 87, 1542-1553

- **CAPPELLINI, G** ; **IVANENKO, Y. P** ; **DOMINICI, N.** ; **POPPELE, R. E.** ; **LACQUANITI, F**. Migration of motor pool activity in the spinal cord reflects body mechanics in human locomotion. *J Neurophysiol*, 2010, vol. 104, 3064-3073

- **CAPOGROSSO, M. et al.** Configuration of electrical spinal cord stimulation through real-time processing of gait kinematics.. *Nat Protoc*, 2018, vol. 13, 2031-2061

- **FORMENTO, E. et al.** Electrical spinal cord stimulation must preserve proprioception to enable locomotion in humans with spinal cord injury.. *Nat Neurosci*, 2018, vol. 21, 1728-1741

- **SCHUETTLER, M** ; **STIESS, S** ; **KING, B. V.** ; **SUANING, G. J.** Fabrication of implantable microelectrode arrays by laser cutting of silicone rubber and platinum foil. *J Neural Eng*, 2005, vol. 2, S121-128

- **MINEV, I. R. et al.** Biomaterials. Electronic dura mater for long-term multimodal neural interfaces. *Science*, 2015, vol. 347, 159-163

- **WENGER, N. et al.** Spatiotemporal neuromodulation therapies engaging muscle synergies improve motor control after spinal cord injury.. *Nature medicine*, 2016, vol. 22, 138-145

- **REVUELTA, G. J.** ; **UTHAYATHAS, S** ; **WAHLQUIST, A. E** ; **FACTOR, S. A** ; **PAPA, S. M**. Non-human primate FOG develops with advanced parkinsonism induced by MPTP Treatment.. *Experimental neurology*, 2012, vol. 237, 464-469

- **PINTO DE SOUZA, C. et al.** Spinal cord stimulation improves gait in patients with Parkinson's disease previously treated with deep brain stimulation.. *Mov Disord*, 2017, vol. 32, 278-282

- **DE LIMA-PARDINI, A. C. et al.** Effects of spinal cord stimulation on postural control in Parkinson's disease patients with freezing of gait. *Elife*, 2018, vol. 7

- **NISHIOKA, K** ; **NAKAJIMA, M**. Beneficial Therapeutic Effects of Spinal Cord Stimulation in Advanced Cases of Parkinson's Disease With Intractable Chronic Pain: A Case Series. *Neuromodulation*, 2015, vol. 18, 751-753

- **ST GEORGE, R. J** ; **NUTT, J. G.** ; **BURCHIEL, K. J.** ; **HORAK, F. B**. A meta-regression of the long-term effects of deep brain stimulation on balance and gait in PD. *Neurology*, 2010, vol. 75, 1292-1299

- **STUART, S. et al.** Cortical activity during walking and balance tasks in older adults and in people with Parkinson's disease: A structured review. *Maturitas*, 2018, vol. 113, 53-72

- **MCCABE, G** ; **MOORE, D**. Introduction to the Practice of Statistics. *Nurse Res*, 2005, vol. 13, 89-90

- **AHMED, M. et al.** Lentiviral overexpression of GRK6 alleviates L-dopa-induced dyskinesia in experimental Parkinson's disease. *Sci Transl Med*, 2010, vol. 2, 28-28

- **BEZARD, E. et al.** Attenuation of levodopa-induced dyskinesia by normalizing dopamine D3 receptor function.. *Nature medicine*, 2003, vol. 9, 762-767

- **BEZARD, E. et al.** The mGluR5 negative allosteric modulator dipraglurant reduces dyskinesia in the MPTP macaque model.. *Movement disorders : official journal of the Movement Disorder Society*, 2014, vol. 29, 1074-1079

- **FASANO, S. et al.** Inhibition of Ras-guanine nucleotide-releasing factor 1 (Ras-GRF1) signaling in the striatum reverts motor symptoms associated with L-dopa-induced dyskinesia.. *Proc Natl Acad Sci U S A*, 2010, vol. 107, 21824-21829

- **PORRAS, G. et al.** PSD-95 expression controls l-DOPA dyskinesia through dopamine D1 receptor trafficking.. *J Clin Invest*, 2012, vol. 122, 3977-3989

- **SHEN, W. et al.** M4 Muscarinic Receptor Signaling Ameliorates Striatal Plasticity Deficits in Models of L-DOPA-Induced Dyskinesia. *Neuron*, 2015, vol. 88, 762-773

- **URS, N. M. et al.** Targeting beta-arrestin2 in the treatment of L-DOPA-induced dyskinesia in Parkinson's disease.. *Proc Natl Acad Sci U S A*, 2015, vol. 112, E2517-2526

- **MEISSNER, W. et al.** Time-course of nigrostriatal degeneration in a progressive MPTP-lesioned macaque model of Parkinson's disease.. *Mol Neurobiol*, 2003, vol. 28, 209-218

- **COURTINE, G. et al.** Kinematic and EMG determinants in quadrupedal locomotion of a non-human primate (Rhesus).. *J Neurophysiol*, 2005, vol. 93, 3127-3145

- **TASS, P. A. et al.** Coordinated reset has sustained aftereffects in Parkinsonian monkeys.. *Ann Neurol*, 2012, vol. 72, 816-820
- **MORAUD, E. M. et al.** Mechanisms Underlying the Neuromodulation of Spinal Circuits for Correcting Gait and Balance Deficits after Spinal Cord Injury.. *Neuron*, 2016, vol. 89, 814-828
- **CAPOGROSSO, M. et al.** A computational model for epidural electrical stimulation of spinal sensorimotor circuits.. *The Journal of neuroscience : the official journal of the Society for Neuroscience*, 2013, vol. 33, 19326-19340
- **BAUFRETON, J et al.** Inhaling xenon ameliorates l-dopa-induced dyskinesia in experimental parkinsonism. *Mov Disord*, 2018
- **ZAMPIERI, C. et al.** The instrumented timed up and go test: potential outcome measure for disease modifying therapies in Parkinson's disease. *J Neurol Neurosurg Psychiatry*, 2010, vol. 81, 171-176
- **PALMERINI, L** ; **MELLONE, S** ; **AVANZOLINI, G** ; **VALZANIA, F** ; **CHIARI, L**. Quantification of motor impairment in Parkinson's disease using an instrumented timed up and go test. *IEEE Trans Neural Syst Rehabil Eng*, 2013, vol. 21, 664-673
- **GALLEGO, J. A.** ; **PERICH, M. G** ; **MILLER, L. E.** ; **SOLLA, S. A**. Neural Manifolds for the Control of Movement.. *Neuron*, 2017, vol. 94, 978-984
- **CHESTEK, C. A. et al.** Long-term stability of neural prosthetic control signals from silicon cortical arrays in rhesus macaque motor cortex. *J Neural Eng*, 2011, vol. 8, 045005
- **SANTHANAM, G. et al.** HermesB: a continuous neural recording system for freely behaving primates.. *IEEE Trans Biomed Eng*, 2007, vol. 54, 2037-2050
- **LINDERMAN, M. D. et al.** Neural recording stability of chronic electrode arrays in freely behaving primates. *Conf Proc IEEE Eng Med Biol Soc*, 2006, vol. 1, 4387-4391
- **DICKEY, A. S** ; **SUMINSKI, A** ; **AMIT, Y** ; **HATSOPOULOS, N. G**. Single-unit stability using chronically implanted multielectrode arrays. *J Neurophysiol*, 2009, vol. 102, 1331-1339
- **ROKNI, U** ; **RICHARDSON, A. G** ; **BIZZI, E.** ; **SEUNG, H. S.** Motor learning with unstable neural representations.. *Neuron*, 2007, vol. 54, 653-666
- **TAYLOR, D. M.** ; **TILLERY, S. I.** ; **SCHWARTZ, A. B.** Direct cortical control of 3D neuroprosthetic devices.. *Science*, 2002, vol. 296, 1829-1832
- **DOWNEY, J. E.** ; **SCHWED, N** ; **CHASE, S. M** ; **SCHWARTZ, A. B** ; **COLLINGER, J. L.** Intracortical recording stability in human brain-computer interface users. *J Neural Eng*, 2018, vol. 15, 046016
- **PERGE, J. A. et al.** Reliability of directional information in unsorted spikes and local field potentials recorded in human motor cortex. *J Neural Eng*, 2014, vol. 11, 046007
- **PERGE, J. A et al.** Intra-day signal instabilities affect decoding performance in an intracortical neural interface system. *J Neural Eng*, 2013, vol. 10, 036004
- **SIMERAL, J. D.** ; **KIM, S. P** ; **BLACK, M. J** ; **DONOGHUE, J. P.** ; **HOCHBERG, L. R**. Neural control of cursor trajectory and click by a human with tetraplegia 1000 days after implant of an intracortical microelectrode array. *J Neural Eng*, 2011, vol. 8, 025027
- **BARRESE, J. C. et al.** Failure mode analysis of silicon-based intracortical microelectrode arrays in non-human primates. *J Neural Eng*, 2013, vol. 10, 066014
- **BJORNSSON, C. S. et al.** Effects of insertion conditions on tissue strain and vascular damage during neuroprosthetic device insertion. *J Neural Eng*, 2006, vol. 3, 196-207
- **SHAIN, W. et al.** Controlling cellular reactive responses around neural prosthetic devices using peripheral and local intervention strategies.. *IEEE Trans Neural Syst Rehabil Eng*, 2003, vol. 11, 186-188
- **WELCH, P**. The use of fast Fourier transform for the estimation of power spectra: A method based on time averaging over short, modified periodograms. *IEEE Transactions on Audio and Electroacoustics*, 1967, vol. 15, 70-73
- **MCLACHLAN, G. J.** ; **PEEL, D**. Finite mixture models.. Wiley, 2000
- **MILEKOVIC, T. et al.** Stable long-term BCI-enabled communication in ALS and locked-in syndrome using LFP signals.. *J Neurophysiol*, 2018, vol. 120, 343-360
- **MILEKOVIC, T** ; **BALL, T** ; **SCHULZE-BONHAGE, A** ; **AERTSEN, A** ; **MEHRING, C**. Detection of error related neuronal responses recorded by electrocorticography in humans during continuous movements. *PLoS One*, 2013, vol. 8, e55235
- **FASANO, A** ; **AQUINO, C. C** ; **KRAUSS, J. K** ; **HONEY, C. R** ; **BLOEM, B. R**. Axial disability and deep brain stimulation in patients with Parkinson disease.. *Nat Rev Neurol*, 2015, vol. 11, 98-110
- **MOREAU, C. et al.** STN-DBS frequency effects on freezing of gait in advanced Parkinson disease.. *Neurology*, 2008, vol. 71, 80-84
- **ROSENBLAD, C. et al.** Vector-mediated l-3,4-dihydroxyphenylalanine delivery reverses motor impairments in a primate model of Parkinson's disease. *Brain : a journal of neurology*, 2019, vol. 142, 2402-2416
- **FRIEDMAN, J.** Regularized Discriminant-Analysis.. *J Am Stat Assoc*, 1989, 165-175
- **GALLEGO, J. A.** ; **PERICH, M. G** ; **CHOWDHURY, R** ; **SOLLA, S. A.** ; **MILLER, L. E**. Long-term stability of cortical population dynamics underlying consistent behavior. *Nature Neurosci*